# EUROPEAN PATENT SPECIFICATION

(11) **EP 0 912 176 B1**
(45) Date of publication and mention of the grant of the patent: **25.09.2002**
(21) Application number: 97928260.5
(22) Date of filing: 20.06.1997
(51) Int. Cl.: A61K 31/44, A61K 31/445, A61K 31/505, A61K 31/55, A61K 31/47, A61K 31/675, A61K 31/535

(54) **USE OF PYRIDYL ALKANE, PYRIDYL ALKENE AND/OR PYRIDYL ALKINE ACID AMIDES IN THE TREATMENT OF TUMORS OR FOR IMMUNOSUPPRESSION**
VERWENDUNG VON PYRIDYL-ALKAN-,PYRIDIYL ALKAN- UND/ODER PYRIDYL-SÄUREN AMIDEN ZUR BEHANDLUNG VON TUMOREN ODER FÜR IMMUNSUPPRESSION
UTILISATION D'AMIDES PYRIDYL-ALCANE, PYRIDYL-ALCENE ET/OU PYRIDYL-ALCYNE ACIDES DANS LE TRAITEMENT DES TUMEURS ET POUR L'IMMUNOSUPPRESSION

(30) Priority: 20.06.1996 DE 19624668
(43) Date of publication of application: 06.05.1999
(73) Proprietor: Fujisawa Deutschland GmbH, 81673 München (DE)
(72) Inventor: BIEDERMANN, Elfi, D-85591 Vaterstetten (DE); HASMANN, Max, D-82061 Neuried (DE); LÖSER, Roland, D-82340 Feldafing (DE); RATTEL, Benno, D-81249 Munich (DE); REITER, Friedemann, D-85640 Putzbrunn (DE); SCHEIN, Barbara, D-85375 Neufahrn (DE); SEIBEL, Klaus, D-82166 Gräfelfing (DE); VOGT, Klaus, D-81541 Munich (DE)
(74) Representative: HOFFMANN - EITLE
(86) International application number: EP9703244
(87) International publication number: WO97048397

(56) References cited:
- EP-A- 0 210 782
- EP-A- 0 330 026
- EP-A- 0 343 307
- WO-A-91/15484
- WO-A-91/15485

## Description

The invention relates to the use of pyridyl aklane, pyridyl alkene and/or pyridyl alkine acid amides, especially in the treatment of tumor conditions and/or as cytostatic agents or as immunosuppressive agents as well as medicaments with an amount of these compounds in combination with other cytostatic agents or immunosuppresive agents.

A strong need exists for the enrichment of cytostatic therapy to provide pharmaceuticals and/or medicaments which not only possess a strong activity, but also exert diminished side effects in comparison to many classical cancerostatic agents, whereby treatment of a broad as possible spectrum of tumors should be made accessible. Furthermore, effective cytostatic agents for an efficient therapy should be made available. Active ingredients of this type should also be exceptionally suitable in the mentioned indications for a combination therapy, be it in connection with other cytostatic agents or with radiation (for example X-rays, radioactive elements, such as cobalt, or linear accelerator, etc.), with operative procedures, heat treatment, etc. As a consequence, further subject-matter of the invention relates to new medicaments in the form of combinations of the compounds defined below and used according to the invention together with other compounds or immunosuppressive agents customary in the therapy of tumors.

In this connection, a strong need also exists in tumor therapy to open up new possibilities which were not usable up to now in these indications, for example for overcoming or preventing resistances.

This object was successfully solved in a completely surprising manner by making available the specially structured pyridyl derivatives defined below.

It was known that various pyridine compounds substituted in a specific manner have pharmacologically useful properties which lie however in completely different indication areas.

Thus, ω-pyridyl alkane and/or alkene amides with anti-allergic activity are described in EP 0 210 782 which are referred to as having a 5-lipoxygenase-inhibiting and antihistamine action, wherein the amide components of these compounds contain a piperazine or homopiperazine ring and the pyridine ring can be linked together in the 2-, 3- or 4-position. JP 63,179,869 describes further pyridyl amides, ω-pyridyl alkane and alkene amides as anti-allergic effective substances containing a substituted piperidine ring in the amine component. Such compounds with the same properties are mentioned in Chem. Pharm. Bull 37, 100-105 (1989) and in J. Med. Chem. 1989, 583-593.

Pyridyl ureas, pyridyl thioureas and pyridyl carbonamides, wherein the amide portion is bound over an aryl substituted alkyl chain with a piperidine ring or piperazine ring, are described for example in EP-A-0 428 434 or in EP-A-0 512 902 as antagonists of the neurokinin receptor and subtance P. Furthermore, pyridyl(alkyl)carbonamides, pyridyl(alkyl)sulfonamides and analogous ureas, wherein the amide portion is bound over an alkyl chain with a piperidine ring are disclosed in EP-A-0 479 601 as active ingredients with anti-arrhythmic properties.

In WO 91/15 485, the production of pyridine-3,5-dicarboxylic acid esters and amides as well as their use for the treatment of tumor conditions is described. These compounds differ from the compounds according to the invention described below in very important structural features, for example by the dicarboxyl grouping on the pyridine ring or the absence of the hydrocarbon chain between the pyridine ring and the amide grouping. The compounds disclosed in WO 89/07 443 in the form of optically pure R(-)-Ni-guldipine and further analogous dihydropyridines with cytotoxic activity have larger structural differences. However, the compounds according to the invention unexpectedly possess a better activity and a wider spectrum of action despite the large structural differences.

Further structurally closely related compounds are represented by the antagonists of the histimine-H₁-receptor described in EP-A-0 343 307 which discloses a series of substituted piperidine derivatives without naming concrete examples for special 3-pyridyl substitutions.

EP-A-0 330 026 also discloses substituted piperidine derivatives with possible, generic pyridyl substitutions for which, however, merely a single concrete example is disclosed, namely (E)-3-(3-pyridyl)-N-[2-(1-benzylpiperidin-4-yl)ethyl]-2-propenamide hydrochloride. These compounds are distinguished by an anti-cholinesterase activity, an anti-amnesia activity as well as activities directed against hyperkinesia, senile demensia, mania and Alzheimer's disease.

In view of this art, the finding that the compounds according to the general formula (I) defined below have activities which make them particularly suitable in an excellent manner for the therapy of tumor illnesses was completely unexpected. Equally unexpected was the pharmacological finding that the compounds according to the invention also possess immunosuppressive properties besides cytostatic activity.

Considering the above-mentioned completely different known medical indications of known piperidine derivatives, such as neurokinin receptor antagonism, hyperkineses, amnesias, allergies, or rhythm disorders, the activity of the compounds used according to the invention with the structural modifications as they are defined below with respect to the present general formula, and the combinations according to the invention in the form of the detected excellent cytostatic or immunomodulatory activity with advantageous therapeutic properties was completely surprising for the person skilled in the art.

Pharmacological test results from which this conclusion must be drawn, as well as the concrete tumor indications and combination possibilities are detailed and illustrated in the last part of the description.

Therefore, subject-matter of the invention relates to the use of one or more compounds of formula (I) 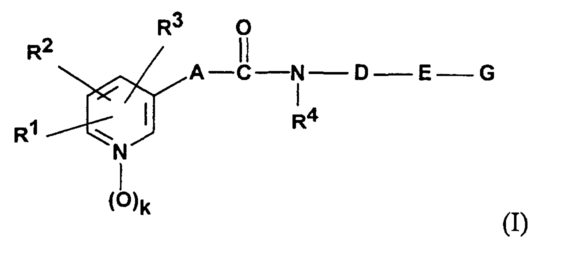 wherein
- **R**^{**1**}: is selected from the group consisting of hydrogen, halogen, cyano, C₁-C₆-alkyl, trifluoromethyl, C₃-C₈-cycloalkyl, C₁-C₄-hydroxyalkyl, hydroxy, C₁-C₄-alkoxy, benzyloxy, C₁-C₄-alkanoyloxy, C₁-C₄-alkylthio, C₂-C₅-alkoxycarbonyl, aminocarbonyl, C₃-C₉-dialkylaminocarbonyl, carboxy, phenyl, phenoxy, pyridyloxy and **NR**^{**5**}**R**^{**6**}, wherein
- **R**^{**5**}: and
- **R**^{**6**}: are selected, independently from each other, from hydrogen and C₁-C₆-alkyl,
- **R**^{**2**}: is selected from the group consisting of hydrogen, halogen, C₁-C₆-alkyl, trifluoromethyl and hydroxy, wherein
- **R**^{**1**} and **R**^{**2**}**,**: in the case they are adjacent, optionally form a bridge which is selected from the group of bridge members -(CH₂)₄- and -(CH=CH)₂- and -CH₂O-**CR**^{**7**}**R**^{**8**}-O-, wherein
- **R**^{**7**}: and
- **R**^{**8**}: are independent from each other, hydrogen or C₁-C₆-alkyl,
- **R**^{**3**}: is selected from hydrogen, halogen and C₁-C₆-alkyl,
- **R**^{**4**}: is selected from hydrogen, C₁-C₆-alkyl, C₃-C₆-alkenyl, hydroxy, C₁-C₆-alkoxy and benzyloxy,
- **k**: is 0 or 1,
- **A**: is selected from C₁-C₆-alkylene, which is optionally substituted once to three-fold by C₁-C₃-alkyl, hydroxy, fluorine or phenyl,
1,2-cyclopropylene,
C₂-C₆-alkenylene, which is optionally substituted one to three-fold by C₁-C₃-alkyl, hydroxy, fluorine, cyano, or phenyl,
C₄-C₆-alkadienylene, which is optionally substituted once or twice by C₁-C₃-alkyl, fluorine, cyano, or phenyl,
1,3,5-hexatrienylene, which is optionally substituted by Cᵢ-C₃-alkyl, fluorine, or cyano,
ethinylene and
C₂-C₆-alkylene, wherein a methylene unit is isosterically replaced by O, S, **NR**^{**9**}**,** CO, SO or SO₂, and wherein the isosteric substitute, with the exception of=CO, is not adjacent to the amide group, and wherein
- **R**^{**9**}: is selected from hydrogen, C₁-C₃-alkyl, C₁-C₆-acyl and methanesulfonyl,
- **D**: is selected from C₁-C₁₀-alkylene, which is optionally substituted once or twice by C₁-C₃-alkyl or hydroxy,
C₂-C₁₀-alkenylene, optionally substituted once or twice by C₁-C₃-alkyl or hydroxy, wherein the double bond optionally is to ring E,
C₃-C₁₀-alkinylene, which is optionally substituted once or twice by C₁-C₃-alkyl or hydroxy, and
the group consisting of C₁-C₁₀-alkylene, C₂-C₁₀-alkenylene or C₃-C₁₀-alkinylene, in which one to three methylene units are isosterically replaced by O, S, **NR**^{**10**}, CO, SO or SO₂, wherein
- **R**^{**10**}: has the same meaning as **R**^{**9**}, but is selected independently therefrom,
- **E**: is selected from 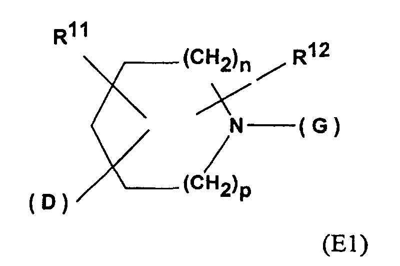 and 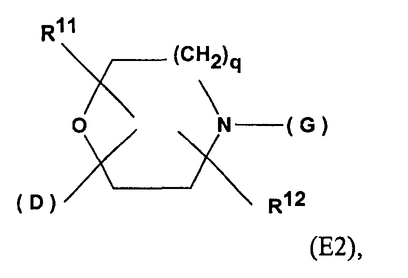 wherein the heterocyclic ring optionally has a double bond and
- **n** and **p**: are, independent of each other, 0, 1, 2 or 3, with the proviso that **n** + **p** ≤ 4,
- **q**: is 1 or 2,
- **R**^{**11**}: is selected from hydrogen, C₁-C₃-alkyl, hydroxy, hydroxymethyl, carboxy and C₂-C₇-alkoxycarbonyl,
- **R**^{**12**}: is selected from hydrogen and an oxo group adjacent to the nitrogen atom,
- **G**: is selected from hydrogen, **G1, G2, G3, G4** and **G5,** wherein
- **G1**: represents the residue

**―(CH**_{**2**}**)**_{**r**}**―(CR**^{**14**}**R**^{**15**}**)**_{**s**}**―R**^{**13**} (G1)

wherein
- **r**: is 0, 1 or 2,
- **s**: is 0 or 1,
- **R**^{**13**}: is selected from hydrogen, C₁-C₆-alkyl, C₃-C₆-alkenyl, C₃-C₆-alkinyl, C₃-C₈-cycloalkyl,
benzyl, phenyl,
the group consisting of monocyclic aromatic five- to six-membered heterocycles, which contain one to.three hetero-atoms from N, S or O and are either bound directly or over a methylene group,
the group consisting of anellated bi- and tricyclic aromatic or partially hydrogenated carbocyclic ring systems with 8 to 16 ring atoms and at least one aromatic ring, whereby the bond occurs either over an aromatic or a hydrogenated ring and either directly or over a methylene group, and the group consisting of anellated bi- and tricyclic aromatic or partially hydrogenated heterocyclic ring systems with 8 to 16 ring atoms and at least one aromatic ring, wherein one to three ring atoms are selected from N, S and O and the bond occurs either over an aromatic or a hydrogenated ring and either directly or over a methylene group,
- **R**^{**14**}: has the same meaning as **R**^{**13**}, but is selected independently thereof,
- **R**^{**15**}: is selected from hydrogen, hydroxy, methyl, benzyl, phenyl,
the group consisting of monocyclic aromatic five- to six-membered heterocycles, which contain one to three hetero-atoms selected from N, S or O and are bound either directly or over a methylene group,
the group consisting of anellated bi- and tricyclic aromatic or partially hydrogenated carbocyclic ring systems with 8 to 16 ring atoms and at least one aromatic ring, wherein the bond occurs either over an aromatic or a hydrogenated ring and either directly or over a methylene group, and
the group consisting of anellated bi- and tricyclic aromatic or partially hydrogenated heterocyclic ring systems with 8 to 16 ring atoms and at least one aromatic ring, wherein one to three ring atoms are selected from N, S or O and the bond occurs either over an aromatic or a hydrogenated ring and either directly or over a methylene group,
- **G2**: is selected from the residues 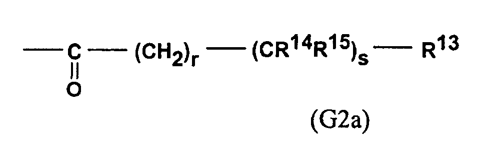 and 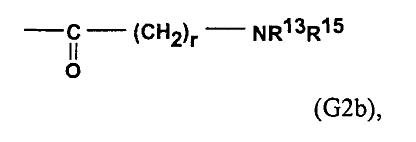 wherein the substituents **R**^{**13**} and **R**^{**15**} have the above meaning, or the group

**―NR**^{**13**}**R**^{**15**}

is a nitrogen-containing heterocycle bound over the nitrogen atom, the nitrogen-containing heterocycle being selected from
the group consisting of saturated and unsaturated monocyclic, four- to eight-membered heterocycles, which, aside from the essential nitrogen atom, optionally contain one or two further hetero-atoms selected from N, S and O, and
the group consisting of saturated and unsaturated bi- or tricyclic, anellated or bridged heterocycles with 8 to 16 ring atoms, which, aside from the essential nitrogen atom, optionally contain one or two further hetero-atoms selected from N, S and O,
- **G3**: is the residue

**―SO**_{**2**}**―(CH**_{**2**}**)**_{**r**} **R**^{**13**} (G3),
- **G4**: is the residue 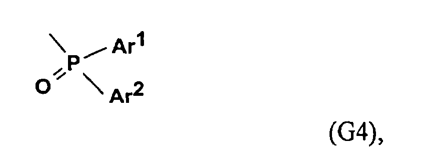 wherein
- **Ar**^{**1**}: and
- **Ar**^{**2**}: are selected independently of each other from phenyl, pyridyl and naphthyl,
- **G5**: is the residue

**―COR**^{**16**} (G5)

wherein
- **R**^{**16**}: is selected from trifluoromethyl, C₁-C₆-alkoxy, C₃-C₆-alkenyloxy and benzyloxy,
wherein aromatic ring systems in the substituents **R**^{**1**}**, R**^{**2**}**, R**^{**4**}**, R**^{**13**}**, R**^{**14**}**, R**^{**15**}**, R**^{**16**}**, Ar**^{**1**} and **Ar**^{**2**} and in the ring system **-NR**^{**13**}**R**^{**15**} optionally carry independently of each other one to three of the same or different substituents selected from the group consisting of halogen, cyano, C₁-C₆-alkyl, trifluoromethyl, C₃-C₈-cycloalkyl, phenyl, benzyl, hydroxy, C₁-C₆-alkoxy, which is optionally entirely or partially substituted by fluorine, benzyloxy, phenoxy, mercapto, C₁-C₆-alkylthio, carboxy, C₁-C₆-alkoxycarbonyl, benzyloxycarbonyl, nitro, amino, mono-C₁-C₆-alkylamino, and di-(C₁-C₆-alkyl)-amino, wherein two adjacent groups of the aromatic ring or ring system optionally form an additional ring over a methylenedioxy bridge
tautomeres in the case of substitution of the heterocycle or in an anellated ring system by free hydroxy, mercapto and/or amino groups,
stereoisomers and/or mixtures thereof and pharmacologically acceptable acid addition salts
for the production of medicaments for cytostatic or immunomodulatory and/or immunosuppressive treatment.

A preferred embodiment according to the invention relates to the use of compounds of formula (I), wherein
- **R**^{**1**}: is selected from hydrogen, halogen, cyano, methyl, trifluoromethyl, hydroxy, C₁-C₄-alkoxy, ethylthio, methoxycarbonyl, tert-butoxycarbonyl, aminocarbonyl, carboxy, and phenoxy,
- **R**^{**2**}: is selected from hydrogen, halogen, trifluoromethyl and hydroxy,
- **R**^{**3**}: is hydrogen or halogen,
- **R**^{**4**}: is selected from hydrogen, C₁-C₃-alkyl, hydroxy and C₁-C₃-alkoxy,
- **k**: is 0 or 1,
- **A**: is selected from the group consisting of C₂-C₆-alkylene, which is optionally substituted once or twice by C₁-C₃-alkyl, hydroxy or fluorine, as
C₂-C₆-alkenylene, which is optionally substituted once or twice by C₁-C₃-alkyl, hydroxy or fluorine,
C₄-C₆-alkadienylene, which is optionally substituted by is C₁-C₃-alkyl or by one or two fluorine atoms,
1,3,5-hexatrienylene, which is optionally substituted by fluorine, and
C₂-C₆-alkylene, wherein a methylene unit is isosterically replaced by O, S, CO or SO₂, and the isosteric substitute, with the exception of =CO is not adjacent to the amide group and,
- **D**: is selected from C₁-C₈-alkylene, which is optionally substituted once or twice by methyl or hydroxy,
C₂-C₈-alkenylene, which is optionally substituted once or twice by methyl or hydroxy, wherein the double bond optionally is to ring E,
C₃-C₈-alkinylene, which is optionally substituted once or twice by methyl or hydroxy, and
the group consisting of C₁-C₈-alkylene, C₂-C₈-alkenylene and C₃-C₈-alkinylene, in which one to three methylene units are isosterically replaced by O, S, NH, N(CH₃), N(COCH₃), N(SO₂CH₃), CO, SO or SO₂,
- **E**: is selected from 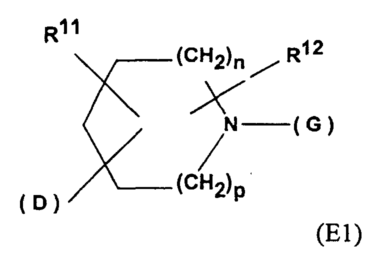 and 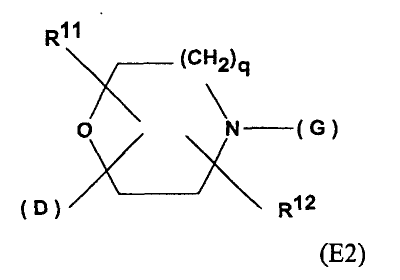 wherein the heterocyclic ring optionally has a double bond and
- **n**: and
- **p**: are independent of each other 0, 1, 2 or 3, with the proviso that **n** + **p** ≤ 3,
- **q**: is 1 or 2,
- **R**^{**11**}: is selected from hydrogen, C₁-C₃-alkyl, hydroxy, and hydroxymethyl,
- **R**^{**12**}: is hydrogen or an oxo group, which is adjacent to the nitrogen atom,
- **G**: is selected from hydrogen, **G1, G2, G3, G4** and **G5,** wherein
- **G1**: represents the residue

**―(CH**_{**2**}**)**_{**r**}**―(CR**^{**14**}**R**^{**15**}**)**_{**s**}**―R**^{**13**} (G1)

wherein
- **r**: is 0, 1 or 2,
- **s**: is 0 or 1,
- **R**^{**13**}: is selected from hydrogen, C₁-C₆-alkyl, C₃-C₈-cycloalkyl, benzyl, phenyl,
the group consisting of benzocyclobutyl, indanyl, indenyl, oxoindanyl, naphthyl, dihydronaphthyl, tetrahydronaphthyl, oxotetrahydronaphthyl, biphenylenyl, fluorenyl, oxofluorenyl, anthryl, dihydroanthryl, oxodihydroanthryl, dioxodihydroanthryl, phenanthryl, dihydrophenanthryl, oxodihydrophenanthryl, dibenzocycloheptenyl, oxodibenzocycloheptenyl, dihydrodibenzocycloheptenyl, oxodihydrodibenzocycloheptenyl, dihydrodibenzocyclooctenyl, tetrahydrodibenzocyclooctenyl and oxotetrahydrodibenzocyclooctenyl, bound directly or over a methylene group, and
the group consisting of furyl, thienyl, pyrrolyl, oxazolyl, isoxazolyl, thiazolyl, isothiazolyl, pyrazolyl, imidazolyl, oxadiazolyl, thiadiazolyl, triazolyl, pyridyl, pyrazinyl, pyridazinyl, pyrimidinyl, triazinyl, imidazothiazolyl, benzofuryl, dihydrobenzofuryl, benzothienyl, dihydrobenzothienyl, indolyl, indolinyl, oxoindolinyl, dioxoindolinyl, benzoxazolyl, oxobenzoxazolinyl, benzisoxazolyl, oxobenzisoxazolinyl, benzothiazolyl, oxobenzthiazolinyl, benzoisothiazolyl, oxobenzoisothiazolinyl, benzimidazolyl, oxobenzimidazolinyl, indazolyl, oxoindazolinyl, benzofurazanyl, benzothiadiazolyl, benzotriazolyl, oxazolopyridyl, oxodihydrooxazolopyridyl, thiazolopyridyl, oxodihydrothiazolopyridyl, isothiazolopyridyl, imidazopyridyl, oxodihydroimidazopyridyl, pyrazolopyridyl, oxodihydropyrazolopyridyl, thienopyrimidinyl, chromanyl, chromanonyl, benzopyranyl, chromonyl, quinolyl, isoquinolyl, dihydroquinolyl, oxodihydroquinolinyl, tetrahydroquinolyl, oxotetrahydroquinolinyl, benzodioxanyl, quinoxalinyl, quinazolinyl, naphthyridinyl, carbazolyl, tetrahydrocarbazolyl, oxotetrahydrocarbazolyl, pyridoindolyl, acridinyl, oxodihydroacridinyl, phenothiazinyl, dihydrodibenzoxepinyl, oxodihydrodibenzoxepinyl, benzocycloheptathienyl, oxobenzocycloheptathienyl, dihydrothienobenzothiepinyl, oxodihydrothienobenzothiepinyl dihydrodibenzothiepinyl, oxodihydrodibenzothiepinyl, octahydrodibenzothiepinyl, dihydrodibenzazepinyl, oxodihydrodibenzazepinyl, octahydrodibenzazepinyl, benzocycloheptapyridyl, oxobenzocycloheptapyridyl, dihydropyridobenzodiazepinyl, dihydrodibenzoxazepinyl, dihydropyridobenzoxepinyl, dihydropyridobenzoxazepinyl, oxodihydropyridobenzoxazepinyl, dihydrodibenzothiazepinyl, oxodihydrodibenzothiazepinyl, dihydropyridobenzothiazepinyl, and oxodihydropyridobenzothiazepinyl, bound directly or over a methylene group,
- **R**^{**14**}: has the same meaning as **R**^{**13**}, but is selected independently therefrom,
- **R**^{**15**}: is selected from hydrogen, hydroxy, methyl, benzyl, phenyl, and
the group consisting of indanyl, indenyl, naphthyl, dihydronaphthyl, tetrahydronaphthyl, furyl, thienyl, pyrrolyl, oxazolyl, isoxazolyl, thiazolyl, isothiazolyl, pyrazolyl, imidazolyl, oxadiazolyl, thiadiazolyl, triazolyl, pyridyl, pyrazinyl, pyridazinyl, pyrimidinyl, triazinyl, benzofuryl, benzothienyl, indolyl, indolinyl, benzoxazolyl, benzothiazolyl, benzimidazolyl, chromanyl, quinolyl and tetrahydroquinolyl, bound directly or over a methylene group,
- **G2**: is selected from the residues 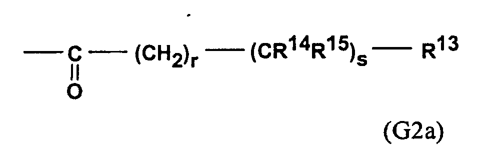 and 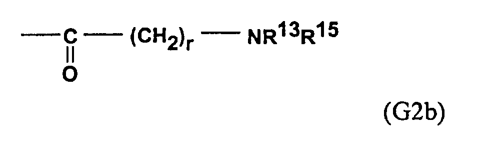 wherein the substituents **R**^{**13**} and **R**^{**15**} have the above meanings, or the group

**―NR**^{**13**}**R**^{**15**}

represents a nitrogen-containing heterocycle bound over the nitrogen atom, the nitrogen-containing heterocycle being selected from the group consisting of azetidine, pyrrolidine, piperidine, (1H)tetrahydropyridine, hexahydroazepine, (1H)tetrahydroazepine, octahydroazocine, pyrazolidine, piperazine, hexyhydrodiazepine, morpholine, hexahydrooxazepine, thiomorpholine, thiomorpholine-1,1-dioxide, 5-aza-bicyclo[2.1.1]hexane, 2-azabicyclo[2.2.1]heptane, 7-aza-bicyclo[2.2.1]heptane, 2,5-diaza-bicyclo[2.2.1]heptane, 2-aza-bicyclo[2.2.2]octane, 8-aza-bicyclo[3.2.1]octane, 2,5-diazabicyclo[2.2.2]octane, 9-azabicyclo[3.3.1]nonane, indoline, isoindoline, (1H)-dihydroquinoline, (1H)-tetrahydroquinoline, (2H)-tetrahydroisoquinoline, (1H)-tetrahydroquinoxaline, (4H)-dihydrobenzoxazine, (4H)-dihydrobenzothiazine, (1H)-tetrahydrobenzo[b]azepine, (1H)-tetrahydrobenzo[c]azepine, (1H)-tetrahydrobenzo[d]azepine, (5H)-tetrahydrobenzo[b]oxazepine, (5H)-tetrahydrobenzo[b]thiazepine, 1,2,3,4-tetrahydro-9H-pyrido[3,4-b]indole, (10H)-dihydroacridine, 1,2,3,4-tetrahydroacridanone, (10H)-phenoxazine, (10H)-phenothiazine,. (5H)-dibenzazepine, (5H)-dihydrodibenzazepine, (5H)-octahydrodibenzazepine, (5H)-dihydrodibenzodiazepine, (11H)-dihydrodibenzo[b,e]oxazepine, (11H)-dihydrodibenzo[b,e]thiazepine, (10H)-dihydrodibenzo[b,f]oxazepine, (10H)-dihydrodibenzo[b,f]thiazepine and (5H)-tetrahydrodibenzazocine,
- **G3**: is the residue

**―SO**_{**2**}**―(CH**_{**2**}**)**_{**r**} **R**^{**13**} (G3),
- **G4**: is the residue 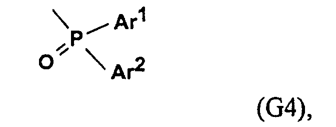 wherein
- **Ar**^{**1**}: and
- **Ar**^{**2**}: are selected independently of each other from phenyl, pyridyl and naphthyl,
- **G5**: is the residue

**―COR**^{**16**} (G5)

wherein
- **R**^{**16**}: is selected from trifluoromethyl, C₁-C₆-alkoxy, C₃-C₆-alkenyloxy and benzyloxy,
wherein aromatic ring systems optionally are substituted independently of each other by one to three of the same or different substituents selected from the group consisting of halogen, cyano, C₁-C₆-alkyl, trifluoromethyl, C₃-C₈-cycloalkyl, phenyl, benzyl, hydroxy, C₁-C₆-alkoxy, C₁-C₆-alkoxy entirely or partially substituted by fluorine; benzyloxy, phenoxy, mercapto, C₁-C₆-alkylthio, carboxy, C₁-C₆-alkoxycarbonyl, benzyloxycarbonyl, nitro, amino, mono-C₁-C₆-alkylamino, and di-(C₁-C₆-alkyl)-amino,
wherein two adjacent groups of the aromatic ring or ring system optionally form an additional ring over a methylenedioxy bridge.

A further preferred embodiment according to the invention relates to the use of compounds of formula (I), wherein:
- **R**^{**1**}: is selected from hydrogen, halogen, cyano, methyl, trifluoromethyl, hydroxy, methoxy and methoxycarbonyl,
- **R**^{**2**}: is hydrogen or halogen,
- **R**^{**3**}: is hydrogen,
- **R**^{**4**}: is hydrogen, C₁-C₃-alkyl or hydroxy,
- **k**: is 0 or 1,
- **A**: is selected from C₂-C₆-alkylene, which is optionally substituted once or twice by hydroxy or fluorine,
C₂-C₆-alkenylene, which is optionally substituted once or twice by hydroxy or fluorine,
C₄-C₆-alkadienylene, which is optionally substituted by one or two fluorine atoms,
1,3,5-hexatrienylene and
C₂-C₆-alkylene, wherein a methylene unit is isosterically replaced by O, S or CO, and the isosteric substitute, with the exception of=CO, is not adjacent to the amide group and,
- **D**: is selected from C₂-C₈-alkylene, which is optionally substituted by methyl or hydroxy
C₂-C₈-alkenylene, which is optionally substituted by methyl or hydroxy, wherein the double bond optionally is to ring E, and
the group consisting of C₂-C₈-alkylene and C₂-C₈-alkenylene, wherein one to three methylene units are isosterically replaced by O, NH, N(CH₃), N(COCH₃), N(SO₂CH₃) or CO,
- **E**: is selected from 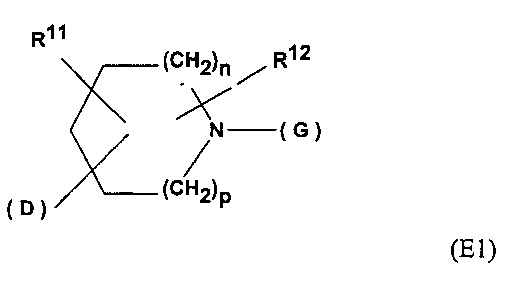 and 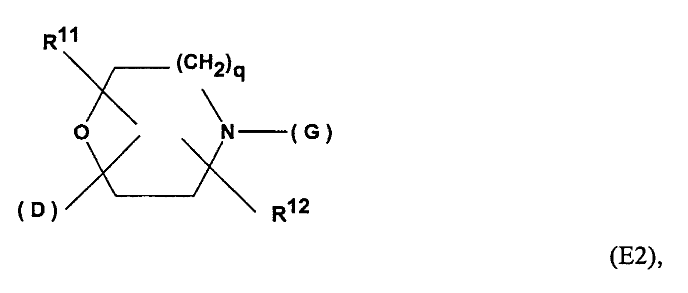 wherein the heterocyclic ring optionally has a double bond and
- **n** and **p**: are, independent of each other, 0, 1, 2 or 3, with the proviso that **n** + **p** ≤ 3,
- **q**: is 1 or 2
- **R**^{**11**}: is selected from the group consisting of hydrogen, methyl and hydroxyl,
- **R**^{**12**}: is hydrogen or an oxo group adjacent to the nitrogen atom,
- **G**: is selected from hydrogen, C₃-C₈-cycloalkyl, methoxycarbonyl, tert-butoxycarbonyl, benzyloxycarbonyl, trifluoroacetyl, diphenylphosphinoyl and the residues

**―(CH**_{**2**}**)**_{**r**}**―(CR**^{**14**}**R**^{**15**}**)**_{**s**}**―R**^{**13**} (G1),

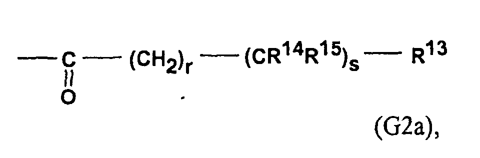

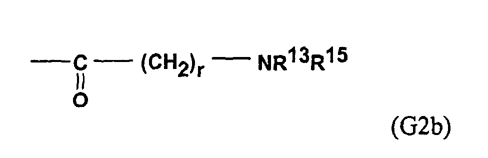

and

**―SO**_{**2**}**―(CH**_{**2**}**)**_{**r**} **R**^{**13**} (G3)

wherein
- **r**: is 0, 1 or 2,
- **s**: is 0 or 1,
- **R**^{**13**}: is selected from hydrogen, methyl, benzyl, phenyl,
the group consisting of indanyl, indenyl, oxoindanyl, naphthyl, dihydronaphthyl, tetrahydronaphthyl, oxotetrahydronaphthyl, fluorenyl, oxofluorenyl, anthryl, dihydroanthryl, oxodihydroanthryl, dioxodihydroanthryl, dibenzocycloheptenyl, oxodibenzocycloheptenyl, dihydrodibenzocycloheptenyl, and oxodihydrodibenzocycloheptenyl bound directly or over a methylene group, and
the group consisting of furyl, thienyl, pyrrolyl, oxazolyl, isoxazolyl, thiazolyl, isothiazolyl, pyrazolyl, imidazolyl, oxadiazolyl, thiadiazolyl, triazolyl, pyridyl, pyrazinyl, pyridazinyl, pyrimidinyl, imidazothiazolyl, benzofuryl, dihydrobenzofuryl, benzothienyl, dihydrobenzothienyl, indolyl, indolinyl, oxoindolinyl, dioxoindolinyl, benzoxazolyl, oxobenzoxazolinyl, benzisoxazolyl, oxobenzisoxazolinyl, benzothiazolyl, oxobenzthiazolinyl, benzoisothiazolyl, oxobenzoisothiazolinyl, benzimidazolyl, oxobenzimidazolinyl, benzofurazanyl, benzothiadiazolyl, benzotriazolyl, oxazolopyridyl, oxodihydrooxazolopyridyl, thiazolopyridyl, oxodihydrothiazolopyridyl, isothiazolopyridyl, imidazopyridyl, oxodihydroimidazopyridyl, pyrazolopyridyl, thienopyrimidinyl, chromanyl, chromanonyl, benzopyranyl, chromonyl, quinolyl, isoquinolyl, dihydroquinolyl, oxodihydroquinolinyl, tetrahydroquinolyl, oxotetrahydroquinolinyl, benzodioxanyl, quinoxalinyl, quinazolinyl, naphthyridinyl, carbazolyl, tetrahydrocarbazolyl, oxotetrahydrocarbazolyl, pyridoindolyl, acridinyl, oxodihydroacridinyl, phenothiazinyl, dihydrodibenzoxepinyl, benzocycloheptathienyl, oxobenzocycloheptathienyl, dihydrothienobenzothiepinyl, oxodihydrothienobenzothiepinyl dihydrodibenzothiepinyl, oxodihydrodibenzothiepinyl, dihydrodibenzazepinyl, oxodihydrodibenzazepinyl, octahydrodibenzazepinyl, benzocycloheptapyridyl, oxobenzocycloheptapyridyl, dihydropyridobenzoxepinyl, dihydrodibenzothiazepinyl, and oxodihydrodibenzothiazepinyl, bound directly or over a methylene group,
- **R**^{**14**}: is selected from the group consisting of hydrogen, methyl, benzyl and phenyl,
- **R**^{**15**}: is selected from hydrogen, hydroxy, methyl, benzyl, phenyl, and
the group consisting of naphthyl, furyl, thienyl, oxazolyl, thiazolyl, pyrazolyl, imidazolyl, oxadiazolyl, thiadiazolyl, pyridyl, benzofuryl, benzothienyl, indolyl, indolinyl, benzoxazolyl, benzothiazolyl, benzimidazolyl, chromanyl, quinolyl and tetrahydroquinolyl, bound directly or over a methylene group,
wherein in formula 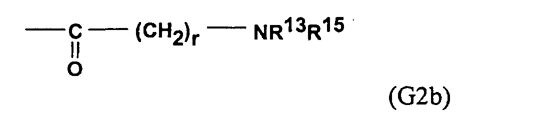 the group **NR**^{**13**}**R**^{**15**} optionally is selected from pyrrolidine, piperidine, (1H)tetrahydropyridine, hexahydroazepine, octahydroazocine, piperazine, hexahydrodiazepine, morpholine, hexahydrooxazepine, 2-azabicyclo[2.2.1]heptane, 7-azabicyclo[2.2.1]heptane, 2,5-diazabicyclo[2.2.1]heptane, 8-azabicyclo[3.2.1]octane, 2,5-diazabicyclo[2.2.2]octane, indoline, isoindoline, (1H)-dihydroquinoline, (1H)-tetrahydroquinoline, (2H)-tetrahydroisoquinoline, (1H)-tetrahydroquinoxaline, (4H)-dihydrobenzoxazine, (4H)-dihydrobenzothiazine, (1H)-tetrahydrobenzo[b]azepine, (1H)-tetrahydrobenzo[d]azepine, (5H)-tetrahydrobenzo[b]oxazepine, (5H)-tetrahydrobenzo[b]thiazepine, 1,2,3,4-tetrahydro-9H-pyrido[3,4-b]indol, (10H)-dihydroacridine, 1,2,3,4-tetrahydroacridanone, (5H)-dihydrodibenzazepine, (5H)-dihydrodibenzodiazepine, (11H)-dihydrodibenzo[b,e]oxazepine, (11H)-dihydrodibenzo[b,e]thiazepine, (10H)-dihydrodibenzo[b,f]oxazepine and (5H)-tetrahydrodibenzazocine.

An even further preferred embodiment according to the invention relates to the use of compounds of formula (I), wherein:
- **R**^{**1**}: is selected from hydrogen, fluorine, chlorine, bromine, methyl, trifluoromethyl and hydroxy,
- **R**^{**2**}: and
- **R**^{**3**}: are hydrogen,
- **R**^{**4**}: is hydrogen or hydroxy,
- **k**: is 0 or 1,
- **A**: is selected from C₂-C₆-alkylene, which is optionally substituted once or twice by hydroxy or fluorine,
C₂-C₄-alkenylene, which is optionally substituted by fluorine, and
1,3-butadienylene, which is optionally substituted by fluorine,
- **D**: is selected from C₂-C₆-alkylene, C₂-C₆-alkenylene, wherein the double bond optionally is to ring E, and the group consisting of C₂-C₆-alkylene and C₂-C₆-alkenylene, wherein a methylene unit is isosterically replaced by O, NH, N(CH₃) or CO, or an ethylene group is isosterically replaced by NH-CO or CO-NH or a propylene group is isosterically replaced by NH-CO-O or O-CO-NH,
- **E**: is selected from pyrrolidine, piperidine, 1,2,5,6-tetrahydropyridine, hexahydroazepine, morpholine and hexahydro-1,4-oxazepine, wherein the heterocyclic ring , optionally is substituted by an oxo group adjacent to the nitrogen atom
- **G**: is selected from **hydrogen**, tert-butoxycarbonyl, diphenylphosphinoyl, and of the residues

**―(CH**_{**2**}**)**_{**r**}**―(CR**^{**14**}**R**^{**15**}**)**_{**s**}**―R**^{**13**} (G1),

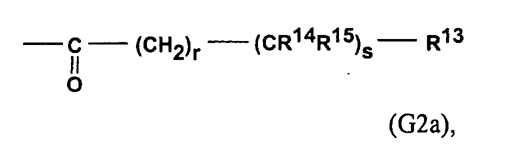

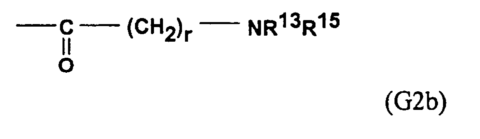

and

**―SO**_{**2**}**―(CH**_{**2**}**)**_{**r**} **R**^{**13**} (G3)

wherein
- **r**: is 0 or 1,
- **s**: is 0 or 1,
- **R**^{**13**}: is selected from hydrogen, methyl, benzyl, phenyl,
the group consisting of indenyl, oxoindanyl, naphthyl, tetrahydronaphthyl, fluorenyl, oxofluorenyl, anthryl, dihydroanthryl, oxodihydroanthryl, dioxodihydroanthryl, dibenzocycloheptenyl, and dihydrodibenzocycloheptenyl bound directly or over a methylene group, and
the group consisting of furyl, thienyl, oxazolyl, thiazolyl, imidazolyl, oxadiazolyl, thiadiazolyl, pyridyl, pyrazinyl, pyrimidinyl, imidazothiazolyl, benzofuryl, benzothienyl, indolyl, oxoindolinyl, dioxoindolinyl, benzoxazolyl, oxobenzoxazolinyl, benzothiazolyl, oxobenzthiazolinyl, benzimidazolyl, oxobenzimidazolinyl, benzofurazanyl, benzotriazolyl, oxazolopyridyl, oxodihydrooxazolopyridyl, thiazolopyridyl, oxodihydrothiazolopyridyl, chromanyl, chromanonyl, benzopyranyl, chromonyl, quinolyl, isoquinolyl, oxodihydroquinolinyl, tetrahydroquinolyl, oxotetrahydroquinolinyl, benzodioxanyl, quinazolinyl, acridinyl, oxodihydroacridinyl, phenothiazinyl, dihydrodibenzoxepinyl, benzocycloheptathienyl, dihydrothienobenzothiepinyl, dihydrodibenzothiepinyl, oxodihydrodibenzothiepinyl, dihydrodibenzazepinyl, oxodihydrodibenzazepinyl, octahydrodibenzazepinyl, benzocycloheptapyridyl, oxobenzocycloheptapyridyl, and dihydrodibenzothiazepinyl, bound directly or over a methylene group,
- **R**^{**14**}: is hydrogen, methyl, benzyl or phenyl,
- **R**^{**15**}: is selected from the group consisting of hydrogen, hydroxy, methyl, benzyl, phenyl, and
the group consisting of naphthyl, furyl, thienyl, pyridyl, benzofuryl, benzothienyl, indolyl, benzoxazolyl, benzothiazolyl, benzimidazolyl, chromanyl, quinolyl and tetrahydroquinolyl bound directly or over a methylene group,
wherein in the formula 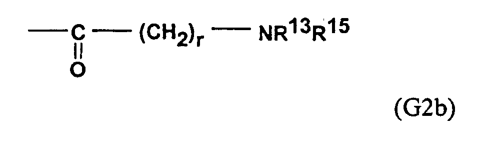 the group **NR**^{**13**}**R**^{**15**} optionally is selected from pyrrolidine, piperidine, hexahydroazepine, morpholine, 2,5-diazabicyclo[2.2.1]heptane, indoline, isoindoline, (1H)-dihydroquinoline, (1H)-tetrahydroquinoline, (2H)-tetrahydroisoquinoline, (1H)-tetrahydrobenzo[b]azepine, (1H)-tetrahydrobenzo[d]azepine, (5H)-tetrahydrobenzo[b]oxazepine, (5H)-tetrahydrobenzo[b]thiazepine, 1,2,3,4-tetrahydroacridanone, (5H)-dihydrodibenzazepine, (11H)-dihydrodibenzo[b,e]-oxazepine and (11H)-dihydrodibenzo[b,e]thiazepine,
wherein aromatic ring systems optionally are substituted, independently of each other, by one to three of the same or different substituents selected from the group consisting of halogen, cyano, C₁-C₆-alkyl, trifluoromethyl, C₃-C₈-cycloalkyl, phenyl, benzyl, hydroxy, C₁-C₆-alkoxy, C₁-C₆-alkoxy which is entirely or partially substituted by fluorine; benzyloxy, phenoxy, mercapto, C₁-C₆-alkylthio, carboxy, C₁-C₆-alkoxycarbonyl, benzyloxycarbonyl, nitro, amino, mono-C₁-C₆-alkylamino and di-(C₁-C₆-alkyl)-amino, wherein two adjacent groups of the aromatic ring or ring system optionally form an additional ring over a methylenedioxy bridge.

An especially preferred embodiment according to the invention relates to the use of compounds of formula (I), wherein:
- **R**^{**1**}: is selected from hydrogen, fluorine, methyl, trifluoromethyl and hydroxy,
- **R**^{**2**}: and
- **R**^{**3**}: are hydrogen,
- **R**^{**4**}: is hydrogen or hydroxy,
- **k**: is 0,
- **A**: is selected from the group consisting of ethylene, propylene and butylene, which are optionally substituted by hydroxy or once or twice by fluorine,
ethenylene, and
1,3-butadienylene
- **D**: is selected from C₂-C₆-alkylene and C₂-C₆-alkenylene, wherein the double bond optionally is to ring E,
- **E**: is selected from pyrrolidine, piperidine, hexahydroazepine and morpholine,
- **G**: is selected from benzyl, phenethyl, fluorenylmethyl, anthrylmethyl, diphenylmethyl, fluorenyl or dihydrodibenzocycloheptenyl, furylmethyl, thienylmethyl, thiazolylmethyl, pyridylmethyl, benzothienylmethyl, quinolylmethyl, phenyl-thienylmethyl, phenyl-pyridylmethyl, dihydrodibenzoxepinyl, dihydrodibenzothiepinyl,
acetyl, pivaloyl, phenylacetyl, diphenylacetyl, diphenylpropionyl, naphthylacetyl, benzoyl, naphthoyl, anthrylcarbonyl, oxofluorenylcarbonyl, oxodihydroanthrylcarbonyl, dioxodihydroanthrylcarbonyl,
furoyl, pyridylcarbonyl, chromonylcarbonyl, quinolylcarbonyl,
naphthylaminocarbonyl, dibenzylaminocarbonyl, benzylphenylaminocarbonyl, diphenylaminocarbonyl, indolinyl-1-carbonyl, dihydrodibenzazepin-N-carbonyl, tetrahydroquinolinyl-N-carbonyl, tetrahydrobenzo[b]azepinyl-N-carbonyl,
methanesulfonyl, phenylsulfonyl, p-toluolsulfonyl, naphthylsulfonyl, quinolinsulfonyl and
diphenylphosphinoyl,
wherein aromatic ring systems optionally are substituted independently of each other by one to three of the same or different substituents selected from the group consisting of halogen, cyano, C₁-C₆-alkyl, trifluoromethyl, C₃-C₈-cycloalkyl, phenyl, benzyl, hydroxy, C₁-C₆-alkoxy, C₁-C₆-alkoxy entirely or partially substituted by fluorine, benzyloxy, phenoxy, mercapto, C₁-C₆-alkylthio, carboxy, C₁-C₆-alkoxycarbonyl, benzyloxycarbonyl, nitro, amino, mono-C₁-C₆-alkylamino and di-(C₁-C₆-alkyl)-amino, wherein two adjacent groups in the ring or ring system optionally form an additional ring over a methylendioxy bridge.

A series of exemplary compounds with the respective substituent definitions are listed in the following Table 1 for illustration of the invention.

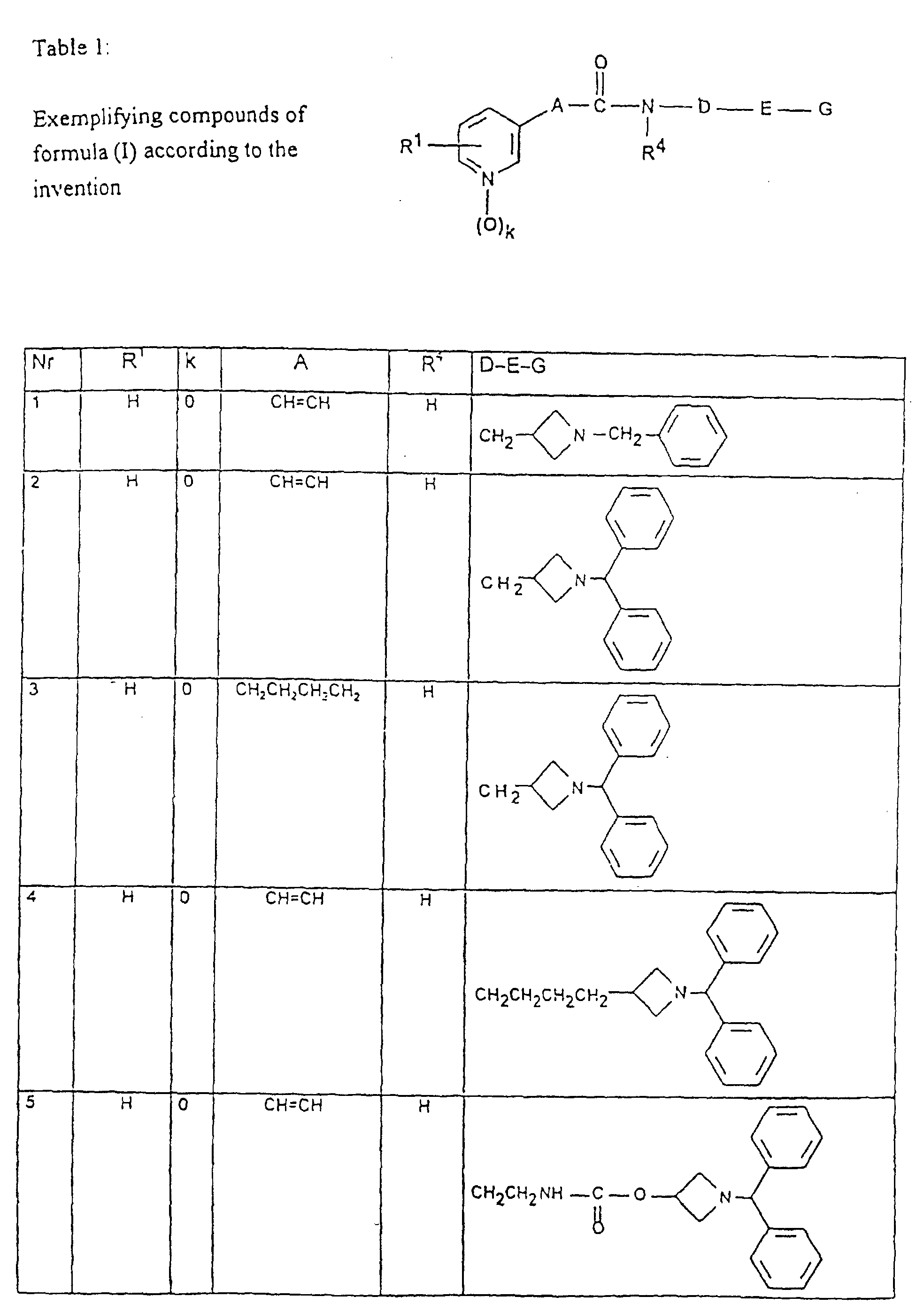

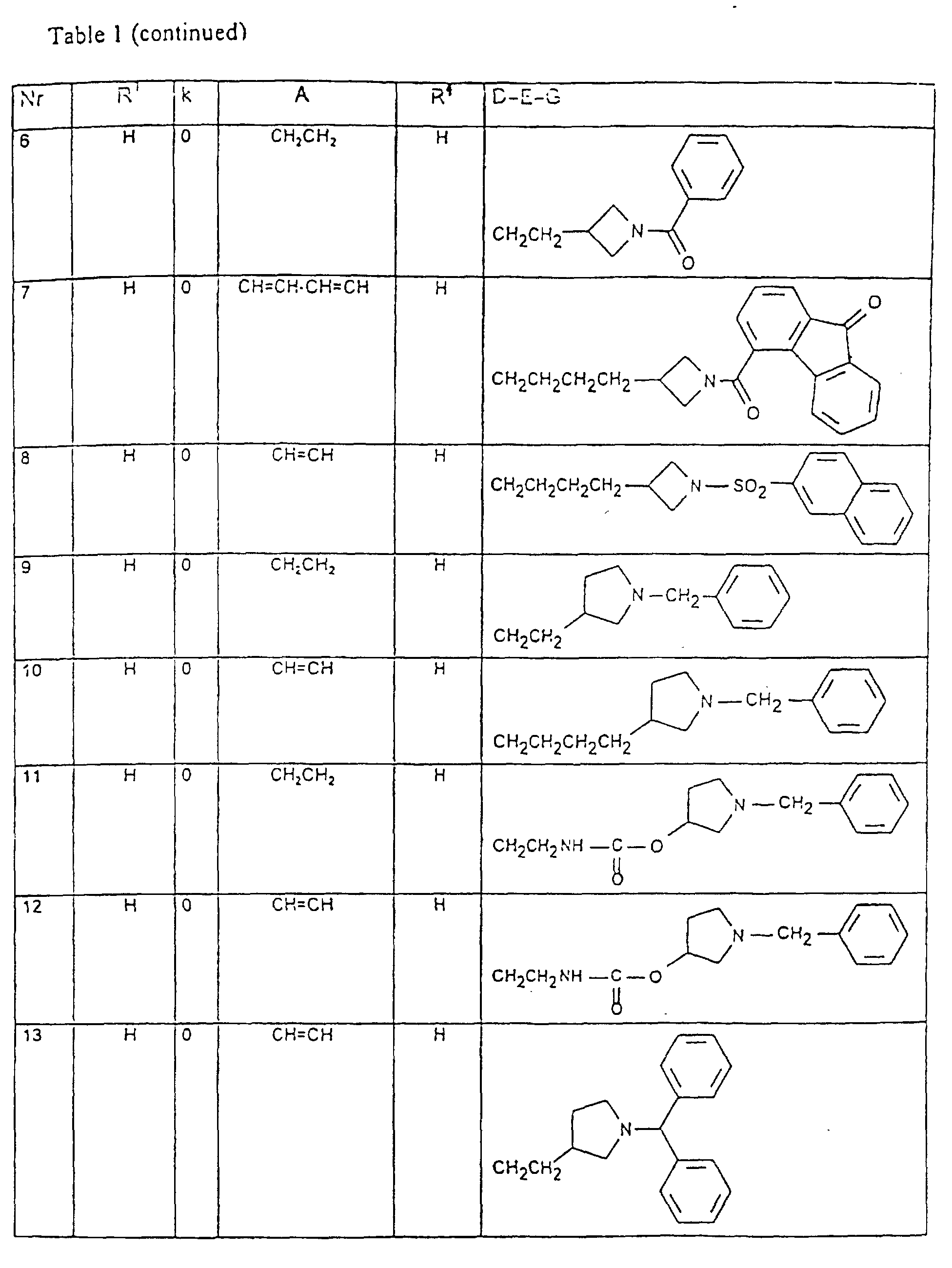

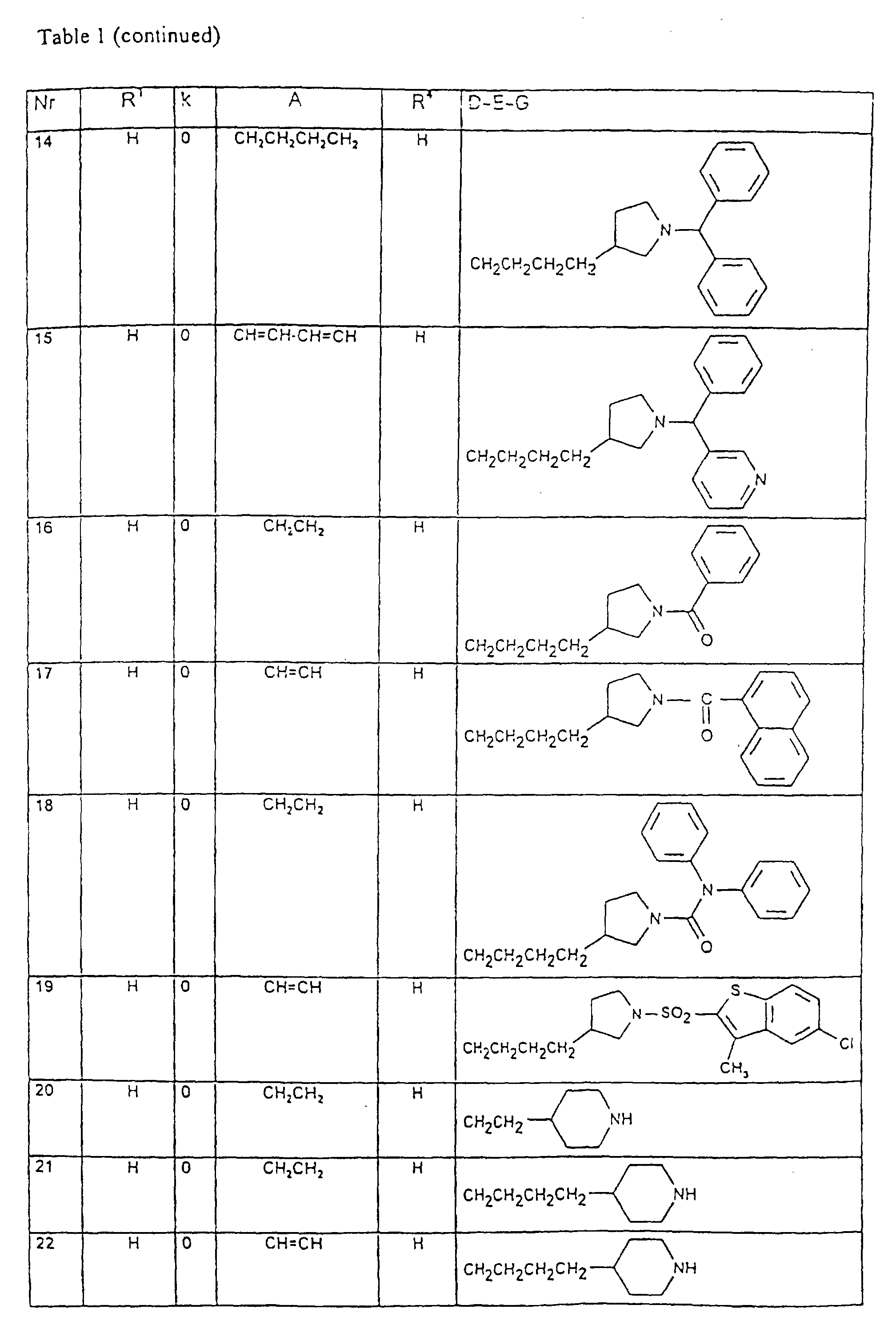

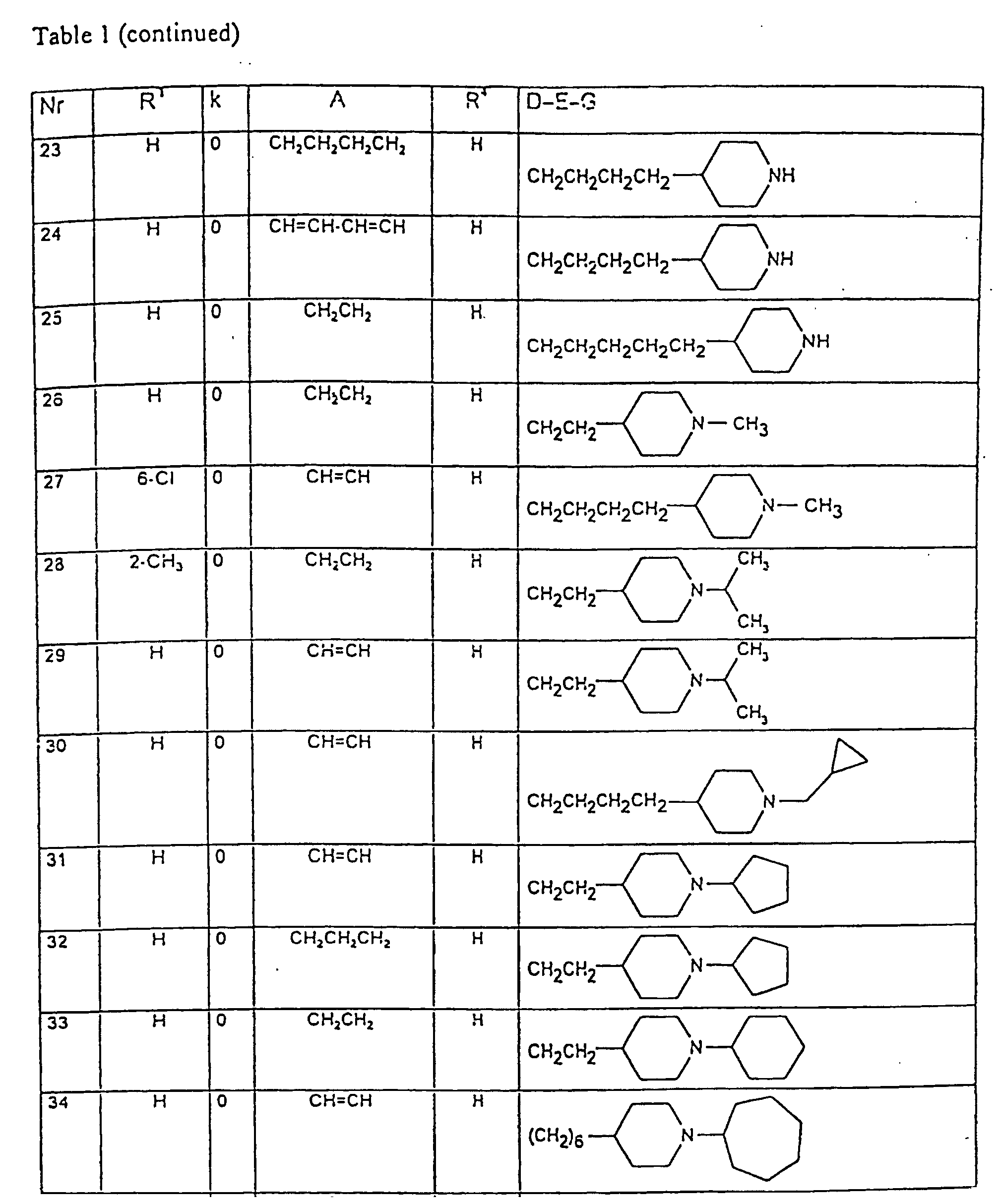

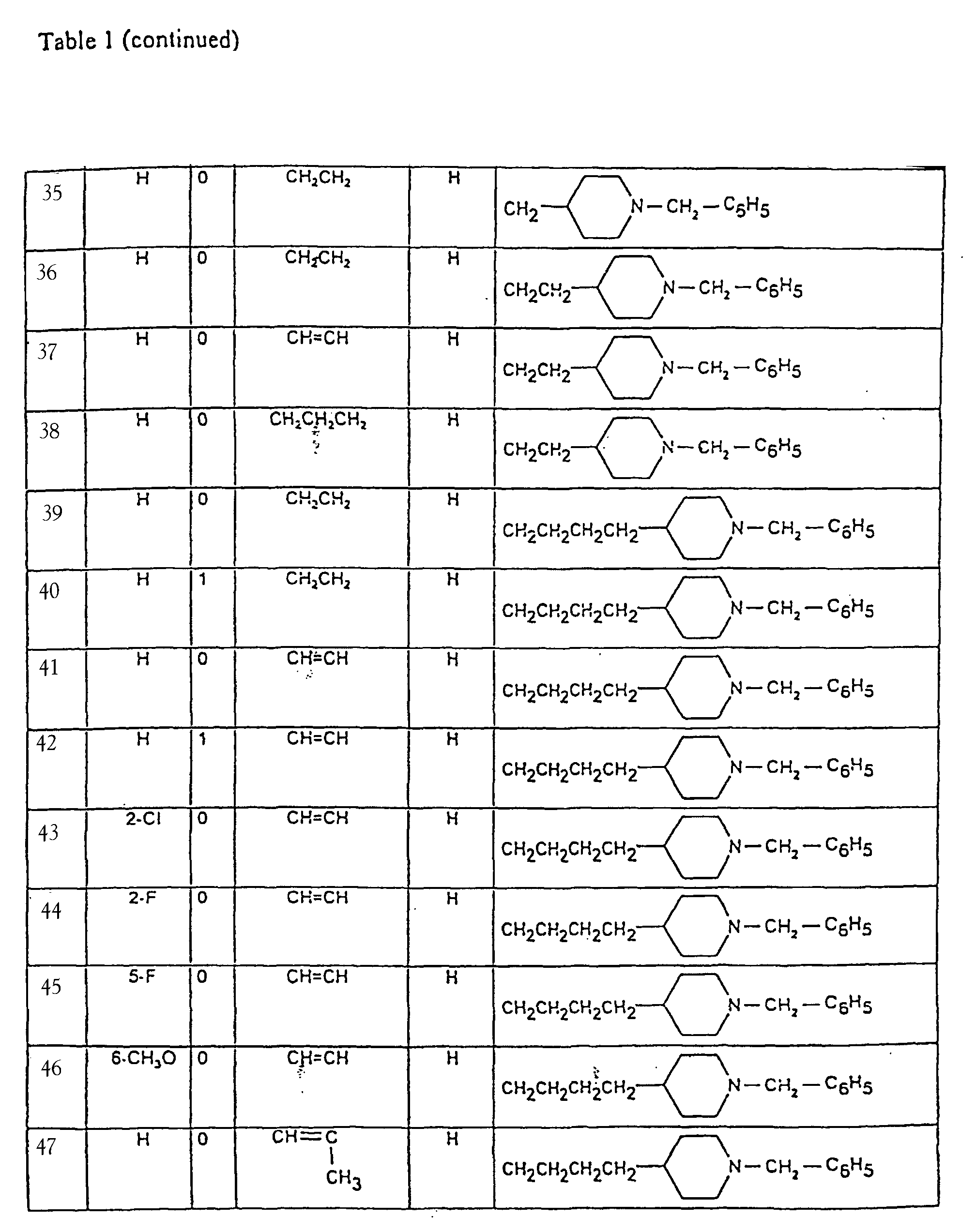

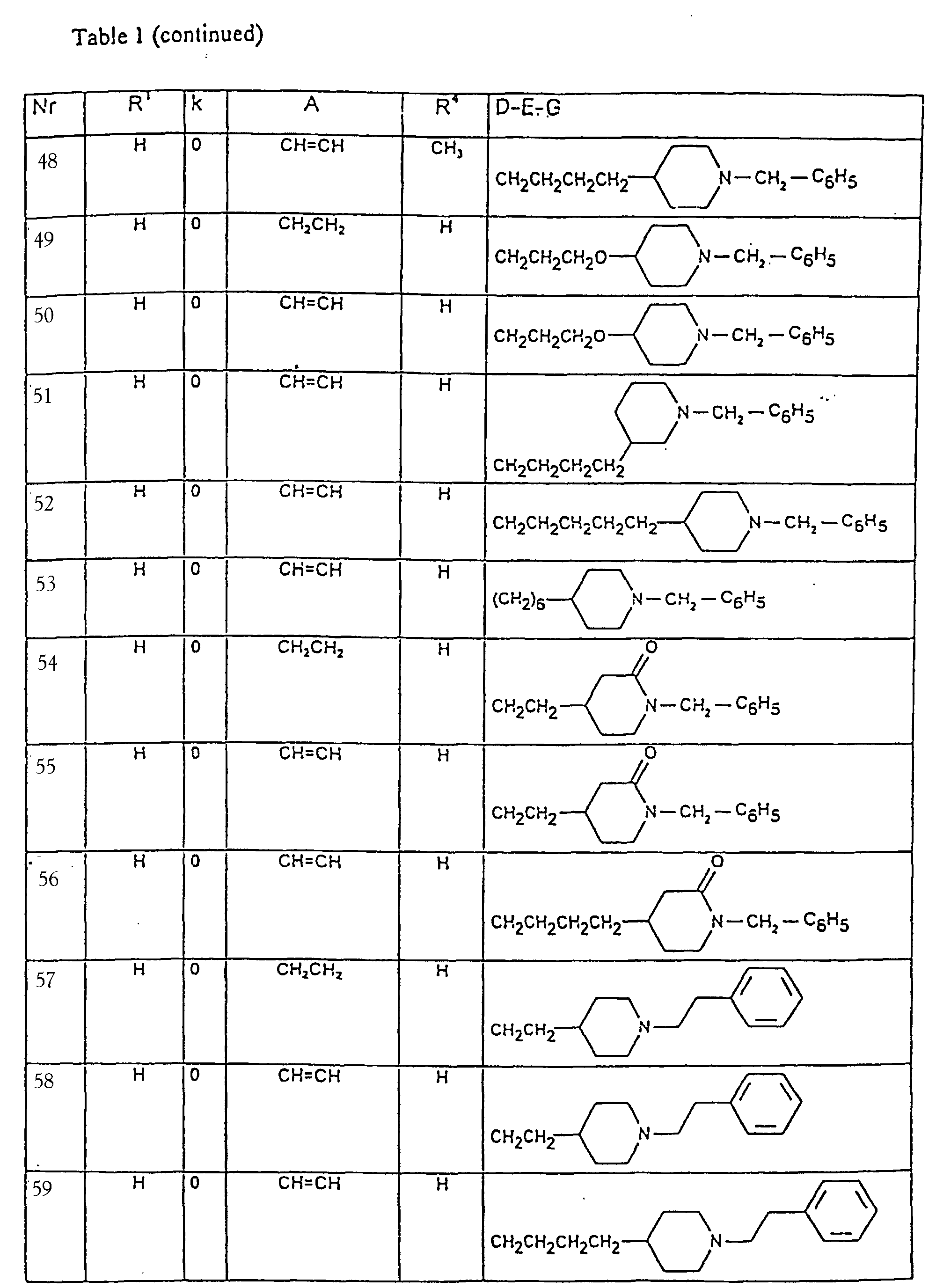

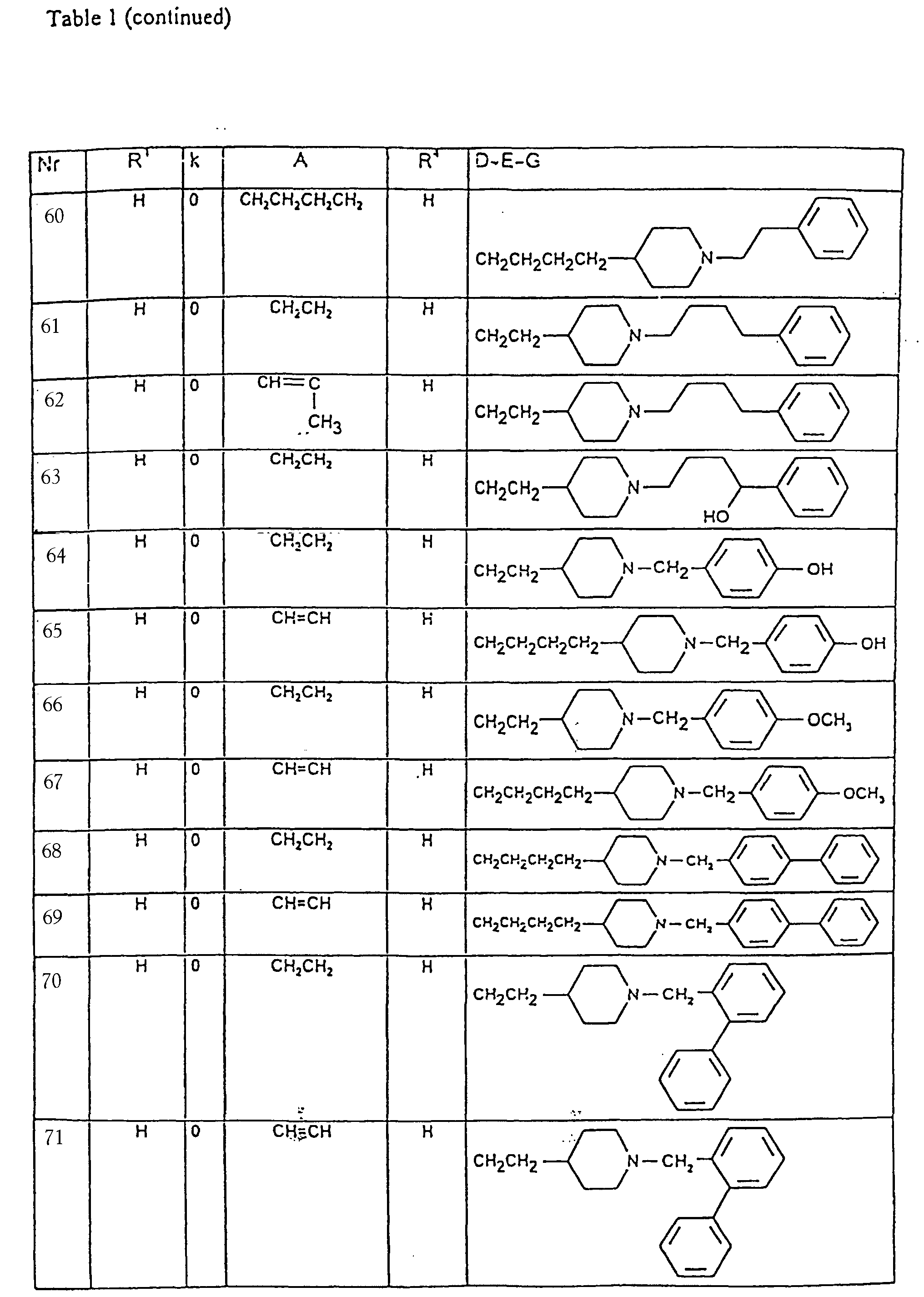

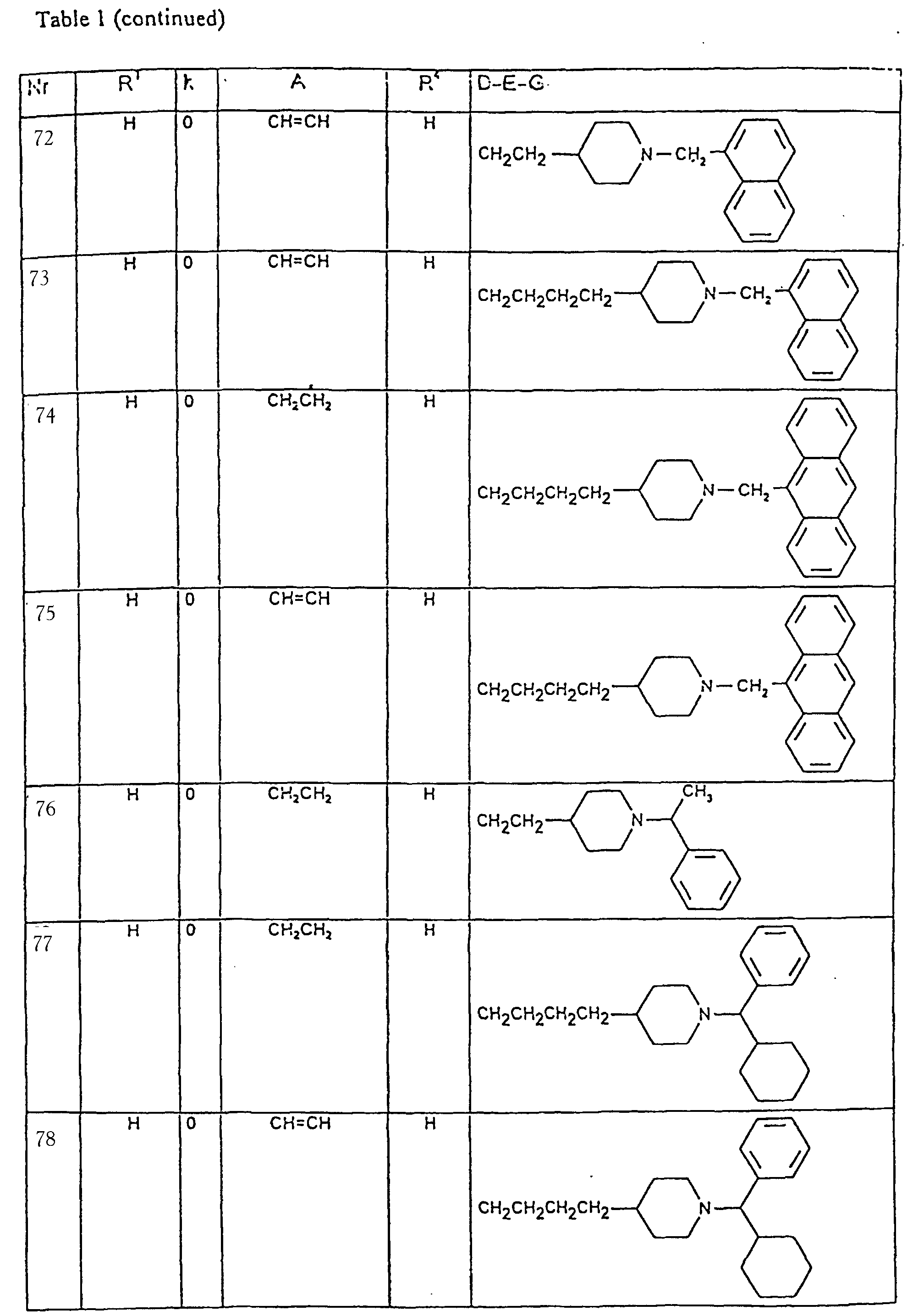

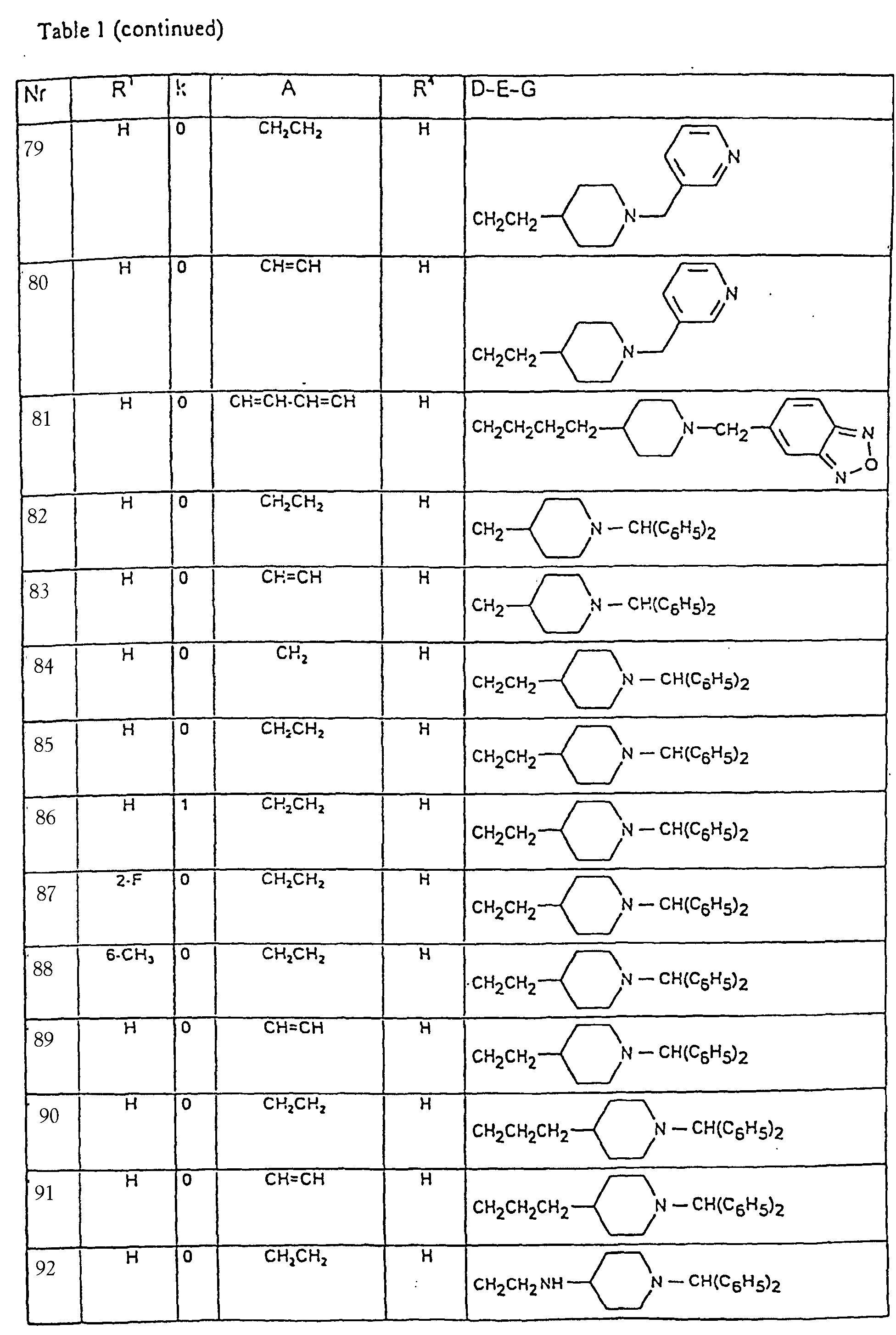

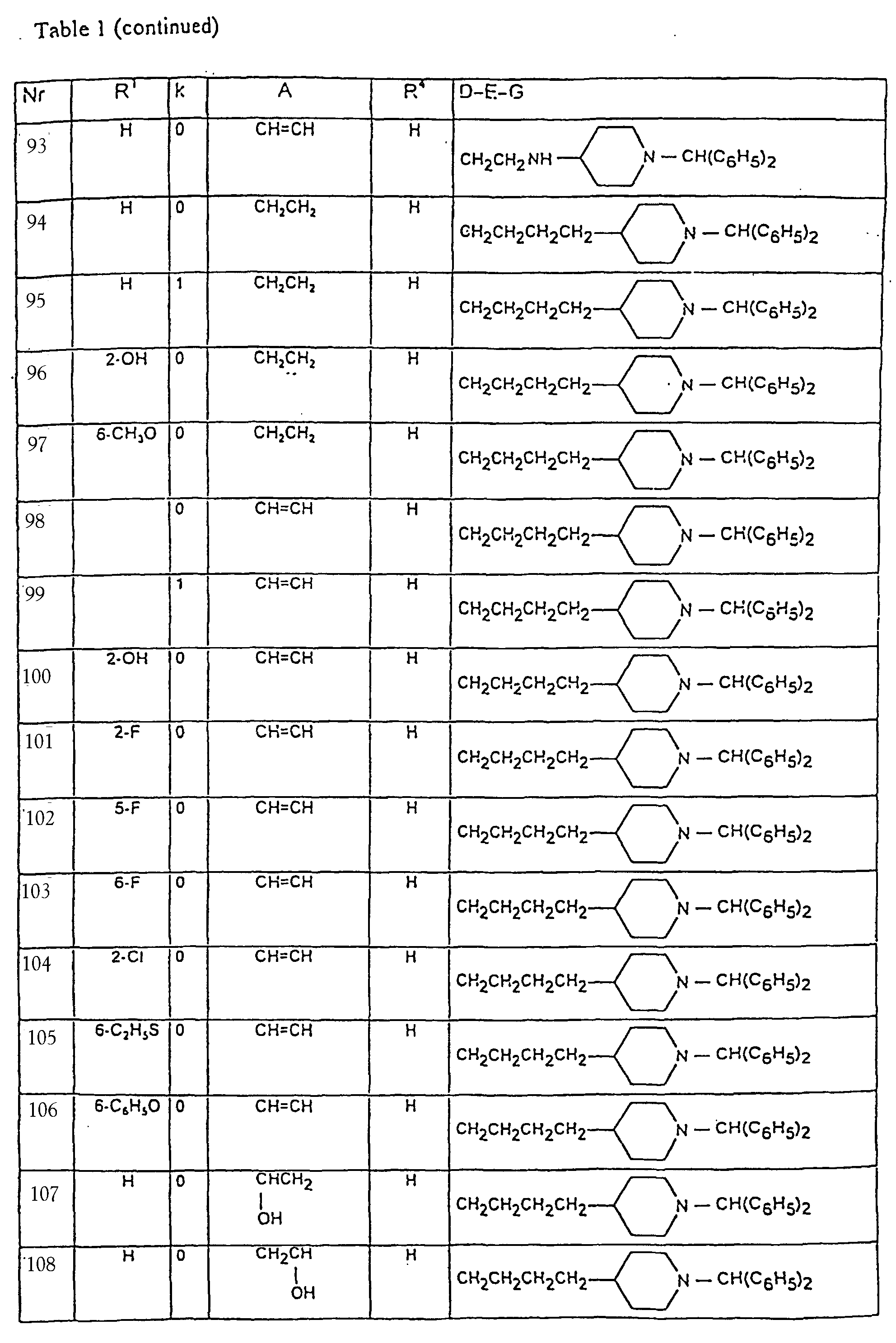

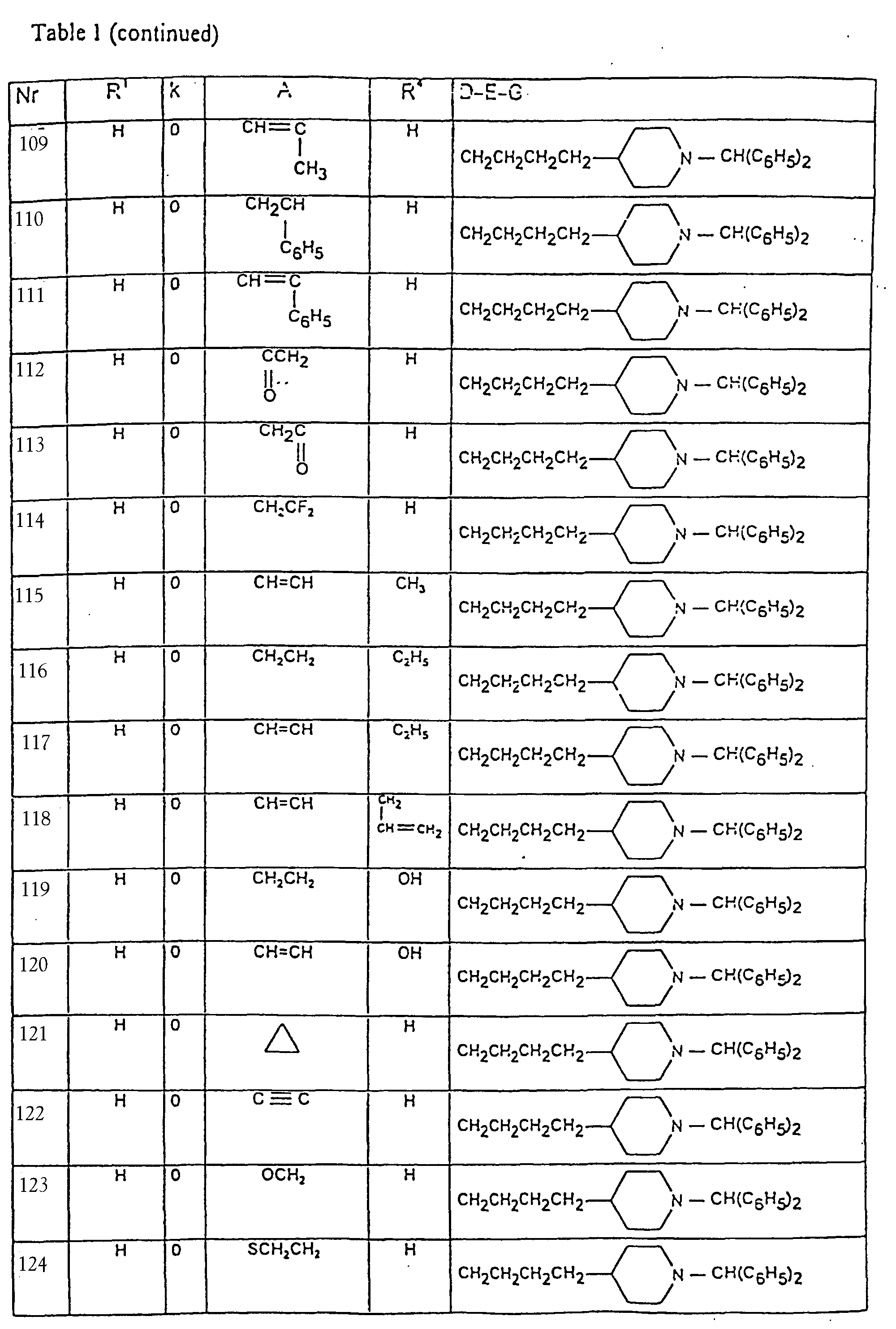

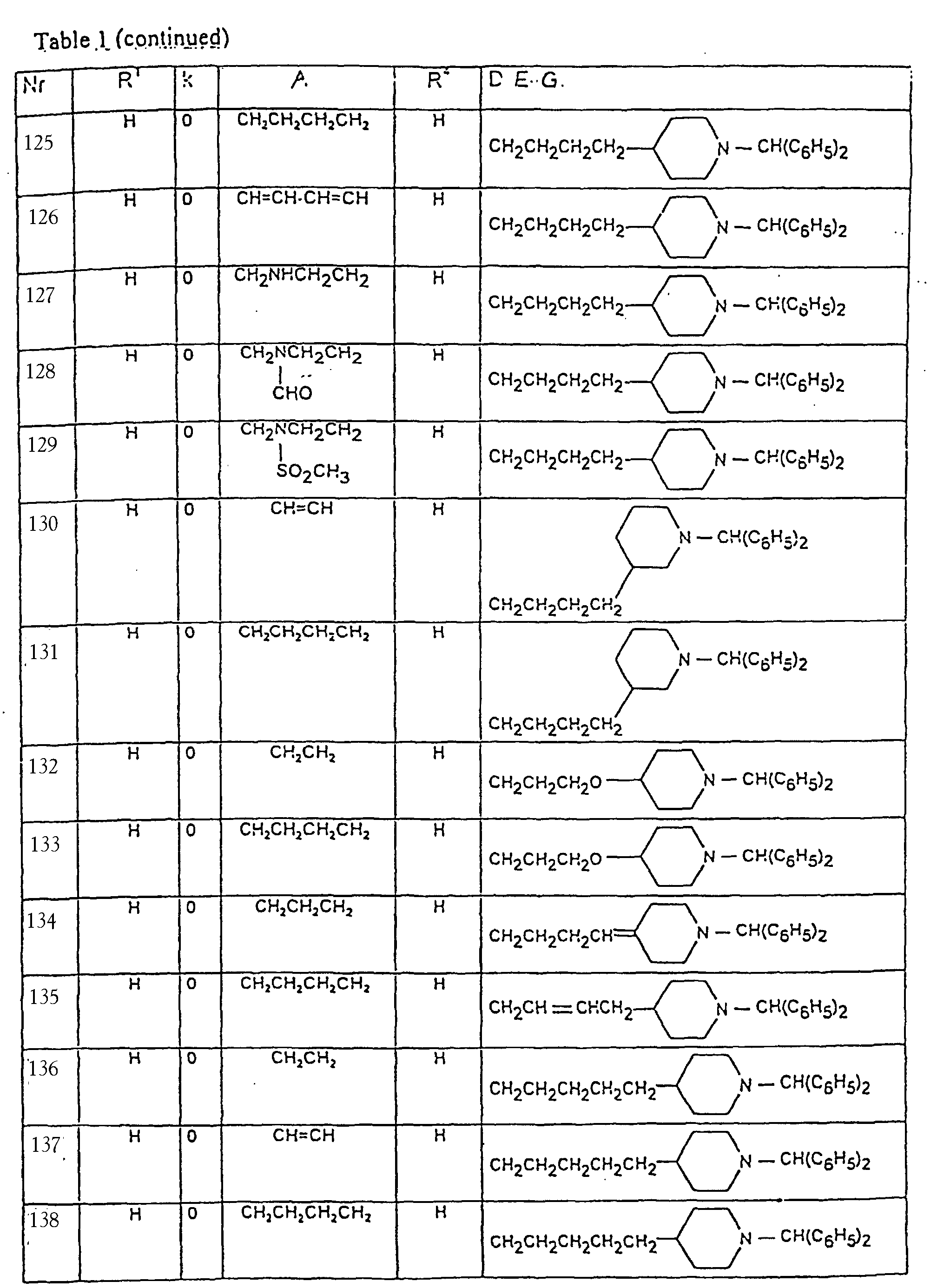

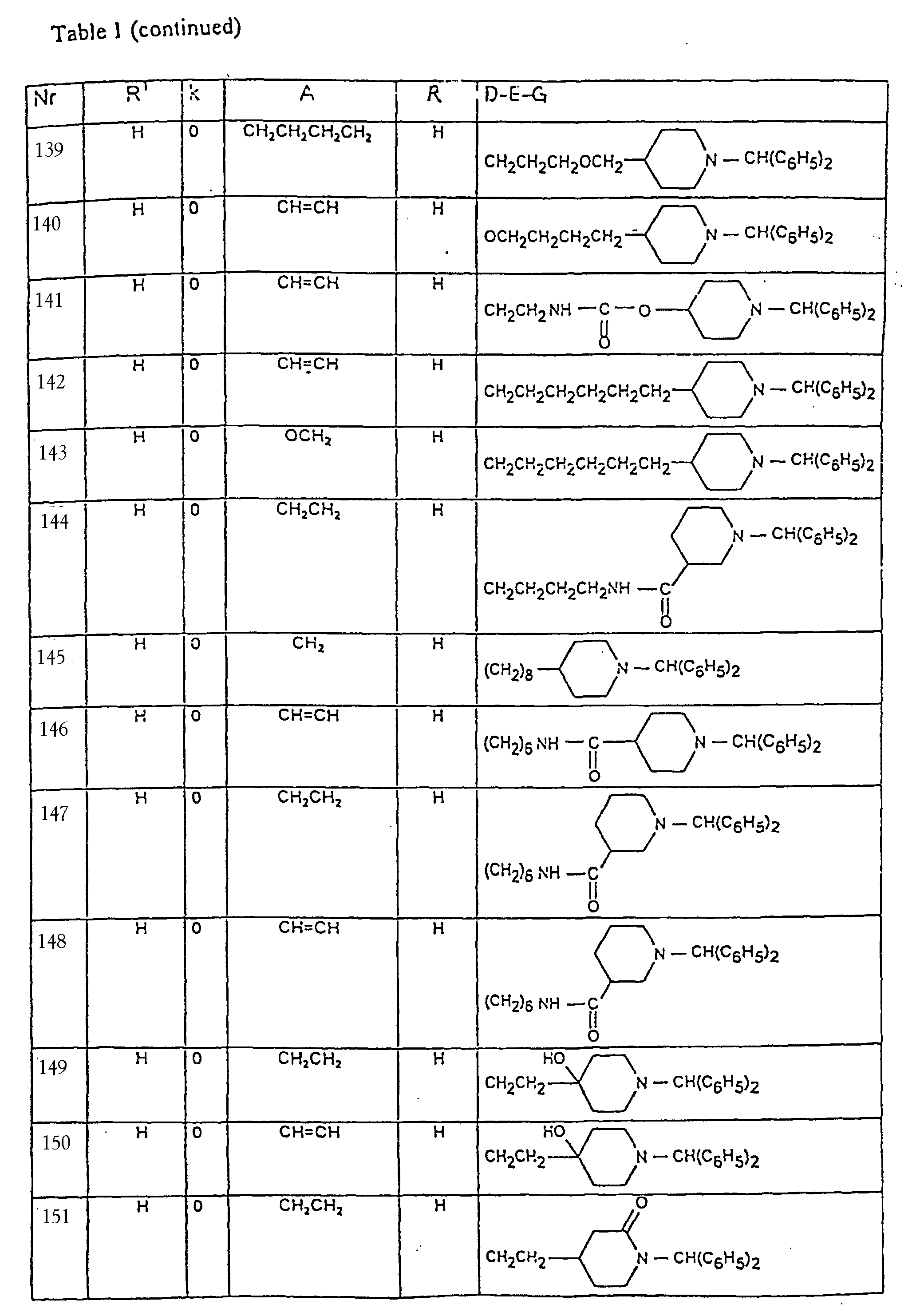

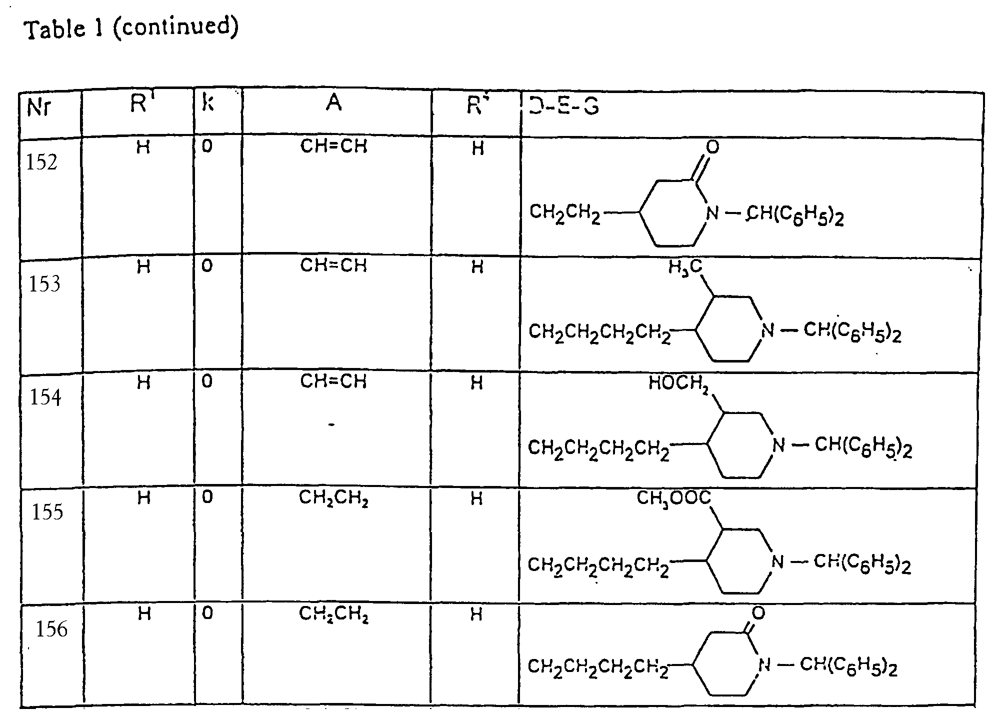

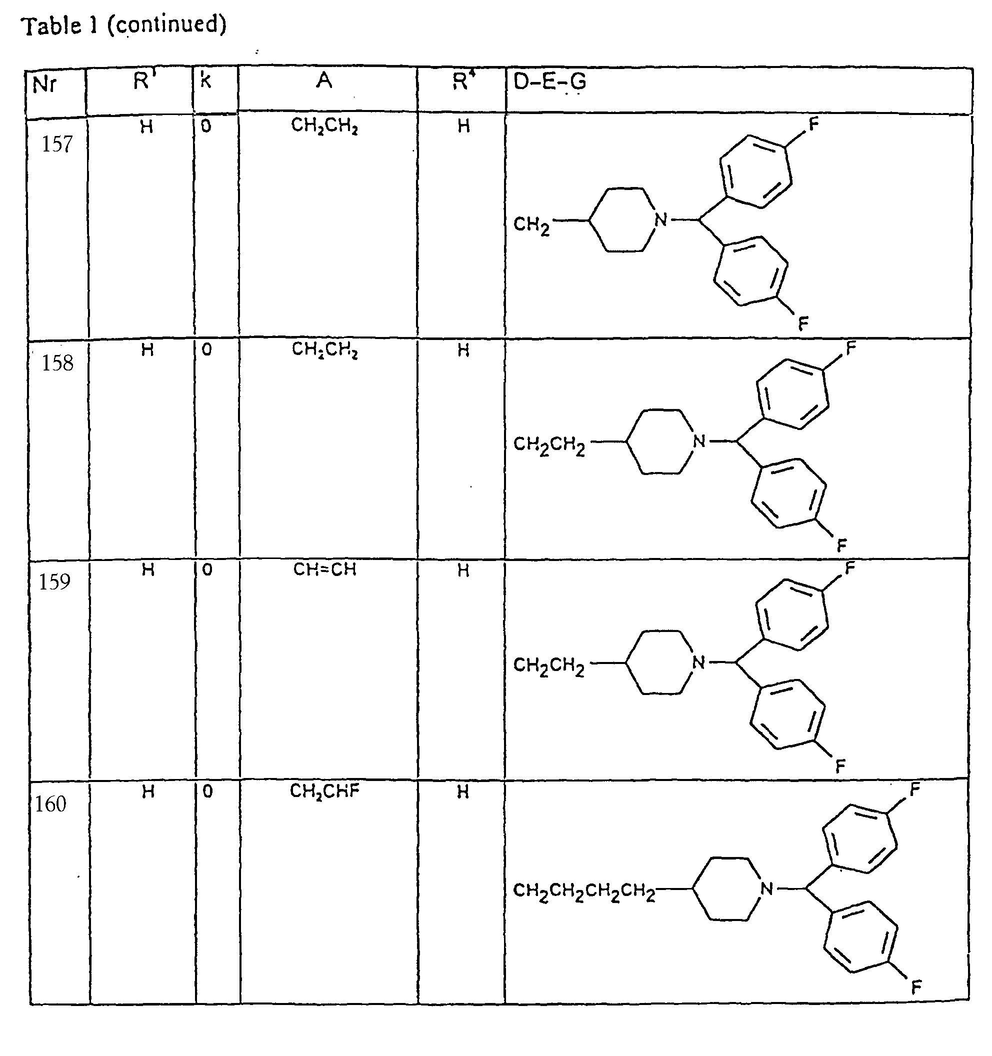

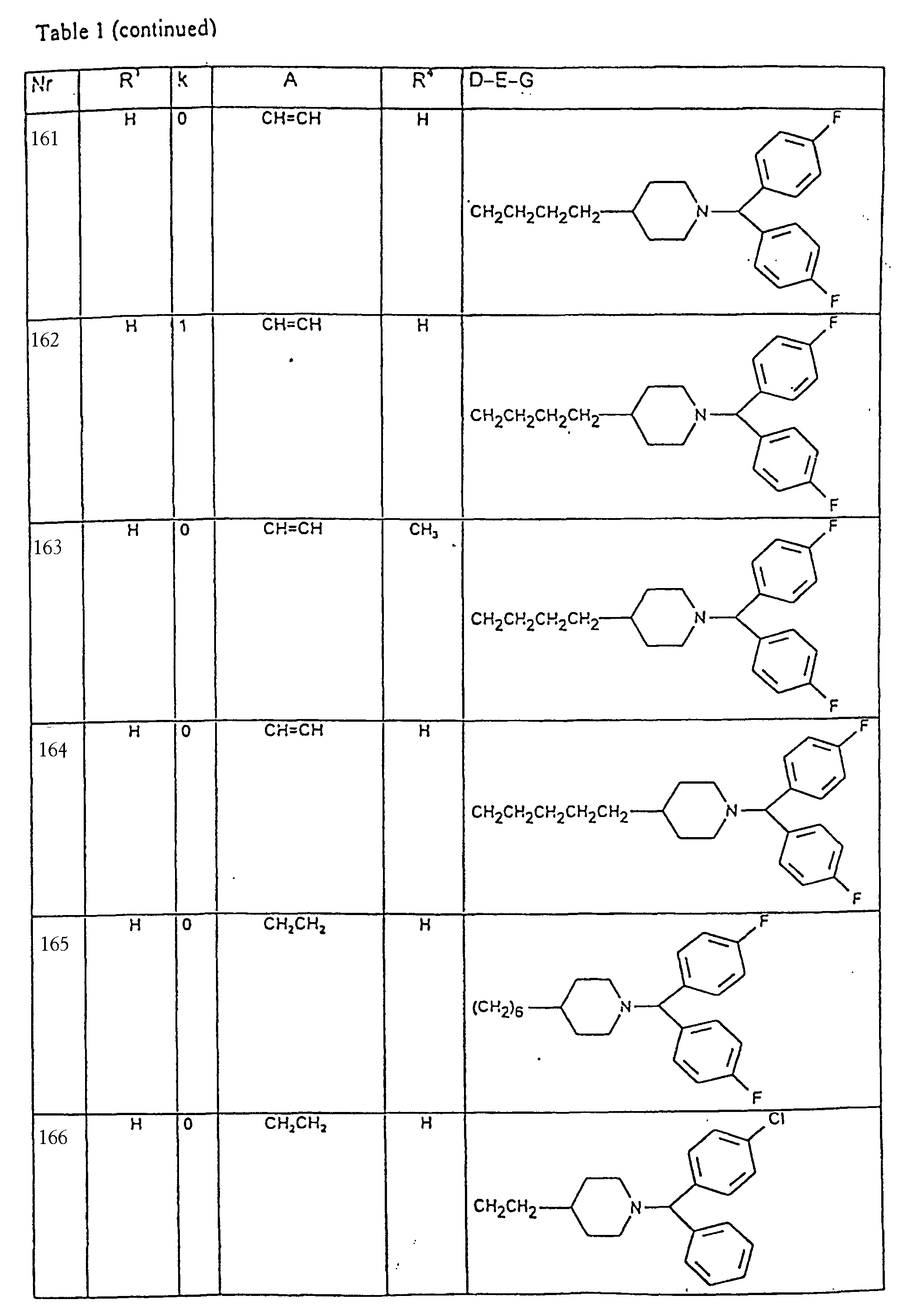

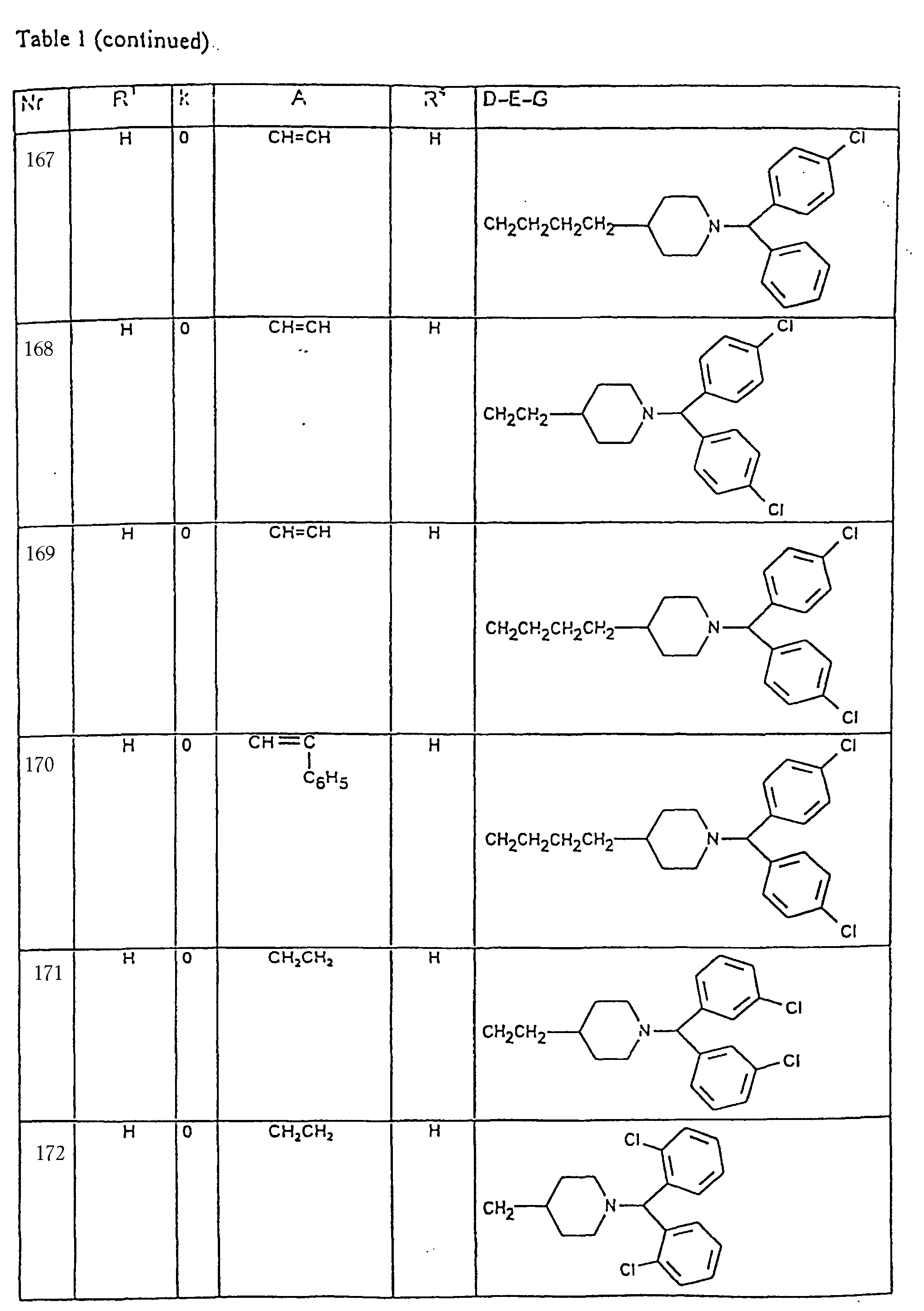

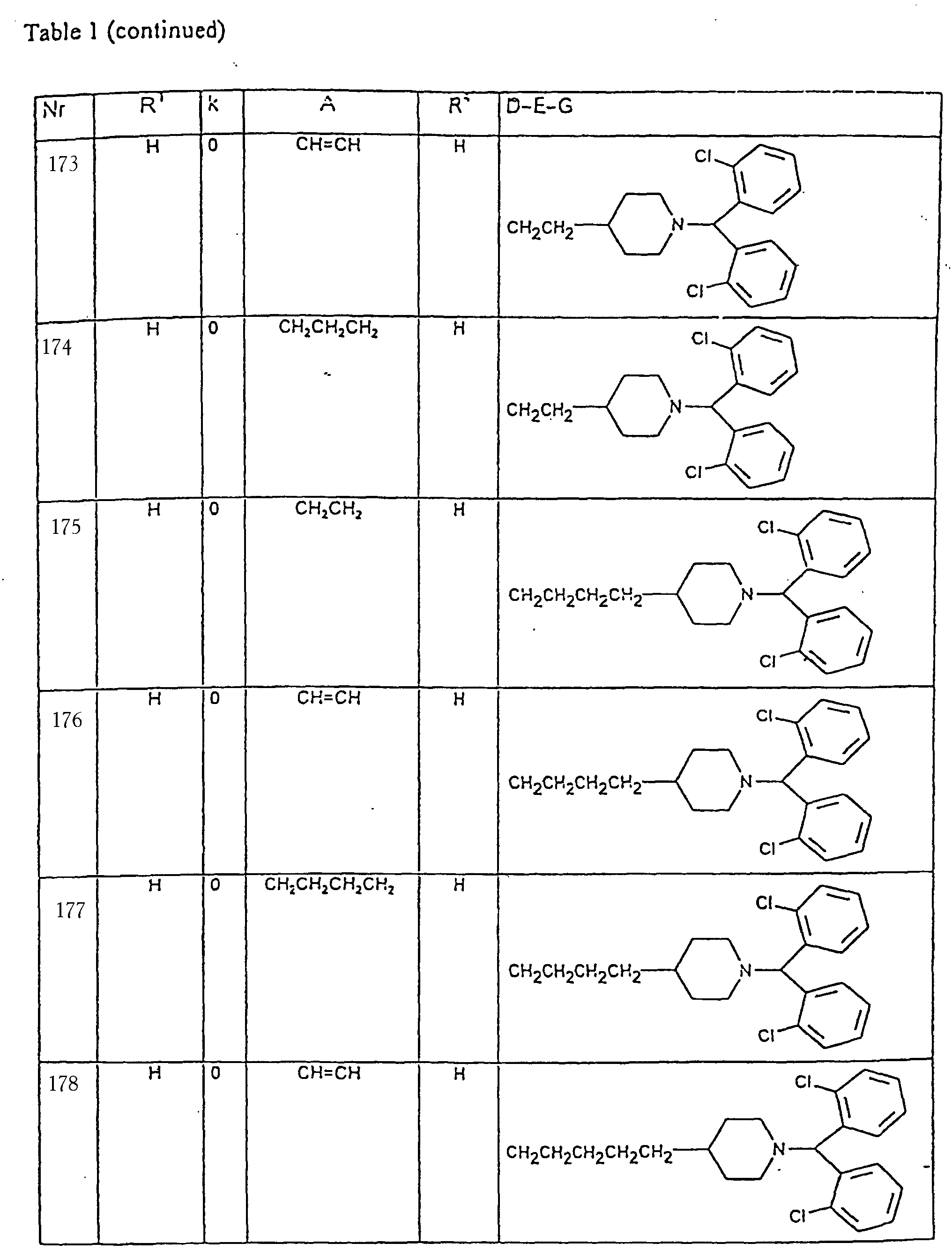

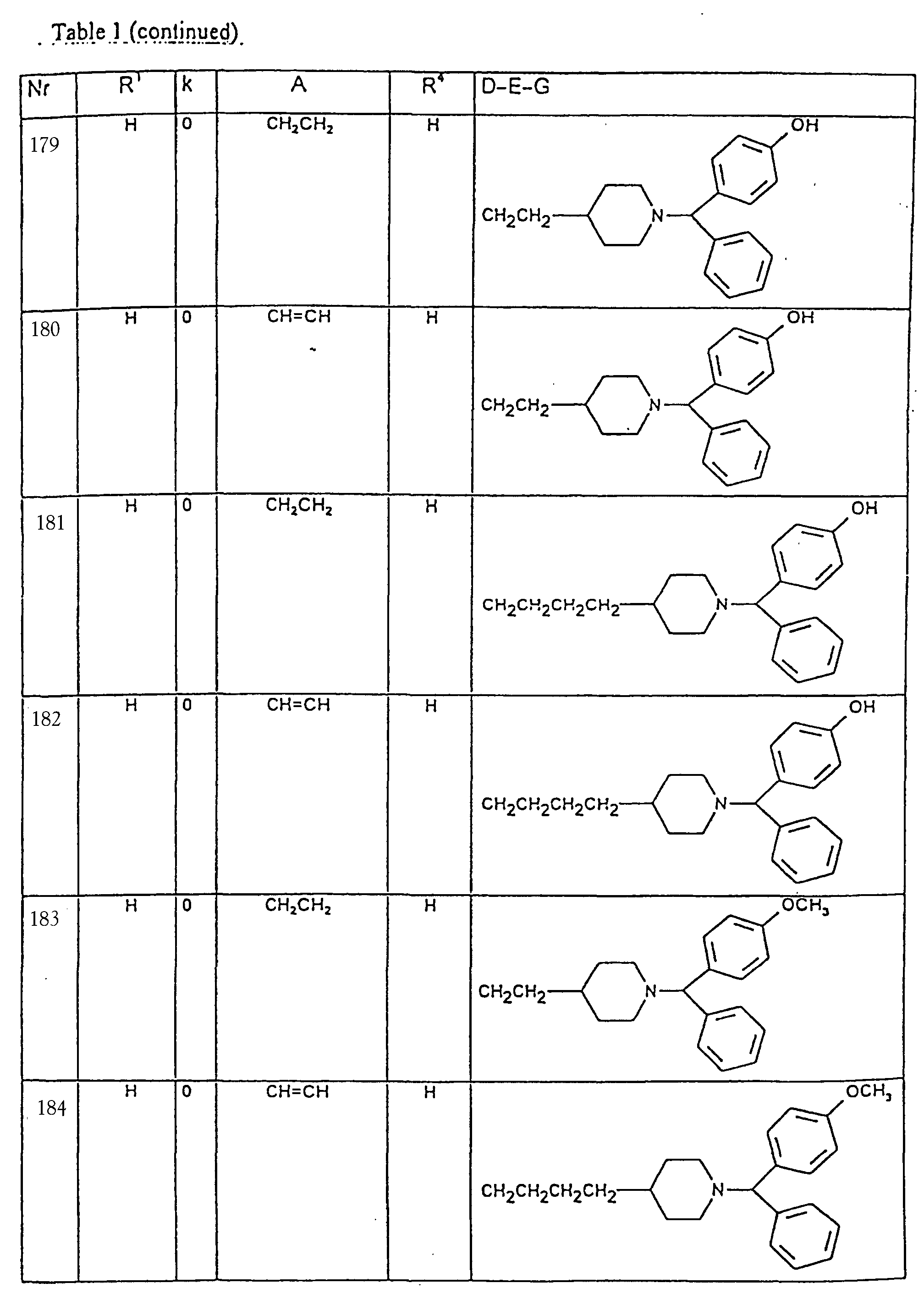

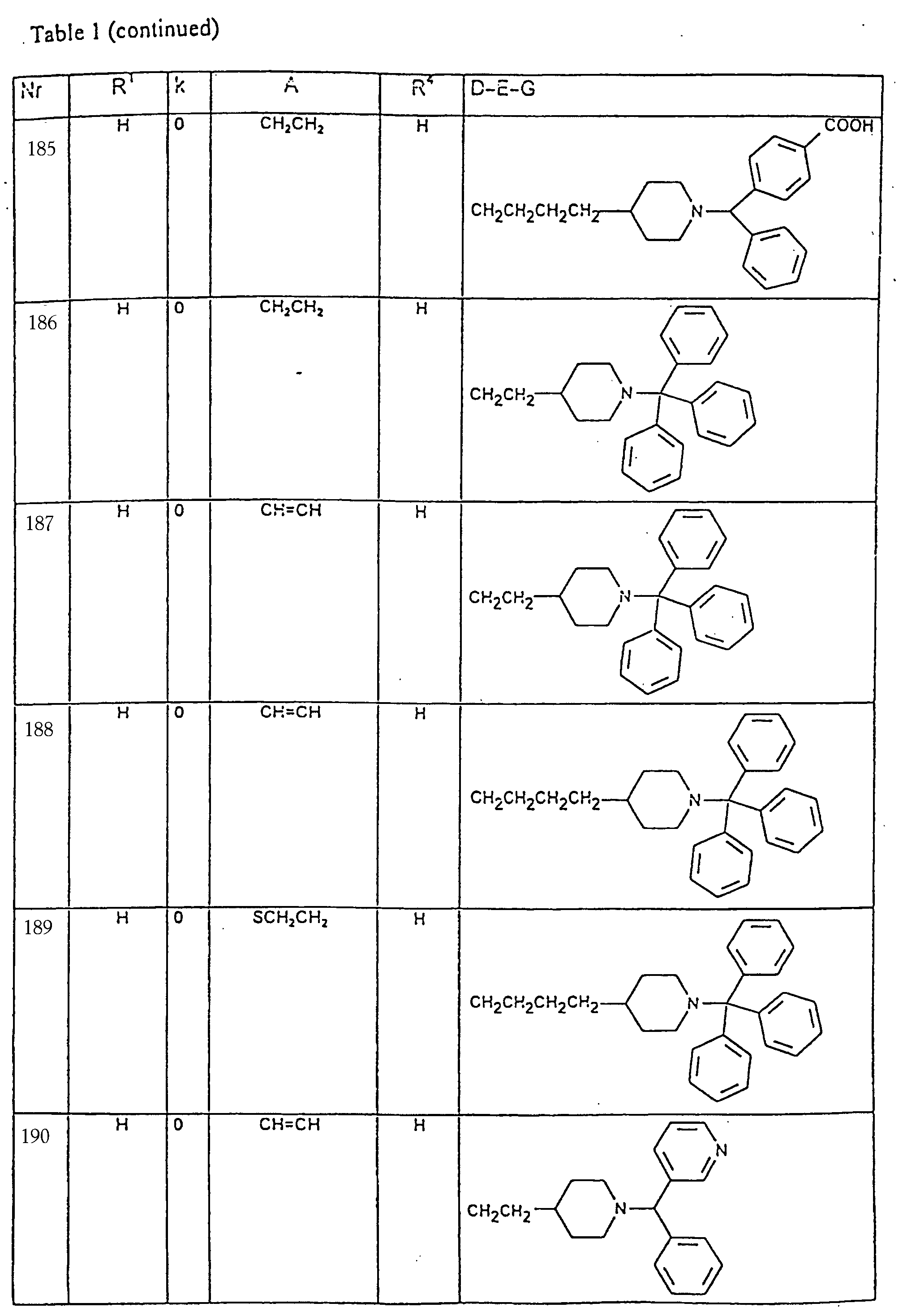

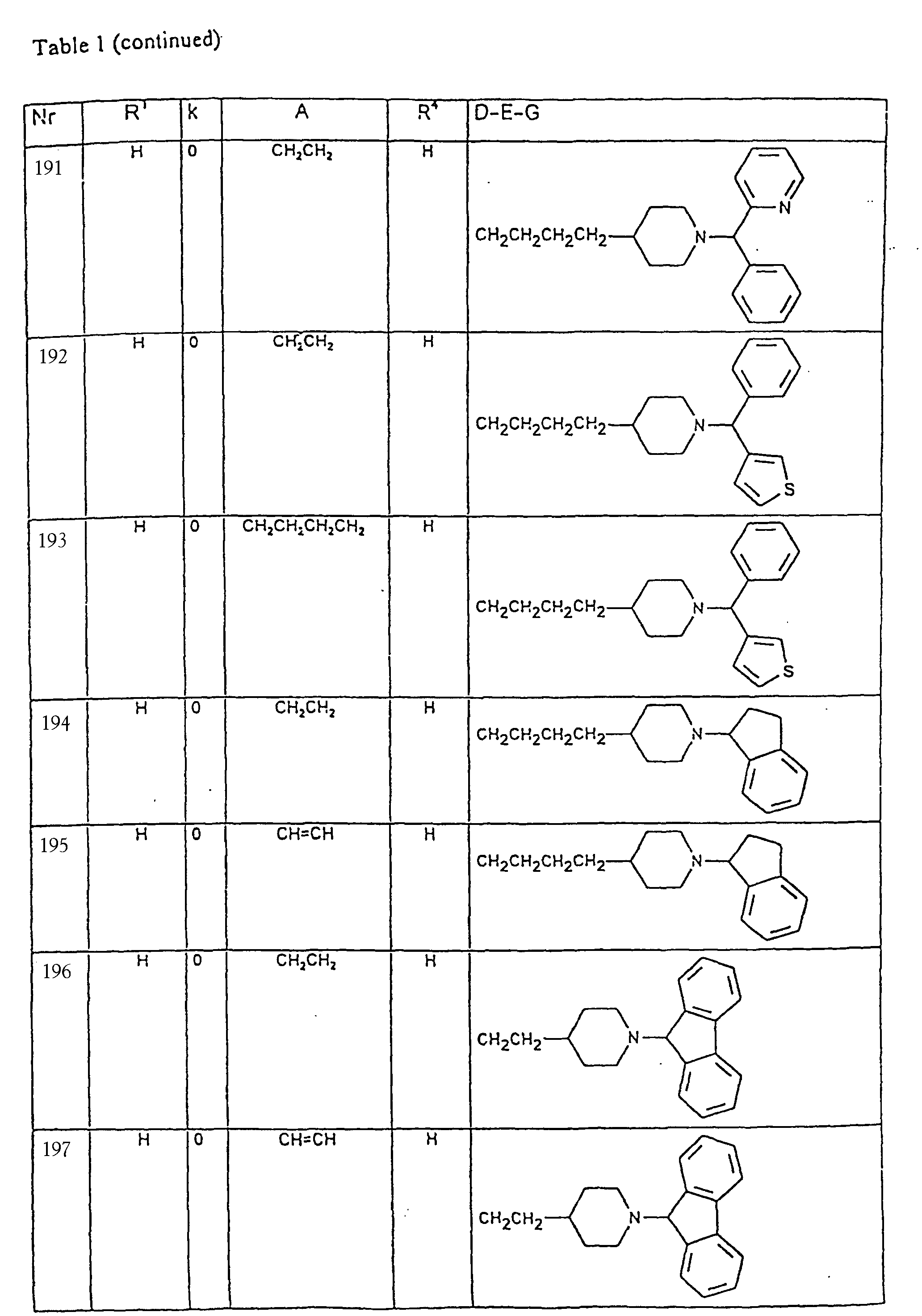

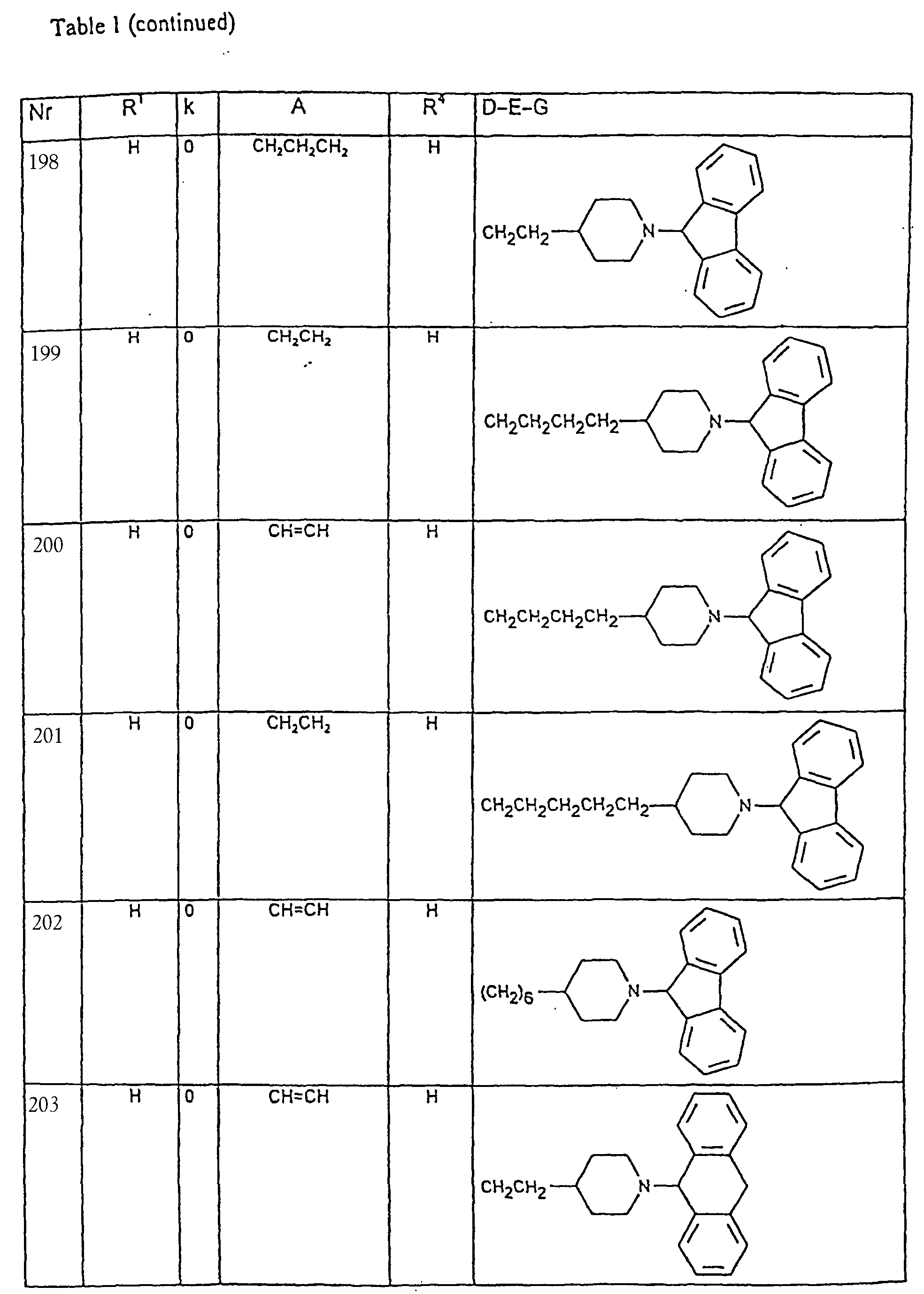

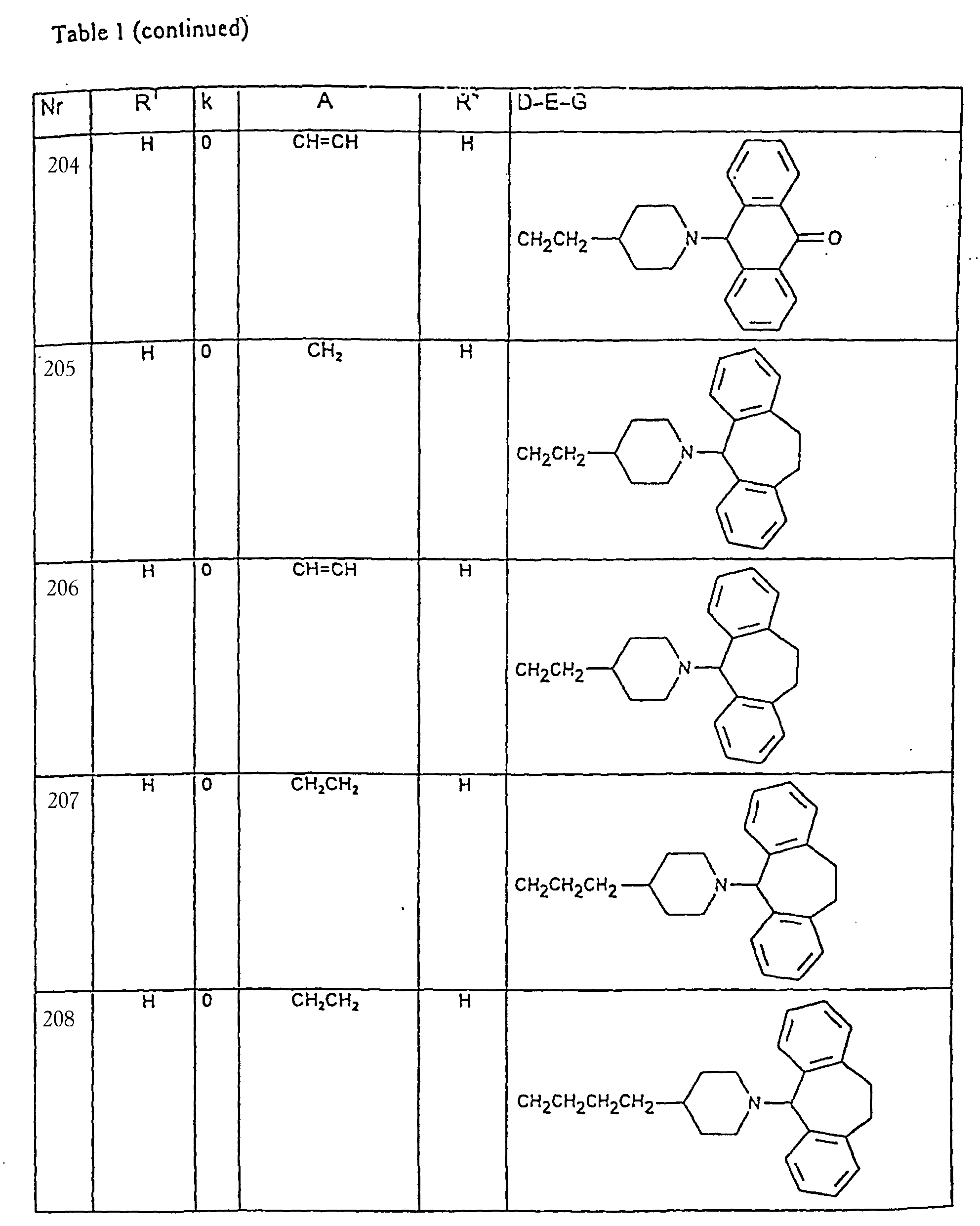

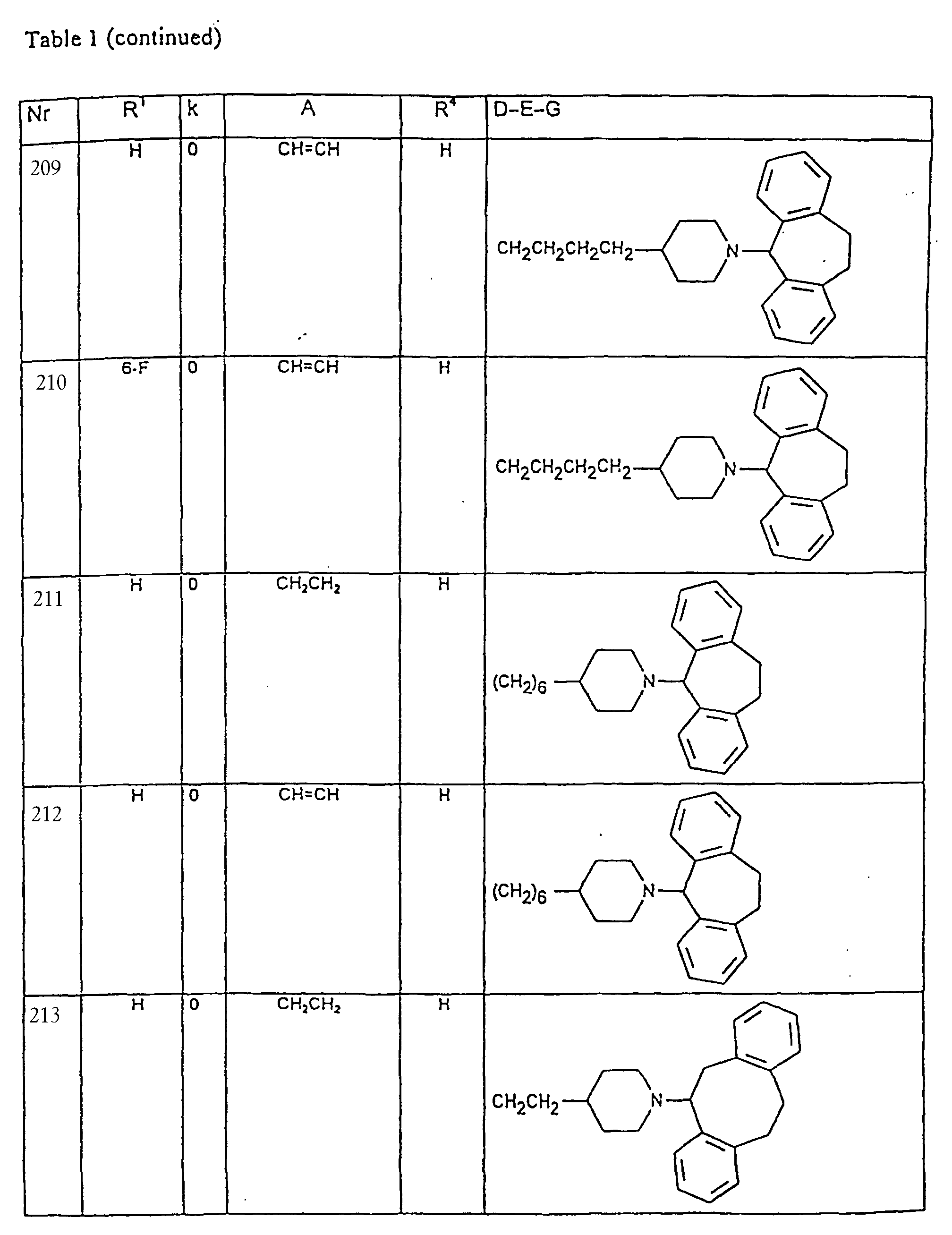

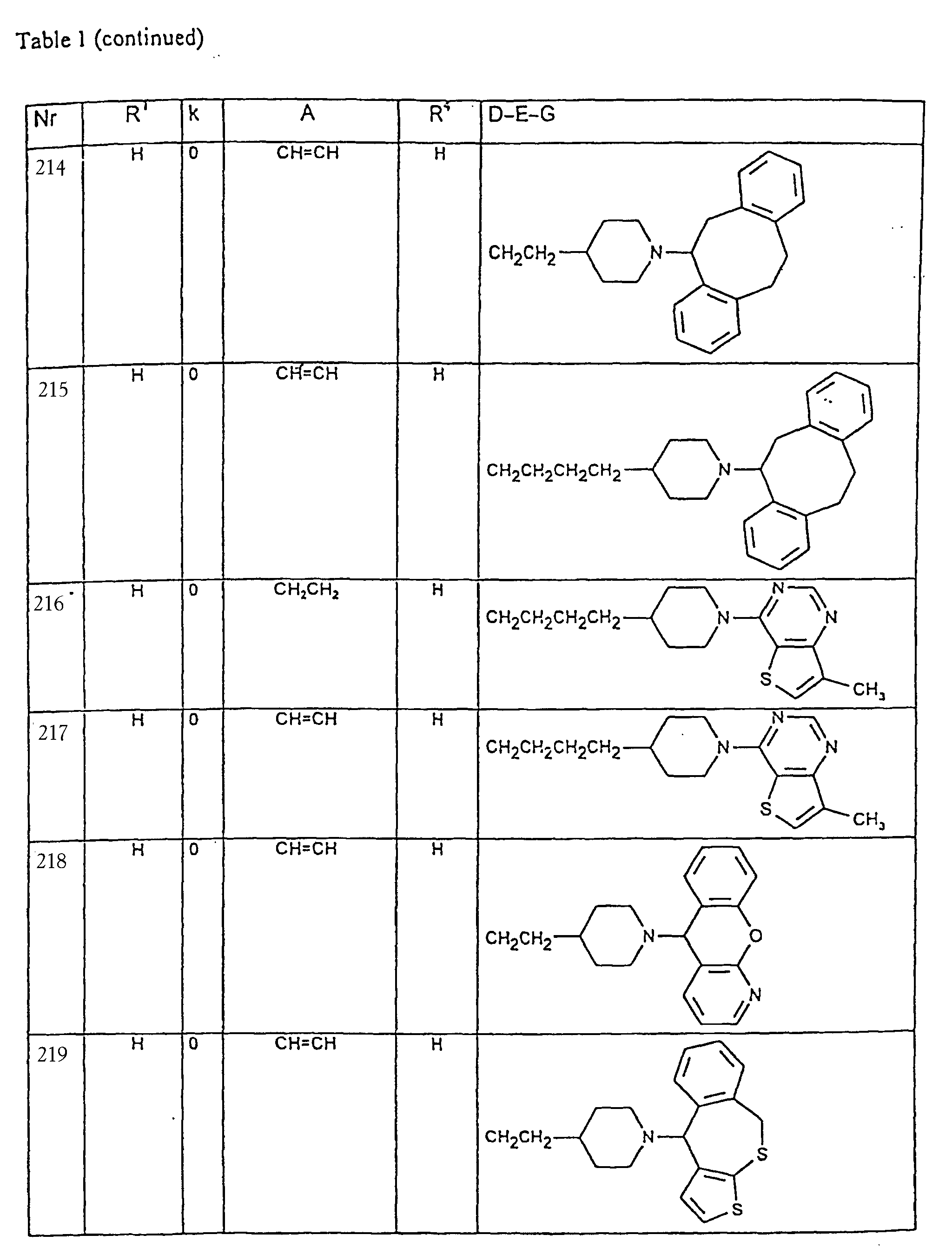

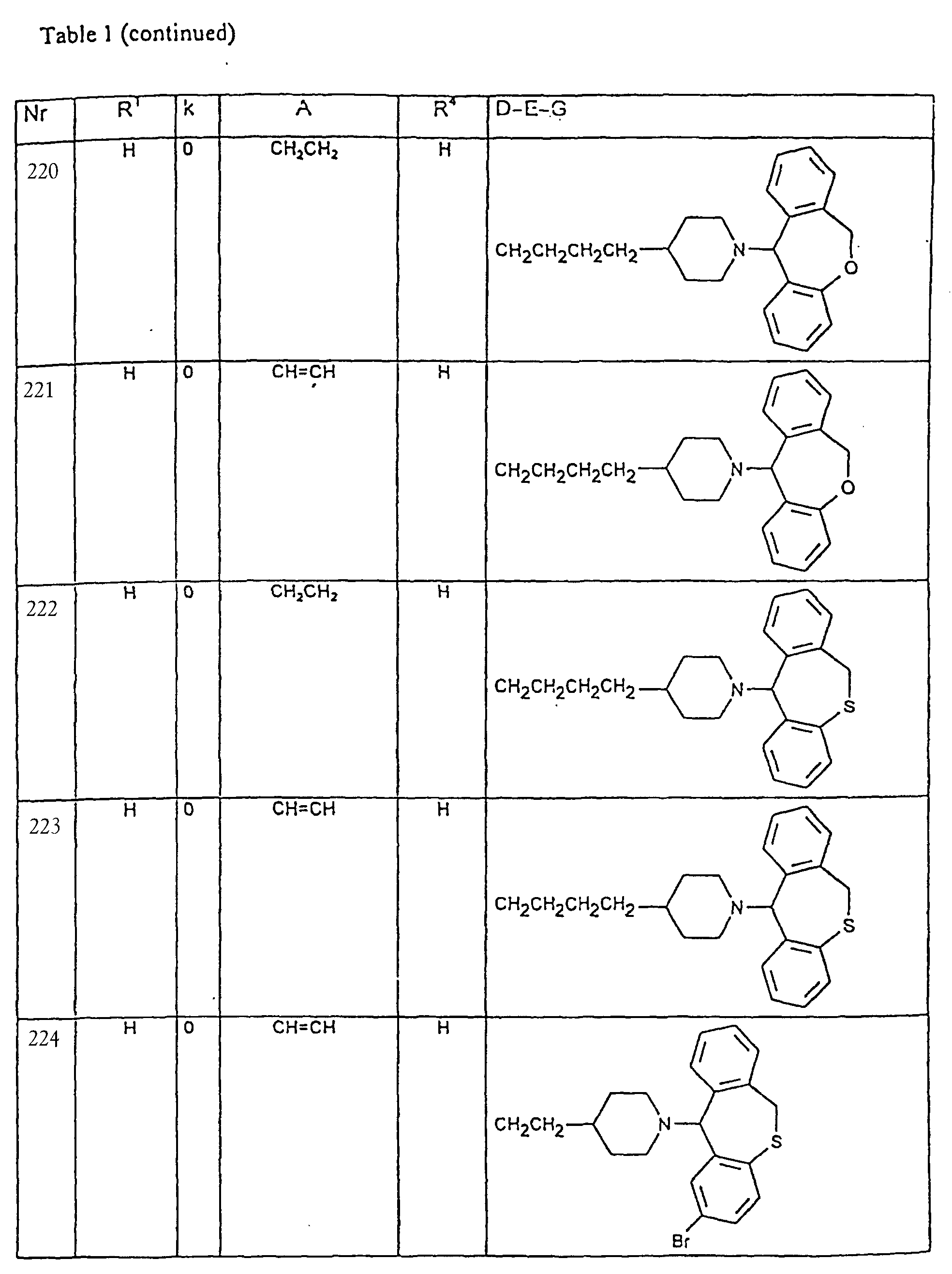

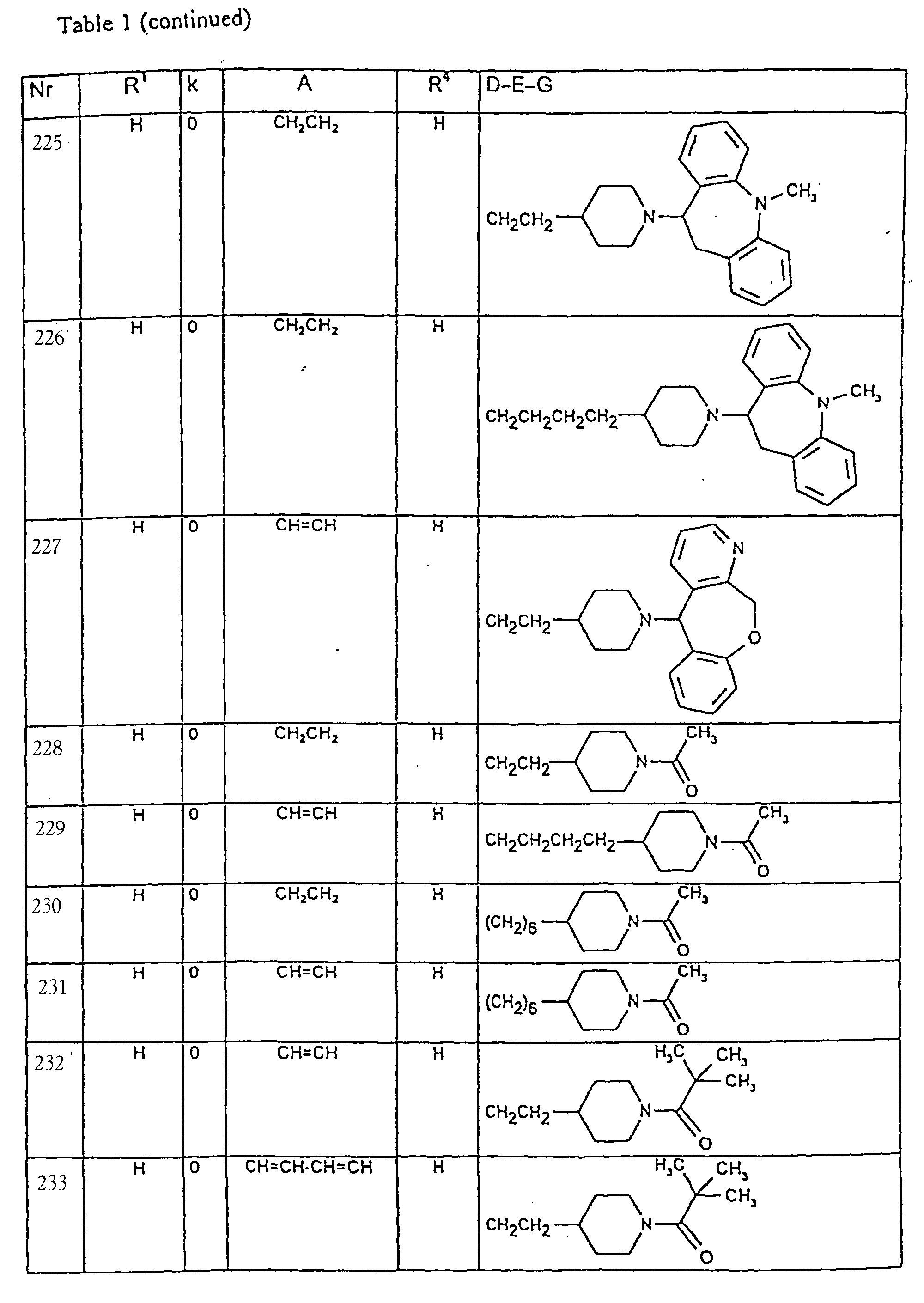

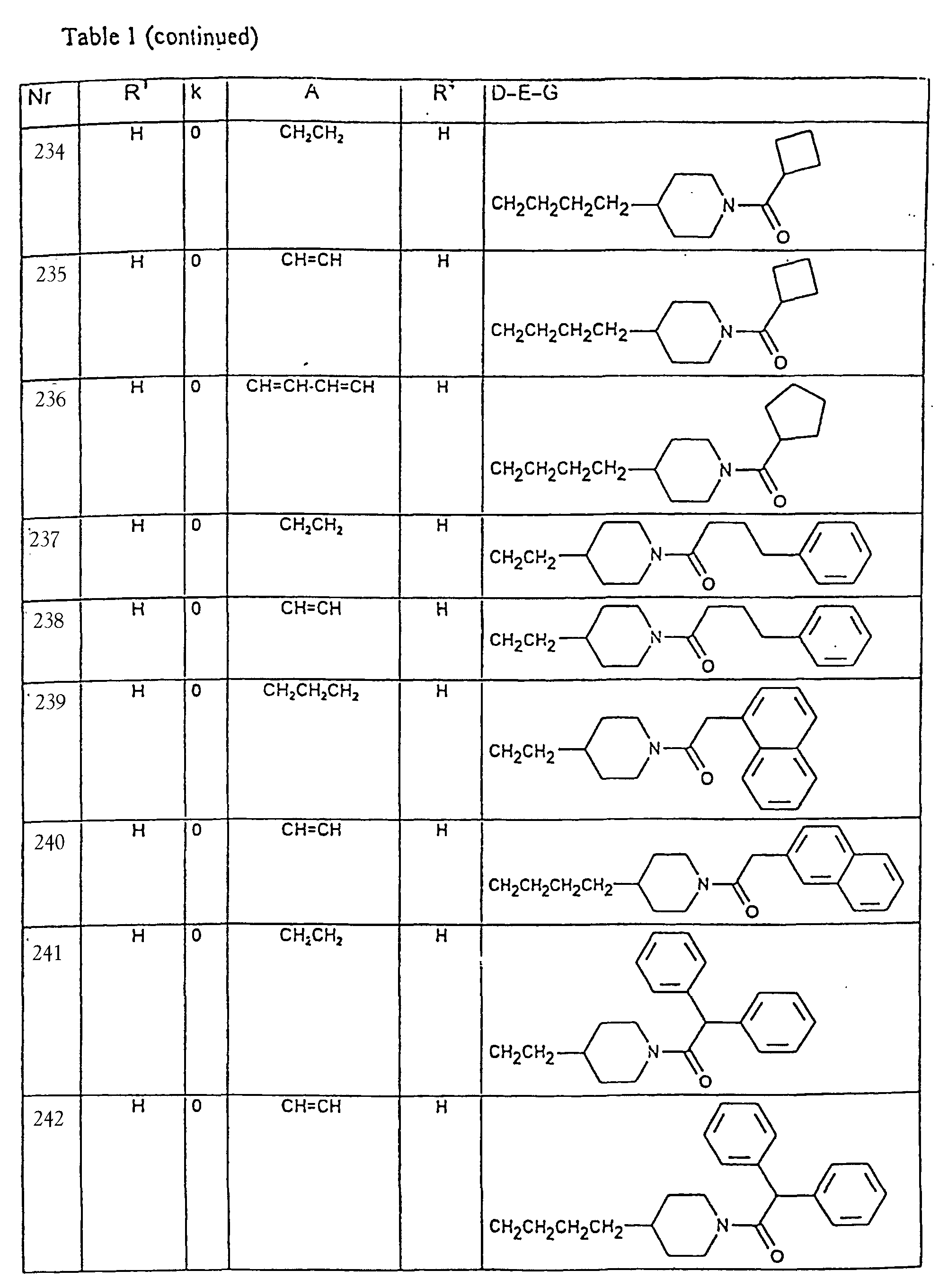

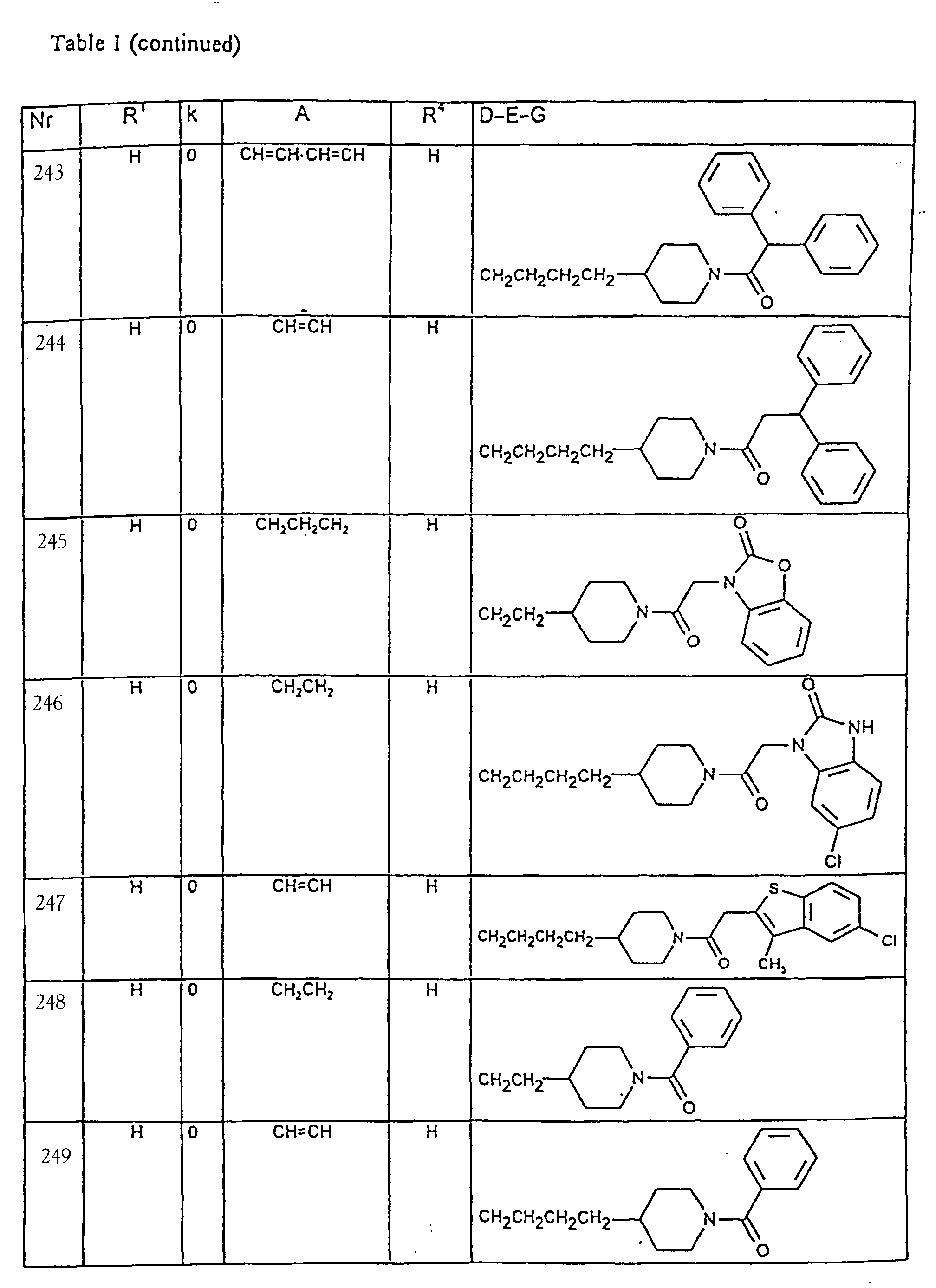

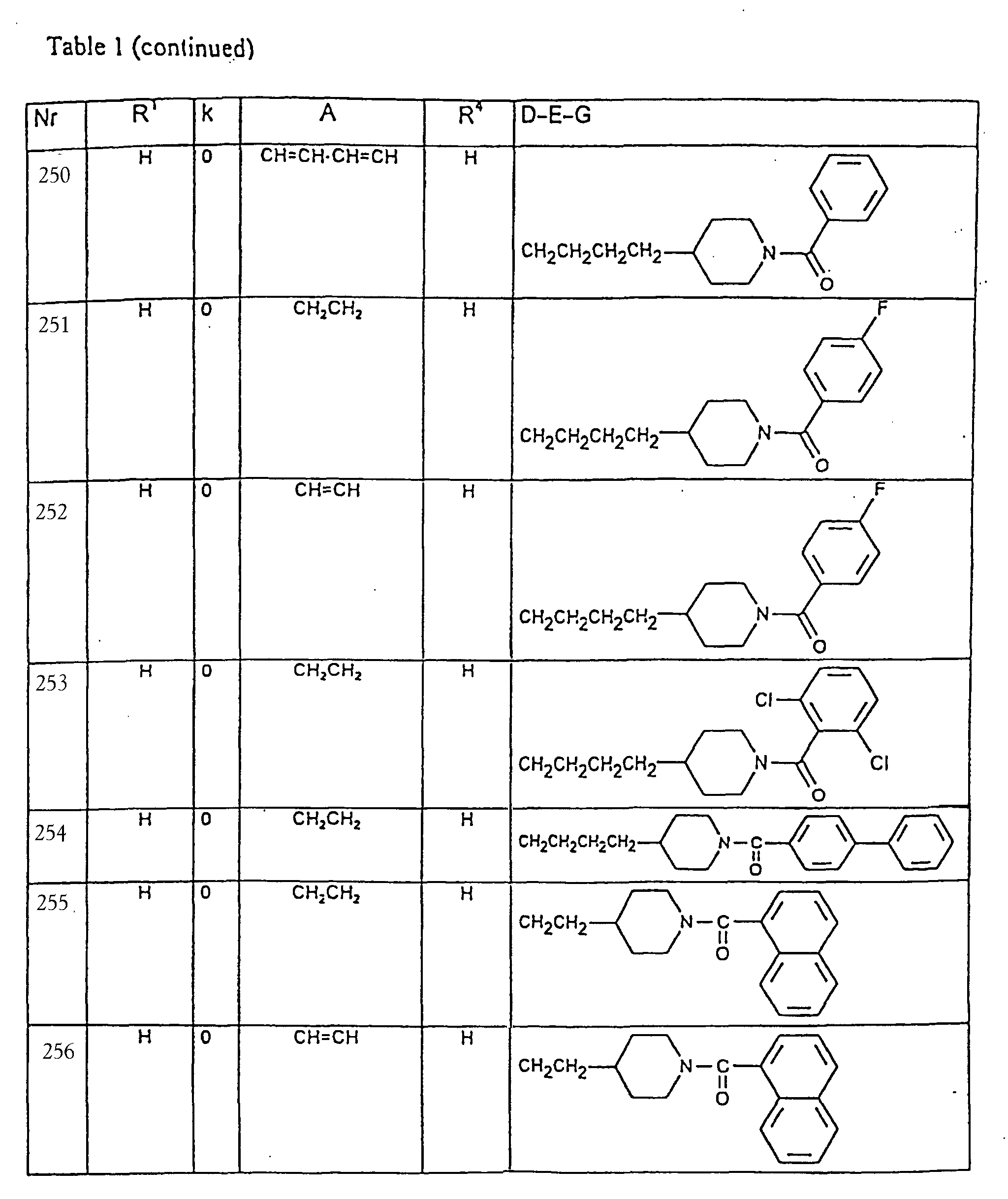

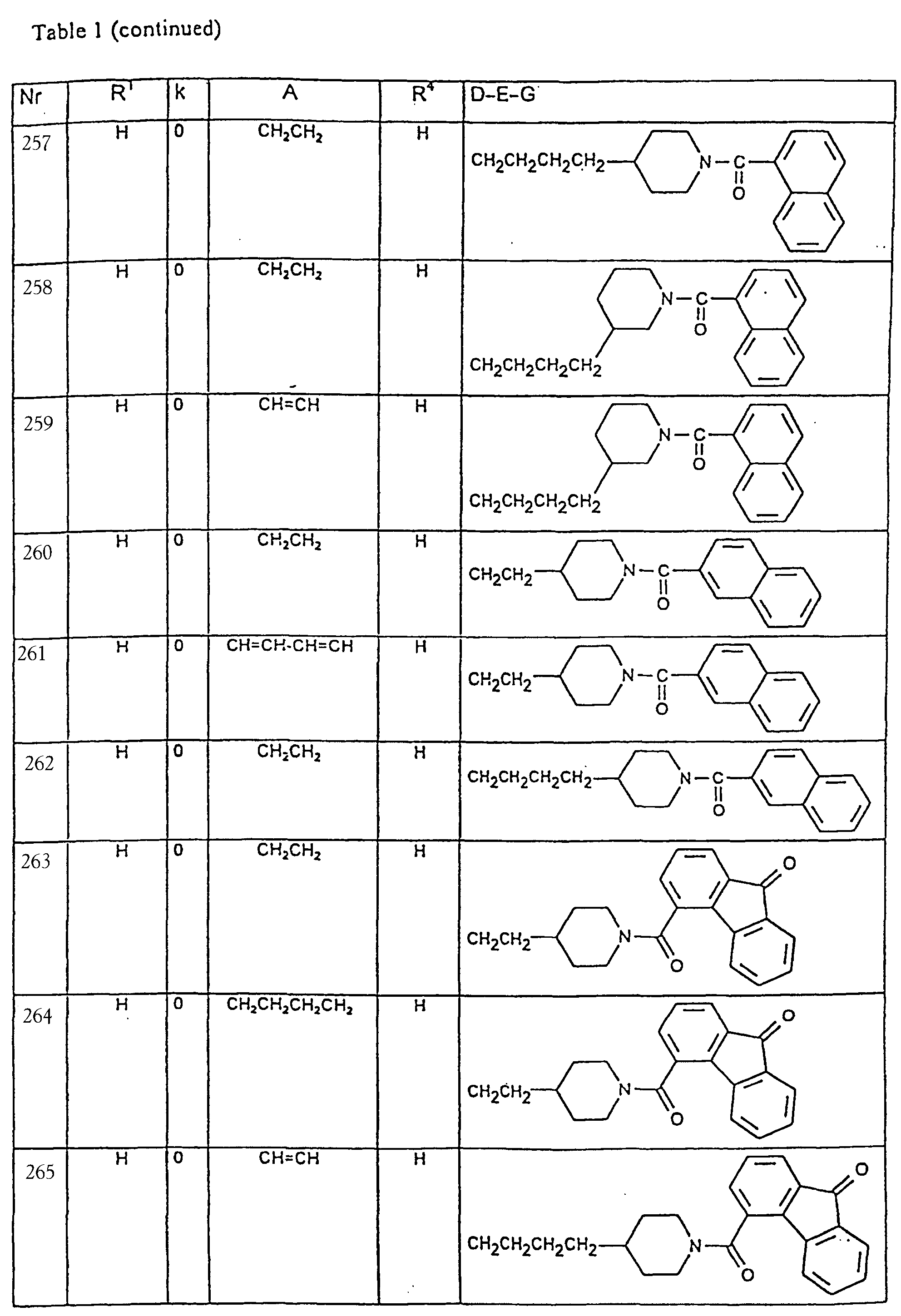

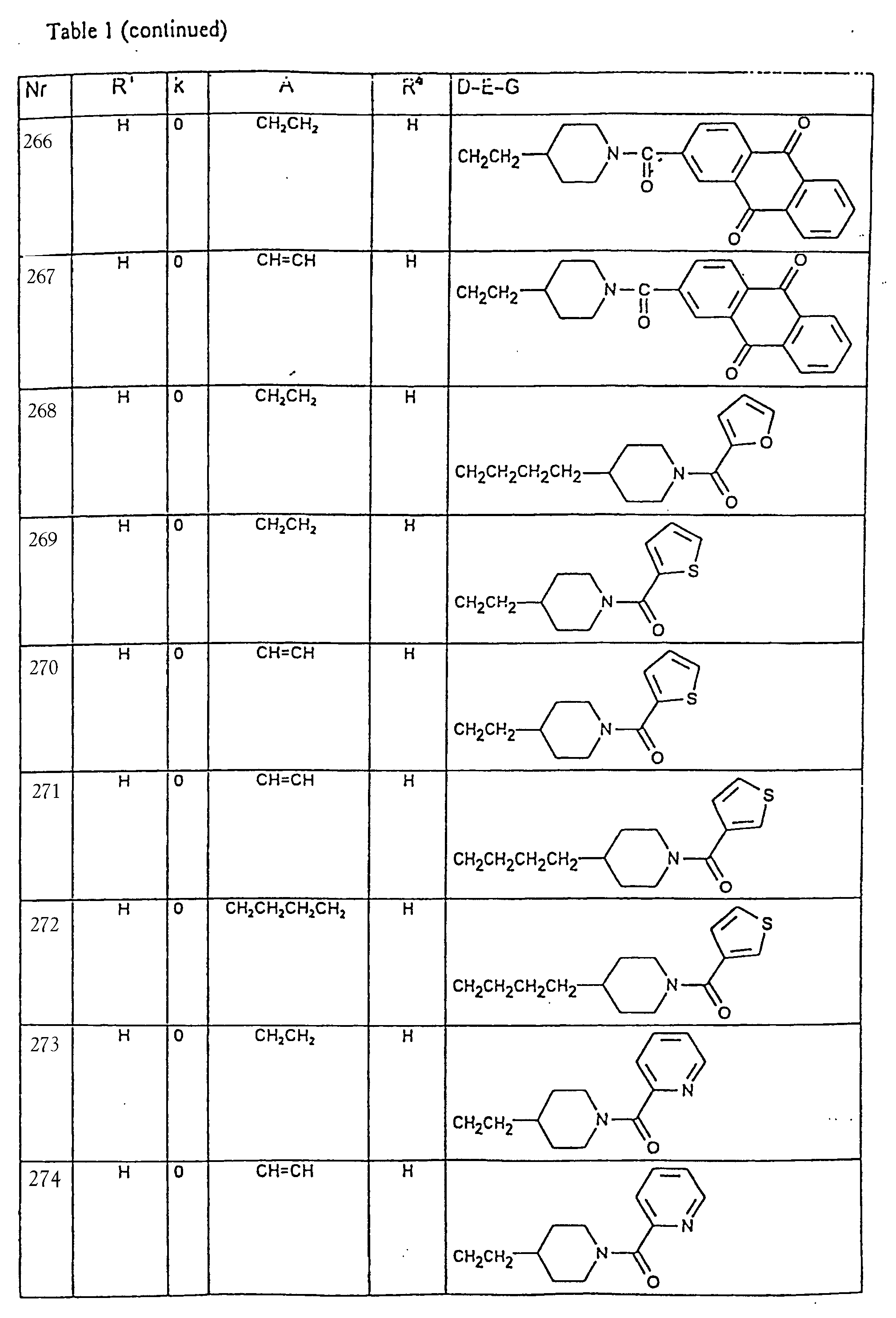

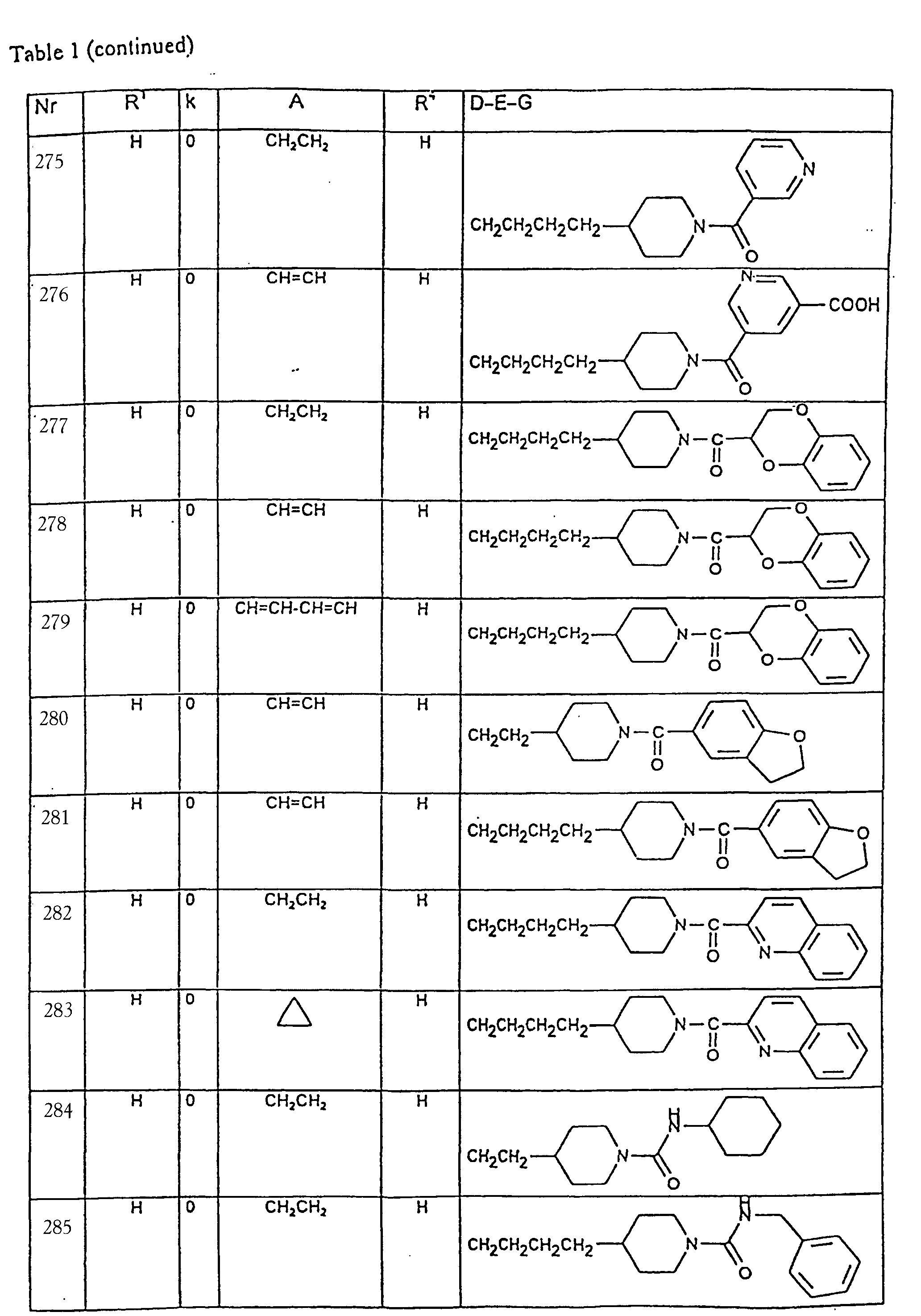

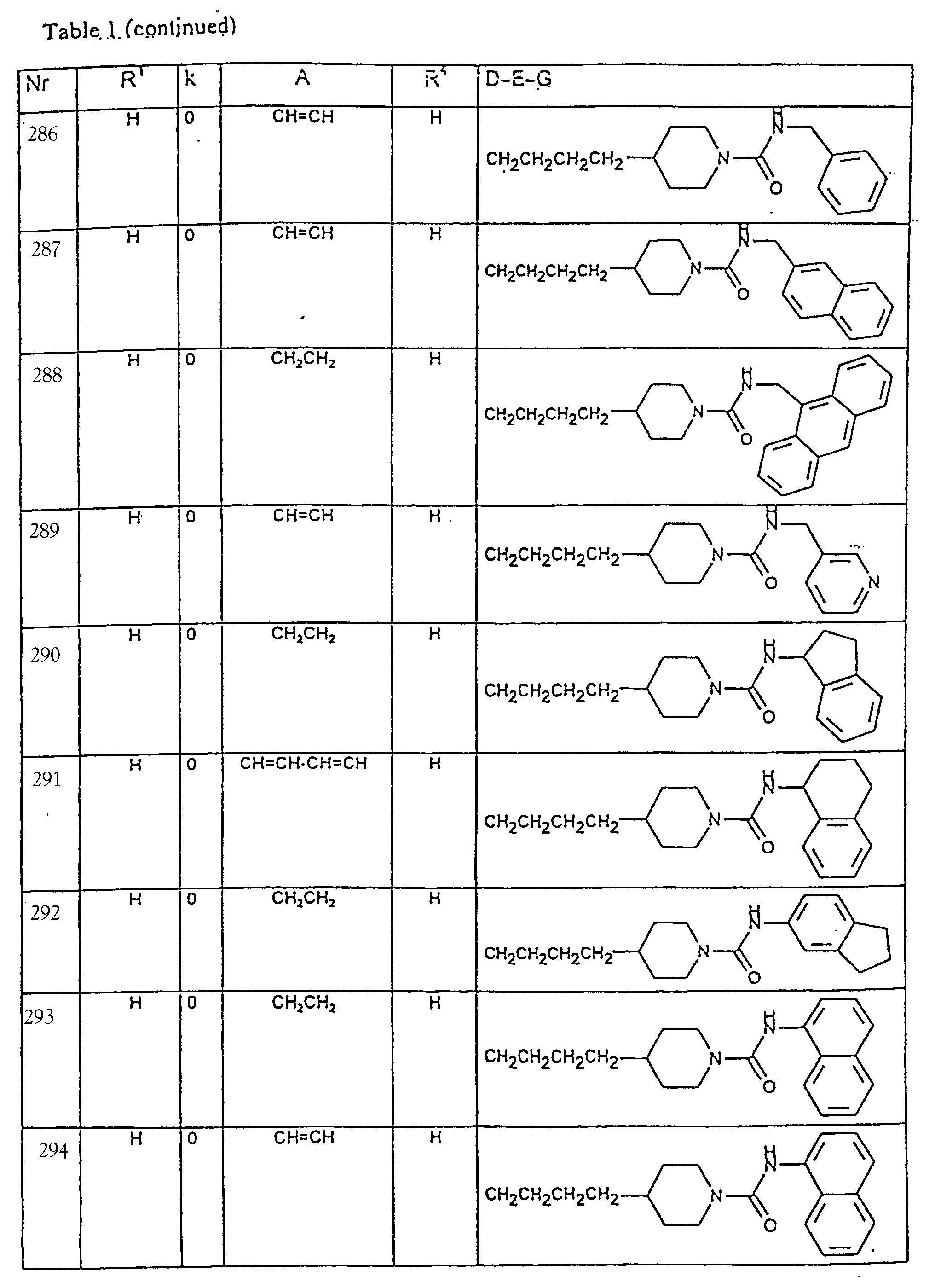

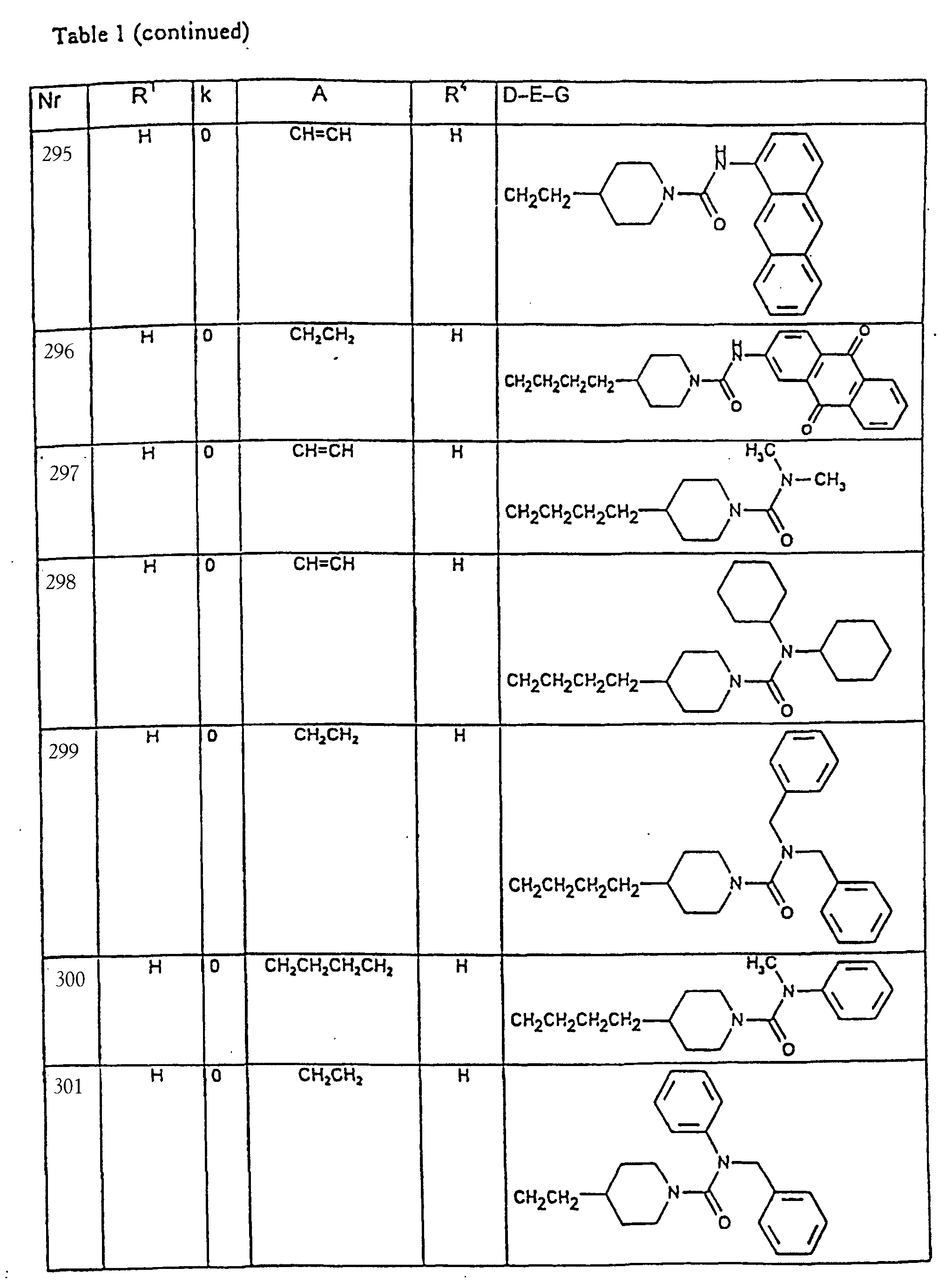

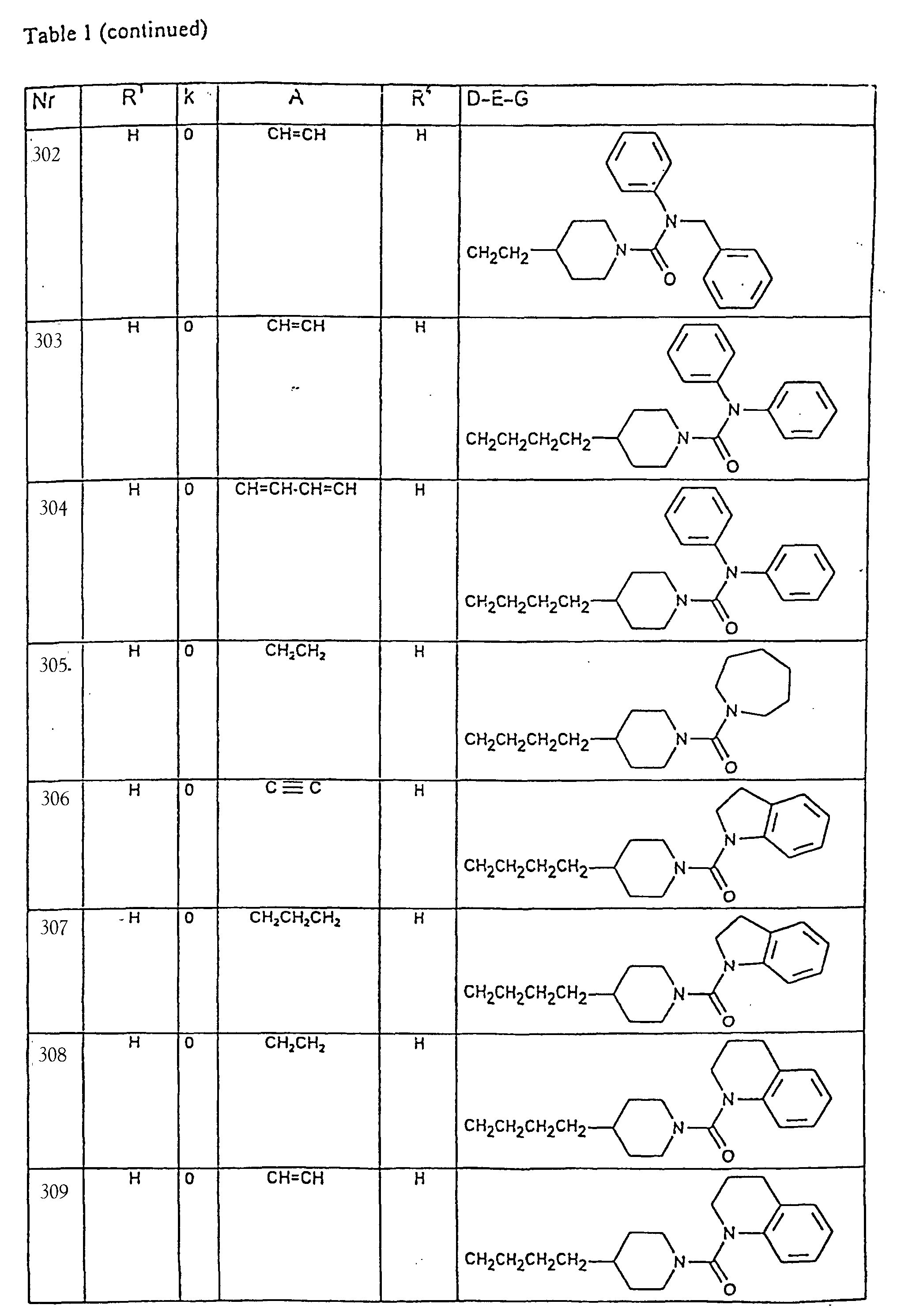

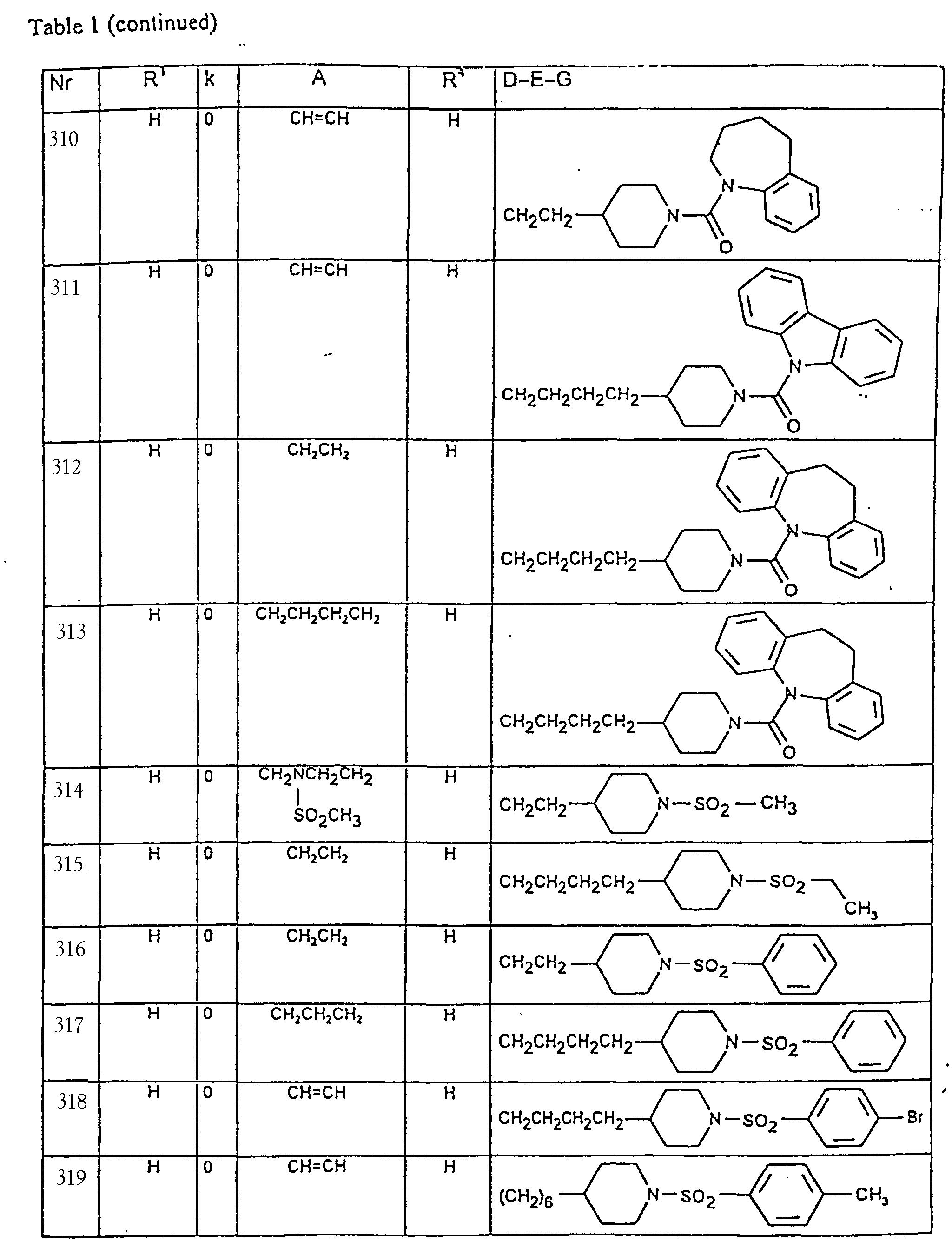

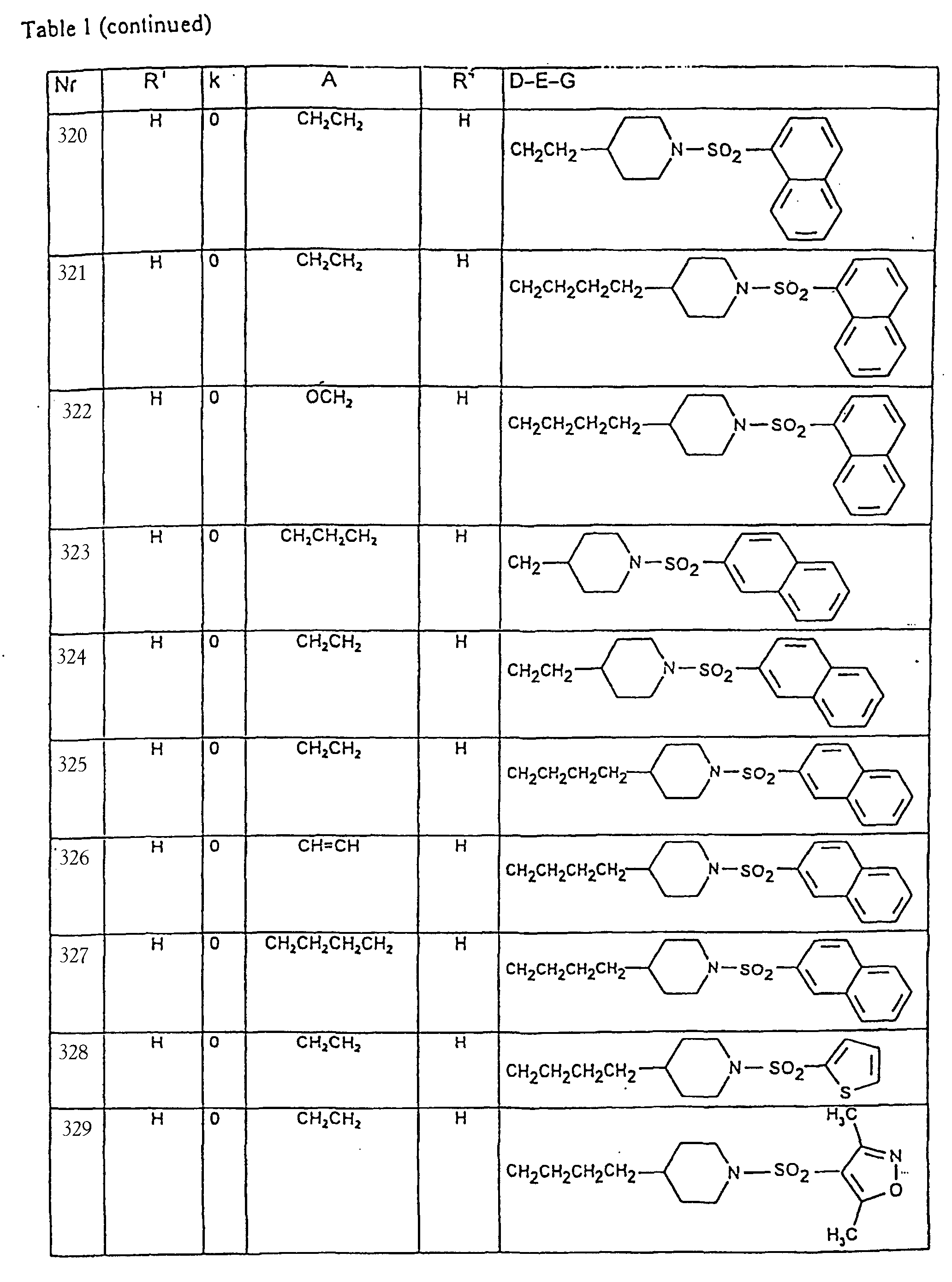

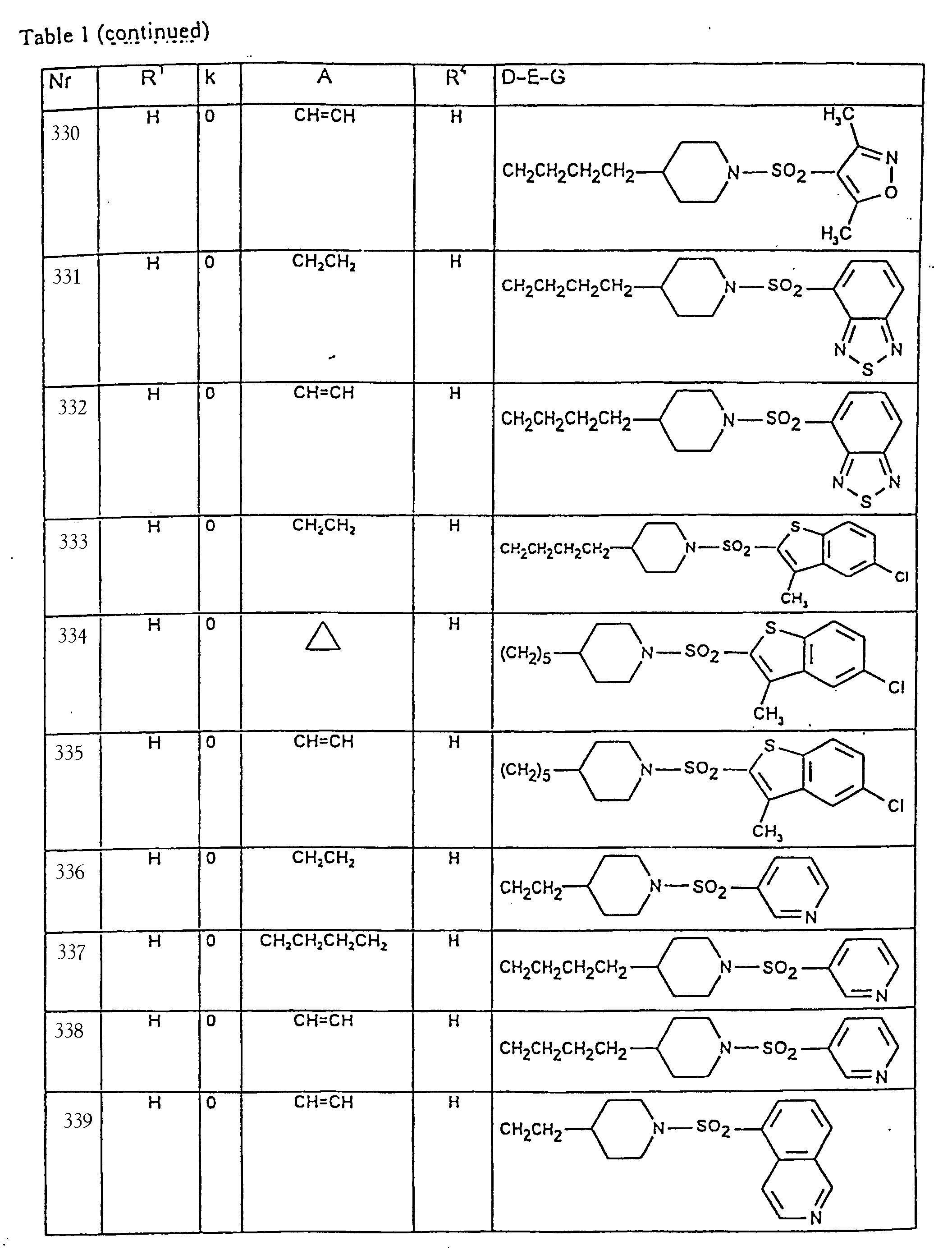

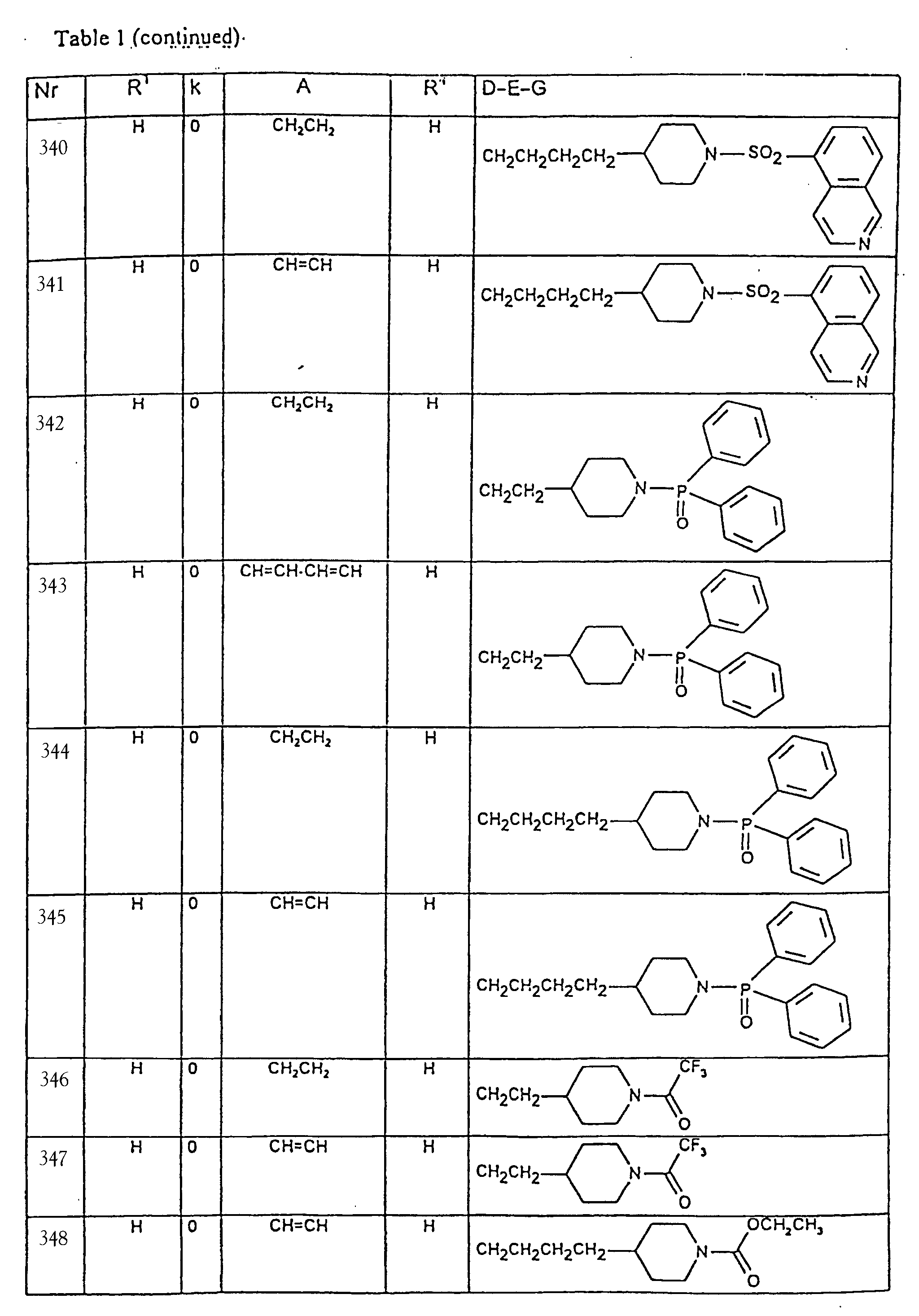

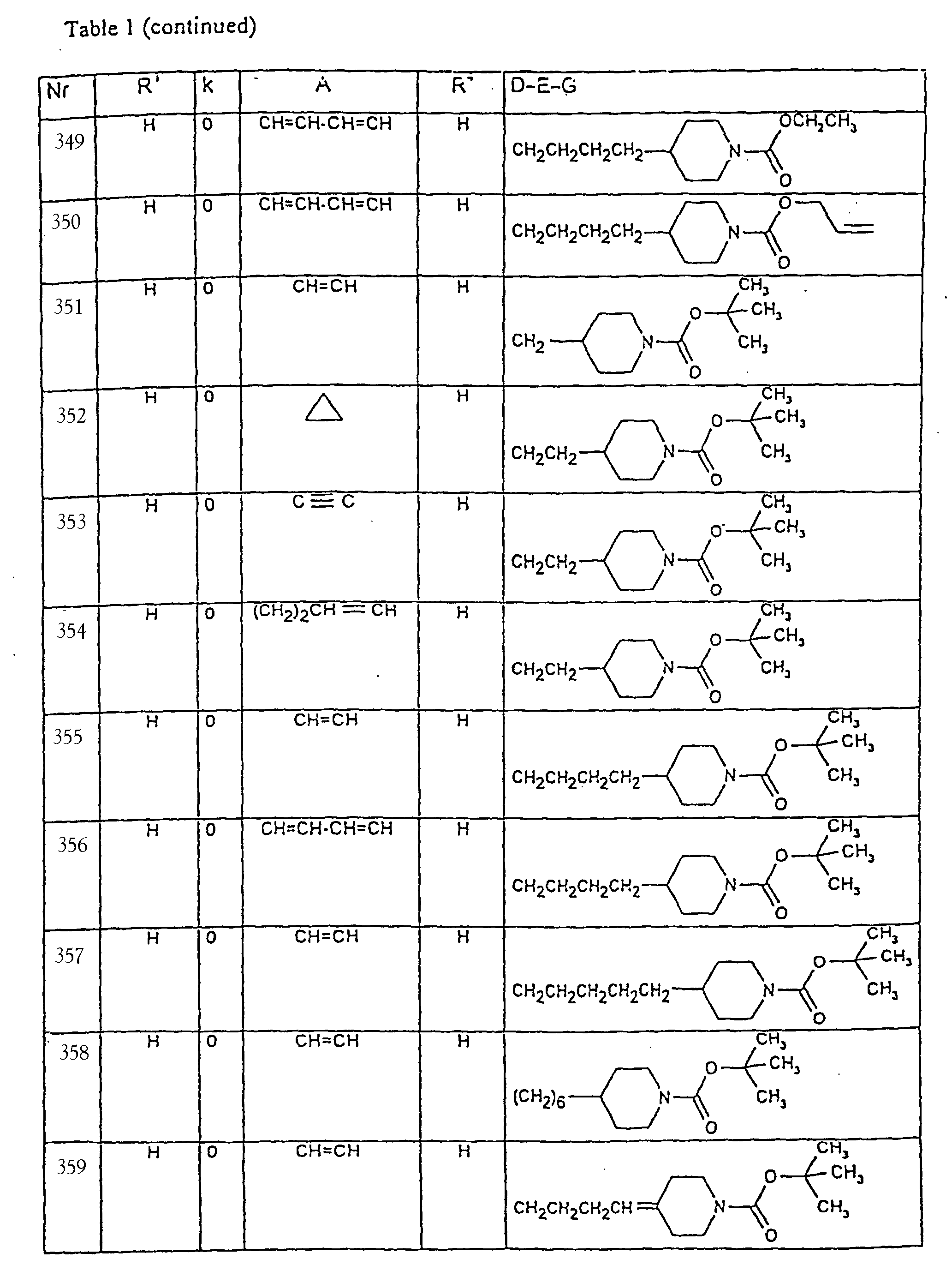

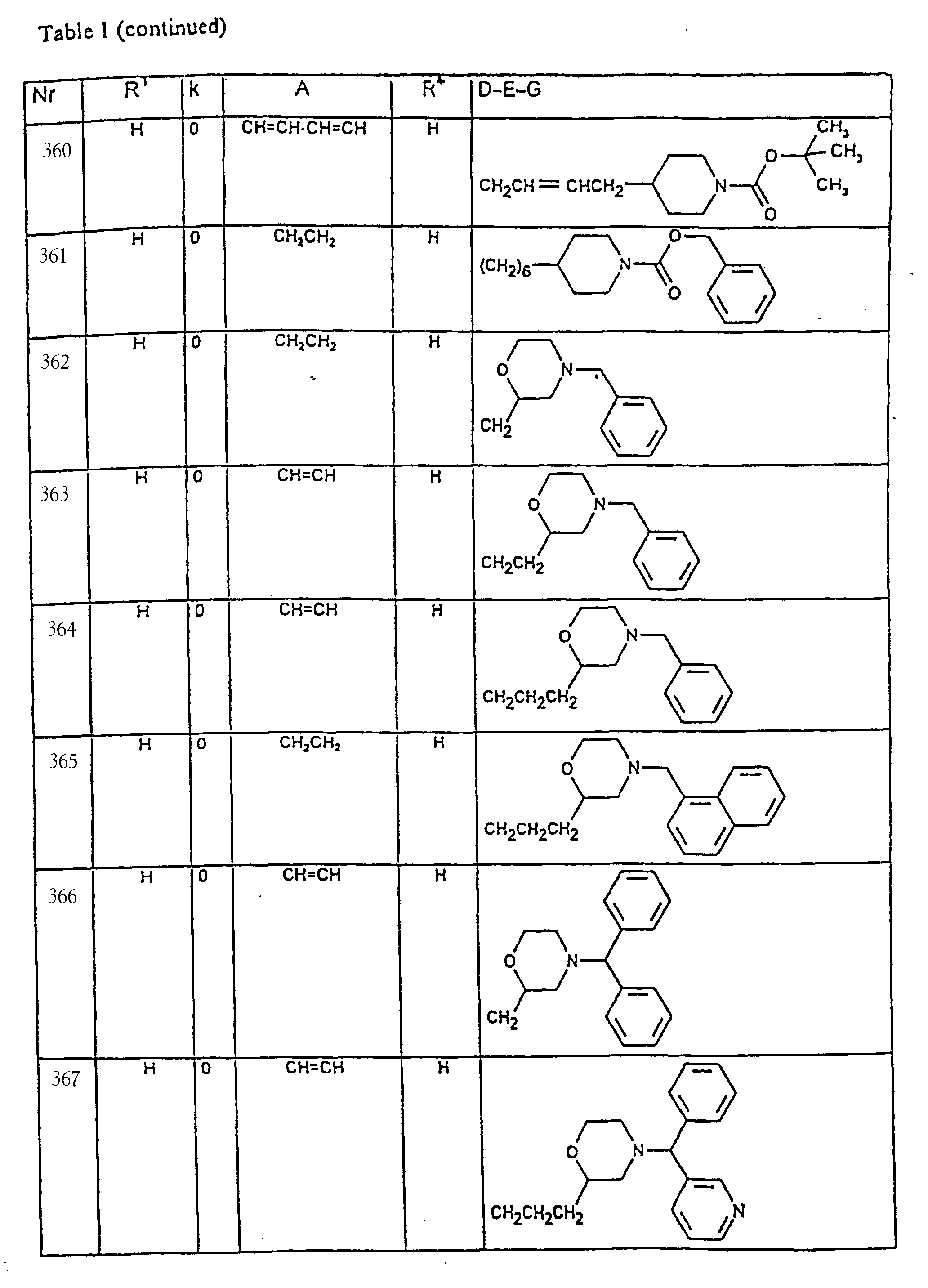

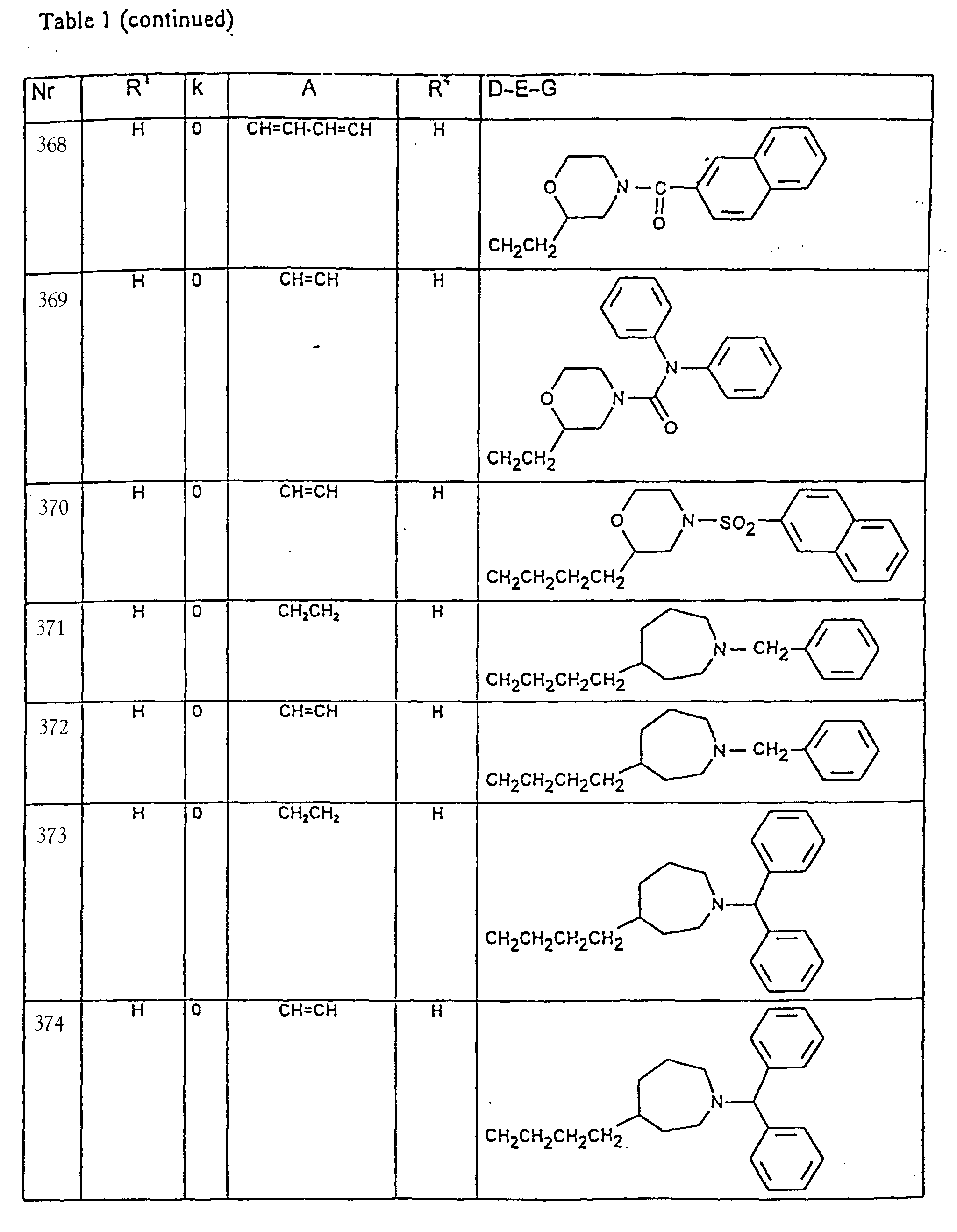

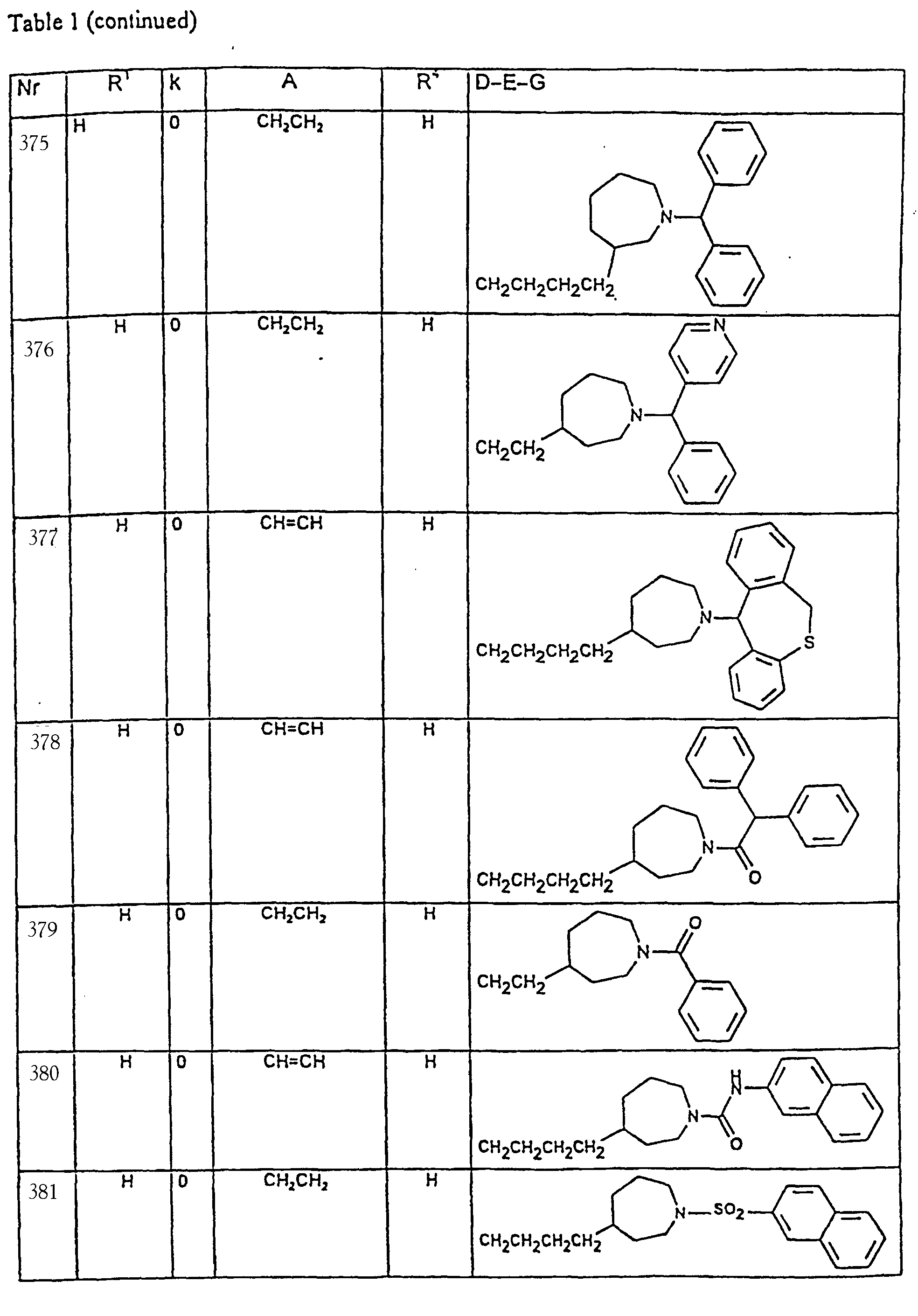

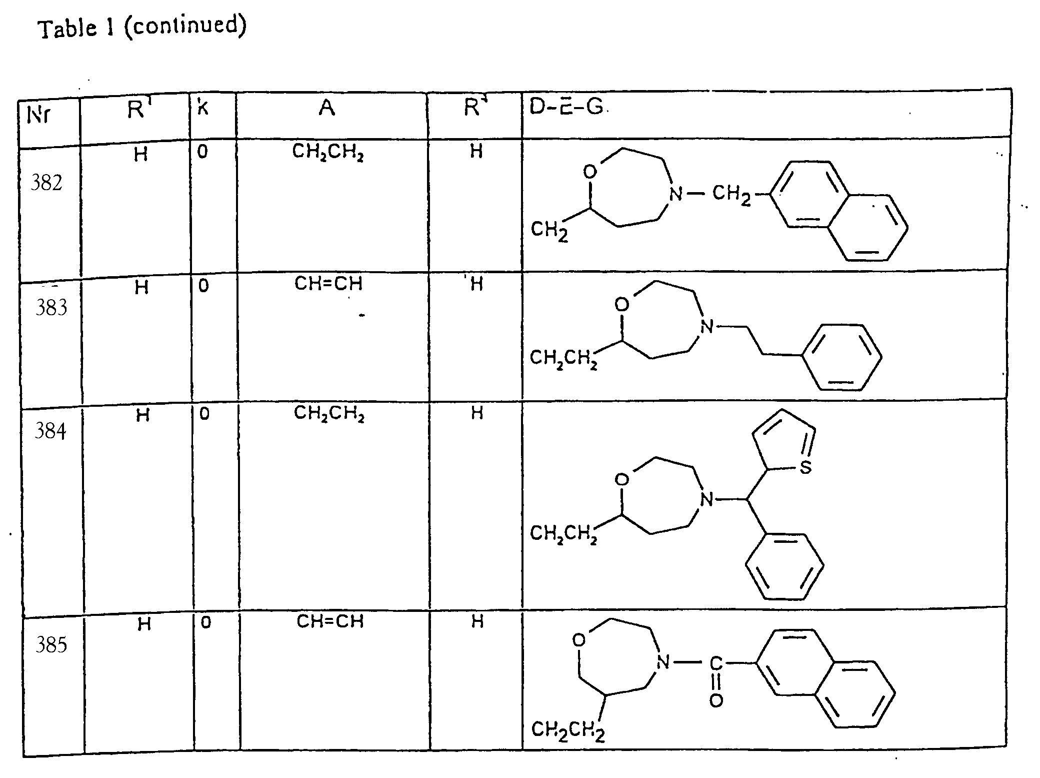

Various modes for synthesis of compounds used according to the invention are described in the following for reasons of simplifying reproducability.

Aside from a few exceptions, the optionally combined compounds presently described and used according to the invention are not previously described in the literature. A smaller portion of these compounds overlaps various previously known generic formulae that are very generally defined with respect to structure and which were named at the beginning as prior art. The synthesis methods for the production of the presently used compounds are entirely known to the person skilled in the art either generally from the relevant literature and/or from the prior art publications named at the beginning; also see the literature information referred to below. Consequently, the presently used compounds of the synthesis encompassed by the defined generic formula are easily accessible by analogous methods, as they are described, for example, in the following.

### Method (A):

Compounds of formula (I) are obtained by reacting carboxylic acids of formula (II) 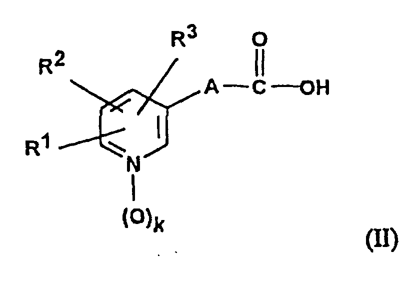 in which R¹, R², R³, A and k have the meaning given above or their reactive derivatives with compounds of formula (III) 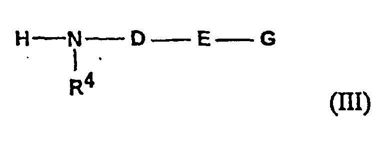 wherein D, E, G and R⁴ have the above meanings.

Reactive derivatives of compound (II) can be present, for example, as activated esters, anhydrides, acid halides, especially acid chlorides, or simple low alkyl esters. Suitable acitivated esters are, for example, p-nitrophenyl ester, 2,4,6-trichlorphenyl ester, pentachlorophenyl ester, cyanomethyl ester, esters of N-hydroxysuccinimide, of N-hydroxyphthalimides, of 1-hydroxybenzotriazol, of N-hydroxypiperidine, of 2-hydroxypyridine or of 2-mercaptopyridine, etc. Anhydrides can be symmetric anhydrides or mixed, as they are obtained, for example, with pivaloyl chloride or with chloroformates. Aromatic (for example chloroformic phenyl ester), araliphatic (for example chloroformic benzyl ester) or aliphatic chloroformates (for example chloroformic methyl ester and/or corresponding -ethyl or -isobutyl ester) can be used for this.

Reaction of compounds (II) with compounds (III) can also be carried out in the presence of condensation agents such as dicyclohexylcarbodiimide, 1-ethyl-3-(3-dimethylaminopropyl)carbodiimide hydrochloride, N,N'-carbonyldiimidazol, 1-ethoxycarbonyl-2-ethoxy-1,2-dihydroquinoline, etc. If carbodiimides are used as the condensation agent, reagents such as N-hydroxysuccinimide, N-hydroxyphthalimide, 1-hydroxybenzotriazol, N-hydroxypiperidine, etc. can be advantageously added.

Compounds of formula (III) can be used for reaction as free bases as well as in the form of their acid addition salts. For this, the salts of inorganic acids are to be preferred, i.e. hydrochlorides, hydrobromides or sulfates.

Reaction of compounds (II) or their reactive derivatives with compounds (III) are normally carried out in a suitable, preferably inert solvent. As examples, aromatic hydrocarbons such as benzene, toluol, xylene, halogenated hydrocarbons (for example dichloromethane, chloroform, 1,2-dichloroethane, trichloroethylene), ethers (for example diethyl ether, tetrahydrofuran, dioxane, glycol dimethyl ether), ethyl acetate, acetonitrile or polar aprotic solvents such as, for example, dimethylsulfoxide, dimethylformamide or N-methylpyrrolidone are to be named. Pure solvents, as well as mixtures of two or more, can be used.

The reaction is optionally carried out in the presence of an auxiliary base. Suitable examples for this are alkali metal carbonates (sodium carbonate, potassium carbonate), alkali metal hydrogen carbonates (sodium hydrogen carbonate, potassium hydrogen carbonate), or organic bases such as, for example, triethylamine, ethyl diisopropylamine, tributylamine, N-methylmorpholine or pyridine. A suitable excess of compound (III) can also be used as a base. If compounds (III) are used in form of their acid addition salts, then it is appropriate to consider the amount of auxiliary base used as equivalent.

The reaction temperatures can - depending on reactivity of the educts - vary in a wide range Generally, the reaction is carried out at temperatures between -40°C and 180°C, preferably between -10°C and 130°C, especially at the boiling point of the solvent used.

The starting compounds (II) and (III) are known and/or can be produced according to known methods in an analogous manner. Moreover, the production of representative examples is further described below.

### Method (B)

Compounds of formula (I) can be produced by reaction of compounds of formula (I), wherein G is hydrogen and which themselves possess the above-named pharmacological activities such as a cytostatic and/or immunomodulatory activity, as intermediate products as well as end products, with a compound of formula (IV),

**L―G** (IV)

in which G has the meaning given above, with the exception of hydrogen, and L is a suitable nucleofuge or reactive group. The type of nucleofuge or reactive group L and the conditions of the reaction are dependent of the nature of group **G**.

### Method (B1)

Compounds of formula (I), in which **G**, with the exception of hydrogen, has the meaning of (G1) according to the above definition can, aside from method (a), also be produced by reacting compounds of formula (I), wherein **G** is hydrogen, with a suitable alkylation agent and/or arylation agent of formula (IV), wherein **G** is an alkyl-, alkenyl-, alkinyl-, cycloalkyl-, aryl-, aralkyl-, heteroaryl- or heteroaralkyl residue according to definition and the leaving group L can be a reactive derivative of an alkohol, for example, a halogen atom such as chlorine, bromine or iodine or a sulfonic acid ester, i.e. for example a methanesulfonyloxy-, trifluoromethanesulfonyloxy-, ethanesulfonyloxy-, benzenesulfonyloxy-, p-toluolsulfonyloxy-, p-bromobenzenesulfonyloxy- or m-nitrobenzenesulfonyloxy residue, etc. A reactive group L can be a terminal epoxide group for example.

The reaction of compounds (I), in which G is a hydrogen, and (IV) is usually conducted in a suitably inert solvent. Such solvents can be, for example, aromatic hydrocarbons (benzene, toluol, xylene), ethers (for example tetrahydrofuran, dioxane, glycol dimethyl ether), ethyl acetate, acetonitrile, ketones (acetone, ethyl methyl ketone), polar protic solvents such as alcohols (ethanol, isopropanol, butanol, glycol monomethyl ether) or polar aprotic solvents such as, for example, dimethylsulfoxide, dimethylformamide or N-methylpyrrolidone. Pure solvents as well as mixtures of two or more can also be used. Preferably, the reactions are carried out in the presence of bases, whereby said bases can be used as in method (a) above. If chlorides or bromides are used as compound (IV), the reaction can be accelerated by the addition of alkali metal iodides (sodium iodide, potassium iodide). The reaction temperatures can vary between 0°C and 180°C depending on the reactivity of the educts, but preferably lie between 20°C and 130°C.

### Method (B2)

Compounds of formula (I), in which **G** represents an acyl residue, a carbamoyl residue, a sulfonyl residue or a phosphinoyl residue according to the above definition, can also be produced, aside from the above method (a), by reacting compounds of formula (I), in which **G** is hydrogen, with a carboxylic acid, carbamic acid, sulfonic acid and/or phosphinic acid of formula (V), in which **G** is an acyl residue, carbamoyl residue, sulfonyl residue or phosphinoyl residue according to definition,

**HO―G** (V)

or their derivatives capable of reaction. Preferred derivatives of carboxylic acids and/or sulfonic acids (V) which are capable of reaction are symmetric or unsymmetric carboxylic acid anhydrides and/or sulfonic acid anhydrides or acyl- and/or sulfonyl halides, especially acyl- and/or sulfonyl chlorides. Preferably, derivatives of carbarnates and/or phosphinic acids which are capable of reaction are the carbamoyl halides and/or phosphinyl halides, especially carbarnyl- and/or phosphinyl chlorides. The reaction of the acids (V) and/or their reactive derivatives with compounds (I), in which G is hydrogen, preferably occurs in the presence of auxiliary bases in solvents and under conditions as they are described in method (A).

### Method (B3)

Compounds of formula (I), in which **G** represents a carbamoyl residue according to the definition G2 with r = 0, i.e. the group 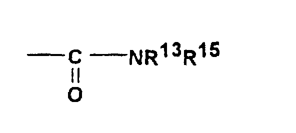 can also be produced, aside from the methods (A) and (B2), by reacting compounds of formula (I), in which **G** is hydrogen with a carbonyl group transmitter to an intermediate product and subsequently reacting this directly with a primary or secondary amine with the formula (VI)

**H―NR**^{**13**}**R**^{**15**} (VI)

in which R¹³ and R¹⁵ and/or the group **― NR**^{**13**}**R**^{**15**} have the meanings according to the above definitions without having to purify or isolate the intermediate product. Trichloromethylcarbonate (triphosgene) and carbonyldiimidazol have been proven as particularly reactive carbonyl group transmitters. The reaction of compounds of formula (I), wherein **G** is hydrogen, with triphosgene and/or carbonyldiimidazol are typically conducted in an absolute, inert solvent in the presence of a tertiary organic amine as an auxiliary base in such a manner that the solution of compounds (I) and the auxiliary base are slowly poured into a solution of an equivalent amount of carbonyl group transmitter. Thereby, the reaction requires molar ratios of 1 : 1 for the reaction of compound (I) and carbonyldiimidazol, and, in contrast, a ratio of 1 : 0.35 for the use of triphosgene. After complete reaction of the components to the intermediate product, compound (VI) is added in stochiometric amounts or in excess as a solution or a solid and the reaction is typically completed at elevated temperature. Suitable inert solvents are, for example hydrocarbons such as hexane, heptane, benzene, toluol, xylene, chlorinated hydrocarbons (for example dichloromethane, chloroform, 1,2-dichloroethane, trichloroethylene), ethers (for example diethyl ether, tetrahydrofuran, dioxane), esters such as ethyl acetate, butyl acetate, acetonitrile or polar aprodic solvents such as formamide or dimethylformamide. Pure solvents as well as mixtures can be used diversely. Sometimes it is of advantage to carry out the first partial reaction at low temperature in a low-viscosity, highly-volatile solvent and to remove the solvent after formation of the intermediate and replace it by a higher boiling solvent. Amines such as for example triethylamine, ethyl diisopropylamine, tributylamine, N-methylmorpholine or pyridine are suitable as auxiliary bases. If compounds (I) or (VI) are used as salts, the amount of the auxiliary base is increased accordingly. The reaction temperatures can lie in between -40°C and 50°C for the first partial reaction, preferably at 0°C bis 30°C, and between 0°C and 150°C for the second partial reaction, preferably at 20°C bis 120°C.

### Method (B4)

Compounds of formula (1), in which **G** represents a carbamoyl residue according to the definition G2 with r = 0 and R¹⁵ = hydrogen, i.e. a group 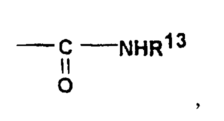 can also be produced, aside from methods A, B2 and B3, by reacting the compounds of formula (I) in which G is hydrogen, with an isocyanate of formula (VII) in which R¹³ has the meaning according to the above definition

**O=C=N―R**^{**13**} (VII)

Reaction of the compounds of formula (I), in which **G** is hydrogen, with the isocyanates of formula (VII) are conducted thereby in an absolute, inert solvent which can be a hydrocarbon such as pentane, hexane, heptane, benzene, toluol, or xylene, chlorinated hydrocarbons (such as dichloromethane, chloroform, 1,2-dichloroethane, trichloroethylene), ethers (for example, diethyl ether, tetrahydrofuran, dioxane), esters such as ethyl acetate, butyl acetate, or polar aprotic solvents such as formamide or dimethylformamide. Mixtures of various solvents can also be used. Thereby, the reaction temperatures can vary in the region from -20°C to 150°C, but preferably lie at 20°C to 100°C.

The above-named intermediate products in the form of compounds according to formula (I), wherein **G** is hydrogen, which have the above-mentioned activities, such as for example a cytostatic activity in an analogous way to the end products themselves, are suitable for the production of a multitude of end products through the synthesis methods B1-B4.

They themselves can, in principle, be produced according to method A by reacting a carboxylic acid of formula (II) with amines of formula (III) in which G is hydrogen as described above. However, since the compounds of formula (III) with hydrogen as G represent α,ω-diamines, the formation of product mixtures is always to be expected in their reaction with carboxylic acids (II) or their reactive derivatives making a subsequent separation necessary.

In contrast, compounds of formula (I), in which **G** is hydrogen, are essentially more advantageously produced from other compounds of formula (I), in which G is a selectively cleavable group under mild conditions, i.e. corresponds to a nitrogen protective group.

Among the compounds according to formula (I) with tumor growth inhibiting properties, compounds are particularly suitable for this in which G represents a benzyl group, a 4-methoxybenzyl group, a diphenylmethyl group, a triphenylmethyl group, a benzyloxycarbonyl group, a methoxy- and/or ethoxycarbonyl group, a tert-butoxycarbonyl group, an allyloxycarbonyl group or a trifluoroacetyl group. For example, compounds according to formula (I) with benzyl, diphenylmethyl, triphenylmethyl or benzyloxycarbonyl groups can already be catalytically transformed into the compounds of formula (I) with hydrogen as G at room temperature under mild conditions with elementary hydrogen or by transfer hydration. Compounds of formula (I) with a 4-methoxylbenzyl group are transformed into compounds of formula (I) with hydrogen as G by selective oxidation with ammonium-cer(IV)-nitrate. The cleavage of simple alkoxycarbonyl groups such as the methoxy- or ethoxycarbonyl group as well as the trifluoroacetyl group as **G** in compounds of formula (I) succeed by alkali hydrolysis under mild conditions without cleaving the A and D linked amide function. This is suitably valid for the cleavage of the triphenylmethyl group and the tert-butoxycarbonyl group as **G** in compounds of formula (I), which occurs in acidic medium under mild conditions. Finally, compounds of formula (I) with an allyloxycarbonyl group as G can be converted into such with hydrogen as G in neutral medium with palladium catalyst. All these methods are fully familiar to the person skilled in the art, and are furthermore also documented in monographs (see for example Greene, Wuts, Protective Groups in Organic Synthesis, New York, 1991).

### Method C

Compounds of formula (I), in which R⁴ is an alkyl, alkenyl, alkinyl or cycloalkyl residue according to the above definition can also be produced, aside from the methods A and B, by reacting compounds of formula (I), in which R⁴ is hydrogen, with a suitable alkylation agent of formula (VIII)

**L―R**^{**4**} (VIII)

in which R⁴ is an alkyl, alkenyl, alkinyl or cycloalkyl residue according to the above definition and L is a suitable nucleofuge, i.e. for example a halogen atom such as chlorine, bromine or iodine or a sulfonic acid ester of an alcohol. Preferred sulfonic acid esters (VIII) contain a methylsulfonyloxy residue, trifluoromethanesulfonyloxy-, p-toluolsulfonyloxy-, p-bromobenzenesulfonyloxy- or m-nitrobenzenesulfonyloxy residue as L. As an amide alkylation in the presence of tertiary amino groups, this reaction requires the use of strong auxiliary bases such as potassium-tert-butylate, sodium hydride, potassium hydride or butyl lithium in aprotic, inert solvents. Such solvents can be for example aliphatic or aromatic hydrocarbons (pentane, hexane, heptane, benzene, toluol), ethers (for example, tetrahydrofuran, dioxane) or polar solvents such as dimethylsulfoxide, dimethylformamide or N-methylpyrrolidone. Depending on the reactivity of the educts, the reaction temperatures can lie between -40°C and 140°C preferably between -20°C and 80°C.

### Method D

Compounds of formula (I) in which A is a saturated alkylene group can also be produced, aside from methods A, B and C, by hydrogenating compounds of formula (I) in which A is an unsaturated group according to the above definition, i.e. an alkenylene group or alkadienyl group with elementary hydrogen in the presence of a suitable catalyst. This method is also applicable when the compounds of formula (I) with an unsaturated group A in the molecule simultaneously contain a principally hydrogenolytically cleavable group B, i.e. -as already mentioned above- a benzyl group, a diphenylmethyl or triphenylmethyl group. In the selection of the conditions, especially the solvent, the temperature and the acid additive in the reaction mixture, the reaction can be controlably driven either to a selective satuation of the C-C multiple bond(s) in the structural element A or to a simultaneous cleavage of the benzyl, diphenylmethyl or triphenylmethyl residue G under formation of the compounds of formula (I) with hydrogen as G.

The hydration is preferably carried out in barely polar, aprotic solvents for selective hydration of one or more C-C multiple bonds of group A in the compounds of formula (I) according to the invention while attaining a simultaneously present hydrogenolytically cleavable benzyl, diphenylmethyl or triphenylmethyl residue as the structural element G. Esters such as ethyl acetate, propyl acetate, butyl acetate or ethers such as tetrahydrofuran, dioxane or ethylene glycol dimethyl ether can be used. Compounds of formula (I) to be hydrated can be present as a free base or entirely or partially in the form of a salt by addition of a sub-maximal to a maximal stochiometric amount of a strong acid, preferably a mineral acid. As a catalyst, palladium is suitable in various proportional amounts from 1, 3, 5 or 10 % on solid supports such as activated carbon, activated aluminum oxide or calcium carbonate. The hydration is carried out under normal pressure and at a temperature of 10 to maximally 30°C, preferably at 20 to 25°C and interrupted after consumption of the amount of hydrogen calculated for the saturation of the multiple bonds.

In contrast, for simultaneous cleavage of the multiple bonds in A and the cleavage of a benzyl, diphenylmethyl or triphenylmethyl group as G in the compound of formula (I), polar, aprotic solvents are used such as methanol, ethanol, isopropanol, methoxyethanol or water or mixtures thereof, whereby a considerable excess of a strong acid compared to the stochiometric salt formation, preferably a mineral acid such as concentrated hydrochloric acid or sulfuric acid is simultaneously added. The molecular ratio of substrate/acid can lie in the range of 1 : 2 to 1 :10 thereby, preferably between 1 : 3 and 1 : 5. The same catalysts which are mentioned above in connection with the selective hydration are suitable as catalysts. The hydration is carried out under normal pressure or slightly increased hydrogen pressure of 2 to 3 bar, preferably under normal pressure, until the termination of the uptake of hydrogen. Depending on uptake speed, the reaction temperature can vary between 10 and 50, 70 or 80°C as a function of the boiling point of the solvent and/or solvent mixture and employed pressure. If, for example, the reaction is carried out in ethanol or ethanol/water under normal pressure, the reaction temperature preferably lies between 40 to 60°C.

The compounds of formula (I) produced according to the method (A), (B1) to (B4), (C) or (D) can be isolated and purified in a known manner, for example by subjecting the residue after distillation of the solvent to partition, extraction, re-precipitation or recrystallization or another purification method For this, column chromatography on a suitable support or preparative, middle or high pressure liquid chromatography are preferred for this.

The compounds (I) are first normally obtained in form of their free bases or their hydrates or solvates, depending on the type of isolation and purification. Their addition salts with pharmaceutically suitable acids are obtained in a typical manner by converting the base with the desired acid in a suitable solvent. Depending on the number of basic centers of compound (I), one or more equivalent acids per mole of base can be bound.

Suitable solvents are, for example, chlorinated hydrocarbons such as dichloromethane or chloroform; ethers such as diethyl ether, dioxane or tetrahydrofuran; acetonitrile; ketones such as acetone or ethyl methyl ketone; esters such as methyl acetate or ethyl acetate or low molecular alcohols such as methanol, ethanol or isopropanol; and water. Pure solvents as well as mixtures of two or three solvents can also be used. The salts can be isolated by crystallization, precipitation or the evaporation of the solvent. Thereby, they optionally accumulate as hydrates or solvates.

The bases can be recovered from the salts by alkalization, for example with aqueous ammonia solution, alkali carbonate or diluted sodium hydroxide solution.

The following listed compounds and/or their pharmaceutically acceptable salts, if not already concretely labelled as such, are particularly preferred.
N-[2-(1-benzylpiperidin-4-yl)-ethyl]-3-(pyridin-3-yl)-propionamide,
N-{2-[1-(2-phenylethyl)-piperidin-4-yl]-ethyl}-3-(pyridin-3-yl)-propionamide
N-{2-[1-(4-phenylbutyl}-piperidin-4-yl]-ethyl}-3-(pyridin-3-yl)-propionamide
N-{2-[1-(4-hydroxy-4-phenylbutyl)-piperidin-4-yl]-ethyl}-3-(pyridin-3-yl)-propionamide
N-[2-(1-diphenylmethylpiperidin-4-yl)-ethyl]-3-(pyridin-3-yl)-propionamide,
N-[3-(1-diphenylmethylpiperidin-4-yl)-propyl)-3-(pyridin-3-yl)-propionamide,
N-[4-(1-diphenylmethylpiperidin-4-yl)-butyl]-3-(pyridin-3-yl)-propionamide,
N-[2-(1-benzylpiperidin-4-yl)-butyl]-3-(pyridin-3-yl)-acrylamide,
N-{4-[1-(2-phenylethyl)-piperidin-4-yl]-butyl}-3-(pyridin-3-yl)-acrylamide
N-{4-[1-(4-biphenylylmethyl)-piperidin-4-yl]-butyl}-3-(pyridin-3-yl)-acrylamide
N-{4-[1-(1-naphthylmethyl)-piperidin-4-yl]-butyl}-3-(pyridin-3-yl)-acrylamide
N-{4-[1-(9-anthrymethyl)-piperidin-4-yl]-butyl}-3-(pyridin-3-yl)-acrylamide
N-{4-[1-(Cyclohexylphenylmethyl)-piperidin-4-yl]-butyl}-3-(pyridin-3-yl)-acrylamide
N-{4-[1-(10,11-dihydro-5H-dienzo[a,d]cycloheptene-5-yl)-piperidin-4-yl]-butyl}-3-(pyridin-3-yl)-acrylamide
N-[2-(1-diphenylmethylpiperidin-4-yl)-ethyl]-3-(pyridin-3-yl)-acrylamide,
N-[3-(1-diphenylmethylpiperidin-4-yl)-propyl]-3-(pyridin-3-yl)-acrylamide,
N-[5-(1-diphenylmethylpiperidin-4-yl)-pentyl]-3-(pyridin-3-yl)-acrylamide,
N-[6-(1-diphenylmethylpiperidin-4-yl)-hexyl]-3-(pyridin-3-yl)-acrylamide,
N-[4-(1-diphenylmethylpiperidin-4-yl)-butyl]-5-(pyridin-3-yl)-2,4-pentadiene acid amide,
N-(4-{1-[bis-(4-fluorophenyl)-methyl]-piperidin-4-yl)-butyl)-3-(pyridin-3-yl)-acrylamide,
N-(4-{1-[bis-(2-chlorophenyl)-methyl]-piperidin-4-yl}-butyl)-3-(pyridin-3-yl)-acrylamide,
N-[4-(1-diphenylmethylpiperidin-4-yl)-butyl]-3-(2-fluoropyridin-3-yl)-acrylamide,
N-[4-(1-diphenylmethylpiperidin-4-yl)-butyl]-3-(6-fluoropyridin-3-yl)-acrylamide,
N-[4-(1-diphenylmethylpiperidin-4-yl)-butyl]-3-(pyridin-3-yl)-acrylamide,
N-[4-(1-diphenylmethylpiperidin-4-yl)-butyl]-3-(pyridin-3-yl)-acrylamide dihydrochloride,
N-[4-(1-diphenylmethylpiperidin-4-yl)-butyl]-3-(pyridin-3-yl)-acrylamide methanesulfonate, and
N-(4-diphenylmethyl-morpholin-2-ylmethyl)-3-(pyridin-3-yl)-acrylamide.

For a better understanding of the use according to the invention and reproducability of the compounds used, a series of synthetic examples is described in the following:

### SYNTHETIC EXAMPLES for the end products of the invention according to formula (I)

In the following production examples for the end products, the abbreviations stand for the following terms:
- MP =: melting point,
- RT =: room temperature,
- THF =: tetrahydrofuran,
- DMF =: dimethylformamide,
- CDI =: carbonyldiimidazol,
- abs. =: absolute,
- EDC =: N-(3-dimethylaminopropyl)-N'-ethyl-carbodiimide hydrochloride,
- HOBT=: 1-hydroxybenzotriazol,
- TEA =: triethylamine.
¹H-NMR-Spectrum = proton resonance spectrum, taken at 100 MHz. The chemical shifts are given in ppm against TMS as a standard (δ = 0.0), whereby
- s: = singlet,
- d: = doublet,
- t: = triplet,
- dt: = doublet-triplet,
- m: = multiplet,
- ar: = aromatic,
- py: = pyridine.

### Example 1

### N-[4-(1-diphenylmethylpiperidin-4-yl)-butyl]-N-ethyl-3-(pyridin-3-yl)-acrylamide (Substance 117)

10 g (22.0 mmol) N-[4-(1-diphenylmethylpiperidin-4-yl)-butyl]-3-(pyridin-3-yl)-acrylamide (substance 98) are dissolved in 100 ml THF and added to 0.73 g (24.3 mmol) 80 % NaH (heavy foaming) and stirred 20 minutes at RT. 2.1 ml (26.4 mmol) ethyl iodine are added dropwise and the mixture is stirred five hours at RT. 0.1 g tetrabutyl ammonium iodine are added and the batch is further stirred at RT overnight. Subsequently, 0.1 g (3 mmol) 80 % NaH are added and this is heated at 50°C for one hour under stirring. The batch is carefully hydrolyzed with 50 ml water after cooling to RT. The aqueous phase is extracted with 100 ml dichloromethane and the combined organic phases are washed with 50 ml water. The organic phase is concentrated in vacuum and the residue is chromatographically pre-purified twice over silica gel with CHCl₃/CH₃OH (95/5 to 90/10 and 98/2 to 95/5), subsequently further purified by flash chromatography with CHCl₃/CH₃OH (100/0 to 98/2) and crystallized three times from 20 ml 1-chlorobutane, 10 ml acetonitrile/diisopropyl ether (1/1) and 8 ml isopropanol/diisopropyl ether (1/1). Yellow crystals with a MP of 115-117°C were recovered; yield: 1.1 g (10 %)

| C₃₂H₃₉N₃O (481.7) | | |
|---|---|---|
| IR-Spectrum (KBr): | ν(C=O) | 1640 cm⁻¹ |
| | ν(C=C) | 1600 cm⁻¹ |

| | |
|---|---|
| ¹H-NMR-Spectrum (CDCl₃): | 0.90 - 1.95 (16H, m, piperidine, piperidine-(CH₂)₃ CH₃) |
| | 2.70 - 3.00 (2H, m, piperidine) |
| | 3.20 - 3.70 (4H, m, CONCH₂, J |
| | 4.21 (1H, s, Ar₂CH) |
| | 6.89 (1H, d, CH=CHCO, J=15.5 Hz) |
| | 7.00 - 7.50(11H, m, ar, py) |
| | 7.69 - 7.60 (1H, d, CH=CHCO, J=15.5 Hz) |
| | 7.70 - 7.95 (1H, m, py) |
| | 8.50 - 8.65 (1H, m, py) |
| | 8.70 - 8.85 (1H, m, py) |

### Example 2

### N-[4-(1-diphenylmethylpiperidin-4-yl)-butyl]-3-(pyridin-3-yl)-acrylamide (Substance 98)

2.0 g (13.6 mmol) 3-(pyridin-3-yl)-acrylic acid are suspended in 60 ml abs. dichloromethane and, after addition of three drops of pyridine, cooled to ca. 0°C in an ice bath under moisture exclusion. 1.8 ml (18.6 mmol) oxalyl chloride are added dropwise and the mixture is stirred at RT overnight. Subsequently, the solvent and excess oxalyl chloride is distilled off in a rotary evaporator. In order to completely remove the oxalyl chloride, the residue is dried further for two hours under high-vacuum. The acid chloride obtained in this manner is suspended in 50 ml abs. dichloromethane and cooled to ca. 0°C in an ice bath under moisture exclusion. 4.0 g (12.4 mmol) 4-(1-diphenylmethylpiperidin-4-yl)-butylamine are dissolved in 30 ml abs. dichloromethane and added dropwise to this suspension. After complete addition, the ice bath is removed and the reaction mixture is stirred for a further two hours at RT. The mixture is subsequently washed with 10 % sodium hydroxide solution. The aqueous phase is extracted with acetic acid ethyl ether. The combined organic phases are dried over sodium sulfate and the solvent is removed under vacuum. The residue is crystallized once from 15 ml isopropanol and then twice from acetic acid ethyl ester. Colorless crystals with a MP of 156°C were recovered; yield: 1.6 g (28 %)

| C₃₀H₃₅N₃O (453.6) | | |
|---|---|---|
| IR-Spectrum (KBr): | ν(NH) | 3310 cm⁻¹ |
| | ν(C=O) | 1660, 1545 cm⁻¹ |
| | ν(C=C) | 1620 cm⁻¹ |

| | |
|---|---|
| ¹H-NMR-Spectrum (CDCl₃): | 0.90 - 2.20 (13H, m, piperidine, piperidine-(CH₂)₃) |
| | 2.65 - 3.05 (2H, m, piperidine) |
| | 3.20 - 3.60 (2H, m, CONCH₂, J |
| | 4.21 (1H, s, Ar₂CH) |
| | 5.75 - 6.15 (1H, m, NH) |
| | 6.45 (1H, d, CH=CHCO, J=15.6) |
| | 6.90 - 8.00 (13H, m, ar, py, CH=CHCO) |
| | 8.45 - 8.70 (1H, m, py) |
| | 8.70 - 8.90 (1H, m, py) |

### Example 3

### N-(4-{1-[bis-(4-fluorophenyl)-methyl]-piperidin-4-yl}-butyl)-3-(pyridin-3-yl)-acrylamide (substance 161)

3.7 g (24.5 mmol) 3-(3-pyridyl)-acrylic acid are suspended in 100 ml abs. dichloromethane and, after addition of three drops of pyridine, cooled to ca. 0°C in an ice bath under moisture exclusion. 2.8 ml (22.1 mmol) oxalyl chloride are added dropwise and the mixture is stirred at RT overnight. Subsequently, the solvent and excess oxalyl chloride is distilled off in a rotary evaporator. In order to completely remove the oxalyl chloride, the residue is dried further for 2 hours under high-vacuum. The acid chloride obtained in this manner is suspended in 50 ml abs. dichloromethane and cooled to ca. 0°C in an ice bath under moisture exclusion. 8.0 g (22.3 mmol) 4-[1-bis-(4-fluorophenyl)-methyl-piperidin-4-yl]-butylamine are dissolved in 50 ml abs. dichloromethane and added dropwise to this suspension. After complete addition, the ice bath is removed and the reaction mixture is stirred for a further two hours at RT. The mixture is subsequently distributed between 10% sodium hydroxide solution and dichloromethane, and the aqueous phase is extracted a further three times with dichloromethane. The combined organic phases are washed with 100 ml water, dried over sodium sulfate and the solvent is removed under vacuum. The residue is chromatographically purified over silica gel with CHCl₃/CH₃OH (99.5/0.5 to 97/3) and crystallized from 30 ml acetic acid ethyl ester after drawing off the solvent. Colorless crystals with a MP of 108°C were recovered; yield 3,5 g (34%).

| C₃₀H₃₃F₂N₃O (489.6) | | |
|---|---|---|
| IR-Spectrum (KBr): | ν(NH) | 3320 cm⁻¹ |
| | ν(C=O) | 1655, 1540 cm⁻¹ |
| | ν(C=C) | 1620 cm⁻¹ |

| | |
|---|---|
| ¹H-NMR-Spectrum (CDCl₃): | 1.00 - 2.00 (13H, m, piperidine, piperidine-(CH₂)₃) |
| | 2.60 - 2.95 (2H, m, piperidine) |
| | 3.38 (2H, dt, CONHCH₂, J=6.6 Hz, J= 12.7 Hz) |
| | 4.20 (1H, s, Ar₂CH) |
| | 5.85-6.10 (1H, m, NH) |
| | 6.47 (1H, d, CH=CHCO, J=15.7 Hz) |
| | 6.80 - 7.50 (9H, m, ar, py) |
| | 7.65 - 7.90 (1H, m, py) |
| | 8.45 - 8.65 (1H, m, py) |
| | 8.65 - 8.85 (1H, m, py) |

### Example 4

### N-{4-[1-(10, 11-dihydro-5H-dibenzo[a,d]cycloheptene-5-yl)-piperidin-4-yl]-butyl}-3-(pyridin-3-yl)-acrylamide (substance 209)

Production occurred analogously to Example 3.

Batch size: 2.6 g (17.6 mmol) 3-(3-pyridyl)-acrylic acid, 2.6 g (20.8 mmol) oxalyl chloride and 5.57 g (16.0 mmol) 4-[1-(10,11-dihydro-5H-dibenzo[a,d]cycloheptene-5-yl)-piperidin-4-yl]-butylamine.

In the work up, the mixture is concentrated under vacuum and subsequently distributed between 100 ml 10 % NaOH and 300 ml acetic acid ethyl ester. The aqueous phase is extracted again with 50 ml acetic acid ethyl ester and the combined organic phases are washed with 50 ml water, dried over sodium sulfate and the solvent is removed under vacuum. The residue is chromatographically purified over silica gel with CHCl₃/CH₃OH (97/3) and crystallized twice from 40 ml and 30 ml acetonitrile after drawing off the solvent. Colorless crystals with a MP of 125-127°C were recovered; yield 2.3 g (30%).

| C₃₂H₃₇F₂N₃O (479:6) | | |
|---|---|---|
| IR-Spectrum (KBr): | ν(NH) | 3300 cm⁻¹ |
| | ν(C=O) | 1655, 1540 cm⁻¹ |
| | ν(C=C) | 1620 cm⁻¹ |

| | |
|---|---|
| ¹H-NMR-Spectrum (CDCl₃): | 0.90 - 2.00 (13H, m, piperidine, piperidine-(CH₂)₃) |
| | 2.55 - 3.00 (4H, m, piperidine, ar -CH-CH-ar) |
| | 3.36 (2H, dt, CONHCH₂, J=6.6 Hz, J= 12.7 Hz) |
| | 3.90 (1H, s, Ar₂CH) |
| | 5.60 - 5.85 (1H, m, NH) |
| | 6.43 (1H, d, CH=CHCO, J=15.7 Hz) |
| | 6.90 - 7.40 (9H, m, ar, py) |
| | 7.60 (1H, d, CH=CHCO, J=15.6 Hz) |
| | 7.65 - 7.90 (1H, m, py) |
| | 8.50 - 8.65 (1H, m, py) |
| | 8.70 - 8.80 (1H, m, py) |

### Example 5

### N-[4-(1-benzylpiperidin-4-yl)-butyl]-3-(pyridin-3-yl)-acrylamide (Substance 41)

6.5 g (18.0 mmol) N-[4-(piperidin-4-yl)-butyl]-3-(pyridin-3-yl)-acrylamide dihydrochloride (substance 22) are suspended in 80 ml acetone added to 9.9 g (72.0 mmol) potassium carbonate. A solution of 3.4 g (19.8 mmol) benzyl bromide in 10 ml acetone is added dropwise to this mixture at RT and stirred overnight. Subsequently, the suspension is filtered and the filtrate is concentrated under vacuum. The residue is taken up in 100 ml CHCl₃ and washed with 30 ml water. The organic phase is dried over sodium sulfate and the solvent is removed under vacuum. The residue is chromatographically purified over silica gel with CHCl₃/CH₃OH (95/5 to 90/10) and crystallized from 15 ml acetonitrile after drawing off the solvent. Beige colored crystals with a MP of 88-90°C were recovered; yield: 1.1 g (16 %)

| C₂₄H₃₁N₃O (377.5) | | |
|---|---|---|
| IR-Spectrum (KBr): | ν(NH) | 3320 cm⁻¹ |
| | ν(C=O) | 1655, 1530 cm⁻¹ |
| | ν(C=C) | 1620 cm⁻¹ |

| | |
|---|---|
| ¹H-NMR-Spectrum (CDCl₃): | 1.00 - 2.10 (13H, m, piperidine, piperidine-(CH₂)₃) |
| | 2.70 - 3.00 (2H, m, piperidine) |
| | 3.20 - 3.55 (4H, m, CONHCH₂, ar, CH₂) |
| | 5.65 - 6.00 (1H, m, NH) |
| | 6.45 (1H, d, CH=CHCO, J=15.6 Hz) |
| | 3.36 (2H, dt, CONHCH₂, J=6.6 Hz, J= 12.7 Hz) |
| | 7.10 - 7.40 (6H, m, ar, py) |
| | 7.62 (1H, d, CH=CHCO, J=15.6 Hz) |
| | 5.60 - 5.85 (1H, m, NH) |
| | 6.43(1H, d, CH=CHCO, J=15.7 Hz) |
| | 7.65 - 7.90 (1H, m, py) |
| | 8.45-8.65 (1H, m, py) |
| | 8.65 - 8.85 (1H, m, py) |

### Example 6

### N-[2-(1-diphenylmethylpiperidin-4-yl)-ethyl]-3-(pyridin-3-yl)-acrylamide (Substance 89)

Production occurred analogously to Example 3:

Batch size: 2.3 g (15.5 mmol) 3-(3-pyridyl)-acrylic acid, 2.7 g (21.3 mmol) oxalyl chloride and 4.1 g (13.9 mmol) 2-(1-diphenylmethylpiperidine-4-yl)-ethylamine in 60 ml abs. dichlormethane.

In the work up, the mixture is concentrated under vacuum and subsequently dispersed between 100 ml 10 % NaOH and 300 ml acetic acid ethyl ester. The aqueous phase is extracted again with 50 ml acetic acid ethyl ester and the combined organic phases are washed with 50 ml water, dried over sodium sulfate and the solvent is removed under vacuum. The residue is chromatographically pre-purified over silica gel with CHCl₃/CH₃OH (95/5) and subsequently purified by flash-chromatography with CHCl₃/CH₃OH (100/0 to 94/6). After drawing off the solvent, this is crystallized from 19 ml acetic acid ethyl ester/petroleum ether. Colorless crystals with a MP of 141-143°C were recovered; yield 0.6 g (10%).

| C₂₈H₃₁N₃O (425.6) | | |
|---|---|---|
| IR-Spectrum (KBr): | ν(NH) | 3820 cm⁻¹ |
| | ν(C=O) | 1660, 1550 cm⁻¹ |
| | ν(C=C) | 1620 cm⁻¹ |

| | |
|---|---|
| ¹H-NMR-Spectrum (CDCl₃): | 1.05 - 2.00 (9H, m, piperidine, piperidine-CH₂) |
| | 2.70 - 3.00 (2H, m, piperidine) |
| | 3.41 (2H, dt, CONHCH₂, J=6.6 Hz, J= 12.7 Hz) |
| | 4.23 (1H, s, Ar₂CH) |
| | 5.55 - 5.80 (1H, m, NH) |
| | 6.43 (1H, d, CH=CHCO, J=15.6 Hz) |
| | 7.00 - 7.55 (11H, m, ar, py) |
| | 7.61 (1H, d, CH=CHCO, J=15.6 Hz) |
| | 7.60 - 7.90 (1H, m, py) |
| | 8.55 - 8.65 (1H, m, py) |
| | 8.65 - 8.80 (1H, m, py) |

### Example 7

### N-{4-[1-(9-anthryl)-methylpiperidin-4-yl]-butyl}3-(pyridin-3-yl)-acrylamide (Substance 75)

5.4 g (15.0 mmol) N-[4-piperidin-4-yl)-butyl]-3-(pyridin-3-yl)-acrylamide dichloride (substance 22) were suspended in 80 ml dichloromethane and added to 5.1 g (50.0 mmol) TEA. To this mixture, a solution of 3.7 g (16.5 mol) (9-anthyl)-methylchloride in dichloromethane is added at RT and stirred overnight. Subsequently, the mixture is washed twice, each with 100 ml water. The organic phase is dried over sodium sulfate and the solvent is removed under vacuum. The residue is chromatographically purified over silica gel with CHCl₃/CH₃OH (95/5 to 94/6) and crystallized first from 100 ml ethanol and then from 83 ml ethanol/diisopropyl ether (75/8) after drawing off the solvent. Yellow crystals with a MP of 162-164°C were recovered; yield: 1.8 g (25 %)

| C₃₂H₃₅N₃O (477.6) | | |
|---|---|---|
| IR-Spectrum (KBr): | ν(NH) | 3360 cm⁻¹ |
| | ν(C=O) | 1680, 1560 cm⁻¹ |
| | ν(C=C) | 1640 cm⁻¹ |

| | |
|---|---|
| ¹H-NMR-Spectrum (CDCl₃): | 0.90 - 1.80 (11H, m, piperidine, piperidine-CH₂)₃) |
| | 2.05 - 2.40 (2H, m, piperidine) |
| | 2.80 - 3.15 (2H, m, piperidine) |
| | 3.20 - 3.55 (2H, m, CONHCH₂) |
| | 4.45 (1H, s, Ar₂CH) |
| | 5.60 - 5.90 (1H, m, NH) |
| | 6.42 (1H, d, CH=CHCO, J=15.6 Hz) |
| | 7.20 - 8.20 (10H, m, ar, py, CH=CHCO) |
| | 8.40 - 8.90 (4H, m, ar, py) |

### Example 8

### N-{4-[1-(cyclohexylphenylmethyl)-piperidin-4-yl]-butyl}-3-(pyridin-3-yl)-acrylamide (Substance 78)

4.5 g (17.7 mmol) (bromcyclohexylphenyl)-methane, 5.6 g (15.5 mmol) N-[4-(piperidin-4-yl)-butyl]-3-(pyridin-3-yl)-acrylamide dihydrochloride (substance 22), 8.5 g (61.8 mmol) potassium carbonate and 2.8 g (16.9 mmol) sodium iodide are stirred in 200 ml DMF 18 hours at ca. 75°C. After cooling, the mixtuure is filtered over a diatomaceous earth layer and the filtrate is concentrated under vacuum. The residue is taken up in 200 ml CHCl₃ and washed twice with 60 ml and 30 ml water. The organic phase is dried over a sodium sulfate and the solvent is removed under vacuum. The residue is chromatographically purified over a silica gel with CHCl₃/CH₃OH (96/4 bis 92/8) and crystallized three times, each from 40 ml acetonitrile and once at the conclusion from 50 ml acetonitrile. Colorless crystals with a MP of 143-145°C were recovered; yield 0,58 g (8%).

| C₃₀H₄₁N₃O (459.7) | | |
|---|---|---|
| IR-Spectrum (KBr): | ν(NH) | 3330 cm⁻¹ |
| | ν(C=O) | 1680, 1570 cm⁻¹ |
| | ν(C=C) | 1640 cm⁻¹ |

| | |
|---|---|
| ¹H-NMR-Spectrum (CDCl₃): | 0.50 - 2.20 (24H, m, piperidine, piperidine-CH₂)₃, cyclohexane) |
| | 2.55 - 2.90 (2H, m, piperidine) |
| | 3.08 (1H, d, Ar-CH, J=9.2 Hz) |
| | 3.35 (2H, dt, CONHCH₂, J=6.5 Hz, J=12.7 Hz) |
| | 5.70 - 6.05 (1H, m, NH) |
| | 6.45 (1H, d, CH=CHCO, J=15.6 Hz) |
| | 7.00 - 7.50 (6H, m, ar, py) |
| | 7.60 (1H, d, CH=CHCO, J=15.6 Hz) |
| | 7.60 - 7.90 (1H, m, py) |
| | 8.45 - 8.65 (1H, m, py) |
| | 8.65 - 8.85 (1H, m, py) |

### Example 9

### N-(4-{1-[bis-(2-chlorphenyl)-methyl]-piperidin-4-yl}-butyl)-3-(pyridin-3-yl)-acrylamid (Substance 176)

Production occurred analogously to Example 3.

Batch size: 1.6 g (10.7 mmol) 3-(3-pyridyl)-acrylic acid, 1.9 g (15.0 mmol) oxalyl chloride and 3.9 g (10.0 mmol) 4-{1-[bis-(2-chlorophenyl)-methyl]-piperidine-4-yl}-butylamine. In the purification, chromatographic purification is done twice over silica gel with CHCl₃/CH₃OH (97/3 and 97/3 to 95/5) and crystallization is from 25 ml acetic acid after drawing off the solvent. Colorless crystals with a MP of 129-131°C were recovered; yield 0.6g (11%).

| C₃₀H₃₃Cl₂N₃O (522.5) | | |
|---|---|---|
| IR-Spectrum (KBr): | ν(NH) | 3240 cm⁻¹ |
| | ν(C=O) | 1655, 1560 cm⁻¹ |
| | ν(C=C) | 1620 cm⁻¹ |

| | |
|---|---|
| ¹H-NMR-Spectrum (CDCl₃): | 0.90 - 1.95 (11H, m, piperidine, piperidine-CH₂)₃) |
| | 1.90 - 2.40 (2H, m, piperidine) |
| | 2.55 - 3.00 (2H, m, piperidine) |
| | 3.38 (2H, dt, CONCH₂ J=6.5 Hz, J=12.4 Hz) |
| | 5.31 (1H, s, Ar₂CH) |
| | 5.55 - 5.90 (1H, m, NH) |
| | 6.44 (1H, d, CH=CHCO, J=15.6 Hz) |
| | 6.90 - 8.00 (11H, m, ar, py, CH=CHCO) |
| | 8.55 - 8.70 (1H, m, py) |
| | 8.70 - 8.95 (1H, m, py) |

### Example 10

### N-[3-(1-diphenylmethylpiperidin-4-yl)-propyl]-3-(pyridin-3-yl)-acrylamide (substance 91)

Production occurred analogously to Example 3.

Batch size: 3.9 g (26.1 mmol) 3-(3-pyridyl)-acrylic acid, 4.1 g (47.4 mmol) oxalyl chloride and 7.3 g (23.7 mmol) 3-(1-diphenylmethylpiperidine-4-yl)-propylamine.

In the purification, crystallization is done first from 1-chlorobutane and subsequently once from acetic acid. Colorless crystals with a MP of 110-113°C were recovered; yield 6.2 g (60%).

| C₂₉H₃₃N₃O (439.6) | | |
|---|---|---|
| IR-Spectrum (KBr): | ν(NH) | 3240 cm⁻¹ |
| | ν(C=O) | 1650, 1555 cm⁻¹ |
| | ν(C=C) | 1605 cm⁻¹ |

| | |
|---|---|
| ¹H-NMR-Spectrum (CDCl₃): | 0.90 - 2.25 (11H, m, piperidine, piperidine-CH₂)₃) |
| | 2.70 - 3.05 (2H, m, piperidine) |
| | 3.36 (2H, dt, CONCH₂ J=6.5 Hz, J=12.8 Hz) |
| | 4.21 (1H, s, Ar₂CH) |
| | 5.85 - 6.20 (1H, m, NH) |
| | 6.46 (1H, d, CH=CHCO, J=15.7 Hz) |
| | 6.90 - 7.70 (11H, m, ar, py) |
| | 7.60 (1H, d, CH=CHCO, J=15.7 Hz) |
| | 7.60 - 7.90 (1H, m, py) |
| | 8.45 - 8.65 (1H, m, py) |
| | 8.65 - 8.85 (1H, m, py) |

### Example 11

### N-[3-(1-diphenylmethylpiperidin-4-yl)-propyl]-3-(pyridin-3-yl)-propionamide (substance 90)

3.0 g (6.8 mmol) N-[3-(1-diphenylmethylpiperidin-4-yl)-propyl]-3-(pyridin-3-yl)-acrylamide (substance 91) are suspended in 60 ml THF and added to 5 drops concentrated hydrochloric acid and 0.35 g palladium (5%) on activated carbon. The mixture is stirred at RT under hydrogen atmosphere until consumption of the theoretical amount of hydrogen to be taken up. The suspension is filtered from the catalyst and the solvent is removed under vacuum. The residue is chromatographically purified over a silica gel with CHCl₃/CH₃OH/NH₄OH (85/15/2) and crystallized twice after drawing off the solvent. Colorless crystals with a MP of 109-110°C were recovered; yield 1.7 g (56%).

| C₂₉H₃₅N₃O₂ (441.6) | | |
|---|---|---|
| IR-Spectrum (KBr): | ν(NH) | 3230 cm⁻¹ |
| | v(C=O) | 1620, 1555 cm⁻¹ |

| | |
|---|---|
| ¹H-NMR-Spectrum (CDCl₃): | 1.00 - 2.00 (11H, m, piperidine, piperidine-CH₂)₂) |
| | 2.44 (2H, t, CO-CH₂, J=7.4 Hz) |
| | 2.75 - 3.15 (4H, m, piperidine, py-CH₂) |
| | 3.15 (2H, dt, CONCH₂ J=6.7 Hz, J=13.0 Hz) |
| | 4.21 (1H, s, Ar₂CH) |
| | 5.30 - 5.60 (1H, m, NH) |
| | 7.00 - 7.70 (12H, m, Ar, pyridine |
| | 6.90 - 7.70 (11H, m, ar, py) |
| | 8.35 - 8.55 (2H, m, pyridine) |

### Example 12

### N-[4-(1-diphenylmethylpiperidin-4-yl)-butyl]-2-(pyridin-3-yloxy)-acetamide (substance 123)

5.0 g (32.6 mmol) 3-pyridyloxyacetic acid and 3.95 g (39.1 mmol) TEA are suspended in 200 ml abs. dichloromethane and cooled to ca. 0°C under moisture exclusion. 6.34 g (41.3 mmol) 88 % HOBT and 7.49 g (39.1 mmol) EDC are added and the mixture is stirred 30 min under ice cooling. 11.56 g (35.9 mmol) N-4-(1-diphenylmethylpiperidine-4-yl)-butylamine are dissolved in 50 ml abs. dichloromethane and added dropwise under ice cooling. The mixture is stirred without further cooling at RT overnight. Subsequently, the batch is washed once with 50 ml 1M NaOH and twice each with 70 ml water. The organic phase is dried over sodium sulfate and the solvent is removed under vacuum. The resinous residue is chromatographically purified over silica gel with CHCl₃/CH₃OH (95/5 to 90/10) and crystallized from 30 ml acetic acid ethyl ester after drawing off the solvent. Colorless crystals with a MP of 103 - 105°C were recovered; yield 3.45 g (23%).

| C₂₉H₃₅N₃O₂ (457.6) | | |
|---|---|---|
| IR-Spectrum (KBr): | ν(NH) | 3360 cm⁻¹ |
| | ν(C=O) | 1660, 1545 cm⁻¹ |

| | |
|---|---|
| ¹H-NMR-Spectrum (CDCl₃): | 0.95 - 2.05 (13H, m, piperidine, piperidine-CH₂)₃) |
| | 2.70 - 3.00 (2H, m, piperidine) |
| | 3.34 (2H, dt, CONHCH₂, J=6.5 Hz, J=12.9 Hz) |
| | 4.21 (1H, s, Ar₂CH) |
| | 4.51 (2H, s, COCH₂O) |
| | 6.40 - 6.70 (1H, m, NH) |
| | 7.00 - 7.60 (12H, m, Ar, py) |
| | 8.20 - 8.45 (2H, m, py) |

### Example 13

### N-[5-(1-diphenylmethylpiperidin-4-yl)-pentyl]-3-(pyridin-3-yl)-propionamide (Substance 136)

2.47 g (16.3 mmol) 3-(3-pyridyl)-priopionic acid are suspended in 40 ml abs. dichloromethane and, after addition of three drops of pyridine, cooled to ca. 0°C in an ice bath under moisture exclusion. 1.90 ml (22.3 mmol) oxalyl chloride are added slowly and the mixture is first stirred under ice cooling for 30 minutes and then at RT overnight. Subsequently, the solvent and excess oxalyl chloride is distilled off in a rotary evaporator. In order to completely remove the oxalyl chloride, the colorless residue is further dried for two hours under high-vacuum. The acid chloride obtained in this manner is suspended in 50 ml abs. dichloromethane and cooled to ca. 0°C in an ice bath under moisture exclusion without further purification. 5.0 g (14.8 mmol) 5-(1-diphenylmethylpiperidin-4-yl)-pentylamine are dissolved in 40 ml abs. dichloromethane and added dropwise to this suspension. After complete addition, the ice bath is removed and the reaction mixture is stirred for a further two hours at RT. The mixture is subsequently concentrated, taken up in 10 % sodium hydroxide solution and extracted three times with acetic acid ethyl ester. The combined organic phases are washed with a saturated NaCl solution, dried over sodium sulfate and the solvent is removed in a vacuum. The residue is chromatographically purified over silica gel with CHCl₃/CH₃OH (96/4) and crystallized from 40 ml acetonitrile after drawing off the solvent. Colorless crystals with a MP of 112-114°C were recovered; yield: 3.5 g (50 %)

| C₃₁H₃₉N₃O (469.7) | | |
|---|---|---|
| IR-Spectrum (KBr): | ν(NH) | 3260 cm⁻¹ |
| | ν(C=O) | 1635, 1550 cm⁻¹ |

| | |
|---|---|
| ¹H-NMR-Spectrum (CDCl₃): | 0.90-2.00 (15H, m, piperidine, piperidine-CH₂)₄) |
| | 2.40 (2H, t, CH-CH₂, J=7.5) |
| | 2.70-3.10 (4H, m, piperidine, py, CH₂) |
| | 3.19 (2H, dt, CONHCH₂, J=6.6 Hz, J=12.6 Hz) |
| | 4.21 (1H, s, Ar₂CH) |
| | 5.30-5.60 (1H, m, NH) |
| | 7.00 - 7.75 (12H, m, Ar, py) |
| | 8.35 - 8.65 (2H, m, py) |

### Example 14

### N-[4-(1-diphenylmethylpiperidin-4-yl)-butyl]-3-(pyridin-3-yl)-propionamide (substance 94)

21.6 g (131 mmol) 3-(3-pyridyl)-propionic acid methyl ester, 35.1 g ml (109 mmol) 4-(1-diphenylmethylpiperidine-4-yl)-butylamine and 9.8 g (54.5 mmol) 30 % sodium methylate solution in methanol are heated to boiling in 480 ml toluenefor five hours. Subsequently, 30 ml of solvent are distilled; thereby, sodium methylate precipitates and the temperature of the suspension increases to 102°C under heavy foaming. The mixture is cooled to 70-80°C and extracted twice with 45 ml and 30 ml of water. The organic phase is azeotropically dried on a moisture separator and cooled to ca. 0°C. The resulting precipitate is filtered off and crystallized from 190 ml toluol. Colorless crystals with a MP of 139°C were recovered; yield 46.3 g (93%).

| C₃₀H₃₇N₃O (455.6) | | |
|---|---|---|
| IR-Spectrum (KBr): | ν(NH) | 3250 cm⁻¹ |
| | ν(C=O) | 1630, 1570 cm⁻¹ |

| | |
|---|---|
| ¹H-NMR-Spectrum (CDCl₃): | 1.00 - 2.10 (13H, m, piperidine, piperidine-CH₂)₃) |
| | 2.43 (2H, t, CO-CH₂, J=7.4 Hz) |
| | 2.70 - 3.10 (4H, m, py-CH₂) |
| | 3.12 (2H, dt,CONHCH₂, J=6.5 Hz, J=12.5 Hz) |
| | 4.21 (1H, s, Ar₂CH) |
| | 5.45 - 5.75 (1H, m, NH) |
| | 7.05 - 7.60 (12H, m, Ar, py) |
| | 8.30 - 8.60 (2H, m, py) |

### Example 15

### N-{4-[1-(6,11-dihydrodibenzo[b,e]-oxepin-11-yl)-piperidine-4-yl]-butyl}-3-(pyridin-3-yl)-propionamide (substance 220)

3.46 g (15 mmol) 11-chloro-6,11-dihydrodibenzo[b,e]oxepine are dissolved in 90 ml abs. dichloromethane and 5.43 g (15 mmol) N-(4-piperidin-4-yl-butyl)-3-(pyridin-3-yl)-propionamide dihydrochloride are added. 5.0 g (49.5 mmol) TEA are dissolved in 20 ml abs. dichloromethane and added dropwise under ice cooling. The mixture is stirred without further cooling for two days at RT. Subsequently, the batch is washed twice, each with 50 ml water. The organic phase is dried over silica gel with CHCl₃/CH₃OH (95/5) and crystallized twice at first, each from 10 ml 1-chlorobutane, and subsequently crystallized once from 10 ml acetic acid. Colorless crystals with a MP of 110-112°C were isolated; yield 0.2 g (3%).

| C₃₁H₃₇Cl₂N₃O₂ (483.6) | | |
|---|---|---|
| IR-Spectrum (KBr): | ν(NH) | 3240 cm⁻¹ |
| | ν(C=O) | 1630, 1570 cm⁻¹ |

| | |
|---|---|
| ¹H-NMR-Spectrum (CDCl₃): | 0.80 - 2.00 (13H, m, piperidine, piperidine-CH₂)₃) |
| | 2.43 (2H, t, CO-CH₂, J=7.5 Hz) |
| | 2.55 - 3.30 (6H, m, piperidine, py-CH₂, CONHCH₂) |
| | 3.83 (1H, s, Ar₂CH) |
| | 4.68 (1H, d, O-CH, J=11.3 Hz) |
| | 5.25 - 5.55 (1H, m, NH) |
| | 6.65 - 7.65 (11H, m, Ar, py, O-CH) |
| | 8.35 - 8.60 (2H, m, py) |

### Example 16

### N-{4-[1-(9H-fluorene)-piperidine-4-yl]-butyl}-3-(pyridin-3-yl)-propionamide (substance 199)

8.0 g (27.7 mmol) N-(4-piperidin-4-yl-butyl)-3-(pyridin-3-yl)-propionamide and 5.6 g (55.3 mmol) TEA are present in 100 ml acetonitrile and cooled to ca. 0°C under moisture exclusion. 6.8 g (27.7 mmol) 9-bromofluorene are added in solid form and the mixture is stirred for 2 days at ca. 65°C and for two days at RT. Subsequently, the solvent is drawn off under vacuum to a large extent and the residue is dispersed betweenCHCl₃ and 10 % NaOH. The organic phase is washed twice with water and dried over sodium sulfate. After the removal of the solvent, the residue is chromatographically purified over silica gel with CHCl₃/CH₃OH (98/2 to 94/6) and crystallized from 30 ml acetonitrile after drawing off the solvent. Colorless crystals with a MP of 131-132°C were recovered; yield 2.5 g (20%).

| C₃₀H₃₅N₃O (453.6) | | |
|---|---|---|
| IR-Spectrum (KBr): | ν(NH) | 3300 cm⁻¹ |
| | ν(C=O) | 1630, 1530 cm⁻¹ |

| | |
|---|---|
| ¹H-NMR-Spectrum (CDCl₃): | 0.95 - 1.80 (11H, m, piperidine, piperidine-CH₂)₃) |
| | 2.25 - 2.80 (6H, m, piperidine, CO-CH₂) |
| | 2.97 (2H, t, py-CH₂, J=7.5 Hz) |
| | 3.19 (2H, dt, CONHCH₂, J=6.5 Hz, J=12.5 Hz) |
| | 4.82 (1H, s, ArCH) |
| | 5.25-5.55 (1H, m, NH) |
| | 7.10-7.80 (10H, m, Ar, py,) |
| | 8.35 - 8.55 (2H, m, py) |

### Example 17

### N-{4-[1-(2-naphthylsulfonyl)-piperidin-4-yl]-butyl}-3-(pyridin-3-yl)-propionamide (substance 325)

3.5 g (12 mmol) N-(4-piperidin-4-yl-butyl)-3-(pyridin-3-yl)-propionamide and 6.7 g (48.1 mmol) TEA are present in 100 ml abs. dichloromethane and cooled to ca. 0°C under moisture exclusion. 3.0 g (13.2 mmol) naphthaline-2-sulfonic acid chloride are dissolved in 40 ml abs. dichloromethane and added dropwise. The mixture is stirred without further cooling at RT overnight. Subsequently, the batch is washed twice, each with 80 ml water. The organic phase is dried over sodium sulfate and the solvent is removed under vacuum. The residue is chromatographically purified over silica gel with CHCl₃/CH₃OH (97/3) and crystallized from acetic acid ethyl ester after drawing off the solvent. Colorless crystals with a MP of 103-105°C were recovered; yield 2.87 g (50%).

| C₂₇H₃₃N₃O₃S (479.6) | | |
|---|---|---|
| IR-Spectrum (KBr): | ν(NH) | 3320 cm⁻¹ |
| | ν(C=O) | 1645, 1530 cm⁻¹ |

| | |
|---|---|
| ¹H-NMR-Spectrum (CDCl₃): | 0.90 - 1.90 (11H, m, piperidine, piperidine-CH₂)₃) |
| | 2.05 - 2.40 (2H, m, piperidine) |
| | 2.42 (2H, t, CO-CH₂, J=7.4 Hz) |
| | 2.80 - 3.30 (4H, t, dt, Py-CH₂, J=7.4 Hz, CONHCH₂) |
| | 3.70 - 4.00 (2H, m, piperidine) |
| | 5.40 - 5.70 (1H, m, NH) |
| | 7.10 - 8.15 (8H, m, Ar, Py,) |
| | 8.25 - 8.55 (3H, m, Ar, Py) |

### Example 18

### N-{4-[1-(naphthylaminocarbonyl)-piperidin-4-yl]-butyl}-3-(pyridin-3-yl)-propionamide (substance 293)

2.6 g (17.7 mmol)1-naphthyl isocyanate are dissolved in 15 ml abs. THF and cooled to 0°C under moisture exclusion. 5.1 g (17.7 mmol) N-(4-piperidine-4-yl-butyl)-3-(pyridine-3-yl)-propionamide are dissolved in 35 ml abs. THF and added dropwise under ice cooling. The mixture is stirred without further cooling at RT overnight. Subsequently, the solution is drawn off under vacuum to a large extent and the residue is chromatographically purified over silica gel with CHCl₃/CH₃OH (90/10) and further purified by flash-chromatography with CHCl₃/CH₃OH (95/5 to 90/10). After drawing off the solvent, crystallization occurs from isopropanol/diisopropanol. Colorless crystals with a MP of 143-144°C were recovered; yield 0.77 g (9%).

| C₂₈H₃₄N₄O₂ (458.6) | | |
|---|---|---|
| IR-Spectrum (KBr): | ν(NH) | 3240 cm⁻¹ |
| | ν(C=O) | 1630, 1560 cm⁻¹ |

| | |
|---|---|
| ¹H-NMR-Spectrum (CDCl₃): | 0.95 - 1.95 (11H, m, piperidine, piperidine-CH₂)₃) |
| | 2.40 (2H, t, CO-CH₂, J=7.4 Hz) |
| | 2.75 - 3.40 (6H, m, piperidine, Py-CH₂, CONHCH₂) |
| | 4.00 - 4.30 (2H, m, piperidine) |
| | 5.55 - 5.85 (1H, m, NH) |
| | 6.77 (1H, s, NH) |
| | 7.10 - 8.00 (9H, m, Ar, Py,) |
| | 8.35 - 8.55 (2H, m, Ar, Py) |

### Example 19

### N-{4-[1-(2-naphthoyl)-piperidin-4-yl]-butyl}-3-(pyridin-3-yl)-propionamide (substance 262)

6.0 g (20.7 mmol) N-(4-piperidine-4-yl-butyl)-3-(pyridine-3-yl)-propionamide and 2.1 g (20.7 mmol) TEA are dissolved in 30 ml abs. dichloromethane and cooled to ca. 0°C under moisture extraction. 3.95 g (20.7 mmol) 2-naphthoylchloride are dissolved in 40 ml abs. dichloromethane and added dropwise under ice cooling. The mixture is stirred without further cooling at RT overnight. Subsequently, the batch is made basic by the addition of 10 % sodium hydroxide solution and washed twice with a small amount of water. The organic phase is dried over sodium sulfate and the solvent is removed under vacuum. The resinous residue is chromatographically purified with CHCl₃/CH₃OH (96/4). Yield of colorless resin: 5.4 g (59%).

| C₂₈H₃₃N₃O₂ (443.6) | | |
|---|---|---|
| IR-Spectrum (KBr): | n(NH) 3300 cm⁻¹ n(C=O) | 1630, 1540 cm⁻¹ |

| | |
|---|---|
| ¹H-NMR-Spectrum (CDCl₃): | 1.00-2.05 (11H, m, piperidin, |
| | piperidine-(CH₂)₃) |
| | 2.55 (2H, t, CH-CH₂, J=7.5 Hz) |
| | 2.70 - 3.45 (6H, m, piperidine, Py-CH₂, CONHCH₂) |
| | 3.65 - 4.15 (1H, m, piperidin) |
| | 4.50 - 5.05 (1H, m, piperidine) |
| | 5.60 - 5.85 (1H, m, NH) |
| | 7.20 - 7.35 (1H, m, Py) |
| | 7.50 - 7.75 (4H, m, Ar, Py) |
| | 7.85 - 8.10(4H, m, Ar) |
| | 8.40 - 8.65 (2H, m, Py) |

### Example 20/1

### N-(4-piperidin-4-yl-butyl)-3-(pyridin-3-yl)-propionamide (substance 21)

100 g (219.5 mmol) N- [4-(1-diphenylmethylpiperidine-4-yl)-butyl]-3-(pyridine-3-yl)-propionamide (substance 100) are dissolved in 500 ml ethanol and mixed with 8.0 g palladium (5%) on activated carbon (moistened with 40 ml water) and 25 ml conc. hydrochloric acid. The mixture is heated to ca. 45°C and stirred under hydrogen atomosphere until consumption of the theoretical amount of hydrogen to be taken up (ca. five hours). After cooling, the catalyst is filtered and the solvent is removed under vacuum. The residue is taken up in 200 ml water and washed three times with a total of 200 ml CHCl₃. The organic phases are discarded and the aqueous phase is made alkaline with 11 g sodium hydroxide and extracted three times, each with 100 ml CHCl₃. After washing the organic phase with 30 ml water, the solvent is removed under vacuum. The oily residue is filtered over silica gel with CHCl₃/CH₃OH/NH₄OH (80/20/2). Yield of the gradually hardening resin: 53.0 g (83%).
For spectroscopic data, see Example 20/2.

### Example 20/2

### N-(4-piperidin-4-yl-butyl)-3-(pyridin-3-yl)-propionamide (substance 21)

100 g (219.5 mmol) N- [4-(1-diphenylmethylpiperidine-4-yl)-butyl]-3-(pyridine-3-yl)-acrylamide (Substance 104) are dissolved in 500 ml ethanol and mixed with 8.0 g palladium (5%) on activated carbon (moistened with 40 ml water) and 25 ml conc. hydrochloric acid. The mixture is heated to ca. 45°C and stirred under hydrogen atomosphere until consumption of the theoretical amount of hydrogen to be taken up (ca. 1 day). After cooling, the catalyst is filtered and the solvent is removed under vacuum. The residue is taken up in 400 ml water and washed twice, each with 100 ml toluol. The organic phases are discarded and the aqueous phase is made alkaline with 400 ml 4M-sodium hydroxide solution and extracted three times each with 200 ml dichloromethane. The combined organic phases are dried over sodium sulfate and the solvent is removed under vacuum. The wax-like residue is filtered over silica gel with CHCl₃/CH₃OH/NH₄OH (90/9/1). Yield: 58.3 g (91%).

| C₂₈H₃₃N₃O₂ (443.6) | | |
|---|---|---|
| IR-Spectrum (KBr): | ν(NH) | 3300 cm⁻¹ |
| | ν(C=O) | 1630, 1540 cm⁻¹ |

| | |
|---|---|
| ¹H-NMR-Spectrum (CDCl₃): | 0.90 - 1.80 (12H, m, piperidin, NH |
| | piperidine-(CH₂)₃) |
| | 2.35 - 2.75 (4H, m, CO-CH₂, piperidine) |
| | 2.80 - 3.35 (6H, m, piperidine, Py-CH₂, CONHCH₂) |
| | 6.05 - 6.40 (1H, m, NH) |
| | 7.10 - 7.35 (1H, m, Py) |
| | 7.40 - 7.60 (1H, m, Py) |
| | 7.20 - 7.35 (1H, m, Py) |
| | 8.30 - 8.55 (2H, m, Py) |

### Example 21

### N-{4-[1-(6,11-dihydrodibenzo[b,e]thiepin-11-yl)-piperidine-4-yl]-butyl}-3-(pyridin-3-yl)-acrylamide (substance 223)

7.02 g (21.5 mmol) N- [4-piperidine-4-yl)-butyl]-3-(pyridine-3-yl)-acrylamide dihydrochloride (substance 22 as dihydrochloride) are suspended in 100 ml abs. dichloromethane and mixed with 7.08 g (70.0 mmol) TEA. The mixture is cooled to ca. 0°C under moisture exclusion and a solution of 5.30 g (21.5 mmol) 11-chloro-6,11-dihydrodibenzo[b,e]thiepine in 10 ml abs. dichloromethane is added dropwise. The mixture is stirred without further cooling for 24 hours at RT. Subsequently, the batch is washed with 50 ml 10 % sodium hydroxide solution and 30 ml water. The organic phase is dried over sodium sulfate and the solution is removed under vacuum. The red-brown residue is chromatographically purified three times over silica gel with CHCl₃/CH₃OH (100/0, 97/3 and 96/4 to 94/6). Subsequently, further purification occurs by means of MPLC with CHCl₃/CH₃OH (98/2). yield: 0.5 g (5%) of a brittle vitreous solid with a MP of 89-91°C.

| C₃₁H₃₅Cl₂N₃OS (497.7) | | |
|---|---|---|
| IR-Spectrum (KBr): | ν(NH) | 3280 cm⁻¹ |
| | ν(C=O) | 1660, 1550 cm⁻¹ |
| | ν(C=C) | 1620 cm⁻¹ |

| | |
|---|---|
| ¹H-NMR-Spectrum (CDCl₃): | 0.90 - 2.00 (13H, m, piperidine, piperidine-CH₂)₃) |
| | 2.55 - 2.95 (2H, m, piperidine) |
| | 3.20 - 3.60 (3H, m, CONHCH₂, SCH₂) |
| | 4.03 (1H, s, Ar₂CH) |
| | 6.10 - 6.35 (1H, m, NH) |
| | 5.95 - 6.30 (1H, m, SCH₂) |
| | 6.44 (1H, d, CH=CHO J=15.7 Hz) |
| | 4.68 (1H, d, O-CH, J=11.3 Hz) |
| | 6.85 - 7.40 (9H, m, Ar, Py) |
| | 8.50 - 8.65 (1H, m, Py) |
| | 8.65 - 8.80 (1H, m, Py) |

### Example 22

### N-[4-(1-diphenylmethylpiperidine-4-yl)-butyl]-5-(pyridin-3-yl)-2,4-pentadienoic acid amide (substance 126)

3.85 g (22.0 mmol) 5- (3-pyridyl)-2,4-pentadieneoic acid are suspended in 90 ml abs. dichloromethane and, after addition of three drops of pyridine, cooled to ca. 0°C in an ice bath under moisture exclusion. 3.8 g (30,0 mmol) oxalyl chloride are added dropwise and the mixture is stirred at RT overnight. Subsequently, the solvent and excess oxalyl chloride are distilled off on a rotary evaporator. In order to completely remove the oxalyl chloride, the residue is dried for a further two hours under high-vacuum. The acid chloride obtained in this manner is suspended in 50 ml abs. dichloromethane and cooled to ca. 0°C. 6.44 g (20.0 mmol) 4-(1-diphenylmethylpiperidine-4-yl)-butylamine are dissolved in 40 ml abs. dichloromethane and added dropwise to the suspension. After complete addition, the ice bath is removed and the reaction is stirred for a further two hours at RT. The mixture is subsequently washed with 10 % sodium hydroxide solution. The organic phase is washed twice, each with 40 ml water, dried over sodium sulfate and the solution is removed under vacuum. The residue is chromatographically purified three times over silica gel with CHCl₃/CH₃OH (98/2 to 95/5) and crystallized twice from 250 ml acetonitrile after removal of the solvent. Beige-colored crystals with a MP of 164-166°C are isolated; yield: 4.7 g (49 %).

| C₃₂H₃₇N₃O (479.6) | | |
|---|---|---|
| IR-Spectrum (KBr): | ν(NH) | 3280 cm⁻¹ |
| | ν(C=O) | 1650, 1550 cm⁻¹ |
| | ν(C=C) | 1600 cm⁻¹ |

| | |
|---|---|
| ¹H-NMR-Spectrum (CDCl₃): | 1.00 - 2.00 (13H, m, piperidine, piperidine-CH₂)₃) |
| | 2.70 - 3.00 (2H, m, piperidine) |
| | 3.34 (2H, dt, CONHCH₂, J=6.6 Hz, J=12.8 Hz) |
| | 4.21 (1H, s, Ar₂CH) |
| | 5.50 - 5.75 (1H, m, NH) |
| | 6.44 (1H, d, CH=CH J=14.7 Hz) |
| | 6.75 - 6.95 (2H, m, CH=CH) |
| | 7.05 - 7.50 (12H, m, Ar, Py, CH=CH) |
| | 7.65 - 7.85 (1H, m, Py) |
| | 8.45 - 8.55 (1H, m, Py) |
| | 8.60 - 8.75 (1H, m, Py) |

### Example 23

### N-[4-(1-benzoylpiperidine-4-yl)-butyl]-3-(pyridin-3-yl)-acrylamide (Substance 249)

5.1 g (36.2 mmol) benzoyl chloride are dissolved in 150 ml abs. dichloromethane and cooled to ca. 0°C under moisture exclusion. 10.4 g (36.2 mmol) N-[4-piperidine-4-yl)-butyl]-3-(pyridin-3-yl)-acrylamide (Substance 22) are dissolved in 50 ml abs. dichloromethane and added dropwise under ice cooling. The mixture is stirred without further cooling at RT overnight. Subsequently, the suspension is added to 60 ml sodium hydroxide solution and extracted twice, each with 80 ml dichloromethane. The combined organic phases are washed twice, each with 60 ml water, dried over sodium sulfate and the solution is removed under vacuum. The residue is chromatographically purified three times over silica gel with CHCl₃/CH₃OH (97/3 to 95/5) and crystallized from 75 ml acetonitrile. Colorless crystals with a MP of 100-102°C were recovered; yield: 9.8 g(69 %).

| C₂₄H₂₉N₃O₂ (391.5) | | |
|---|---|---|
| IR-Spectrum (KBr): | ν(NH) | 3280 cm⁻¹ |
| | ν(C=O) | 1670, 1545 cm⁻¹ |
| | ν(C=C) | 1630 cm⁻¹ |

| | |
|---|---|
| ¹H-NMR-Spectrum (CDCl₃): | 0.80 - 2.00 (11H, m, piperidine, piperidine-CH₂)₃) |
| | 2.55 - 4.00 (5H, m, piperidine, CONHCH₂) |
| | 4.40-4.90 (1H, piperidine) |
| | 6.00 - 6.25 (1H, m, NH) |
| | 6.48 (1H, d, CH=CHCO, J=15.7 Hz) |
| | 8.50 - 8.65 (1H, m, Py) |
| | 8.65 - 8.80 (1H, m, Py) |

### Example 24/

### N-(1-diphenylmethylazetidin-3-ylmethyl)-3-(pyridin-3-yl)-acrylamide (substance 2)

Production occurred analogously to Example 22.

Batch size: 4.3 g (28.7 mmol) 3-(3-pyridyl)-acrylic acid, 6.7 ml (78.4 mmol) oxalyl chloride and 6.6 g (26.1 mmol) 3-(1-diphenylmethylazetidine-3-ylmethyl)-amine.

In the work up, the reaction mixture is washed with sodium hydroxide solution. The aqueous phase is extracted twice, each with 50 ml dichloromethane. The combined organic phases are dried over sodium sulfate and the solvent is removed under vacuum. The residue is chromatigraphically pre-purified over silica gel with CHCl₃/CH₃OH (98/2 to 95/5) and subsequently purified twice by flash chromatography with CHCl₃/CH₃OH (99/1 to 95/5). An amorphous solid with a MP of 72-74°C remains after the removal of the solvent; yield 0.75 g (7%).

| C₂₅H₂₅N₃O (383.5) | | |
|---|---|---|
| IR-Spectrum (KBr): | ν(NH) | 3320 cm⁻¹ |
| | ν(C=O) | 1680, 1570 cm⁻¹ |
| | ν(C=C) | 1640 cm⁻¹ |

| | |
|---|---|
| ¹H-NMR-Spectrum (CDCl₃): | 2.40 - 2.80 (1H, m, azetidine) |
| | 2.80 - 3.10 (2H, m, azetidine) |
| | 3.10 - 3.40 (2H, m, azetidine) |
| | 3.60 (2H, dd, CONCH₂ J=6.5 Hz, J=12.8 Hz) |
| | 436 (1H, Ar₂CH) |
| | 6.45 - 6.75 (1H, m, NH) |
| | 6.50 (1H, d, CH=CHCO, J=15.7 Hz) |
| | 7.00 - 7.50 (11H, m, Ar, Py) |
| | 7.62 (1H, d, CH=CHCO, J=15.7 Hz) |
| | 7.65 - 7.90 (1H, m, Py) |
| | 8.50 - 8.70 (1H, m, Py) |
| | 8.70 - 8.85 (1H, m, Py) |

### Example 25

### N-(4-diphenylmethylmorpholin-2-ylmethyl)-3-(pyridin-3-yl)-acrylamide (substance 366)

Production occurred analogously to Example 22.

Batch size: 2.3 g (15.6 mmol) 3-(3-pyridyl)-acrylic acid, 5.4 g (42.5 mmol) oxalyl chloride and 3.6 g (14.7 mmol) 2-aminomethyl-4-diphenylmethylmorpholine.

In the work up, 40 ml 10 % sodium hydroxide solution are added to the reaction solution. The aqueous phase is extracted with 15 ml dichloromethane. The combined organic phases are washed twice, each with 15 ml water, dried over sodium sulfate and the solvent is removed under vacuum. The residue is chromatigraphically purified three times over silica gel with CHCl₃/CH₃OH (95/5, 90/10 and 90/10). An amorphous solid with a MP of 71-74°C remains after the removal of the solvent; yield 0.8 g (13%).

| C₂₆H₂₇N₃O₂ (413.5) | | |
|---|---|---|
| IR-Spectrum (KBr): | ν(NH) | 3370 cm⁻¹ |
| | ν(C=O) | 1655, 1540 cm⁻¹ |
| | ν(C=C) | 1620 cm⁻¹ |

| | |
|---|---|
| ¹H-NMR-Spectrum (CDCl₃): | 1.70 - 2.30 (2H, m, morpholine) |
| | 2.55 - 2.90 (2H, m, morpholine) |
| | 3.00 - 3.35 (1H, m, morpholine) |
| | 3.50 - 4.00 (4H, m, CONHCH₂, morpholine) |
| | 4.20 (1H, Ar₂CH) |
| | 6.00 - 6.25 (1H, m, NH) |
| | 6.47 (1H, d, CH=CHCO, J=15.7 Hz) |
| | 4.36 (1H, Ar₂CH) |
| | 7.60 (1H, d, CH=CHCO, J=15.7 Hz) |
| | 7.00 - 7.55 (11H, m, Ar, Py) |
| | 7.60 (1H, d, CH=CHCO, J=15.7 Hz) |
| | 7.65 - 7.90 (1H, m, Py) |
| | 8.50 - 8.70 (1H, m, Py) |
| | 8.70 - 8.80 (1H, m, Py) |

### Example 26

### N-{4-[1-(9-oxo-9H-fluorene-4-carbonyl)-piperidine-4-yl]-butyl}-3-(pyridin-3-yl)-acrylamide (substance 265)

5.0 g (20.0 mmol) 95 % 9-fluorene-1-acyl chloride were dissolved in 70 ml abs. dichloromethane and 6.5 g (18.2 mmol) N-[4-(piperidine-4-yl)-butyl]-3-(pyridine-3-yl)-acrylamide dihydrochloride (substance 22) are added. The mixture is cooled to ca. 0°C under moisture exclusion and 4.0 g (40.0 mmol) TEA dissolved in 10 ml abs. dichloromethane is added dropwise. The batch is stirred without cooling at RT overnight. In the work up, 150 ml 10 % sodium hydroxide solution are added to the reaction solution and extracted by shaking. The organic phase is washed with 100 ml water, dried over sodium sulfate and the solution is removed under vacuum. The residue is pre-purified over silica gel with CHCl₃/CH₃OH (96/4 to 95/5) and subsequently purified by flash chromatography with CHCl₃/CH₃OH (95/5). The product remains as a yellow, vitreous solid with MP of 80-82°C after removal of the solvent; yield: 2.3 g (25%)

| C₃₁H₃₁N₃O₃ (493.6) | | |
|---|---|---|
| IR-Spectrum (KBr): | ν(NH) | 3320 cm⁻¹ |
| | ν(C=O) | 1730, 1640 cm⁻¹ |
| | ν(C=C) | 1620 cm⁻¹ |

| | |
|---|---|
| ¹H-NMR-Spectrum (CDCl₃): | 0.70 - 2.05 (11H, m, piperidine, piperidine-CH₂)₃) |
| | 2.60 - 3.80 (5H, m, piperidine, CONHCH₂) |
| | 4.70 - 5.05 (1H, piperidine) |
| | 5.85 - 6.20 (1H, m, NH) |
| | 6.47 (1H, d, CH=CHO J=15.7 Hz) |
| | 7.15 - 7.90 (10H, m, Ar, PyCH=CHCO, J=15.7 Hz) |
| | 8.50 - 8.65 (1H, m, Py) |
| | 8.65 - 8.85 (1H, m, Py) |

### Example 27

### N-[3-(1-benzylpiperidine-4-yloxy)-propyl]-3-(pyridin-3-yl)-acrylamide (substance 50)

2.4 g (16.2 mmol) 3-(3-pyridyl)-acrylic acid and 2.3 g (16.2 mmol) TEA were suspended in 50 ml abs. tolueneand a solution of 1.5 ml (15.5 mmol) chloroformic ethyl ester in 20 ml abs. tolueneis added dropwise under moisture exclusion and gentle cooling. This yellow suspension is stirred two hours at RT and then a solution of 3.5 g (14.1 mmol) 3-(1-benzylpiperidine-4-yloxy)-propylamine in 20 ml abs. tolueneis added dropwise. The mixture is stirred at RT and subsequently extracted by shaking in the heat three times with 10 ml water, 2M sodium hydroxide solution and again with water, respectively. The organic phase is concentrated under vacuum and the orange colored, oily residue is chromatographically purified twice over silica gel with CHCl₃/CH₃OH/NH₄OH (90/9/1 and 95/5/0 to 90/10/0) and crystallized twice from 10 ml acetic acid ethyl ester. Colorless crystals with a MP of 100-102°C were recovered; yield: 1.9 g (35 %).

| C₂₃H₂₉N₃O₂ (379.5) | | |
|---|---|---|
| IR-Spectrum (KBr): | ν(NH) | 3290 cm⁻¹ |
| | ν(C=O) | 1650, 1530 cm⁻¹ |
| | ν(C=C) | 1610 cm⁻¹ |

| | |
|---|---|
| ¹H-NMR-Spectrum (CDCl₃): | 1.50 - 2.45 (8H, m, piperidine, C-CH₂-C) |
| | 2.70 - 3.00 (2H, m, piperidine) |
| | 3.25 - 3.80 (7H, m, piperidine, CONHCH₂, Ar-CH₂, O-CH₂) |
| | 6.54 (1H, d, CH=CHCO, J=15.7 Hz) |
| | 7.25 - 7.50 (6H, m, Ar, Py) |
| | 7.69 (1H, d, CH=CHCO, J=15.7 Hz) |
| | 7.80 - 8.00 (1H, m, Py) |
| | 8.60 - 8.75 (1H, m, Py) |
| | 8.75 - 8.90 (1H, m, Py) |

### Example 28

### N-[4-(1-benzoylpiperidine-3-yl)-butyl]-3-(pyridin-3-yl)-acrylamide (substance 51)

Production occurred analogously to Example 27.

Batch size: 2.6 g (17.4 mmol) 3-(3-pyridyl)-acrylic acid, 1.6 ml (19.0 mmol) oxalyl chloride and 3.9 g (15.8 mmol) 4-(1-benzlypiperidine-3-yl)-butylamine in 100 ml abs. dichloromethane.

The reaction time is increased to 6 hours at RT. In the work up, the batch is washed with 50 ml sodium hydroxide solution and the aqueous phase is extracted with 50 ml dichloromethane. The combined organic phases are concentrated under vacuum and the residue is chromatigraphically purified twice over silica gel with CHCl₃/CH₃OH (93/7 and 95/5), subsequently further purified by flash chromatography with CHCl₃/CH₃OH, 95/5 and 97/3) and crystallized from 5 ml acetic acid ethyl ester. Colorless crystals with a MP of 80-82°C were recovered; yield 0.9 g (15%).

| C₂₄H₃₁N₃O (377.5) | | |
|---|---|---|
| IR-Spectrum (KBr): | ν(NH) | 3300 cm⁻¹ |
| | ν(C=O) | 1650, 1530 cm⁻¹ |
| | ν(C=C) | 1610 cm⁻¹ |

| | |
|---|---|
| ¹H-NMR-Spectrum (CDCl₃): | 1.00 - 2.10 (13H, m, piperidine, piperidine-CH₂)₃) |
| | 2.65 - 2.95 (2H, m, piperidine) |
| | 3.37 (2H, dt, CONHCH₂, J= 6.5 Hz, J= 12.7 Hz) |
| | 3.50 (2H, s, Ar-CH₂) |
| | 5.65 - 5.95 (1H, m NH) |
| | 6.46 (1H, d, CH=CHCO, J=15.6 Hz) |
| | 7.10-7.40(6H, m, Ar, Py) |
| | 7.62 (1H, d, CH=CHCO, J=15.6 Hz) |
| | 7.65 - 7.90 (1H, m, Py) |
| | 8.50 - 8.65 (1H, m, Py) |
| | 8.70 - 8.80 (1H, m, Py) |

### Example 29

### N-[4-(1-tert-butoxycarbonylpiperidine-4-yl)-butyl]-3-(pyridin-3-yl)-acrylamide (substance 355)

Production occurred analogously to Example 22. TEA is also added dropwise with the addition of the amine.

Batch size: 16.4 g (110 mmol) 3-(3-pyridyl)-acrylic acid, 18.9 g (150 mmol) oxalyl chloride and 25.6 g (100 mmol) 4-(1-tert-butoxycarbonylpiperidine-4-yl)-butylamine and 10.1 g (100 mmol) TEA in 300 ml abs. dichloromethane.

In the work up, 100 ml 10 % sodium hydroxide solution are added to the reaction solution. The aqueous phase is extracted with 30 ml dichloromethane. The combined organic phases are washed twice, each with 25 ml water and the solution is removed under vacuum. The residue is dissolved in CHCl₃/CH₃OH (90/10) and filtered through a thin silica gel layer. The crude product remains as a red oil after the revomal of the solvent (44.0 g). For purification this is chromatographed with CHCl₃/CH₃OH, (95/5) on a silica gel; yield 26.5 g (68%) as a yellow viscous oil.

| C₂₂H₃₃N₃O₃ (387.50) | | |
|---|---|---|
| IR-Spectrum (KBr): | ν(NH) | 3250 cm⁻¹ |
| | ν(C=O) | 1670, 1540 cm⁻¹ |
| | ν(C=C) | 1600 cm⁻¹ |

| | |
|---|---|
| ¹H-NMR-Spectrum (CDCl₃): | 0.80 - 1.90 (20H, m, piperidine, piperidine-CH₂)₃, tert.butyl) |
| | 2.30 - 2.90 (2H, m, piperidine) |
| | 3.10-3.60 (2H, m, piperidine) |
| | 3.80 - 4.30 (2H, m, CONHCH₂) |
| | 6.15 - 6.55 (1H, m NH) |
| | 6.43 (1H, d, CH=CHCO, J=15.6 Hz) |
| | 3.50 (2H, s, Ar-CH₂) |
| | 8.35 - 8.55 (2H, m, Py) |
| | 8.55 - 8.70 (1H, m, Py) |

### Example 30

### N-[4-(piperidine-4-yl)-butyl]-3-(pyridin-3-yl)-acrylamide dihydrochloride (substance 22 as dihydrochloride)

44.0 g (<113.5 mmol) crude N-[4-(1-tert-butoxycarbonylpiperidine-4-yl)-butyl]-3-(pyridin-3-yl)-acrylamide (substance 367) are dissolved in 400 ml ethanol and added to 26.0 ml concentrated hydrochloric acid. The mixture is heated to boiling for three hours and the solvent is removed under vacuum after cooling. The yellow residue is crystallized from 500 ml isopropanol. Beige colored crystals with a MP of 178-188°C were recovered; yield: 32.6 g (90 %).

| C₁₇H₂₅N₃O₂ (360.3) | | |
|---|---|---|
| IR-Spectrum (KBr): | ν(NH) | 3260 cm⁻¹ |
| | ν(C=O) | 1670, 1545 cm⁻¹ |
| | ν(C=C) | 1630 cm⁻¹ |

| | |
|---|---|
| ¹H-NMR-Spectrum (CDCl₃): | 0.95 - 1.95 (11H, m, piperidine, piperidine CH₂)₃) |
| | 2.60 - 3.00 (2H, m, piperidine) |
| | 3.00 - 3.40 (4H, m, piperidine, CONHCH₂,) |
| | 6.73 (1H, d, CH=CHCO, J=15.9 Hz) |
| | 7.41 (1H, d, CH=CHCO, J=15.9 Hz). |
| | 7.80 - 8.00 (1H, m, Py) |
| | 8.50 - 8.65 (2H, m, Py) |
| | 8.65 - 8.90 (1H, m, Py) |

Further examples of the synthesized compounds are listed in the following Table 2, giving the structural features and melting points, for the further illustration of the compounds used according to the invention with the above characterized pharacological activities.

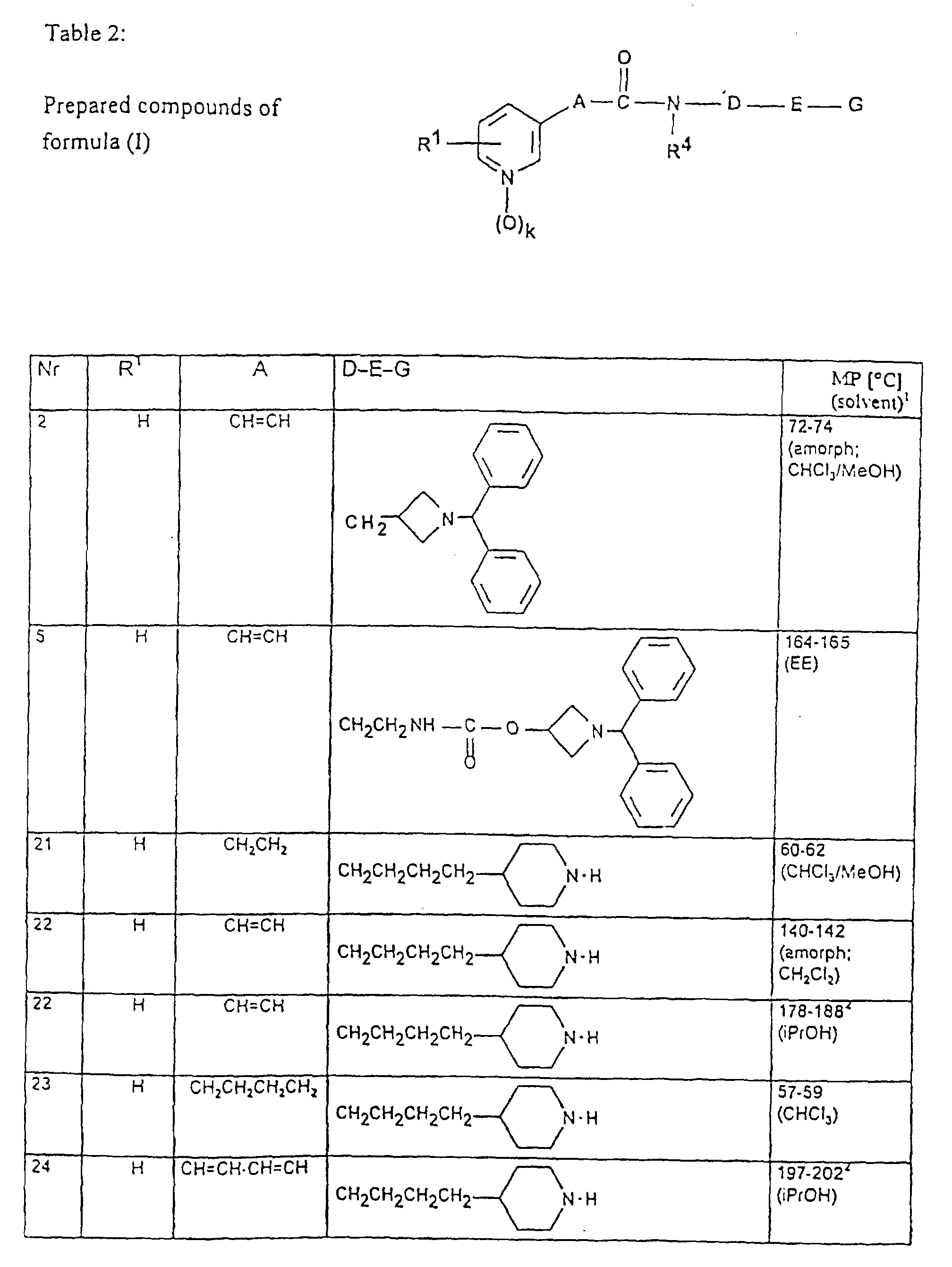

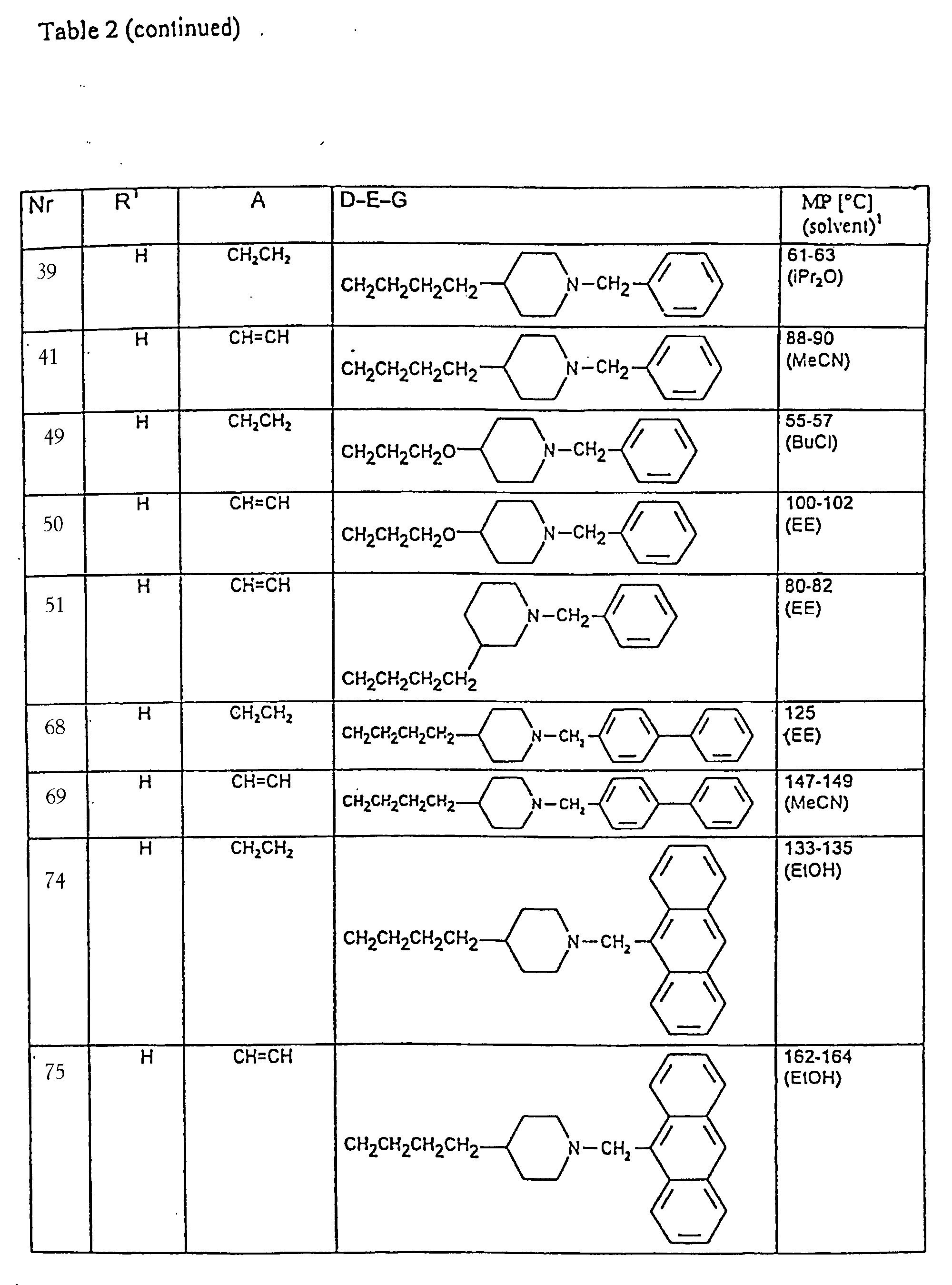

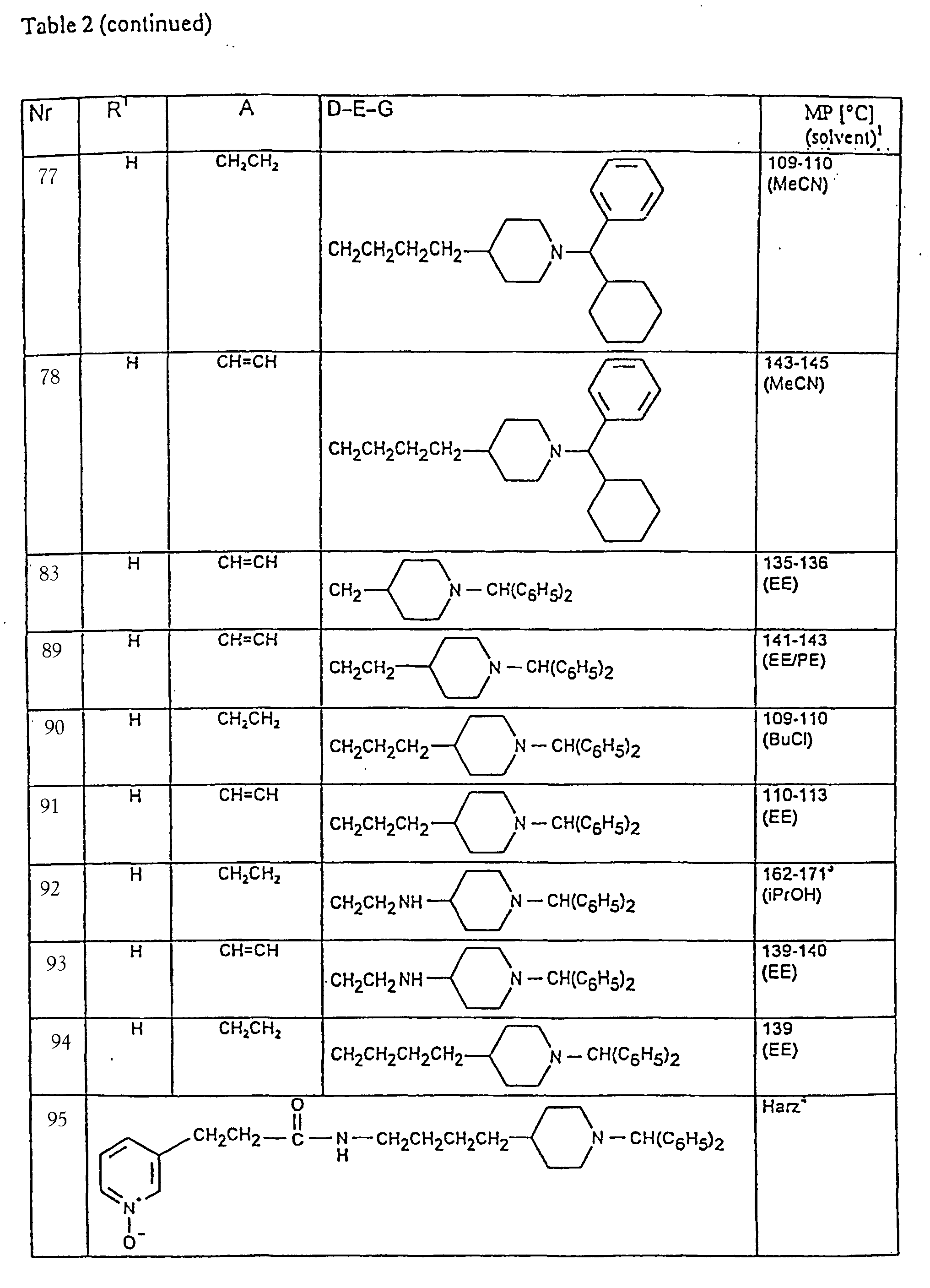

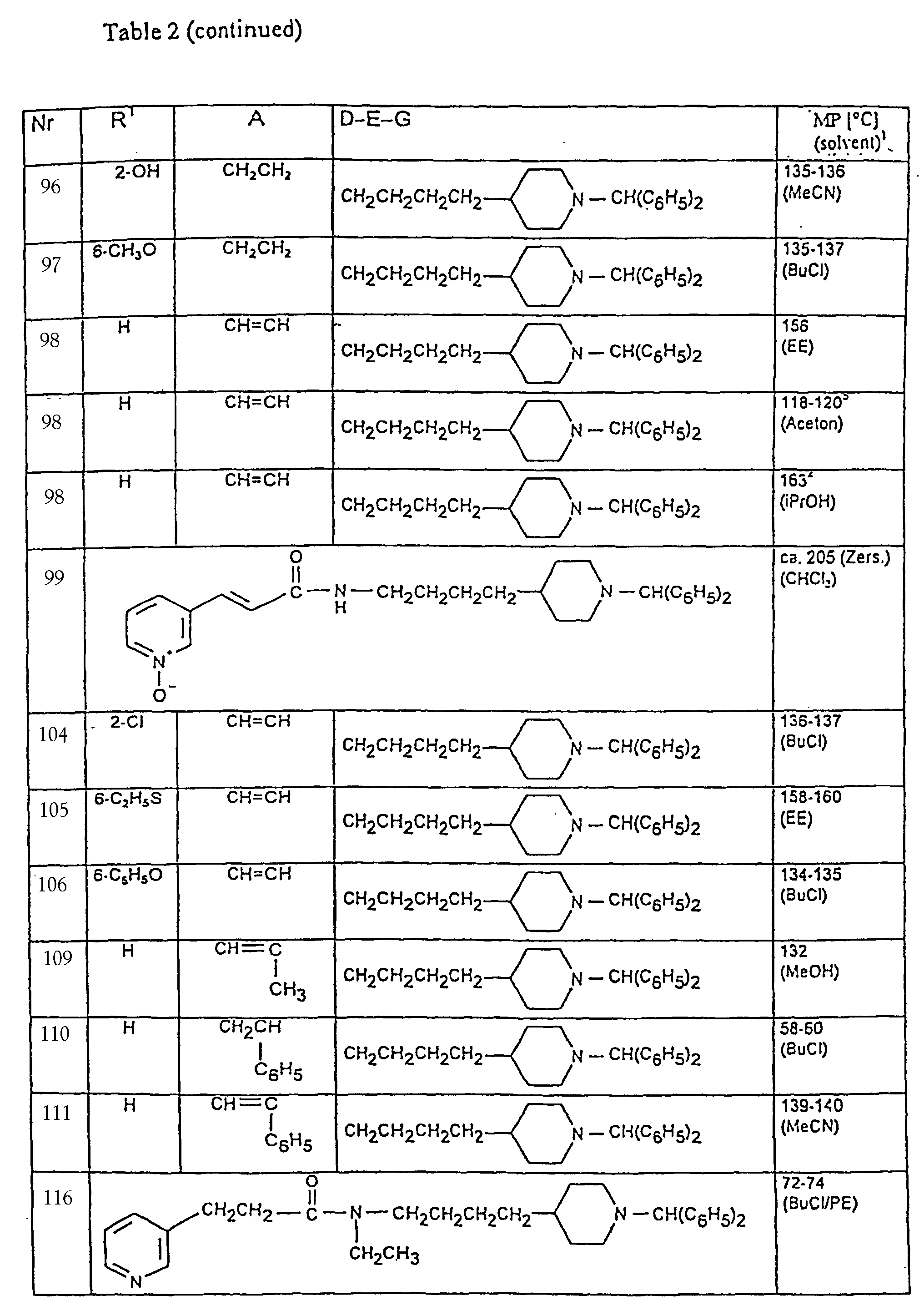

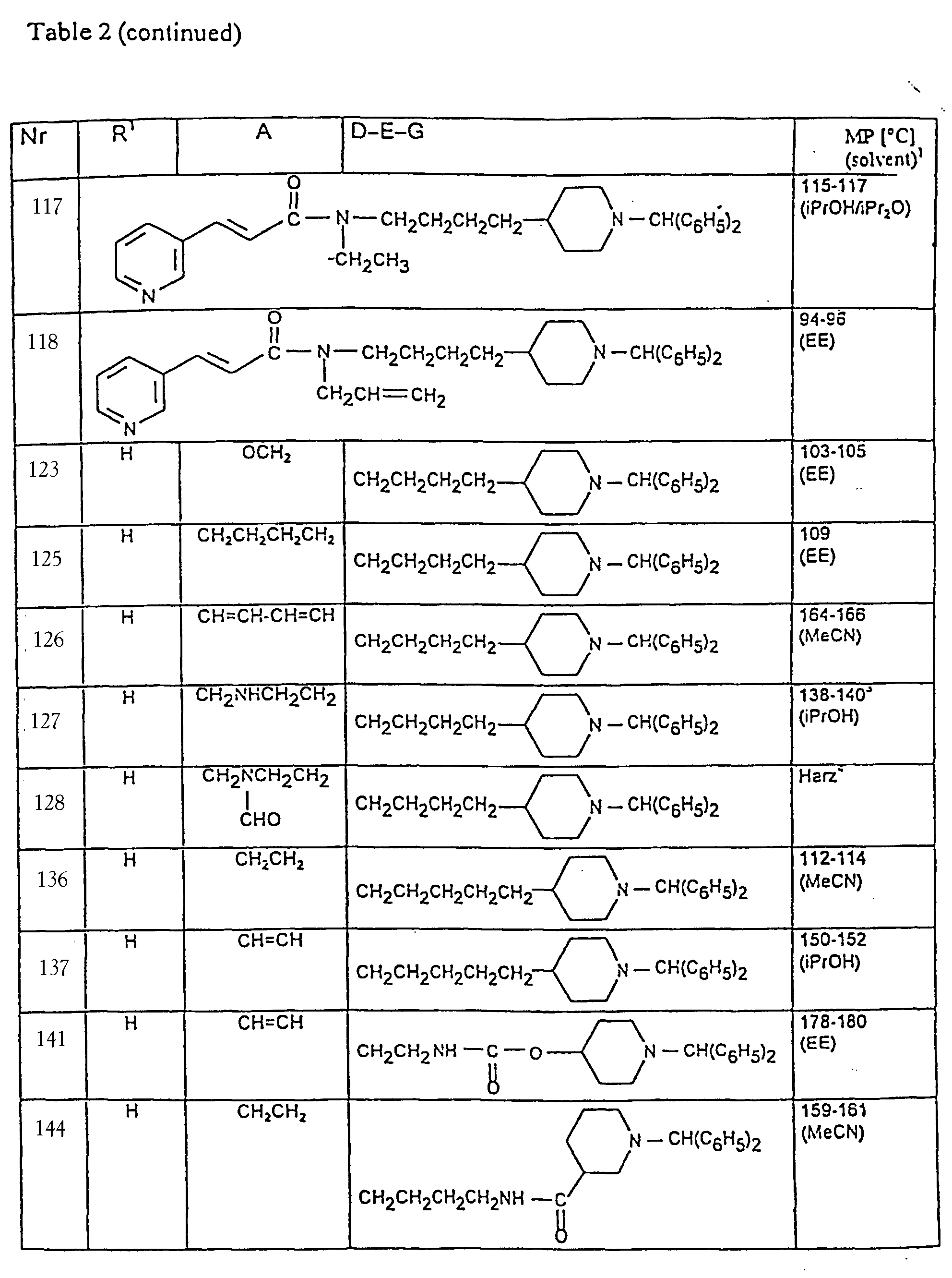

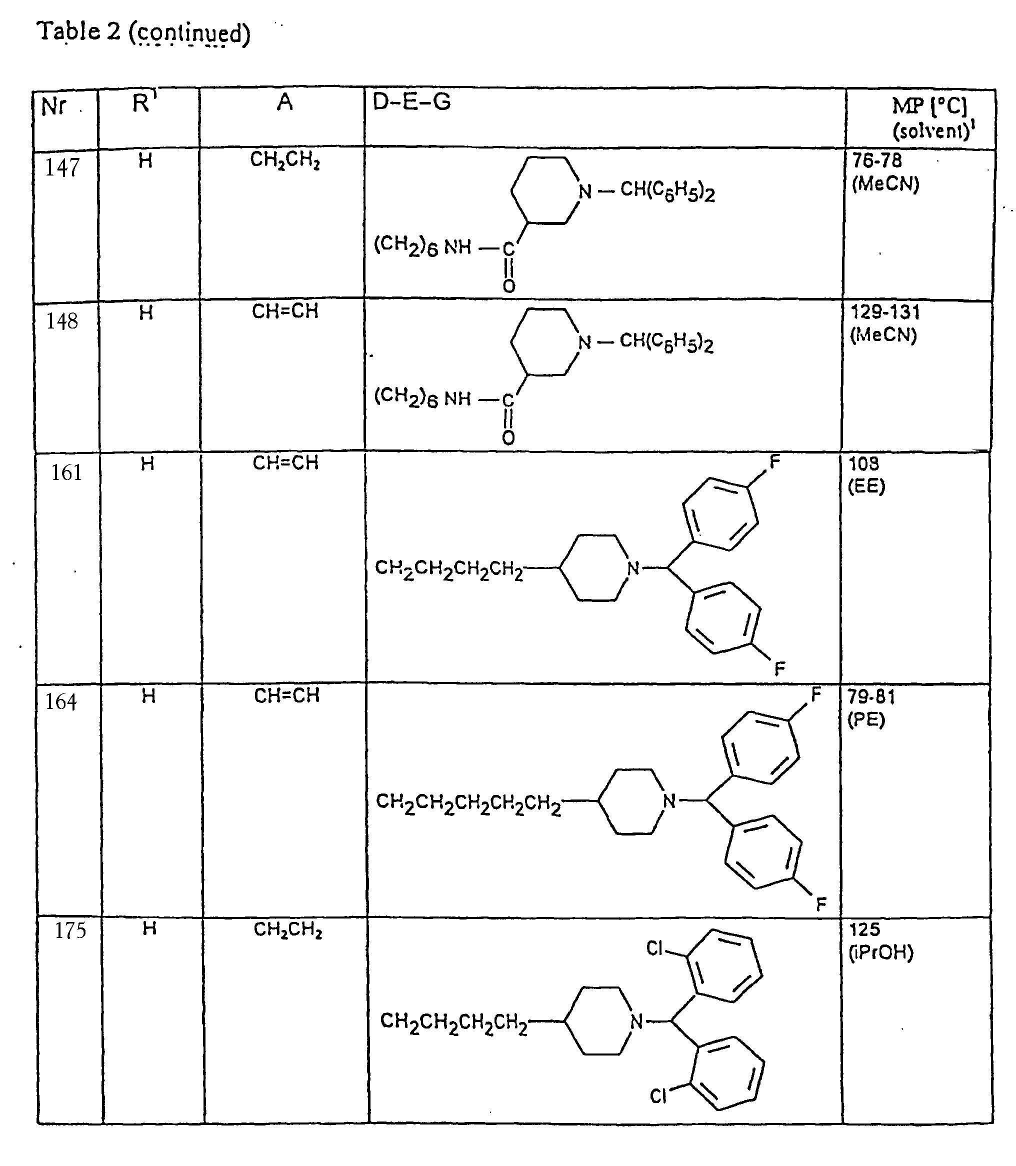

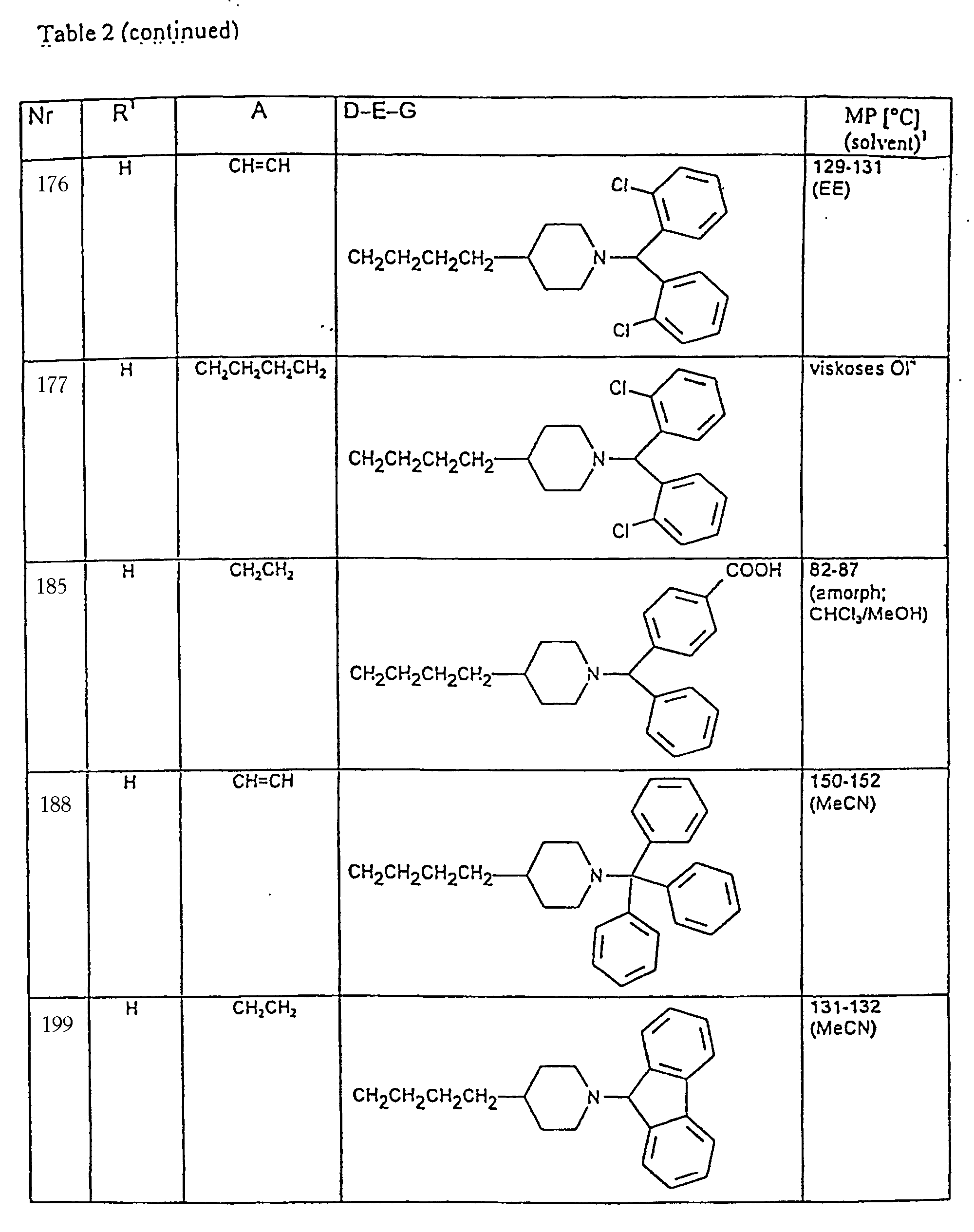

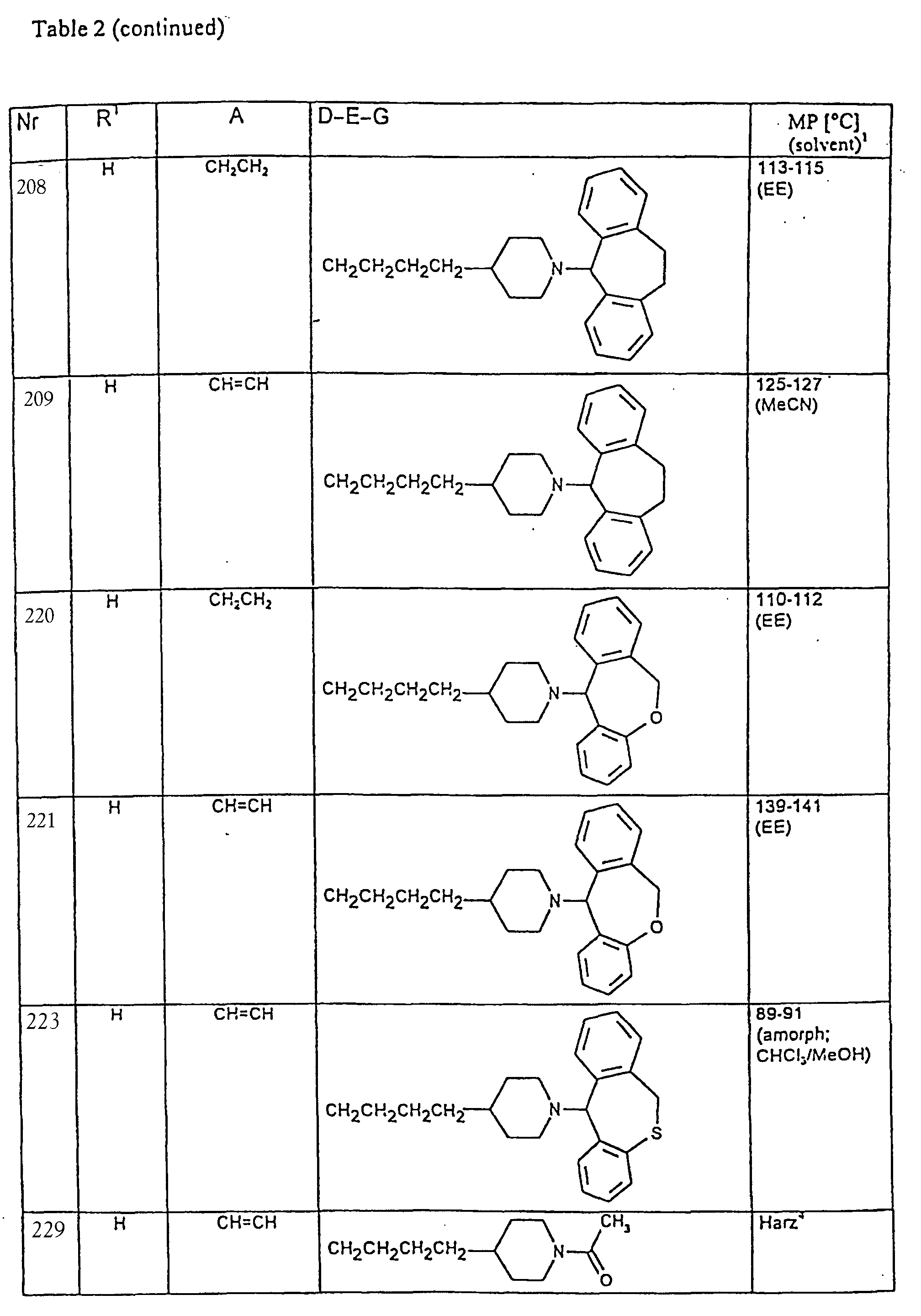

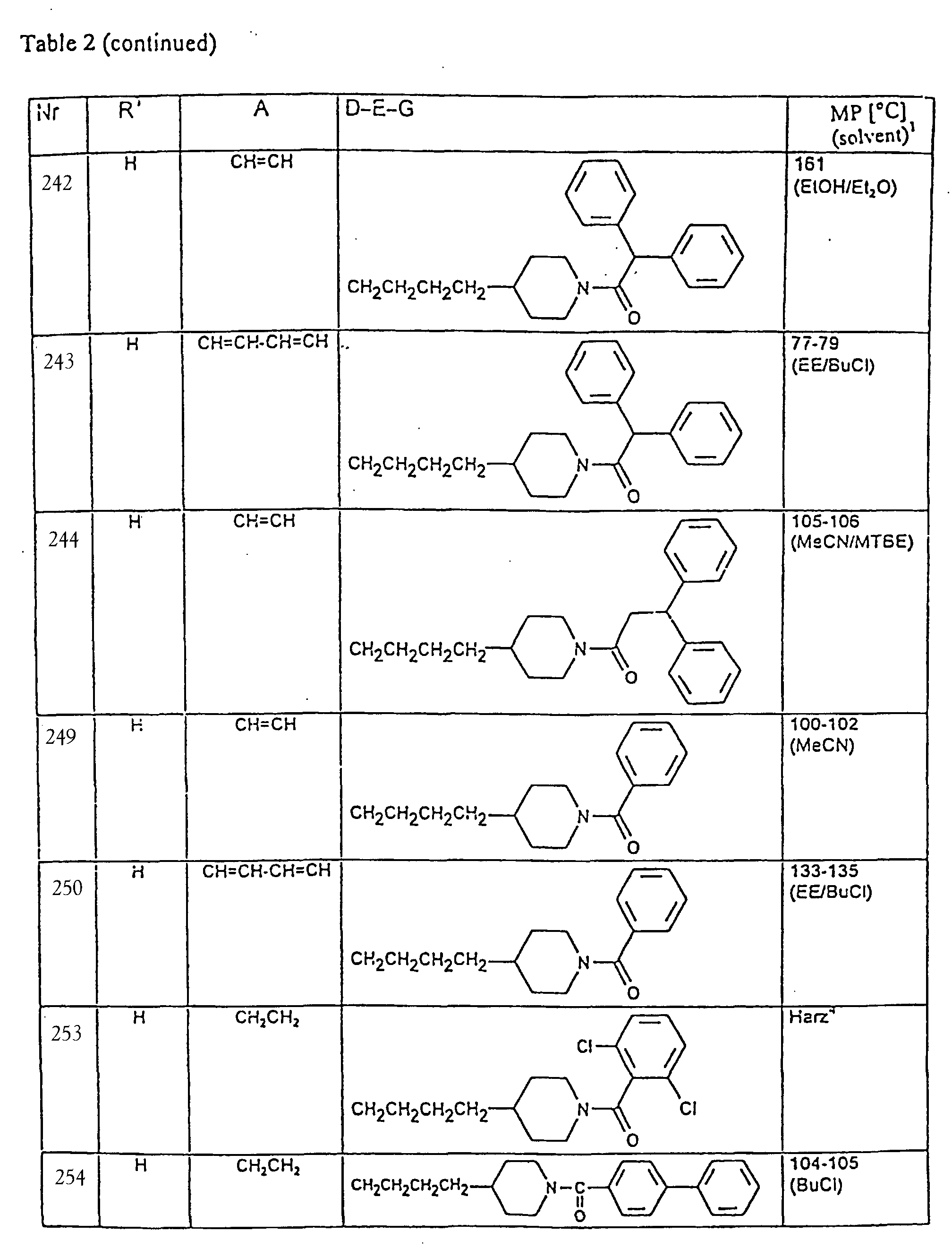

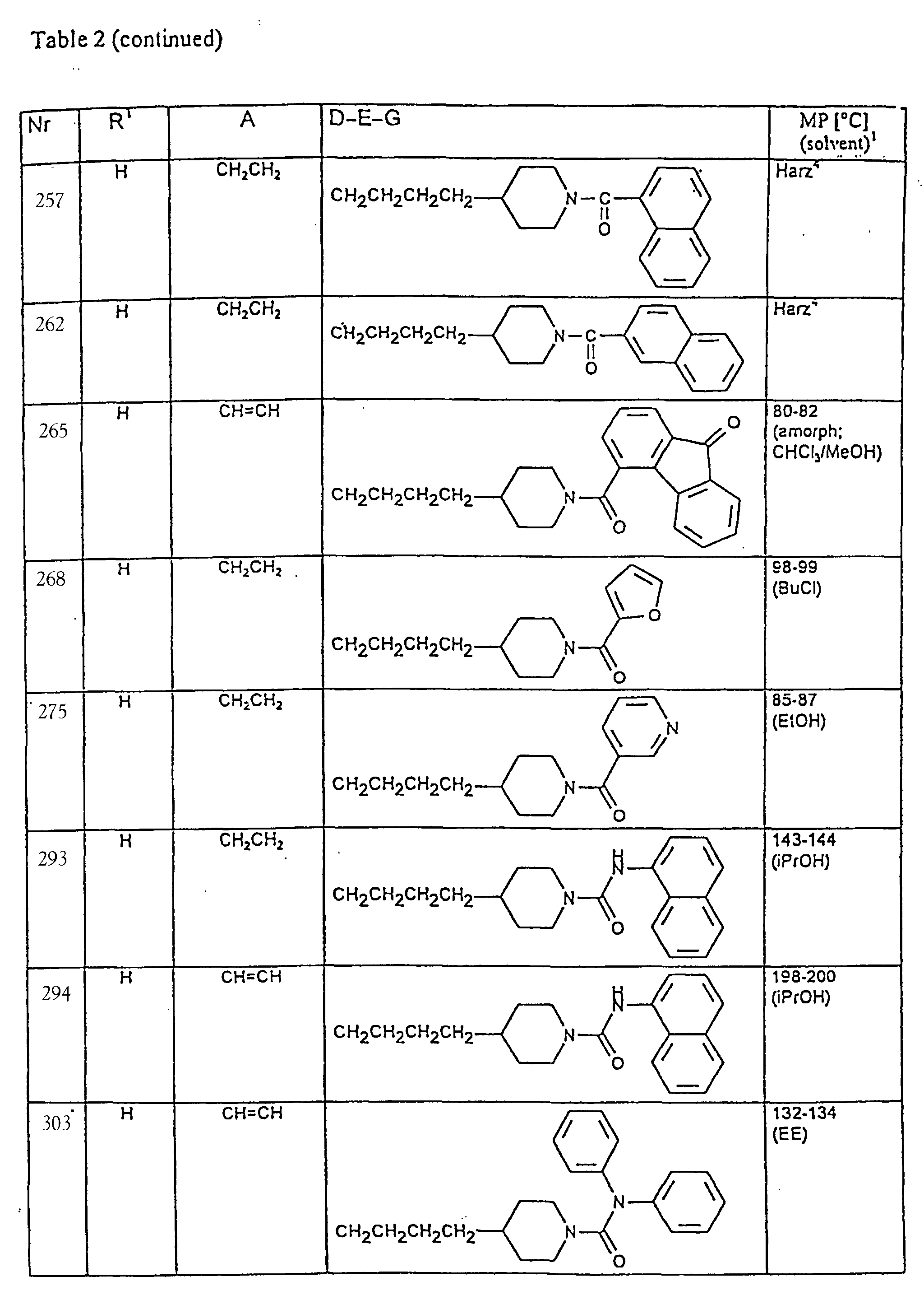

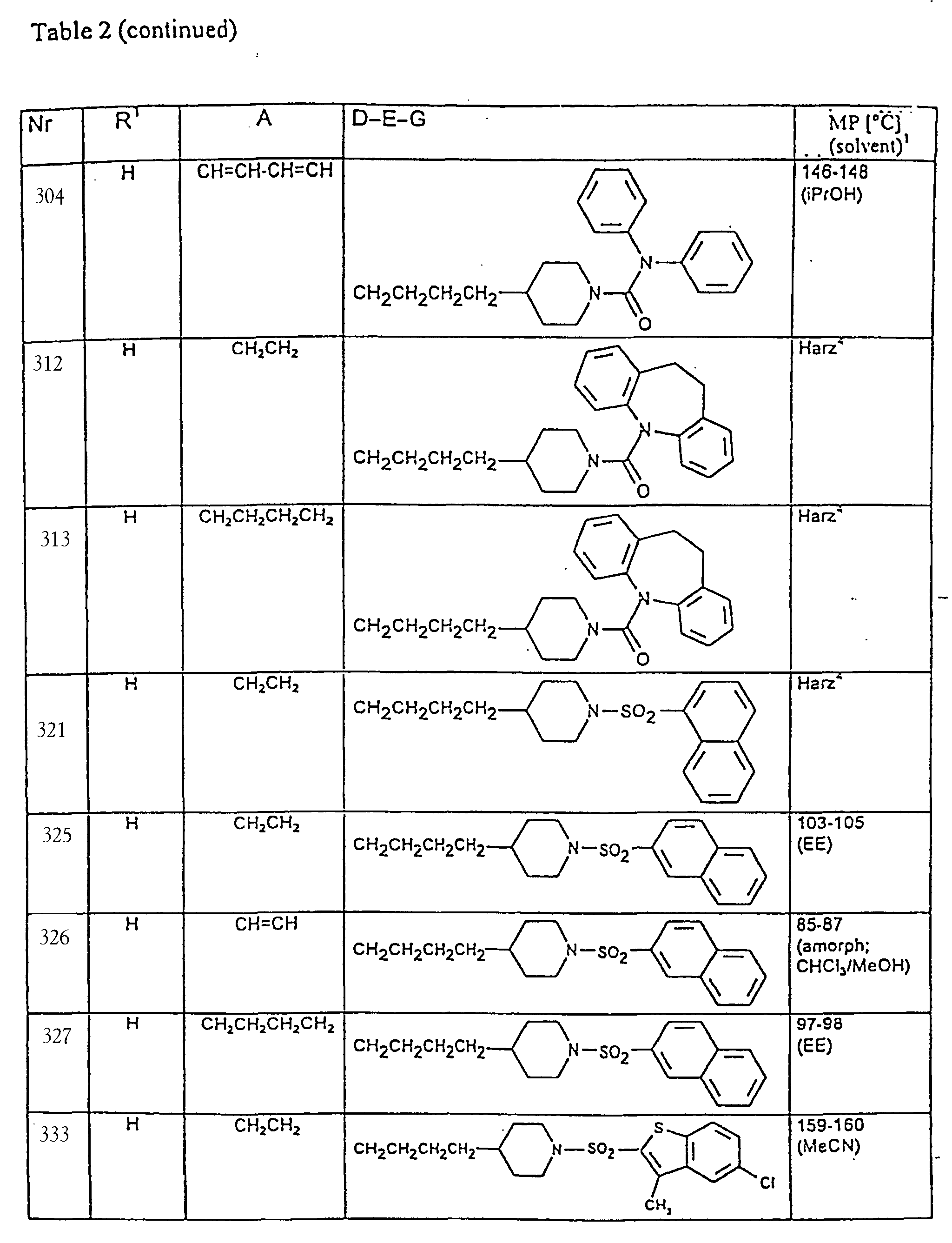

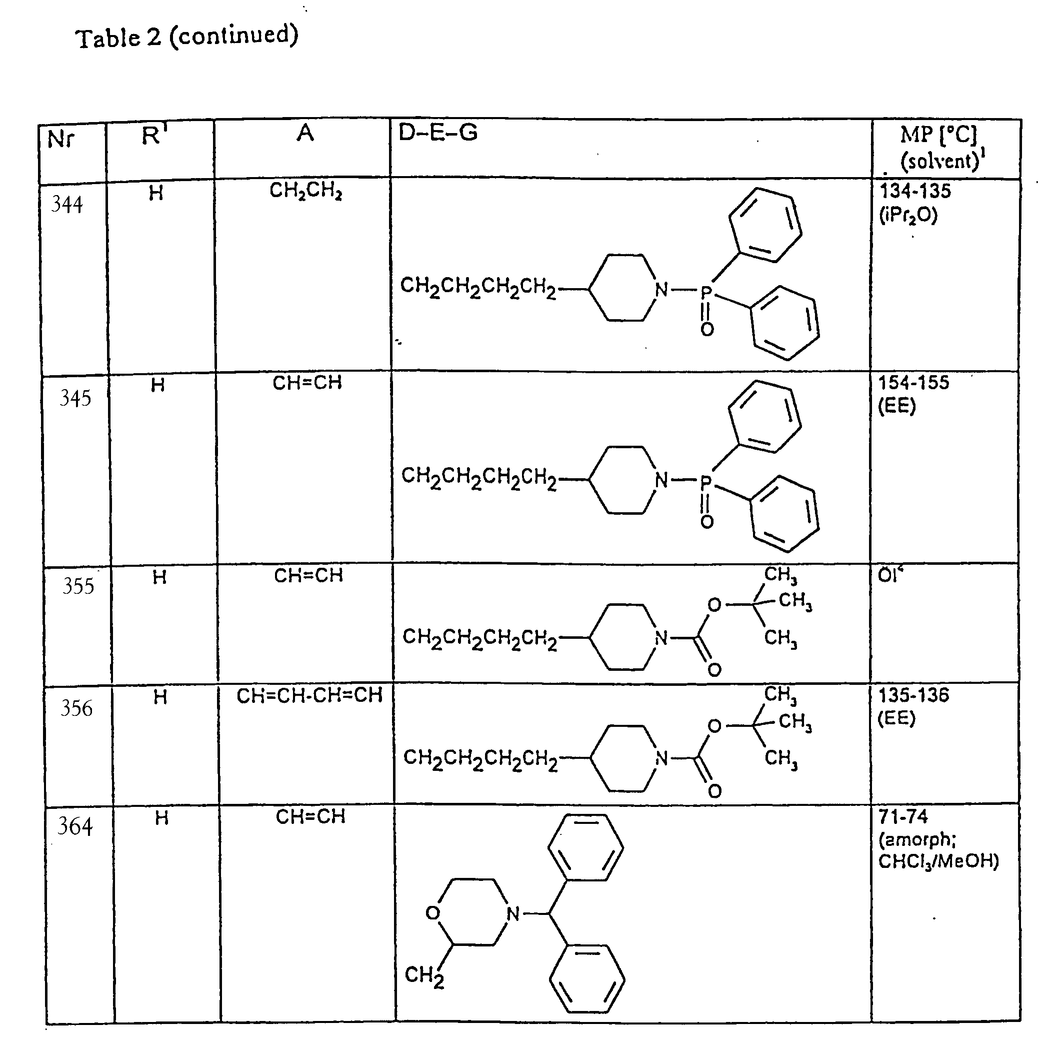

**Table annotation**

| | |
|---|---|
| ^{**1**} | **MeOH = methanol** |
| | **EE = ethyl acetate** |
| | **iPrOH = isopropanol** |
| | **iPr**_{**2**}**O = diisopropyl ether** |
| | **MeCN = acetonitrile** |
| | **BuCl = 1-chlorobutane** |
| | **EtOH = ethanol** |
| | **PE = petroleum ether** |
| | **Et**_{**2**}**O = diethyl ether** |
| | **MTBE = methyl tert-butyl ether** |
| ^{**2**} | **as a dihydrochloride** |
| ^{**3**} | **as a trihydrochloride** |
| ^{**4**} | **purified by column chromatography** |
| ^{**5**} | **as a methanesulfonate** |

In the following, the production of the starting materials necessary for illustration of the reproducibility for providing the compounds used according to the invention is described by means of several examples.

### Example 1A

### 4-{1-[bis(4-fluorophenyl)-methyl]-piperidin-4-yl)-butane-1-ol

20 g (103 mmol) 4-piperidin-4-yl-butan-1-ol hydrochloride are suspended in 70 ml 3,4-dihydro-2H-pyrane and added to 10 g pyridinium tosylate. The mixture is stirred for two days at RT. After addition of 5 g potassium carbonate, this is concentrated under vacuum to dryness. The resulting 4-[4-tetrahydropyran-2-yloxy)-butyl]-piperidine is dissolved without further purification in 100 ml acetonitrile and added to 25.1 g (105 mmol) bis-(4-fluorophenyl)-chloromethane, 30 g (217 mmol) potassium carbonate and 50 g (30 mmol) potassium iodide and stirred for four days at RT. The mixture is filtered and the solvent is removed under vacuum. The resulting 1-[bis-(4-fluorophenyl)-methyl]-4-[4-(tetrahydropyran-2-yloxy)-butyl]-piperidine is dissolved without further purification in 150 ml methanol, added to enough 6 M methanolic hydrochloric acid until the pH of the mixture is acidic and left to stand for two days at RT. Subsequently, the solvent is drawn off under vacuum and the residue is dispersed between sodium hydroxide solution and acetic acid ethyl ester. The aqueous solution is extracted three times with acetic acid. The combined organic phases are dried over sodium sulfate and the residue is chromatographically purified over silica gel with CH₂Cl₂/CH₃OH(99/l to 94/4). Yield: 23.8 g (63%).

### Example 2A

### 2-(4-{1-[bis(4-fluorophenyl)-methyl]-piperidin-4-yl}-butyl)-isoindol-1,3-dione

23.1 g (62.5 mmol) 4-{1-[bis(4-fluorophenyl)-methyl]-piperidin-4-yl}-butan-1-ol, 16.4 g (62.5 mmol) triphenylphosphine and 9.2 g (62.5 mmol) phthalimide are suspended in THF and 10.9 g (62.5 mmol) azodicarboxylic acid diethyl ester is added dropwise under a protective atmosphere and light cooling (ca. 15-25°C). The mixture is stirred for three hours at RT and subsequently the solvent is removed under vacuum. The residue is chromatographically purified over silica gel with CH₂Cl₂/CH₃OH (99.5/0.5) Yield: 27.6 g (90%).

### Example 3A

### 4-[1-bis (4-fluorophenyl)-methylpiperidin-4-yl]-butylamine

27.6 g (56.3 mmol) 2-(4-{1-[bis(4-fluorophenyl)-methyl]-piperidin-4-yl}-butyl)-isoindol-1,3-dione are suspended in 120 ml ethanol and added to 5.6 g (112 mmol) hydrazine hydrate and heated to boiling for four hours. After cooling, the mixture is filtered and the solvent is removed under vacuum. The residue is dispersed between 10 % sodium hydroxide solution and acetic acid ethyl ester. The aqueous phase is extracted three times with acetic acid ethyl ester. The combined organic phases are dried over sodium sulfate. The solvent is removed under vacuum and the residue is chromatographically purified over silica gel with CHCl₃/CH₃OH/NH₄OH (95/5/0 to 90/10/1). Yield: 14.6 g (73%).

### Example 4A

### 4-[1-(10,11-dihydro-5H-dibenzo[a,d]cyclohepten-5-yl)-piperidin-4-yl]-butane-1-ol

26.3 g (<109 mmol) 4-[4-tetrahydropyran-2-yloxy)-butyl]-piperidine (crude product) and 23.3 g (229 mmol) TEA are dissolved in 150 ml acetonitrile and added in portions to 25.0 g (109 mmol) 5-chloro-10,11-dihydro-5H-dibenzo[a,d]cycloheptene under cooling. The mixture is stirred at RT overnight. Subsequently, the solvent is removed under vacuum and the residue is dispersed betweenacetic acid ethyl ester and water. The organic phase is dried over sodium sulfate and the solvent is removed under vacuum. The resulting 1-(10,11-dihydro-5H-dibenzo[a,d]cycloheptene-5-yl)-4-[4-(tetrahydropyran-2-yloxy)-butyl]-piperidine is dissolved without further purification in 200 ml methanol, added to enough 6 M methanolic hydrochloric acid until the pH of the mixture is acidic and stirred for five hours at RT. Subsequently, the solvent is removed under vacuum and the residue is dispersed between 10 % sodium hydroxide solution and dichloromethane. The organic phase is dried over sodium sulfate and the solvent is removed under vacuum. The residue is processed further without additional purification. Yield: 38.2 g.

### Example 5A

### 2-{4-[1-(10,11-dihydro-5H-dibenzo[a,d]cyclohepten-5-yl)-piperidin-4-yl]-butyl}-isoindol-1,3-dione

Production occurs analogously to Example 2a.

Batch size: 15.0 g (<43 mmol) 4-[1-(10,11-dihydro-5H-dibenzo[a,d]cyclohepten-5-yl)-piperidin-4-yl]-butan-1-ol (crude product), 11.3 g (43.1 mmol) triphenylphosphine, 6.4 g (43.5 mmol) phthalimide and 7.5 g (43.0 mmol) azodicarboxylic acid diethyl ester in 200 ml THF. For purification, this is chromatographed over silica gel first with dichloromethane and then with petroleum ether/acetic acid ethyl ester (10/1 to 5/1). Yield: 12.0 g (57%)

### Example 6A

### 4-[1-(10,11-dihydro-5H-dibenzo[a,d]cyclohepten-5-yl)-piperidin-4-yl]-butylamine

Production occurs analogously to Example 3a.

Batch size: 11.5 g (24.0 mmol) 2-{4-[1-(10,11-dihydro-5H-dibenzo[a,d]cyclohepten-5-yl)-piperidin-4-yl]-butyl}-isoindol-1,3-dione and 2.4 g (48.0 mmol) hydrazine hydrate in 100 ml ethanol.

For work up, the reaction solution is filtered and the filter cake is dispersed between300 ml acetic acid ethyl ester and 100 ml 10 % sodium hydroxide solution. The organic phase is dried over sodium sulfate and the solvent is removed under vacuum. The residue is chromatographically purified over silica gel with CHCl₃/CH₃OH/TEA (95/5/0 to 94/5/1). Yield: 5.6 g (67%)

### Example 7A

### (1-diphenylmethylpiperidine-4-yliden)-acetonitrile

2.9 g (97 mmol) 80 % sodium hydride are suspended in abs. THF and a solution of 21.5 g (121 mmol) diethyl-(cyanomethyl)-phosphonate in 150 ml abs. THF is added dropwise under light cooling. The mixture is subsequently stirred 30 minutes at RT and then a solution of 26.5 g (100 mmol) N-(diphenylmethyl)-4-piperidone in 80 ml abs. THF is added dropwise. The suspension is left to stand overnight and subsequently added to 200 ml acetic acid ethyl ester and 100 ml water. The organic phase is separated and washed with 100 ml water, dried over sodium sulfate and the solvent is removed under vacuum. The residue is chromatographically purified over silica gel with dichloromethane. Yield: 22.7 g (78%).

### Example 8A

### (1-diphenylmethylpiperidine-4-yl)-acetonitrile

9.8 g (33.5 mmol) (1-diphenylmethylpiperidine-4-ylidine)-acetonitrile are dissolved in a mixture of 60 ml dioxane and 70 ml ethanol and added dropwise to 1.5 g palladium (5%) on activated carbon. The mixture is stirred at RT under hydrogen atomosphere until consumption of the theoretical amount of hydrogen to be taken up (ca. 14 hours).The mixture is filtered from the catalyst and the solvent is removed under vacuum. The residue is chromatographically purified over silica gel with CHCl₃/CH₃OH (96/4). Yield of yellow oil: 8.4 g (85%).

### Example 9A

### 2-(1-diphenylmethylpiperidine-4-yl)-ethylamine

2.2 g (58.2 mmol) sodium borohydroxide are suspended in 50 ml abs. THF and cooled to ca. 0°C under moisture exclusion. 6.7 g (23.1 mmol) (1-diphenylmethylpiperidin-4-yl)-acetonitrile are added and the mixture is subsequently cooled to -5°C to -10°C and added dropwise to 1.5 ml (26.7 mmol) 95 % sulfuric acid (vigorous foaming). The suspension is left to stand for two days at RT without further cooling. Under renewed cooling to ca. 0°C, 40 ml 2 M sodium hydroxide solution is added dropwise. The aqueous phase is extracted with 30 ml THF and thereafter the combined organic phases are washed twice, each with 30 ml saturated NaCl solution, dried over sodium sulfate and the solution is removed under vacuum. The residue is chromatographically purified over silica gel with CHCl₃/CH₃OH/NH₄OH (90/10/1). Yield: 3.9 g (57%).

### Example 10A

### 4-{1-[bis(2-chlorophenyl)-methyl]-piperidin-4-yl}-butyronitrile

20 g (106 mmol) 4-(3-cyanoprop-1-yl)-piperidin hydrochloride, 40.2 g (127 mmol) bis(2-chlorophenyl)-bromomethane and 35.2 g (254 mmol) potassium carbonate are heated to boiling in 90 ml acetone for six hours. After cooling, the mixture is freed from solution under vacuum. The residue is dispersed between 90 ml tolueneand 90 ml water. The aqueous phase is extracted with 10 ml tolueneand the combined organic phases are washed with 10 ml water. The organic phase is concentrated and the residue is chromatographically purified over silica gel with CHCL₃. Yield: 11.2 g (27 %)

### Example 11A

### 4-{1-[bis(2-chlorophenyl)-methyl]-pipendin-4-yl}-butylamine

8.0 g (20.6 mmol) 4-{1-[bis(2-chlorophenyl)-methyl]-piperidin-4-yl}-butyronitrile are dissolved in 240 ml dioxane/ethanol (1/5) and added to 1.5 g Raney-nickel. The mixture is stirred at RT under hydrogen atmosphere until uptake of the theoretical amount of hydrogen. The reaction solution is filtered from the catalyst and the solvent is removed under vacuum. The residue is dispersed between 100 ml 10 % sodium hydroxide solution and 300 ml acetic acid ethyl ester. The organic phase is dried with sodium sulfate and the solvent is removed under vacuum. The resin is processed further without additional purification.

### Example 12A

### 3-(1-diphenylmethylpiperidin-4-yl)-propan-1-ol

20 g (111 mmol) 3-piperidin-4-yl-propan-1-ol hydrochloride are suspended in 70 ml 3,4-dihydro-2H-pyrane and added to 0.5 g pyridinium tosylate. The mixture is stirred for two days at RT. After addition of 1 g potassium carbonate, this is concentrated under vacuum to dryness. The resulting 4-[3-tetrahydropyran-2-yloxy)-propyl]-piperidine is dissolved in 90 ml acetonitrile without further purification and added to 35 g (135 mmol) diphenyl bromomethane (95%) and 29 g (210 mmol) potassium carbonate and stirred for four days at RT. The mixture is filtered and the solvent is removed under vacuum. The resulting 1-diphenylmethyl-4-[3-(tetrahydropyran-2-yloxy)-propyl]-piperidine is dissolved without further purification in 130 ml methanol and added to 25 ml conc. hydrochloric acid, and the mixture is stirred at RT overnight. Subsequently, the solvent is removed under vacuum and the residue is taken up with 300 ml water and extracted with 300 ml acetic acid ethyl ester. The organic phase is discarded and the aqueous phase is made alkaline with 17 g sodium hydroxide and extracted with 300 g acetic acid ethyl ester. After washing the organic phase with 40 ml water, this is dried over sodium sulfate and the solvent is removed under vacuum The oil is dried under high-vacuum and processed further without purification. Yield: 22.4 g (65%).

### Example 13A

### 2-[3-(1-diphenylmethylpiperidine-4-yl)-propyl]-isoindol-1,3-dione

20.3 g (65.6 mmol) 3-(1-diphenylmethylpiperidin-4-yl)-propan-1-ol, 17.2 g (65.6 mmol) triphenylphosphine and 9.7 g (65.6 mmol) phthalimide are suspended in 220 ml THF and a solution of 10.4 ml (65.6 mmol) azodicarbonic acid diethyl ester in 50 ml THF is added dropwise within one hour under protective atmosphere and light cooling (ca 15-25°C). After a further three hours, the solvent is removed under vacuum and the residue is crystallized from 60 ml 1-chlorobutane. Yield: 12.9 g (45%)

### Example 14A

### 3-(1-diphenylmethylpiperidin-4-yl)-propanylamine

12.8 g (29.2 mmol) 2-[3-(1-diphenylmethylpiperidine-4-yl)-propyl]-isoindol-1,3-dione are suspended in 110 ml ethanol and added to 2.2 ml (58.4 mmol) hydrazine hydrate and heated to boiling for three hours. After cooling, the mixture is concentrated under vacuum. The residue is dispersed between 300 ml dichloromethane and 50 ml 10 % sodium hydroxide solution. The aqueous phase is extracted twice, each with 200 ml dichloromethane. The combined organic phases are washed twice, each with 20 ml water, and dried over sodium sulfate. The solvent is removed under vacuum and the residue is processed fruther without additional purification. Yield 7.3 g (81 %)

### Example 15A

### 4-(1-diphenylmethylpiperidin-4-yl)-butan-1-ol

120 g (620 mmol) 4-piperidin-4-yl-butan-1-ol hydrochloride are suspended in 400 ml 3,4-dihydro-2H-pyran and added to 1.5 g pyridinium tosylate and 5 ml 8 M methanolic hydrochloric acid. This is stirred for three hours and left to stand at RT overnight. After addition of 5 g potassium carbonate, this is concentrated under vacuum to dryness. The resulting 4-[4-tetrahydropyran-2-yloxy)-butyl]-piperidine is dissolved in 500 ml acetonitrile without further purification and added to 193 g (742 mmol) diphenylmethylbromide (95%) and 160 g (1157 mmol) potassium carbonate and stirred for three days at RT. The mixture is filtered and the filtrate is stirred for one day at RT with a further 20 g (76.8 mmol) diphenylmethylbromide (95%) and 16 g (115.8 mmol) potassium carbonate. The mixture is filtered and the solvent is removed under vacuum. The resulting 1-diphenylmethyl-4-[4-(tetrahydropyran-2-yloxy)-butly]-piperidine is dissolved in 700 ml methanol without further purification, added to 120 ml conc. hydrochloric acid, and the mixture is left to stand for two days at RT. Subsequently, the solvent is removed under vacuum and the residue is taken up with 1500 ml water and extracted with 1500 ml acetic acid ethyl ester. The organic phase is discarded and the aqueous phase is adjusted to alkaline with sodium hydroxide and extracted with 700 ml acetic acid ethyl ester. After washing the organic phase with 200 ml water, this is dried over sodium sulfate and the solvent is removed under vacuum. The brown oil is dried under high-vacuum and processed further without purification. Yield: 145 g (72%).

### Example 16A

### 2-[4-(1-diphenylmethylpiperidin-4-yl)-butyl]-isoindol-1,3-dione

135.0 g (417 mmol) 4-(1-diphenylmethylpiperidin-4-yl)-butan-1-ol, 109.5 g (417 mmol) triphenylphosphine and 61.3 g (417 mmol) phthalimide are suspended in THF and 72.6 g (417 mmol) azodicarboxylic acid diethyl ester are added dropwise within 2½ hours under protective atmosphere and light cooling (ca. 15-25°C). After a further hour, the solvent is removed under vacuum and the residue is crystallized three times from acetic acid ester (1000 ml, 1200 ml and 1100 ml). Colorless crystals with a MP of 150-152 were recovered. Yield: 103 g (54.5%).

### Example 17A

### 4-(1-diphenylmethylpiperidin-4-yl)-butylamine

88.9 g (196 mmol) 2-[4-(1-diphenylmethylpiperidin-4-yl)-butyl]-isoindol-1,3-dione are suspended in 1000 ml ethanol and added to 22.0 g (440 mmol) hydrazine hydrate and heated to boiling for four hours. After cooling, the mixture is filtered and the solvent is removed under vacuum. The solid residue is distributed together with the filter residue under heat between 300 ml 10 % sodium hydroxide solution and 300 ml acetic acid ethyl ester. The aqueous phase is extracted once again with 150 ml warm acetic acid ethyl ester. The combined organic phases are extracted twice, each with 300 ml 10 % hydrochloric acid. The combined aqueous phases are adjusted to alkaline with 10 % sodium hydroxide solution and extracted twice, each with 400 ml acetic acid ethyl ester. The combined organic phases are washed with 100 ml water and dried over sodium sulfate. The solvent is removed under vacuum, the residue is dried under high-vacuum and further processed without additional purification. Yield of gradually solidifying resin: 63 g (99%)

### Example 18A

### 5-(1-diphenylmethylpiperidin-4-yl)-pentanitrile

24.9 g (69.2 mmol) 4-(1-diphenylmethylpiperidin-4-yl)-butan-1-ol hydrochloride are suspended in 160 ml abs. dichloromethane and cooled to ca. 0°C under moisture exclusion. At this temperature, 16.0 g (159 mmol) TEA is first added, and thereafter, a solution of 10.3 g (90.0 mmol) methanesulfonic acid chloride in a little abs dichloromethane is added dropwise. The mixture is subsequently stirred for three hours at RT and then placed in ice water. The organic phase is washed once with 50 ml water, dried over sodium sulfate and the solvent is removed under vacuum. The resulting methanesulfonic acid-4-(1-diphenylmethylpiperidin-4-yl)-butyl ester is dissolved in 120 ml DMF without further purification and added to 7.7 g (158 mmol) sodium cyanide and two drops 15-crown-5 and stirred six hours at 65°C. After cooling, the mixture is poured in ice water. The precipitated solid is drawn off and dried under high-vacuum at 40°C. The solid is processed further without additional purification. Yield: 19.9 g (86%).

### Example 19A

### 5-(1-diphenylmethylpiperidin-4-yl)-pentylamine

19.0 g (57.1 mmol) 5-(1-diphenylmethylpiperidin-4-yl)-pentanitrile are dissolved in 240 ml dioxane/ethanol (1/1) and added to 3.2 g Raney-Nickel. The mixture is stirred at RT under hydrogen atmosphere until uptake of the theoretical amount of hydrogen. The mixture is filtered from the catalyst and the solvent is removed under vacuum. The residue is dispersed between 100 ml water and 250 ml acetic acid ethyl ester. The organic phase is dried with sodium sulfate and the solvent is removed under vacuum. The resin is processed further without additional purification. Yield: 17.0 g (88%).

### Example 20A

### 4-(1-tert-butoxycarbonylpiperidin-4-yl)-butan-1-ol

100 g (458 mmol) 4-piperidin-4-yl-butan-1-ol hydrochloride are dissolved in 120 ml water, added to 216 ml (1550 mmol) TEA and cooled to ca 5-10°C. 122 g (559 mmol) di-tert-butyl dicarbonate dissolved in 400 ml THF are added dropwise within four hours under further cooling. The mixture is left to stand at RT overnight without further cooling. Subsequently, the THF is largely removed under vacuum and the residue is extracted twice with 300 ml and 200 ml CHCl₃ and the combined organic phases are washed twice, each with 20 ml water. The solvent is removed under vacuum, the residue dried under high-vacuum and processed further without additional purification. Yield: 136g.

### Example 21A

### 2-[4-(1-tert-butoxycarbonylpiperidin-4-yl)-butyl]-isoindol-1,3-dione

136.0 g (<528 mmol) 4-(1-tert-butoxycarbonylpiperidin-4-yl)-butan-1-ol (crude product), 135.3 g (516 mmol) triphenylphosphine and 75.9 g (516 mmol) phthalimide are suspended in THF and 89.9 g (516 mmol) azodicarboxylic acid diethyl ester are added dropwise within 3 hours under protective atmosphere and light cooling (ca 15°C). The mixture is left to stand at RT overnight without further cooling. Subsequently, the solvent is removed under vacuum and the oily residue is dissolved in 500 ml acetic acid ethyl ester and held overnight at 0°C. The sedimented precipitate is filtered and discarded. The solution is concentrated under vacuum and the oily residue is chromatographically purified over silica gel with CHCl₃ and crystallized from 200 ml isopropanol after drawing off the solvent. Colorless crystals with a MP of 100-102°C were recovered; yield: 108.5g (57%).

### Example 22A

### 4-(1-tert-butoxycarbonylpiperidin-4-yl)-butylamine

113.0 g (292 mmol) 2-[4-(1-tert-butoxycarbonylpiperidin-4-yl)-butyl]-isoindol-1,3-dione are dissolved in 600 ml ethanol and added to 29.3 g (585 mmol) hydrazine hydrate are added thereto and heated to boiling for three hours. After cooling the solution, the mixture is filtered and the filtrate is concentrated under vacuum. The residue is distributed under heat between 500 ml tolueneand 500 ml 10 % sodium hydroxide solution. The organic phase is washed once with 50 ml 10 % sodium hydroxide solution and twice, each with 50 ml water. The solvent is removed under vacuum, the residue is dried under high-vacuum at 70°C and further processed without additional purification. Yield of colorless oil: 64.0 g (85%).

### Example 23A

### (1-diphenylmethylazetidin-3-ylmethyl)-amine

A solution of 10 g (40 mmol) 1-diphenylmethylazetidin-3-carbonitrile in 20 ml abs. THF are added dropwise at RT to a suspension of 3.1 g (80 mmol) lithium aluminum hydride in 80 ml abs. THF and stirred overnight. 2 ml ethanol are carefully added to the batch and the batch is filtered. The filtrate is concentrated under vacuum and dispersed between CHCl₃ and water. The aqueous phase is extracted twice, each with 50 ml CHCl₃, and the combined organic phases are dried over sodium sulfate and the solvent is removed under vacuum. The residue is chromatographically purified over silica gel with CHCl₃/CH₃OH/NH₄OH (90/10/0 to 90/10/1). Yield: 5.6 g (57%) of slowly solidifying resin.

### Example 24A

### 3-(1-benzylpiperidin-4-yloxy)-propylamine

100 g (40.9 mmol) 3-(1-benzylpiperidin-4-yloxy)-propionitrile are dissolved in 100 ml ethanol and added to a spatula tip of Raney-Nickel. The mixture is stirred at RT under hydrogen atmosphere until the uptake of the theoretical amount of hydrogen (ca. 2 days). The mixture is filtered from the catalyst and the solvent is removed under vacuum. The residue is distilled in a ball-pipe apparatus. Yield of colorless oil: 7.5 g (73%)

### Example 25A

### 4-(1-benzylpiperidin-3-ylidene)-butyronitrile

77.3 g (188.3 mmol) 3-cyanopropyl triphenylphosphonium bromide are suspended in 300 ml tolueneand added to 22.0 g (191.9 mmol) potassium-tert-butylate. The mixture is cooled to ca. 0°C under moisture exclusion and a solution of 34.6 g (182.8 mmol) 1-benzyl-3-piperidone in 50 ml tolueneare added dropwise under cooling. The batch is left to stand overnight at ca. 0°C, subsequently diluted with 200 ml tolueneand washed twice, each with 100 ml water. The organic phase is extracted with 150 ml half-concentrated hydrochloric acid. Subsequently, the aqueous phase is made basic with 200 ml 10 % sodium hydroxide solution and extracted twice, each with 250 ml toluol. The solvent is removed under vacuum and the residue is chromatographically purified over silica gel with CHCl₃/CH₃OH (97/3). After drawing off the solvent, a light brown oil remains which is further processed without additional purification. Yield: 47.4 g (90%).

### Example 26A

### 4-(1-benzylpiperidin-3-yl)-butylamine

8.0 g (33.3 mmol) 4-(1-benzylpiperidin-3-ylidene)-butyronitrile are dissolved in 80 ml ethanol and added to a spatula tip of Raney-Nickel. The mixture is stirred under hydrogen atmosphere until consumption of the theoretical amount of hydrogen to be taken up (ca. 5 days). The mixture is filtered from the catalyst and the solvent is removed under vacuum. The residue is chromatographically purified twice over silica gel with CHCl₃/CH₃OH/NH₄OH (90/10/1). After drawing off the solvent, a colorless oil remains which is further processed without additional purification. Yield: 3.9 g (47%).

The active ingredients used according to the invention can be processed to the desired medicaments in the form of their acid addition salts, hydrates or solvates individually or in combination with each other or with others, optionally under addition of other active ingredients, for the indications tumor treatment or immunosuppression. In the case of the combination of active ingredients according to the invention with other medicinal forms, these can also optionally be separately present next to each other in the medicine packaging, for example as tablets next to viles, depending on the requirements.

Therefore, further subject-matter of the invention is a medicament with an amount of compounds according to the above defined general formula (I) in combination with the further cytostatic agent, cancerostatic agent, immunosuppressing agent and/or immunomodulatory agent. Therewith, a method for the production of medicaments with an amount of one or more compounds according to formula (I), optionally together with another cytostatic agent or immunosuppressive agent and optionally next to further active ingredients and additives customary and suitable for these indications together with respective pharmaceutically acceptable carriers and adjuvents for providing the finished medical form also falls within the scope of protection according to the invention.

The invention is more closely illustrated in the following by means of the production of respective medicaments suitable for the use according to the invention and the combinations according to the invention as well as by means of a series of examples for various medical forms suitable for the respective indications.

### Therapeutic Administration Forms

The production of medicaments with an amount of one or more compounds according to the invention and/or their use in the application according to the invention occurs in the customary manner by means of common pharmaceutical technology methods. For this, the active ingredients as such or in the form of their salts are processed together with suitable, pharmaceutically acceptable adjuvents and carriers to medicinal forms suitable for the various indications and types of application. Thereby, the medicaments can be produced in such a manner that the respective desired release rate is obtained, for example a quick flooding and/or a sustained or depot effect.

Preparations for parenteral use, to which injections and infusions belong, are among the most important systemically employed medicaments for tumor treatment as well as for other indications.

Preferably, injections are administered for the treatment of tumors. These are prepared either in the form of vials or also as so-called ready-to-use injection preparations, for example as ready-to-use syringes or single use syringes in addition to perforation bottles for multiple withdrawals. Administration of the injection preparations can occur in the form of subcutaneous (s.c.), intramuscular (i.m.), intravenous (i.v.) or intracutaneous (i.c.) application. The respective suitable injection forms can especially be produced as solutions, crystal suspensions, nanoparticular or colloid-disperse systems, such as for example, hydrosols.

The injectable formulations can also be produced as concentrates which can be adjusted with aqueous isotonic dilution agents to the desired active ingredient dosage. Furthermore, they can also be produced as powders, such as for example lyophilisates, which are then preferably dissolved or dispersed immediately before application with suitable diluents. The infusions can also be formulated in the form of isotonic solutions, fat emulsions, liposome formulations, microemulsions and liquids based on mixed micells, for example, based on phospholipids. As with injection preparations, infusion formulations can also be prepared in the form of concentrates to dilute. The injectable formulations can also be applied in the form of continuous infusions as in stationary as well as in out-patient therapy, for example in the form of mini-pumps

Albumin, plasma expanders, surface active compounds, organic solvents, pH influencing compounds, complex forming compounds or polymeric compounds can be added to the parenteral medicinal forms, especially as substances for influencing the adsorption of the active ingredients to protein or polymers or also with the aim of decreasing the adsorption of the active ingredient to materials such as injection instruments or packaging materials, for example plastic or glass.

The active ingredients can be bound to nanoparticles in the preparations for parenteral use, for example on finely dispersed particles based on poly(meth)acrylates, polyacetates, polyglycolates, polyamino acids or polyether urethanes. The parenteral formulations can also be constructively modified as depot preparations, for example on the multiple unit principle, where the active ingredients are incorporated in a most finely distributed and/or dispersed, suspended form or as crystal suspensions, or on the single unit principle, where the active ingredient is enclosed in a medicinal form, for example, a tablet or a seed which is subsequently implanted. Often, these implantations or depot medicaments in single unit and multiple unit medicinal forms consist of so-called biodegradable polymers, such as for example, polyether urethanes of lactic and glycolic acid, polyether urethanes, polyamino acids, poly(meth)acrylates or polysaccharides.

Sterilized water, pH value influencing substances, such as for example organic and inorganic acids or bases as well as their salts, buffer substances for setting the pH value, agents for isotonicity, such as for example sodium chloride, monosodium carbonate, glucose and fructose, tensides and/or surface active substances and emulsifiers, such as for example, partial fatty acid esters of polyoxyethylene sorbitan (Tween®) or for example fatty acid esters of polyoxethylene (Cremophor®), fatty oils such as for example peanut oil, soybean oil and castor oil, synthetic fatty acid esters, such as for example ethyl oleate, isopropyl myristate and neutral oil (Miglyol®) as well as polymer adjuvents such as for example gelatin, dextran, polyvinylpyrrolidone, organic solvent additives which increase solubility, such as for example propylene glycol, ethanol, N,N-dimethylacetamide, propylene glycol or complex forming compounds such as for example citrates and urea, preservatives, such as for example hydroxypropyl benzoate and hydroxymethyl benzoate, benzyl alcohol, anti-oxidants, such as for example sodium sulfite and stabilizers, such as for example EDTA, are suitable as adjuvents and carriers in the production of preparations for parenteral use.

In suspensions, addition of thickening agents to prevent the settling of the active ingredients from tensides and peptizers, to secure the ability of the sediment to be shaken, or complex formers, such as EDTA, ensues. This can also be achieved with the various polymeric agent complexes, for example with polyethylene glycols, polystyrol, carboxymethylcellulose, Pluronics® or polyethylene glycol sorbitan fatty acid esters. The active ingredient can also be incorporated in liquid formulations in the form of inclusion compounds, for example with cyclodextrins. As further adjuvents, dispersion agents are also suitable. For production of lyophilisates, builders are also used, such as for example mannite, dextran, saccharose, human albumin, lactose, PVP or gelatin varieties.

As long as the active ingredients are not incorporated in the liquid medicinal formulations in the form of a base, they are used in the form of their acid addition salts, hydrates or solvates in the preparations for parenteral use.

A further systemic application form of importance is peroral administration as tablets, hard or soft gelatin capsules, coated tablets, powders, pellets, microcapsules, oblong compressives, granules, chewable tablets, lozenges, gums or sachets. These solid peroral administration forms can also be prepared as sustained action and/or depot systems. Among these are medicaments with an amount of one or more micronized active ingredients, diffusions and erosion forms based on matrices, for example by using fats, wax-like and/or polymeric compounds, or so-called reservoir systems. As a retarding agent and/or agent for controlled release, film or matrix forming substances, such as for example ethylcellulose, hydroxypropylmethylcellulose, poly(meth)acrylate derivatives (for example Eudragit®), hydroxypropylmethylcellulose phthalate are suitable in organic solutions as well as in the form of aqueous dispersions. In this connection, so-called bio-adhesive preparations are also to be named in which the increased retention time in the body is achieved by intensive contact with the mucus membranes of the body. An example of a bio-adhesive polymer is the group of Carbomers®.

For sublingual application, compressives, such as for example non-disintegrating tablets in oblong form of a suitable size with a slow release of active ingredient, are especially suitable. For purposes of a targeted release of active ingredients in the various sections of the gastrointestinal tract, mixtures of pellets which release at the various places are employable, for example mixtures of gastric fluid soluble and small intestine soluble and/or gastric fluid resistant and large intestine soluble pellets. The same goal of releasing at various sections of the gastrointestinal tract can also be conceived by suitably produced laminated tablets with a core, whereby the coating of the agent is quickly released in gastric fluid and the core of the agent is slowly released in the small intestine milieu. The goal of controlled release at various sections of the gastrointestinal tract can also be attained by multilayer tablets. The pellet mixtures with differentially released agent can be filled into hard gelatin capsules.

Anti-stick and lubricant and separating agents, dispersion agents such as flame dispersed silicone dioxide, disintegrants, such as various starch types, PVC, cellulose esters as granulating or retarding agents, such as for example wax-like and/or polymeric compounds on the basis of Eudragit®, cellulose or Cremophor® are used as a further adjuvents for the production of compressives, such as for example tablets or hard and soft gelatin capsules as well as coated tablets and granulates.

Anti-oxidants, sweetening agents, such as for example saccharose, xylite or mannite, masking flavors, aromatics, preservatives, colorants, buffer substances, direct tableting agents, such as for example microcrystalline cellulose, starch and starch hydrolysates (for example Celutab®), lactose, polyethylene glycols, polyvinylpyrrolidone and dicalcium phosphate, lubricants, fillers, such as lactose or starch, binding agents in the form of lactose, starch varieties, such as for example wheat or corn and/or rice starch, cellulose derivatives, for example methylcellulose, hydroxypropylcellulose or silica, talcum powder, stearates, such as for example magnesium stearate, aluminum stearate, calcium stearate, talc, siliconized talc, stearic acid, acetyl alcohol and hydrated fats are used

In this connection, oral therapeutic systems constructed especially on osmotic principles, such as for example GIT (gastrointestinal therapeutic system) or OROS (oral osmotic system), are also to be mentioned.

Effervescent tablets or tabs, both of which represent immediately drinkable instant medicinal forms which are quickly dissolved or suspended in water are among the perorally administratable compressives. Among the perorally administratable forms are also solutions, for example drops, juices and suspensions, which can be produced according to the above given method, and can still contain preservatives for increasing stability and optionally aromatics for reasons of easier intake, and colorants for better differentiation as well as antioxidants and/or vitamins and sweeteners such as sugar or artificial sweetening agents This is also true for inspisated juices which are formulated with water before ingestion. Ion exchange resins in combination with one or more active ingredients are also to be mentioned for the production of liquid ingestable forms.

A special release form consists in the preparation of so-called floating medicinal forms, for example based on tablets or pellets which develop gas after contact with body fluids and therefore float on the surface of the gastric fluid. Furthermore, so-called electronically controlled release systems can also be formulated by which active ingredient release can be selectively adjusted to individual needs.

A further group of systemic administration and also optionally topically effective medicinal forms are represented by rectally applicable medicaments. Among these are suppositories and enema formulations. The enema formulations can be prepared based on tablets with aqueous solvents for producing this administration form Rectal capsules can also be made available based on gelatin or other carriers.

Hardened fat, such as for example Witepsol®, Massa Estarinum®, Novata®, coconut fat, glycerol-gelatin masses, glycerol-soap-gels and polyethylene glycols are suitable as suppository bases.

For long-term application with a systematic active ingredient release up to several weeks, pressed implants are suitable which are preferably formulated on the basis of so-called biodegradable polymers.

As a further important group of systemically active medicaments, transdermal systems are also to be emphasized which distinguish themselves, as with the above-mentioned rectal forms, by circumventing the liver circulation system and/or liver metabolism. These plasters can be especially prepared as transdermal systems which are capable of releasing the active ingredient in a controlled manner over longer or shorter time periods based on different layers and/or mixtures of suitable adjuvents and carriers. Aside from suitable adjuvents and carriers such as solvents and polymeric components, for example based on Eudragit®, membrane infiltration increasing substances and/or permeation promoters, such as for example oleic acid, Azone®, adipinic acid derivatives, ethanol, urea, propylglycol are suitable in the production of transdermal systems of this type for the purpose of improved and/or accelerated penetration.

As topically, locally or regionally administration medicaments, the following are suitable as special formulations: vaginally or genitally applicable emulsions, creams, foam tablets, depot implants, ovular or transurethral adminstration installation solutions. For opthalmological application, highly sterile eye ointments, solutions and/or drops or creams and emulsions are suitable.

In the same manner, corresponding otological drops, ointments or creams can be designated for application to the ear. For both of the above-mentioned applications, the adminstration of semi-solid formulations, such as for example gels based on Carbopols® or other polymer compounds such as for example polyvinylpyrolidone and cellulose derivatives is also possible.

For customary application to the skin or also to the mucus membrane, normal emulsions, gels, ointments, creams or mixed phase and/or amphiphilic emulsion systems (oil/water-water/oil mixed phase) as well as liposomes and transfersomes can be named. Sodium alginate as a gel builder for production of a suitable foundation or celluolose derivatives, such as for example guar or xanthene gum, inorganic gel builders, such as for example aluminum hydroxides or bentonites (so-called thixotropic gel builder), polyacrylic acid derivatives, such as for example Carbopol®, polyvinylpyrolidone, microcrystalline cellulose or carboxymethylcellulose are suitable as adjuvents and/or carriers. Furthermore, amphiphilic low and high molecular weight compounds as well as phospholipids are suitable. The gels can be present either as hydrogels based on water or as hydrophobic organogels, for example based on mixtures of low and high molecular paraffin hydrocarbons and vaseline.

Anionic, cationic or neutral tensides can be employed as emulsifiers, for example alkalized soaps, methyl soaps, amine soaps, sulfanated compounds, cationic soaps, high fatty alcohols, partial fatty acid esters of sorbitan and polyoxyethylene sorbitan, for example lanette types, wool wax, lanolin, or other synthetic products for the production of oil/water and/or water/oil emulsions.

Hydrophilic organogels can be formulated, for example, on the basis of high molecular polyethylene glycols. These gel-like forms are washable. Vaseline, natural or synthetic waxes, fatty acids, fatty alcohols, fatty acid esters, for example as mono-, di-, or triglycerides, paraffin oil or vegetable oils, hardened castor oil or coconut oil, pig fat, synthetic fats, for example based on acrylic, caprinic, lauric and stearic acid, such as for example Softisan® or triglyceride mixtures such as Miglyol® are employed as lipids in the form of fat and/or oil and/or wax-like components for the production of ointments, creams or emulsions.

Osmotically effective acids and bases, such as for example hydrochloric acid, citric acid, sodium hydroxide solution, potassium hydroxide solution, monosodium carbonate, further buffer systems, such as for example citrate, phosphate, Tris-buffer or triethanolamine are used for adjusting the pH value.

Preservatives, for example such as methyl- or propyl benzoate (parabenes) or sorbic acid can be added for increasing stability.

Pastes, powders or solutions are to be mentioned as further topically applicable forms. Pastes often contain lipophilic and hydrophilic auxiliary agents with very high amounts of fatty matter as a consistency-giving base.

Powders or topically applicable powders can contain for example starch varieties such as wheat or rice starch, flame dispersed silicon dioxide or silica, which also serve as diluents, for increasing flowability as well as lubricity as well as for preventing agglomerates.

Nose drops or nose sprays serve as nasal application forms. In this connection, nebulizers or nose creams or ointments can come to use.

Furthermore, nose spray or dry powder formulations as well as controlled dosage aerosols are also suitable for systemic administeration of the active ingredients.

These pressure and/or controlled dosage aerosols and dry powder formulations can be inhaled and/or insufflated. Administration forms of this type also certainly have importance for direct, regional application in the lung or bronchi and larynx. Thereby, the dry powder compositions can be formulated for example as active ingredient-soft pellets, as an active ingredient-pellet mixture with suitable carriers, such as for example lactose and/or glucose. For inhalation or insufflation, common applicators are suitable which are suitable for the treatment of the nose, mouth and/or pharynx. The active ingredients can also be applied by means of an ultrasonic nebulizing device. As a propellant gas for aerosol spray formulations and/or controlled dosage aerosols, tetrafluoroethane or HFC 134a and /or heptafluoropropane or HFC 227 are suitable, wherein non-fluorinated hydrocarbons or other propellants which are gaseous at normal pressure and room temperature, such as for example propane, butane or dimethyl ether can be preferred. Instead of controlled dosage aerosols, propellant-free, manual pump systems can also be used.

The propellant gas aerosols can also suitably contain surface active adjuvents, such as for example isopropyl myristate, polyoxyethylene sorbitan fatty acid ester, sorbitan trioleate, lecithins or soya lecithin.

For regional application in situ, solutions for installation, for example for transurethral administration in bladder tumors or genital tumors, or for profusion in liver tumors or other organ carcinomas are suitable.

The respective suitable medicinal forms can be produced in accordance with the prescription and procedures based on pharmaceutical-physical fundamentals as they are described for example in the following handbooks and are included in the present inventive subject-matter with respect to the production of the respective suitable medicaments:
Physical Pharmacy (A.N. Martin, J. Swarbrick, A. Cammarata), 2nd Ed., Philadelphia Pennsylvania, (1970), German version: Physikalische Pharmazie, (1987), 3rd edition, Stuttgart;
R. Voigt, M. Bornschein, Lehrbuch der pharmazeutischen Technologie, Verlag Chemie, Weinheim, (1984), 5th edition;
P.H. List, Arzneimformenlehre, Wissenschaftliche Verlagsgesellschaft mbH, Stuttgart, (1985), 4th edition;
H. Sucker, P. Fuchs, P. Speiser, Pharmazeutische Technologie, Georg Thieme Verlag, Stuttgart - New York, (1991), 2nd edition;
A.T. Florence, D. Attwood, Physicochemical Principles of Pharmacy, The Maximillan Press Ltd., Hong Kong, (1981);
L.A. Trissel, Handbook on Injectable Drugs, American Society of Hospital Pharmacists, (1994), 8th edition;
Y.W. Chien, Transdermal Controlled Systemic Medications, Marcel Dekker Inc., New York - Basel, (1987);
K.E. Avis, L. Lachmann, H.A. Liebermann, Pharmaceutical Dosage Forms: Parenteral Medications, volume 2, Marcel Dekker Inc., New York - Basel, (1986);
B.W. Müller, Controlled Drug Delivery, Paperback APV, volume 17, Wissenschaftliche Verlagsgesellschaft mbH, Stuttgart, (1987);
H. Asch, D. Essig, P.C. Schmidt, Technologie von Salben, Suspensionen und Emulsionen, Wissenschaftliche Verlagsgesellschaft mbH, Stuttgart, (1984);
H.A. Liebermann, L. Lachman, J.B. Schwartz, Pharmaceutical Dosage forms: Tablets, Volume 1, Marcel Dekker Inc., New York, 2nd Edition (1989);
D. Chulin, M. Deleuil, Y. Pourcelot, Powder Technology and Pharmaceutical Processes, in J.C. Williams, T. Allen, Handbook of Powder Technology, Elsevier Amsterdam - London - New York - Tokyo, (1994);
J.T. Carstensen, Pharmaceutical Principles of Solid Dosage Forms, Technomic Publishing Co, Inc., Lancaster - Basel, (1993).

### PRODUCTION EXAMPLES

### 1. Injection therapeutics

### a) Parenteral Solution

| | |
|---|---|
| active ingredient used according to the invention | 5.000 g |
| acid sodium phosphate | 5.000 g |
| sodium tartrate | 12.000 g |
| benzyl alcohol | 7.500 g |
| water for injection purposes | to 1000.000 ml |

The solution is produced according to the customary method, sterilized and filled into 10 ml vials. One vial contains 50 mg of the compound according to the invention.

### b) Penteral Solution

| | |
|---|---|
| active ingredient used according to the invention | 1.000 g |
| hydrochloric acid, dilute | 5.000 g |
| sodium chloride | 6.000 g |
| water for injection purposes to | 1000.000 ml |

The solution is produced according to a customary method by stirring; the medicinal form is adjusted to a suitable pH value by acid addition and subsequently filled into 100 ml vials and sterilized. A vial contains 100 mg of the compound according to the invention.

### c) Parenteral Dispersion

| | |
|---|---|
| active ingredient used according to the invention | 10.000 g |
| soya lecithin | 20.000 g |
| saturated triglycerides | 100.000 g |
| sodium hydroxide | 7.650 g |
| water for injection purposes | to 1000.000 ml |

The active ingredient(s) used according to the invention is dispersed in the saturated triglycerides. Then the soya lecithin is added under stirring, and subsequent to this, the aqueous solution of sodium hydroxide is added with subsequent homogenization.The dispersion is sterilized and filled into 10 ml vials. A vial contains 50 mg of the compound according to the invention.

### d) Biodegradable Parenteral Depot Medicinal Form

| | |
|---|---|
| active ingredient used according to the invention | 10.000 |
| polylactic acid /polygylcolic acid polymer | 70.000 |
| polyvinylpyrrolidone | 0.200 |
| gelatin | 2.000 |
| soya lecithin | 2.000 |
| isotonic sodium chloride solution | to 1000.000 ml |

First, the active ingredient is incorporated into the biodegradable polymer comprising polylactic acid and polyglycolic acid by a suitable method (spray drying, solvent-evaporation or phase separation) and subsequently subjected to a sterilization process. The particles are introduced into a 2-chamber ready-made syringe in which the adjuvent solution, which is also produced in a sterile manner, is filled. The biodegradable microparticles are mixed with the dispersion agent shortly before application and dispersed. The content of a ready-made syringe is measured such that this contains 200 mg of the active ingredient according to the invention.

### e) Parenteral Dispersion for Subcutaneous Installation

| | |
|---|---|
| active ingredient used according to the invention | 25,000 g |
| soya lecithin | 25,000 g |
| arachis oil | 400,000 g |
| benzyl alcohol | 50,000 g |
| Miglyole® | to 1000,000 g |

The active ingredient is dispersed together with soya lecithin and arachis oil. The benzyl alcohol is dissolved in Miglyole® and added to the dispersion. The entire dispersion is sterilized and subsequently filled into vials with 2 ml content. A vial contains 50 mg active ingredient.

### f) Parenteral Perfusions Solution

The solution named under example b) can also be used for perfusion of liver for example.

According to need, instead of ampules with injection solution, so-called perforation bottles (vials), which can also be optionally preserved, and infusion solutions with an amount of one or more active ingredients according to the invention can also be made available in the customary manner under addition of buffer substances for adjustment of physiological pH value and/or the isotonicity and/or a best possible suitable pH value for the medicinal form (euhydria) and optional further required nutrients, vitamins, amino acids, stablizers and other necessary adjuvents, possibly in combination with further medicinal agents suitable for the mentioned indications.

### 2. Solid, Peroral Administration Medicaments

### a) Tablets

| | |
|---|---|
| active ingredient used according to the invention | 10,000 g |
| lactose | 5,200 g |
| starch, soluble | 1,800 g |
| hydroxypropylmethylcellulose | 900 g |
| magnesium stearate | 100 g |

The above components are mixed with each other and compacted in a conventional manner, wherein a tablet weight of 180 mg is set. Each tablet contains 100 mg active ingredient. If desired, the tablets obtained in this manner are coated, provided with a film coat and/or enterically coated.

### b) Coated Tablet Core

| | |
|---|---|
| active ingredient used according to the invention | 10,000 g |
| flame dispersed silicon dioxide | 500 g |
| corn starch | 2,250 g |
| stearic acid | 350 g |
| ethanol | 3.0 l |
| gelatin | 900 g |
| purified water | 10.0 l |
| talcum | 300 g |
| magnesium stearate | 180 g |

From these components, a granulate is produced which is pressed to the desired coated tablet cores. Each core contains 50 mg of active ingredient. The core can be further processed in a customary manner to coated tablets. If desired, a gastric fluid resistant or retarding film coat can be applied in a known manner.

### c) Drink Suspension in Vials

| | |
|---|---|
| active ingredient used according to the invention | 0.050 g |
| glycerin | 0.500 g |
| sorbite, 70% solution | 0.500 g |
| sodium saccharinate | 0.010 g |
| methyl-p-hydroxybenzoate | 0.040 g |
| aromatic agent | q.s. |
| sterile wasser | q.s. to 5 ml |

The above-mentioned components are mixed in a customary manner to a suspension and filled in a suitable drink vial having 5 ml content.

### d) Poorly Soluble Sublingual Tablets

| | |
|---|---|
| active ingredient used according to the invention | 0.030 g |
| lactose | 0.100 g |
| stearic acid | 0.004 g |
| talcum purum | 0.015 g |
| sweetener | q.s. |
| aromatic agent | q.s. |
| rice starch | q.s. to 0.500 g |

The active ingredient is compacted together with the adjuvents under high pressure to sublingual tablets, favorably in oblong form.

### e) Soft Gel Capsule

| | |
|---|---|
| active ingredient used according to the invention | 0.050 g |
| fatty acid glyceride mixture (Miglyole®) | q.s. to 0.500 g |

The active ingredient is impasted together with the fluid carrier mixture and mixed together with further adjuvents suitable for the incapsulation and filled into elastic soft gelatin capsules which are sealed.

### f) Hard Gelatin Capsules

| | |
|---|---|
| active ingredient used according to the invention | 0.150 g |
| microcrystalline cellulose | 0.100 g |
| hydroxypropylmethylcellulose | 0.030 g |
| mannite | 0.100 g |
| ethylcellulose | 0.050 g |
| triethyl citrate | 0.010 g |

The active ingredient is mixed together with the adjuvents, microcrystalline cellulose, hydroxypropylmethylcellulose and mannite, wet with granulation liquid and formed into pellets. These are subsequently coated with a solution of ethylcellulose and triethyl citrate in organic solvents in a fluidized-bed apparatus. A hard gelatin capsule contains 150 mg of active ingredient.

### 3. Topically Administratable Medicinal Forms

### a) Hydrophilic Ointment

| | |
|---|---|
| active ingredient used according to the invention | 0.500 g |
| Eucerinum® anhydricum | 60.000 g |
| microcrystalline wax | 15.000 g |
| vaseline oil | q.s. to 100.000 g |

The above-mentioned adjuvents are melted and further processed together with the active ingredient to an ointment in a customary manner.

### b) Lipophilic Ointment

| | |
|---|---|
| active ingredient used according to the invention | 10.000 g |
| propylene glycol | 50.000 g |
| paraffin, liquid | 100.000 g |
| paraffin wax | 100.000 g |
| vaseline | to 1000.000 ml |

The active ingredient(s) used according to the invention is dissolved in propylene glycol at ca 60°C. At the same time, the lipophilic components are melted at 60-70°C and subsequently combined with the active ingredient solution. The ointment is emulsified at first at 60-70°C and subsequently cooled to 35-40°C under constant emulsification and then filled in 10 g tubes. A tube contains 100 mg of the compound according to the invention.

### 4. Inhalation Therapeutic

Further subject-matter is a pharmaceutical formulation which is characterized in that it contians an active ingredient(s) used according to the invention as a base or a physiologically acceptable salt thereof together with carriers and/or diluents customary for this and suitable for administration by means of inhalation.

In this connection, particularly suitable physiologically acceptable salts of the active ingredients are, as already illustrated in the synthesis section, acid addition salts derived from inorganic or organic acids such as for example especially hydrochloride, hydrobromide, sulfate, phosphate, maleate, tartrate, citrate, benzoate, 4-methoxybenzoate, 2- or 4-hydroxybenzoate, 4-chlorobenzoate, p-toluolsulfonate, methanosulfonate, ascorbate, salicylate, acetate, formate, succinate, lactate, glutarate, gluconate or tricarballylate.

The administration of the active ingredient(s) used of the invention by means of inhalation occurs according to the invention in conventional ways customary for administrations of this form, for example in the form of a commercial controlled dosage aerosol or in combination with a spacer. In controlled dosage aerosols, a metering valve is delivered with whose help, a dosed amount of the composition is administered. For spraying, the present compositions can be formulated for example as aqueous solutions or suspensions and be administered by means of an atomizer. Aerosol spray formulations in which the active ingredient is either suspended with one or two stabilizers in a propellant as a carrier and/or diluent, for example tetrafluoroethane or HFC 134a and/or heptafluoropropane or HFC 227 can equally be used, whereby however, non-fluorinated hydrocarbons or other propellants which are gaseous at normal pressure and room temperature, such as propane, butane or dimethyl ether, can be preferred. Thereby, propellant-free manual pump systems or dry powder systems as desribed below can also be used

Suitably, the propellant aerosols can also contain surface active adjuvents, such as for example isopropyl myristate, polyoxyethylene sorbitan fatty acid ester, sorbitan trioleate, lecithins, oleic acid.

For administration by means of inhalation and/or insufflation, the medicaments with an amount of compounds according to the invention can also be formulated in the form of dry powder compositions, for example as active ingredient-soft pellets or as an active ingredient-powder mixture with a suitable carrier, such as for example lactose and/or glucose The powder compositions can be formulated and administered as single doses or as multiple doses.

The compounds according to the invention are preferably administered by means of a controlled dosage aerosol or in the form of a dry powder dosage formulation, wherein the latter preferably contains glucose and/or lactose as a carrier substance.

As applicators for inhalation of the pharmaceutical formulations containing one or more of the active ingredient(s) used according to the invention, all applicators are generally suitable which are suitable for controlled dosage aerosols and/or a dry powder dosage formulation, such as for example usual applicators for the nose, mouth and or pharynx, or also devices standing under propellant gas for the delivery of a spray (as controlled dosage aerosol or dry powder dosage formulation) as they are also used for inhalations in the nose, mouth and/or pharynx.

A further embodiment can also consist of an aqueous solution of the active ingredient(s) used according to the invention, which also optionally contains further active ingredients and/or additives, which are applied by means of an ultrasound atomizer.

### a) Controlled Dosage Aerosol

| | Intended dose per stroke | per aerosol % by weight |
|---|---|---|
| active ingredient used according to the invention | 0.500 mg | 0.66 |
| stabilizer | 0.075 mg | 0.10 |
| HFC 134a | 75.500 mg | 99.24 |

### b) Controlled Dosage Aerosol

| | Intended dose per stroke | per aerosol % by weight |
|---|---|---|
| active ingredient used according to the invention | 0.250 mg | 0.32 |
| Stabilizer | 0.038 mg | 0.05 |
| HFC 227 | 79.180 mg | 99.63 |

In the examples a) and b) the micronized active ingredient is, after previous dispersion in a small amount of the stabilizer, placed in a suspension vessel in which the bulk amount of propellant gas solution is found. The corresponding suspension is dispersed by means of a suitable stirring system (for example high performance mixer or ultrasound mixer) until an ultra-fine dispersion results. The suspension is then continuously held in flux in a filling apparatus suitable for cold propellants or pressure fillings. Alternatively, the suspension can also be produced in a suitable cooled stabilizer solution in HFC 134a/227.

The examples c) to d) describe the composition and production of dosage dry powder formulations.

### c) Dosage-Dry Powder Formulation

| | mg/dose |
|---|---|
| active ingredient used according to the invention | 0.500 mg |

### d) Dosage-Dry Powder Formulation

| | mg/dose |
|---|---|
| active ingredient used according to the invention | 0.500 mg |
| lactose Ph.Eur. | to 2.5 mg or |
| | to 5.0 mg |

### e) Dosage-Dry Powder Formulation

| | mg/dose |
|---|---|
| active ingredient used according to the invention | 0.250 mg |
| lactose Ph.Eur. | to 2.5 mg or |
| | to 5.0 mg |

Im example c) the active ingredient is formulated after micronization under addition of steam as pellets with an MMAD between 0,1 and 0,3 mm diameter and brought to use in a multi-dose powder applicator.

In the examples d) and e) the active ingredient is micronized, thereafter, bulk material is mixed with the lactose in the given amounts, and subsequently, filled in a multi-dose powder inhalator.

In all of the examples set forth above, the active ingredient or the medicinal agent in the form of the respective suitable pharmaceutical acceptable salt and/or acid addition salts can be present, insofar as the base is not preferred in each case.

In the following, the pharmaceutical test results obtained in connection with the newly found indications based, in a representative manner, on the specifically structured compounds falling under formula (I) are reproduced and the experimental results are discussed.

### PHARMACEUTICAL EXPERIMENTAL SECTION

### 1. Growth Inhibition of Human Tumor Cells

The tumor growth inhibiting activity of the substances was determined on human tumor cells in standardized in vitro test systems. In the screening tests, the substances gave IC₅₀-values in a concentration range of 0.1 nM bis 10 µM.

### Example:

HepG2 cells plated at a density of 20,000 cells/ml in 12-well plastic dishes. Cultivation occured in Richters IMEM-ZO nutrient medium with 5% fetal calf serum (FCS) in a tissue culture incubator with a gas mixture of 5% CO₂ and 95% air at a temperature of 37°C. One day after plating, the culture medium was aspirated from the cells and replaced by fresh medium which contained the respective concentrations of the test substances. For the individual concentrations and the controls without test substances, three-fold batches were done for each. Three days after the beginning of treatment, the medium was again renewed with the test compounds. After six days of substance incubation, the test was ended and the protein amount in the individual wells was determined with the sulforhodamin-B-method (according to P. Skehan et al.: New Colorimetric Cytotoxicity Assay for Anticancer-Drug Screening. J. Natl. Cancer Inst. 82: 1107-1112, 1990). The IC₅₀-values (defined as that concentration in which the cell growth was inhibited by 50%) was taken from the dose-response curves and given as a comparative measurement for the activity of the test compounds.

The following results were obtained:

| **Test substance No.** | **IC**_{**50**}**-value [µM]** |
|---|---|
| 41 | 0.04 |
| 69 | 0.2 |
| 75 | 0.07 |
| 78 | 0.02 |
| 89 | 0.03 |
| 91 | 0.08 |
| 98 | 0.01 |
| 137 | 0.02 |
| 161 | 0.05 |
| 164 | 0.05 |
| 176 | 0.02 |
| 188 | 0.08 |
| 209 | 0.02 |

### 2. Indications

The compounds of formula (1) and their salts permit a therapeutic use in malignant illnesses of humans and animals through their excellent inhibition of the growth of tumor cells. The anti-neoplastic activity of the described substances can be used for prophylactic, adjunct, palliative, and curative treatment of solid tumors, leukemic illnesses and lymphomas as well as for decreasing or preventing metastasis formation in humans and animals. The therapeutic use is possible in the following illnesses for example: gynocological tumors, such as of the vulva or the uterus, ovarian carcinomas, testicle tumors, prostate carcinomas, skin cancer, kidney cancer, bladder tumors, esophagus carcinomas, stomach cancer, rectal carcinomas, pancreas carcinomas, thyroid cancer, adrenal tumors, various types of leukemia and lymphomas, Hodgkin's disease, tumor illnesses of the CNS, soft-tissue sarcomas, bone sarcomas, benign and malignant mesotheliomas, especially intestine cancer, liver cancer, breast cancer, bronchial and lung carcinomas, melanomas, acute and chronic leukemias. Benign papillomatosis tumors can also be inhibited in their growth with the named substances.

The broad activity of the new compounds was tested in-vitro according to the method described under point 1. Thereby, the following IC₅₀-values were obtained for compound number 98:

| **Cell-Line Source** | **IC**_{**50**}**-value [µM]** |
|---|---|
| NCI-H69 small cell lung carcinoma | 0.02 |
| WERI-Rb-1 retinoblastoma | 0.008 |
| THP-1 monocytic leukemia | 0.02 |

With respect to the compounds used according to the invention, an independent activity profile is suggested by the findings with the observed activity against the various tumor types. Thus, tumors which are resistant to customary cytostatic agents, for example, respond entirely to these substances. In addition, based on the independent characteristics of the compounds used according to the invention, combinations of these compounds together with known chemo-therapeutically used pharmaceuticals, for example cytostatic agents or immunosuppresive agents, etc., are created especially taking a possible complimentation of their properties into consideration.

The integration of the presently used compounds with their specific structures in a therapy scheme is successful with one or more substances from the following classes for example: anti-metabolites (for example cytarabine, 5-fluorouracil, 6-mercaptopurine, methotrexate), alkylating agents (for example busulfan, carmustine, cisplatin, carboplatin, cyclophosphamide, dacarbazine, melphalane, thiotepa), DNA-intercalating substances and topoisomerase inhibitors (for example actinomycin D, daunorubicin, doxorubicin, mitomycin C, mitoxantrone, etoposide, teniposide, topotecan, irinotecan), spindle poisons (for example vincristine, navelbin, taxol, taxoter), hormonally active agents (for example tamoxifen, flutamide, formestan, goserelin) or other cytostatic agents with complex modes of action (for example L-asparaginase, bleomycin, hydroxyurea). Resistant tumor cells can be made sensitive again by interaction of the new compounds with a mechanism of resistance for common cytostatic agents (for example P-glycoprotein, MRP, glutathione-S-transferase, metallothionein).
A combination with other therapeutic physical measures or other measures for tumor patients, such as for example radiotherapy, hyperthermia or immuno therapy is also possible with the compounds used according to the invention. Therefore, further subject-matter under the invention is also combinations of this type in form of new medicaments.

### 3. Immuno Suppressing Activity

Many anti-tumor agents have not only a cytotoxic effect on tumor cells, but also on the blood cell system. This leads to a weakening of the immune defence, which can, in turn, be specifically employed to suppress the rejection reaction after an organ transplantation for example. Therefore, a use of the main compounds, optionally in combination with other compounds effective for these indications is suitable in diseases such as psoriasis or autoimmune diseases. In order to test the possibility for a therapeutic use in illnesses of this type, the substance activity was tested on freshly isolated lymphocytes as follows:
The spleen of a Swiss mouse served as a lymphocyte source. The lymphocyte population was isolated from the spleen cell suspension over a ficoll gradient and taken up in IMEM-ZO culture medium with 0,1% dextran 70,000 and 2% fetal calf serum. The cells were plated at a density of ca. 500,000 cells/well/ml in a 12-well plate, 1 ml doubly concentrated test substance solution was pipetted per well and this was subsequently incubated in a tissue culture incubator at 37°C and 5% CO₂. After 2 days, a 1 ml-aliquot with 5 µl of the fluorescent dye solutions propidium iodide (8 mg/ml) and 3,3'-dihexyloxacarbocyanin iodide (40 µg/ml) each was added per well, and incubated for 3 minutes at room temperature. Subsequently, 10,000 cells per each sample were measured on a flow-through cytometer and the percentage amount of vital cells in the population was determined. By means of the dose-response curves, IC₅₀-values were calculated which were also employed in the following Tables for the characterization of the individual substances:

| Test Substance No. | IC₅₀ [µM] |
|---|---|
| 41 | 0.03 |
| 242 | 0.0002 |
| 244 | 0.00008 |
| 294 | 0.002 |
| 303 | 0.00004 |

Hence, the special, independent class of the compounds used according to the invention is also suitable for a combination with known immunosuppressive agents from the class of macrolides, for example cyclosporin A, tacrolimus, rapamycin, or anti-metabolites, for example azathioprin or methotrexate and glucocorticoids. Combinations of this and/or medicaments with an amount of compounds used according to the invention together with known immunosuppressive agents as well as a method for their production represents further subject-matter of the invention.

The invention is in no way limited to the present respective concretely named active ingredient concentrations, dosages, combinations with one or more other cytostatic agents, tumor inhibitors, cancerostatic agents, immunosuppressive agents or further medicinal agents suitable for the respective specific indications or the type of tumor to treated or immunological illness, etc.

## Claims

1. Use of one or more of the compounds of formula (I) 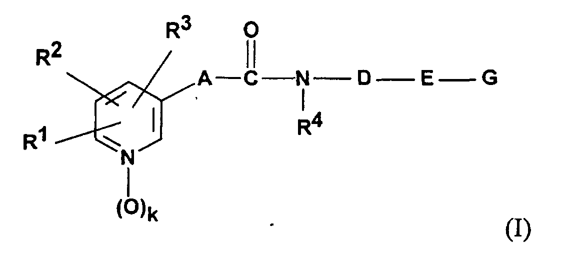 wherein
**R**^{**1**} is selected from the group consisting of hydrogen, halogen, cyano, C₁-C₆-alkyl, trifluoromethyl, C₃-C₈-cycloalkyl, C₁-C₄-hydroxyalkyl, hydroxy, C₁-C₄-alkoxy, benzyloxy, C₁-C₄-alkanoyloxy, C₁-C₄-alkylthio, C₂-C₅-alkoxycarbonyl, aminocarbonyl, C₃-C₉-dialkylaminocarbonyl, carboxy, phenyl, phenoxy, pyridyloxy and **NR**^{**5**}**R**^{**6**}, wherein
**R**^{**5**} and
**R**^{**6**} are selected, independently from each other, from hydrogen and C₁-C₆-alkyl,
**R**^{**2**} is selected from the group consisting of hydrogen, halogen, C₁-C₆-alkyl, trifluoromethyl and hydroxy, wherein
**R**^{**1**} and **R**^{**2**}, in the case they are adjacent, optionally form a bridge which is selected from the group of bridge members -(CH₂)₄- and -(CH=CH)₂- and -CH₂O-**CR**^{**7**}**R**^{**8**}-O-, wherein
**R**^{**7**} and
**R**^{**8**} are independent from each other, hydrogen or C₁-C₆-alkyl,
**R**^{**3**} is selected from hydrogen, halogen and C₁-C₆-alkyl,
**R**^{**4**} is selected from hydrogen, C₁-C₆-alkyl, C₃-C₆-alkenyl, hydroxy, C₁-C₆-alkoxy and benzyloxy,
**k** is 0 or 1,
**A** is selected from C₁-C₆-alkylene, which is optionally substituted once to three-fold by C₁-C₃-alkyl, hydroxy, fluorine or phenyl,
1,2-cyclopropylene,
C₂-C₆-alkenylene, which is optionally substituted one to three-fold by C₁-C₃-alkyl, hydroxy, fluorine, cyano, or phenyl,
C₄-C₆-alkadienylene, which is optionally substituted once or twice by C₁-C₃-alkyl, fluorine, cyano, or phenyl,
1,3,5-hexatrienylene, which is optionally substituted by C₁-C₃-alkyl, fluorine, or cyano,
ethinylene and
C₂-C₆-alkylene, wherein a methylene unit is isosterically replaced by O, S, **NR**^{**9**}, CO, SO or SO₂, and wherein the isosteric substitute, with the exception of =CO, is not adjacent to the amide group, and wherein
**R**^{**9**} is selected from hydrogen, C₁-C₃-alkyl, C₁-C₆-acyl and methanesulfonyl,
**D** is selected from C₁-C₁₀-alkylene, which is optionally substituted once or twice by C₁-C₃-alkyl or hydroxy,
C₂-C₁₀-alkenylene, optionally substituted once or twice by C₁-C₃-alkyl or hydroxy, wherein the double bond optionally is to ring E,
C₃-C₁₀-alkinylene, which is optionally substituted once or twice by C₁-C₃-alkyl or hydroxy, and
the group consisting of C₁-C₁₀-alkylene, C₂-C₁₀-alkenylene or C₃-C₁₀-alkinylene, in which one to three methylene units are isosterically replaced by O, S, **NR**^{**10**}, CO, SO or SO₂, wherein
**R**^{**10**} has the same meaning as **R**^{**9**}**,** but is selected independently therefrom,
**E** is selected from 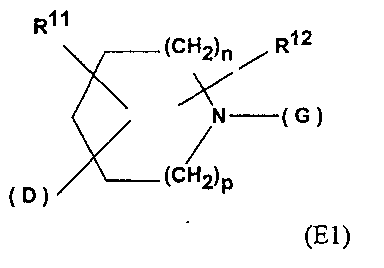 and 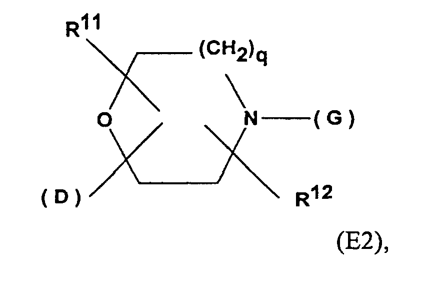 wherein the heterocyclic ring optionally has a double bond and
**n** and **p** are, independent of each other, 0, 1, 2 or 3, with the proviso that **n** + **p** ≤ 4,
**q** is 1 or 2,
**R**^{**11**} is selected from hydrogen, C₁-C₃-alkyl, hydroxy, hydroxymethyl, carboxy and C₂-C₇-alkoxycarbonyl,
**R**^{**12**} is selected from hydrogen and an oxo group adjacent to the nitrogen atom,
**G** is selected from hydrogen, **G1, G2, G3, G4** and **G5,** wherein
**G1** represents the residue
**―(CH**_{**2**}**)**_{**r**}**―(CR**^{**14**}**R**^{**15**}**)**_{**s**}**―R**^{**13**} (G1)
wherein
**r** is 0, 1 or 2,
**s** is 0 or 1,
**R**^{**13**} is selected from hydrogen, C₁-C₆-alkyl, C₃-C₆-alkenyl, C₃-C₆-alkinyl, C₃-C₈-cycloalkyl,
benzyl, phenyl,
the group consisting of monocyclic aromatic five- to six-membered heterocycles, which contain one to three hetero-atoms from N, S or O and are either bound directly or over a methylene group,
the group consisting of anellated bi- and tricyclic aromatic or partially hydrogenated carbocyclic ring systems with 8 to 16 ring atoms and at least one aromatic ring, whereby the bond occurs either over an aromatic or a hydrogenated ring and either directly or over a methylene group, and
the group consisting of anellated bi- and tricyclic aromatic or partially hydrogenated heterocyclic ring systems with 8 to 16 ring atoms and at least one aromatic ring, wherein one to three ring atoms are selected from N, S and O and the bond occurs either over an aromatic or a hydrogenated ring and either directly or over a methylene group,
**R**^{**14**} has the same meaning as **R**^{**13**}, but is selected independently thereof,
**R**^{**15**} is selected from hydrogen, hydroxy, methyl, benzyl, phenyl,
the group consisting of monocyclic aromatic five- to six-membered heterocycles, which contain one to three hetero-atoms selected from N, S or O and are bound either directly or over a methylene group,
the group consisting of anellated bi- and tricyclic aromatic or partially hydrogenated carbocyclic ring systems with 8 to 16 ring atoms and at least one aromatic ring, wherein the bond occurs either over an aromatic or a hydrogenated ring and either directly or over a methylene group, and
the group consisting of anellated bi- and tricyclic aromatic or partially hydrogenated heterocyclic ring systems with 8 to 16 ring atoms and at least one aromatic ring, wherein one to three ring atoms are selected from N, S or O and the bond occurs either over an aromatic or a hydrogenated ring and either directly or over a methylene group,
**G2** is selected from the residues 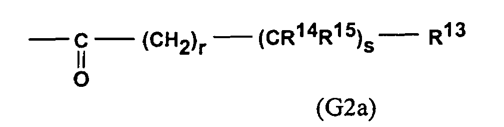 and 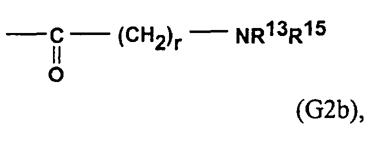 wherein the substituents **R**^{**13**} and **R**^{**15**} have the above meaning, or the group
**―NR**^{**13**}**R**^{**15**}
is a nitrogen-containing heterocycle bound over the nitrogen atom, the nitrogen-containing heterocycle being selected from
the group consisting of saturated and unsaturated monocyclic, four- to eight-membered heterocycles, which, aside from the essential nitrogen atom, optionally contain one or two further hetero-atoms selected from N, S and O, and
the group consisting of saturated and unsaturated bi- or tricyclic, anellated or bridged heterocycles with 8 to 16 ring atoms, which, aside from the essential nitrogen atom, optionally contain one or two further hetero-atoms selected from N, S and O,
**G3** is the residue
**―SO**_{**2**}**―(CH**_{**2**}**)**_{**r**} **R**^{**13**} (G3),
**G4** is the residue 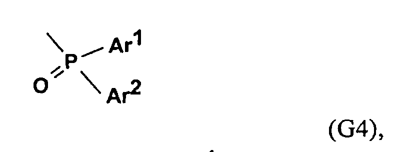 wherein
**Ar**^{**1**} and
**Ar**^{**2**} are selected independently of each other from phenyl, pyridyl and naphthyl,
**G5** is the residue
**―COR**^{**16**} (G5)
wherein
**R**^{**16**} is selected from trifluoromethyl, C₁-C₆-alkoxy, C₃-C₆-alkenyloxy and benzyloxy,
wherein aromatic ring systems in the substituents **R**^{**1**}**, R**^{**2**}**, R**^{**4**}**, R**^{**13**}**, R**^{**14**}**, R**^{**15**}**, R**^{**16**}**, Ar**^{**1**} and **Ar**^{**2**} and in the ring system **-NR**^{**13**}**R**^{**15**} optionally carry independently of each other one to three of the same or different substituents selected from the group consisting of halogen, cyano, C₁-C₆-alkyl, trifluoromethyl, C₃-C₈-cycloalkyl, phenyl, benzyl, hydroxy, C₁-C₆-alkoxy, which is optionally entirely or partially substituted by fluorine, benzyloxy, phenoxy, mercapto, C₁-C₆-alkylthio, carboxy, C₁-C₆-alkoxycarbonyl, benzyloxycarbonyl, nitro, amino, mono-C₁-C₆-alkylamino, and di-(C₁-C₆-alkyl)-amino, wherein two adjacent groups of the aromatic ring or ring system optionally form an additional ring over a methylenedioxy bridge
tautomeres in the case of substitution of the heterocycle or in an anellated ring system by free hydroxy, mercapto and/or amino groups,
stereoisomers and/or mixtures thereof and pharmacologically acceptable acid addition salts
for the production of medicaments for cytostatic or immunomodulatory and/or immunosuppressive treatment.

2. The use of compounds according to claim 1, wherein
**R**^{**1**} is selected from hydrogen, halogen, cyano, methyl, trifluoromethyl, hydroxy, C₁-C₄-alkoxy, ethylthio, methoxycarbonyl, tert-butoxycarbonyl, aminocarbonyl, carboxy, and phenoxy,
**R**^{**2**} is selected from hydrogen, halogen, trifluoromethyl and hydroxy,
**R**^{**3**} is hydrogen or halogen,
**R**^{**4**} is selected from hydrogen, C₁-C₃-alkyl, hydroxy and C₁-C₃-alkoxy,
**k** is 0 or 1,
**A** is selected from the group consisting of C₂-C₆-alkylene, which is optionally substituted once or twice by C₁-C₃-alkyl, hydroxy or fluorine, as
C₂-C₆-alkenylene, which is optionally substituted once or twice by C₁-C₃-alkyl, hydroxy or fluorine,
C₄-C₆-alkadienylene, which is optionally substituted by is C₁-C₃-alkyl or by one or two fluorine atoms,
1,3,5-hexatrienylene, which is optionally substituted by fluorine, and
C₂-C₆-alkylene, wherein a methylene unit is isosterically replaced by O, S, CO or SO₂, and the isosteric substitute, with the exception of=CO is not adjacent to the amide group and,
**D** is selected from C₁-C₈-alkylene, which is optionally substituted once or twice by methyl or hydroxy,
C₂-C₈-alkenylene, which is optionally substituted once or twice by methyl or hydroxy, wherein the double bond optionally is to ring E,
C₃-C₈-alkinylene, which is optionally substituted once or twice by methyl or hydroxy, and
the group consisting of C₁-C₈-alkylene, C₂-C₈-alkenylene and C₃-C₈-alkinylene, in which one to three methylene units are isosterically replaced by O, S, NH, N(CH₃), N(COCH₃), N(SO₂CH₃), CO, SO or SO₂,
**E** is selected from 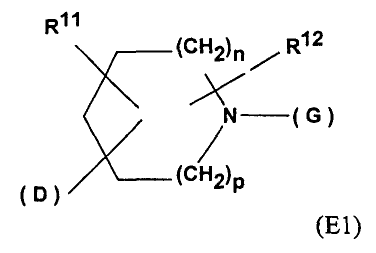 and 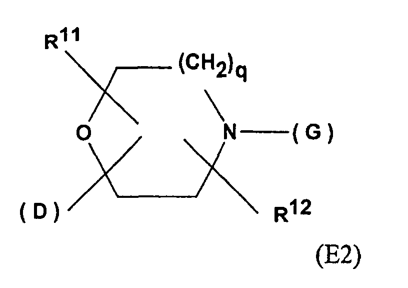 wherein the heterocyclic ring optionally has a double bond and
**n** and
**p** are independent of each other 0, 1, 2 or 3, with the proviso that **n** + **p** ≤ 3,
**q** is 1 or 2,
**R**^{**11**} is selected from hydrogen, C₁-C₃-alkyl, hydroxy, and hydroxymethyl,
**R**^{**12**} is hydrogen or an oxo group, which is adjacent to the nitrogen atom,
**G** is selected from hydrogen, **G1, G2, G3, G4** and **G5,** wherein
**G1** represents the residue
**―(CH**_{**2**}**)**_{**r**}**―(CR**^{**14**}**R**^{**15**}**)**_{**s**}**―R**^{**13**} (G1)
wherein
**r** is 0, 1 or 2,
**s** is 0 or 1,
**R**^{**13**} is selected from hydrogen, C₁-C₆-alkyl, C₃-C₈-cycloalkyl, benzyl, phenyl,
the group consisting of benzocyclobutyl, indanyl, indenyl, oxoindanyl, naphthyl, dihydronaphthyl, tetrahydronaphthyl, oxotetrahydronaphthyl, biphenylenyl, fluorenyl, oxofluorenyl, anthryl, dihydroanthryl, oxodihydroanthryl, dioxodihydroanthryl, phenanthryl, dihydrophenanthryl, oxodihydrophenanthryl, dibenzocycloheptenyl, oxodibenzocycloheptenyl, dihydrodibenzocycloheptenyl, oxodihydrodibenzocycloheptenyl, dihydrodibenzocyclooctenyl, tetrahydrodibenzocyclooctenyl and oxotetrahydrodibenzocyclooctenyl, bound directly or over a methylene group, and
the group consisting of furyl, thienyl, pyrrolyl, oxazolyl, isoxazolyl, thiazolyl, isothiazolyl, pyrazolyl, imidazolyl, oxadiazolyl, thiadiazolyl, triazolyl, pyridyl, pyrazinyl, pyridazinyl, pyrimidinyl, triazinyl, imidazothiazolyl, benzofuryl, dihydrobenzofuryl, benzothienyl, dihydrobenzothienyl, indolyl, indolinyl, oxoindolinyl, dioxoindolinyl, benzoxazolyl, oxobenzoxazolinyl, benzisoxazolyl, oxobenzisoxazolinyl, benzothiazolyl, oxobenzthiazolinyl, benzoisothiazolyl, oxobenzoisothiazolinyl, benzimidazolyl, oxobenzimidazolinyl, indazolyl, oxoindazolinyl, benzofurazanyl, benzothiadiazolyl, benzotriazolyl, oxazolopyridyl, oxodihydrooxazolopyridyl, thiazolopyridyl, oxodihydrothiazolopyridyl, isothiazolopyridyl, imidazopyridyl, oxodihydroimidazopyridyl, pyrazolopyridyl, oxodihydropyrazolopyridyl, thienopyrimidinyl, chromanyl, chromanonyl, benzopyranyl, chromonyl, quinolyl, isoquinolyl, dihydroquinolyl, oxodihydroquinolinyl, tetrahydroquinolyl, oxotetrahydroquinolinyl, benzodioxanyl, quinoxalinyl, quinazolinyl, naphthyridinyl, carbazolyl, tetrahydrocarbazolyl, oxotetrahydrocarbazolyl, pyridoindolyl, acridinyl, oxodihydroacridinyl, phenothiazinyl, dihydrodibenzoxepinyl, oxodihydrodibenzoxepinyl, benzocycloheptathienyl, oxobenzocycloheptathienyl, dihydrothienobenzothiepinyl, oxodihydrothienobenzothiepinyl dihydrodibenzothiepinyl, oxodihydrodibenzothiepinyl, octahydrodibenzothiepinyl, dihydrodibenzazepinyl, oxodihydrodibenzazepinyl, octahydrodibenzazepinyl, benzocycloheptapyridyl, oxobenzocycloheptapyridyl, dihydropyridobenzodiazepinyl, dihydrodibenzoxazepinyl, dihydropyridobenzoxepinyl, dihydropyridobenzoxazepinyl, oxodihydropyridobenzoxazepinyl, dihydrodibenzothiazepinyl, oxodihydrodibenzothiazepinyl, dihydropyridobenzothiazepinyl, and oxodihydropyridobenzothiazepinyl, bound directly or over a methylene group,
**R**^{**14**} has the same meaning as **R**^{**13**}, but is selected independently therefrom,
**R**^{**15**} is selected from hydrogen, hydroxy, methyl, benzyl, phenyl, and
the group consisting of indanyl, indenyl, naphthyl, dihydronaphthyl, tetrahydronaphthyl, furyl, thienyl, pyrrolyl, oxazolyl, isoxazolyl, thiazolyl, isothiazolyl, pyrazolyl, imidazolyl, oxadiazolyl, thiadiazolyl, triazolyl, pyridyl, pyrazinyl, pyridazinyl, pyrimidinyl, triazinyl, benzofuryl, benzothienyl, indolyl, indolinyl, benzoxazolyl, benzothiazolyl, benzimidazolyl, chromanyl, quinolyl and tetrahydroquinolyl, bound directly or over a methylene group,
**G2** is selected from the residues 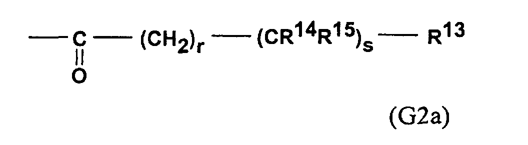 and 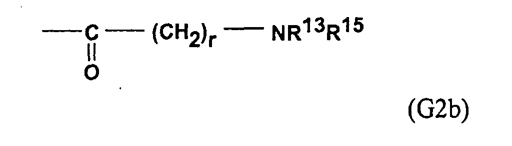 wherein the substituents **R**^{**13**} and **R**^{**15**} have the above meanings, or the group
**―NR**^{**13**}**R**^{**15**}
represents a nitrogen-containing heterocycle bound over the nitrogen atom, the nitrogen-containing heterocycle being selected from the group consisting of azetidine, pyrrolidine, piperidine, (1H)tetrahydropyridine, hexahydroazepine, (1H)tetrahydroazepine, octahydroazocine, pyrazolidine, piperazine, hexyhydrodiazepine, morpholine, hexahydrooxazepine, thiomorpholine, thiomorpholine-1,1-dioxide, 5-aza-bicyclo[2.1.1]hexane, 2-azabicyclo[2.2.1]heptane, 7-aza-bicyclo[2.2.1]heptane, 2,5-diaza-bicyclo[2.2.1]-heptane, 2-aza-bicyclo[2.2.2]octane, 8-aza-bicyclo[3.2.1]octane, 2,5-diazabicyclo[2.2.2]octane, 9-azabicyclo[3.3.1]nonane, indoline, isoindoline, (1H)-dihydroquinoline, (1H)-tetrahydroquinoline, (2H)-tetrahydroisoquinoline, (1H)-tetrahydroquinoxaline, (4H)-dihydrobenzoxazine, (4H)-dihydrobenzothiazine, (1H)-tetrahydrobenzo[b]azepine, (1H)-tetrahydrobenzo[c]azepine, (1H)-tetrahydrobenzo[d]azepine, (5H)-tetrahydrobenzo[b]oxazepine, (5H)-tetrahydrobenzo[b]thiazepine, 1,2,3,4-tetrahydro-9H-pyrido[3,4-b]indole, (10H)-dihydroacridine, 1,2,3,4-tetrahydroacridanone, (10H)-phenoxazine, (10H)-phenothiazine, (5H)-dibenzazepine, (5H)-dihydrodibenzazepine, (5H)-octahydrodibenzazepine, (5H)-dihydrodibenzodiazepine, (11H)-dihydrodibenzo[b,e]oxazepine, (1lH)-dihydrodibenzo[b,e]thiazepine, (10H)-dihydrodibenzo[b,f]oxazepine, (10H)-dihydrodibenzo[b,f]thiazepine and (5H)-tetrahydrodibenzazocine,
**G3** is the residue
**―SO**_{**2**}**―(CH**_{**2**}**)**_{**r**} **R**^{**13**} (G3),
**G4** is the residue 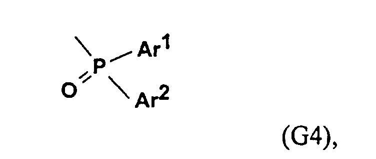 wherein
**Ar**^{**1**} and
**Ar**^{**2**} are selected independently of each other from phenyl, pyridyl and naphthyl,
**G5** is the residue
**―COR**^{**16**} (G5)
wherein
**R**^{**16**} is selected from trifluoromethyl, C₁-C₆-alkoxy, C₃-C₆-alkenyloxy and benzyloxy,
wherein aromatic ring systems optionally are substituted independently of each other by one to three of the same or different substituents selected from the group consisting of halogen, cyano, C₁-C₆-alkyl, trifluoromethyl, C₃-C₈-cycloalkyl, phenyl, benzyl, hydroxy, C₁-C₆-alkoxy, C₁-C₆-alkoxy entirely or partially substituted by fluorine; benzyloxy, phenoxy, mercapto, C₁-C₆-alkylthio, carboxy, C₁-C₆-alkoxycarbonyl, benzyloxycarbonyl, nitro, amino, mono-C₁-C₆-alkylamino, and di-(C₁-C₆-alkyl)-amino, wherein two adjacent groups of the aromatic ring or ring system optionally form an additional ring over a methylenedioxy bridge.

3. The use of compounds according to claim 1 or 2, wherein
**R**^{**1**} is selected from hydrogen, halogen, cyano, methyl, trifluoromethyl, hydroxy, methoxy and methoxycarbonyl,
**R**^{**2**} is hydrogen or halogen,
**R**^{**3**} is hydrogen,
**R**^{**4**} is hydrogen, C₁-C₃-alkyl or hydroxy,
**k** is 0 or 1,
**A** is selected from C₂-C₆-alkylene, which is optionally substituted once or twice by hydroxy or fluorine,
C₂-C₆-alkenylene, which is optionally substituted once or twice by hydroxy or fluorine,
C₄-C₆-alkadienylene, which is optionally substituted by one or two fluorine atoms,
1,3,5-hexatrienylene and
C₂-C₆-alkylene, wherein a methylene unit is isosterically replaced by O, S or CO, and the isosteric substitute, with the exception of=CO, is not adjacent to the amide group and,
**D** is selected from C₂-C₈-alkylene, which is optionally substituted by methyl or hydroxy
C₂-C₈-alkenylene, which is optionally substituted by methyl or hydroxy, wherein the double bond optionally is to ring E, and
the group consisting of C₂-C₈-alkylene and C₂-C₈-alkenylene, wherein one to three methylene units are isosterically replaced by O, NH, N(CH₃), N(COCH₃), N(SO₂CH₃) or CO,
**E** is selected from 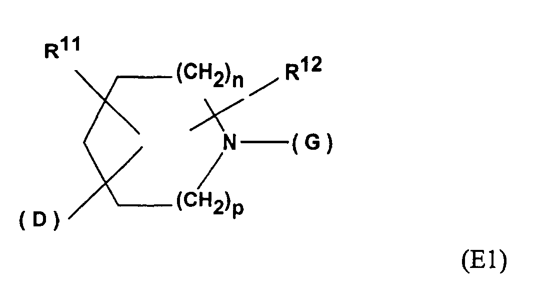 and 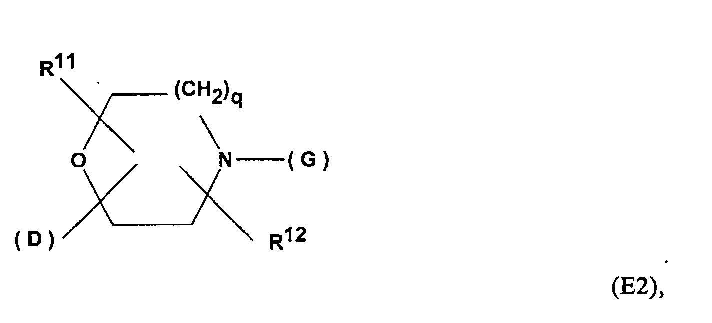 wherein the heterocyclic ring optionally has a double bond and
**n** and p are, independent of each other, 0, 1, 2 or 3, with the proviso that **n** + **p** ≤ 3,
**q** is 1 or 2
**R**^{**11**} is selected from the group consisting of hydrogen, methyl and hydroxyl,
**R**^{**12**} is hydrogen or an oxo group adjacent to the nitrogen atom,
**G** is selected from hydrogen, C₃-C₈-cycloalkyl, methoxycarbonyl, tert-butoxycarbonyl, benzyloxycarbonyl, trifluoroacetyl, diphenylphosphinoyl and the residues
**―(CH**_{**2**}**)**_{**r**}**―(CR**^{**14**}**R**^{**15**}**)**_{**s**}**―R**^{**13**} (G1), 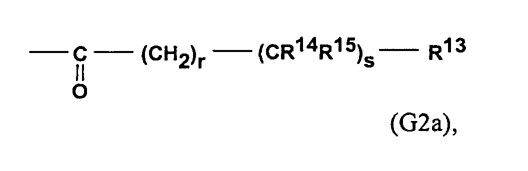 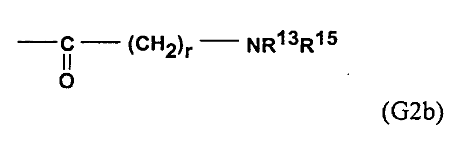
and
**―SO**_{**2**}**―(CH**_{**2**}**)**_{**r**} **R**^{**13**} (G3)
wherein
**r** is 0, 1 or 2,
**s** is 0 or 1,
**R**^{**13**} is selected from hydrogen, methyl, benzyl, phenyl,
the group consisting of indanyl, indenyl, oxoindanyl, naphthyl, dihydronaphthyl, tetrahydronaphthyl, oxotetrahydronaphthyl, fluorenyl, oxofluorenyl, anthryl, dihydroanthryl, oxodihydroanthryl, dioxodihydroanthryl, dibenzocycloheptenyl, oxodibenzocycloheptenyl, dihydrodibenzocycloheptenyl, and oxodihydrodibenzocycloheptenyl bound directly or over a methylene group, and
the group consisting of furyl, thienyl, pyrrolyl, oxazolyl, isoxazolyl, thiazolyl, isothiazolyl, pyrazolyl, imidazolyl, oxadiazolyl, thiadiazolyl, triazolyl, pyridyl, pyrazinyl, pyridazinyl, pyrimidinyl, imidazothiazolyl, benzofuryl, dihydrobenzofuryl, benzothienyl, dihydrobenzothienyl, indolyl, indolinyl, oxoindolinyl, dioxoindolinyl, benzoxazolyl, oxobenzoxazolinyl, benzisoxazolyl, oxobenzisoxazolinyl, benzothiazolyl, oxobenzthiazolinyl, benzoisothiazolyl, oxobenzoisothiazolinyl, benzimidazolyl, oxobenzimidazolinyl, benzofurazanyl, benzothiadiazolyl, benzotriazolyl, oxazolopyridyl, oxodihydrooxazolopyridyl, thiazolopyridyl, oxodihydrothiazolopyridyl, isothiazolopyridyl, imidazopyridyl, oxodihydroimidazopyridyl, pyrazolopyridyl, thienopyrimidinyl, chromanyl, chromanonyl, benzopyranyl, chromonyl, quinolyl, isoquinolyl, dihydroquinolyl, oxodihydroquinolinyl, tetrahydroquinolyl, oxotetrahydroquinolinyl, benzodioxanyl, quinoxalinyl, quinazolinyl, naphthyridinyl, carbazolyl, tetrahydrocarbazolyl, oxotetrahydrocarbazolyl, pyridoindolyl, acridinyl, oxodihydroacridinyl, phenothiazinyl, dihydrodibenzoxepinyl, benzocycloheptathienyl, oxobenzocycloheptathienyl, dihydrothienobenzothiepinyl, oxodihydrothienobenzothiepinyl dihydrodibenzothiepinyl, oxodihydrodibenzothiepinyl, dihydrodibenzazepinyl, oxodihydrodibenzazepinyl, octahydrodibenzazepinyl, benzocycloheptapyridyl, oxobenzocycloheptapyridyl, dihydropyridobenzoxepinyl, dihydrodibenzothiazepinyl, and oxodihydrodibenzothiazepinyl, bound directly or over a methylene group,
**R**^{**14**} is selected from the group consisting of hydrogen, methyl, benzyl and phenyl,
**R**^{**15**} is selected from hydrogen, hydroxy, methyl, benzyl, phenyl, and
the group consisting of naphthyl, furyl, thienyl, oxazolyl, thiazolyl, pyrazolyl, imidazolyl, oxadiazolyl, thiadiazolyl, pyridyl, benzofuryl, benzothienyl, indolyl, indolinyl, benzoxazolyl; benzothiazolyl, benzimidazolyl, chromanyl, quinolyl and tetrahydroquinolyl, bound directly or over a methylene group,
wherein in formula 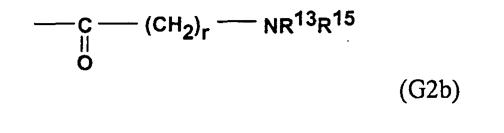 the group **NR**^{**13**}**R**^{**15**} optionally is selected from
pyrrolidine, piperidine, (1H)tetrahydropyridine, hexahydroazepine, octahydroazocine, piperazine, hexahydrodiazepine, morpholine, hexahydrooxazepine, 2-azabicyclo[2.2.1]heptane, 7-azabicyclo[2.2.1]heptane, 2,5-diazabicyclo[2.2.1]heptane, 8-azabicyclo[3.2.1]octane, 2,5-diazabicyclo[2.2.2]octane, indoline, isoindoline, (1H)-dihydroquinoline, (1H)-tetrahydroquinoline, (2H)-tetrahydroisoquinoline, (1H)-tetrahydroquinoxaline, (4H)-dihydrobenzoxazine, (4H)-dihydrobenzothiazine, (1H)-tetrahydrobenzo[b]azepine, (1H)-tetrahydrobenzo [d]azepine, (5H)-tetrahydrobenzo[b]oxazepine, (5H)-tetrahydrobenzo[b]thiazepine, 1,2,3,4-tetrahydro-9H-pyrido[3,4-b]indol, (10H)-dihydroacridine, 1,2,3,4-tetrahydroacridanone, (5H)-dihydrodibenzazepine, (5H)-dihydrodibenzodiazepine, (11H)-dihydrodibenzo[b,e]oxazepine, (11H)-dihydrodibenzo[b,e]thiazepine, (10H)-dihydrodibenzo[b,f]oxazepine and (5H)-tetrahydrodibenzazocine.

4. The use of compounds according to claims 1 to 3, wherein
**R**^{**1**} is selected from hydrogen, fluorine, chlorine, bromine, methyl, trifluoromethyl and hydroxy,
**R**^{**2**} and
**R**^{**3**} are hydrogen,
**R**^{**4**} is hydrogen or hydroxy,
**k** is 0 or 1,
**A** is selected from C₂-C₆-alkylene, which is optionally substituted once or twice by hydroxy or fluorine ,
C₂-C₄-alkenylene, which is optionally substituted by fluorine, and
1,3-butadienylene, which is optionally substituted by fluorine,
**D** is selected from C₂-C₆-alkylene, C₂-C₆-alkenylene, wherein the double bond optionally is to ring E, and the group consisting of C₂-C₆-alkylene and C₂-C₆-alkenylene, wherein a methylene unit is isosterically replaced by O, NH, N(CH₃) or CO, or an ethylene group is isosterically replaced by NH-CO or CO-NH or a propylene group is isosterically replaced by NH-CO-O or O-CO-NH,
**E** is selected from pyrrolidine, piperidine, 1,2,5,6-tetrahydropyridine, hexahydroazepine, morpholine and hexahydro-1,4-oxazepine, wherein the heterocyclic ring , optionally is substituted by an oxo group adjacent to the nitrogen atom
**G** is selected from **hydrogen**, tert-butoxycarbonyl, diphenylphosphinoyl, and of the residues
**―(CH**_{**2**}**)**_{**r**}**―(CR**^{**14**}**R**^{**15**}**)**_{**s**}**―R**^{**13**} (G1), 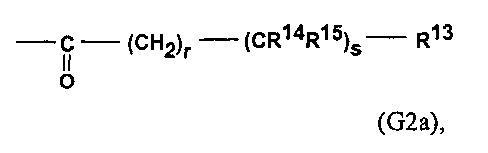 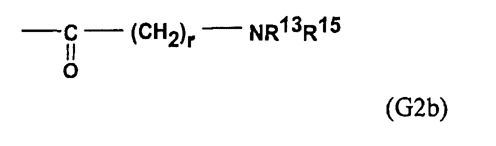
and
**―SO**_{**2**}**―(CH**_{**2**}**)**_{**r**} **R**^{**13**} (G3)
wherein
**r** is 0 or 1,
**s** is 0 or 1,
**R**^{**13**} is selected from hydrogen, methyl, benzyl, phenyl,
the group consisting of indenyl, oxoindanyl, naphthyl, tetrahydronaphthyl, fluorenyl, oxofluorenyl, anthryl, dihydroanthryl, oxodihydroanthryl, dioxodihydroanthryl, dibenzocycloheptenyl, and dihydrodibenzocycloheptenyl bound directly or over a methylene group, and
the group consisting of furyl, thienyl, oxazolyl, thiazolyl, imidazolyl, oxadiazolyl, thiadiazolyl, pyridyl, pyrazinyl, pyrimidinyl, imidazothiazolyl, benzofuryl, benzothienyl, indolyl, oxoindolinyl, dioxoindolinyl, benzoxazolyl, oxobenzoxazolinyl, benzothiazolyl, oxobenzthiazolinyl, benzimidazolyl, oxobenzimidazolinyl, benzofurazanyl, benzotriazolyl, oxazolopyridyl, oxodihydrooxazolopyridyl, thiazolopyridyl, oxodihydrothiazolopyridyl, chromanyl, chromanonyl, benzopyranyl, chromonyl, quinolyl, isoquinolyl, oxodihydroquinolinyl, tetrahydroquinolyl, oxotetrahydroquinolinyl, benzodioxanyl, quinazolinyl, acridinyl, oxodihydroacridinyl, phenothiazinyl, dihydrodibenzoxepinyl, benzocycloheptathienyl, dihydrothienobenzothiepinyl, dihydrodibenzothiepinyl, oxodihydrodibenzothiepinyl, dihydrodibenzazepinyl, oxodihydrodibenzazepinyl, octahydrodibenzazepinyl, benzocycloheptapyridyl, oxobenzocycloheptapyridyl, and dihydrodibenzothiazepinyl, bound directly or over a methylene group,
**R**^{**14**} is hydrogen, methyl, benzyl or phenyl,
**R**^{**15**} is selected from the group consisting of hydrogen, hydroxy, methyl, benzyl, phenyl, and
the group consisting of naphthyl, furyl, thienyl, pyridyl, benzofuryl, benzothienyl, indolyl, benzoxazolyl, benzothiazolyl, benzimidazolyl, chromanyl, quinolyl and tetrahydroquinolyl bound directly or over a methylene group,
wherein in the formula 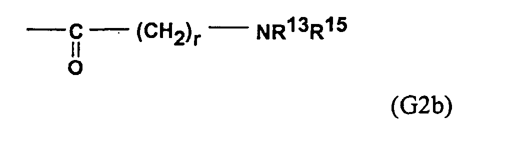 the group **NR**^{**13**}**R**^{**15**} optionally is selected from pyrrolidine, piperidine, hexahydroazepine, morpholine, 2,5-diazabicyclo[2.2.1]heptane, indoline, isoindoline, (1H)-dihydroquinoline, (1H)-tetrahydroquinoline, (2H)-tetrahydroisoquinoline, (1H)-tetrahydrobenzo[b]azepine, (1H)-tetrahydrobenzo[d]azepine, (5H)-tetrahydrobenzo[b]oxazepine, (5H)-tetrahydrobenzo[b]thiazepine, 1,2,3,4-tetrahydroacridanone, (5H)-dihydrodibenzazepine, (11H)-dihydoodibenzo[b,e]-oxazepine and (11H)-dihydrodibenzo[b,e]thiazepine,
wherein aromatic ring systems optionally are substituted, independently of each other, by one to three of the same or different substituents selected from the group consisting of halogen, cyano, C₁-C₆-alkyl, trifluoromethyl, C₃-C₈-cycloalkyl, phenyl, benzyl, hydroxy, C₁-C₆-alkoxy, C₁-C₆-alkoxy which is entirely or partially substituted by fluorine; benzyloxy, phenoxy, mercapto, C₁-C₆-alkylthio, carboxy, C₁-C₆-alkoxycarbonyl, benzyloxycarbonyl, nitro, amino, mono-C₁-C₆-alkylamino and di-(C₁-C₆-alkyl)-amino, wherein two adjacent groups of the aromatic ring or ring system optionally form an additional ring over a methylenedioxy bridge.

5. The use of compounds according to claims 1 to 4, wherein
**R**^{**1**} is selected from hydrogen, fluorine, methyl, trifluoromethyl and hydroxy,
**R**^{**2**} and
**R**^{**3**} are hydrogen,
**R**^{**4**} is hydrogen or hydroxy,
**k** is 0,
**A** is selected from the group consisting of ethylene, propylene and butylene, which are optionally substituted by hydroxy or once or twice by fluorine,
ethenylene, and
1,3-butadienylene
**D** is selected from C₂-C₆-alkylene and C₂-C₆-alkenylene, wherein the double bond optionally is to ring E,
**E** is selected from pyrrolidine, piperidine, hexahydroazepine and morpholine,
**G** is selected from benzyl, phenethyl, fluorenylmethyl, anthrylmethyl, diphenylmethyl, fluorenyl or dihydrodibenzocycloheptenyl, furylmethyl, thienylmethyl, thiazolylmethyl, pyridylmethyl, benzothienylmethyl, quinolylmethyl, phenyl-thienylmethyl, phenyl-pyridylmethyl, dihydrodibenzoxepinyl, dihydrodibenzothiepinyl,
acetyl, pivaloyl, phenylacetyl, diphenylacetyl, diphenylpropionyl, naphthylacetyl, benzoyl, naphthoyl, anthrylcarbonyl, oxofluorenylcarbonyl, oxodihydro-anthrylcarbonyl, dioxodihydroanthrylcarbonyl,
furoyl, pyridylcarbonyl, chromonylcarbonyl, quinolylcarbonyl,
naphthylaminocarbonyl, dibenzylaminocarbonyl, benzylphenylaminocarbonyl, diphenylaminocarbonyl, indolinyl-1-carbonyl, dihydrodibenzazepin-N-carbonyl, tetrahydroquinolinyl-N-carbonyl, tetrahydrobenzo[b]azepinyl-N-carbonyl,
methanesulfonyl, phenylsulfonyl, p-toluolsulfonyl, naphthylsulfonyl, quinolinsulfonyl and
diphenylphosphinoyl,
wherein aromatic ring systems optionally are substituted independently of each other by one to three of the same or different substituents selected from the group consisting of halogen, cyano, C₁-C₆-alkyl, trifluoromethyl, C₃-C₈-cycloalkyl, phenyl, benzyl, hydroxy, C₁-C₆-alkoxy, C₁-C₆-alkoxy entirely or partially substituted by fluorine, benzyloxy, phenoxy, mercapto, C₁-C₆-alkylthio, carboxy, C₁-C₆-alkoxycarbonyl, benzyloxycarbonyl, nitro, amino, mono-C₁-C₆-alkylamino and di-(C₁-C₆-alkyl)-amino, wherein two adjacent groups in the ring or ring system optionally form an additional ring over a methylendioxy bridge.

6. Use according to one of the claims 1 to 5, wherein one or more of the following compounds and/or pharmacologically acceptable acid addition salts thereof are used for the production of the medicament
N-[2-(1-benzylpiperidin-4-yl)-ethyl]-3-(pyridin-3-yl)-propionamide,
N-{2-[1-(2-phenylethyl)-piperidin-4-yl]-ethyl}-3-(pyridin-3-yl)-propionamide,
N-[2-(1-diphenylmethylpiperidin-4-yl)-ethyl]-3-(pyridin-3-yl)-propionamide,
N-[3-(1-diphenylmethylpiperidin-4-yl)-propyl]-3-(pyridin-3-yl)-propionamide, or
N-[4-(1-diphenylmethylpiperidin-4-yl)-butyl]-3-(pyridin-3-yl)-propionamide.

7. Use according to one of the claims 1 to 4, wherein the following compound and/or pharmacologically acceptable acid addition salts thereof are used for the production of a medicament
N-{2-[1-(4-phenylbutyl)-piperidin-4-yl]-ethyl}-3-(pyridin-3-yl)-propionamide.

8. Use of
N-{2-[1-(4-hydroxy-4-phenylbutyl)-piperidin-4-yl]-ethyl}-3-(pyridin-3-yl)-propionamide,
stereoisomers, mixtures and/or pharmacologically acceptable acid addition salts thereof for the production of a medicament for cytostatic or immunomodulatory and/or immunosuppressive treatment.

9. Use according to any one of the claims 1 to 5, wherein one or more of the following compounds and/or pharmacologically acceptable acid addition salts thereof are used for the production of a medicament
N-[4-(1-benzylpiperidin-4-yl)-butyl]-3-(pyridin-3-yl)-acrylamide,
N-{4-[1-(2-phenylethyl)-piperidin-4-yl]-butyl}-3-(pyridin-3-yl)-acrylamide,
N-{4-[1-(4-biphenylylmethyl)-piperidin-4-yl]-butyl }-3-(pyridin-3-yl)-acrylamide,
N- {4-[1-(9-anthrylmethyl)-piperidin-4-yl]-butyl}-3-(pyridin-3-yl)-acrylamide, or
N- {4-[1-(10,11-dihydro-5H-dibenzo[a,d]cycloheptene-5-yl)-piperidin-4-yl]-butyl}-3-(pyridin-3-yl)-acrylamide.

10. Use according to one of the claims 1 to 4, wherein the following compound and/or pharmacologically acceptable acid addition salts thereof are used for the production of a medicament:
N-{4-[1-(1-naphthylmethyl)-piperidin-4-yl]-butyl}-3-(pyridin-3-yl)-acrylamide.

11. Use according to claim 1 or 2, wherein the following compound and/or pharmacologically acceptable acid addition salts thereof are used for the production of a medicament:
N-{4-[1-(cyclohexylphenylmethyl)-piperidin-4-yl]-butyl}-3-(pyridin-3-yl)-acrylamide.

12. Use according to any one of the claims 1 to 5, wherein one or more of the following compounds and/or pharmacologically acceptable acid addition salts thereof are used for the production of a medicament
N-[2-(1-diphenylmethylpiperidin-4-yl)-ethyl]-3-(pyridin-3-yl)-acrylamide,
N-[3-(1-diphenylmethylpiperidin-4-yl)-propyl]-3-(pyridin-3-yl)-acrylamide,
N-[5-(1-diphenylmethylpiperidin-4-yl)-pentyl]-3-(pyridin-3-yl)-acrylamide,
N-[6-(1-diphenylmethylpiperidin-4-yl)-hexyl]-3-(pyridin-3-yl)-acrylamide, or
N-[4-(1-diphenylmethylpiperidin-4-yl)-butyl]-5-(pyridin-3-yl)-2,4-pentadiene acid amide.

13. Use according to any one of the claims 1 to 5, wherein one or more of the following compounds and/or pharmacologically acceptable acid addition salts thereof are used for the production of a medicament
N-(4- {1-[bis-(4-fluorophenyl)-methyl]-piperidin-4-yl}-butyl)-3-(pyridin-3-yl)-acrylamide,
N-(4- {1-[bis-(2-chlorophenyl)-methyl]-piperidin-4-yl}-butyl)-3-(pyridin-3-yl)-acrylamide,
N-[4-(1-diphenylmethylpiperidin-4-yl)-butyl]-3-(2-fluoropyridin-3-yl)-acrylamide, or
N-[4-(1-diphenylmethylpiperidin-4-yl)-butyl]-3-(6-fluoropyridin-3-yl)-acrylamide.

14. Use according to any one of the claims 1 to 5, wherein one or more of the following compounds are used for the production of a medicament
N-[4-(1-diphenylmethylpiperidin-4-yl)-butyl]-3-(pyridin-3-yl)-acrylamide,
N-[4-(1-diphenylmethylpiperidin-4-yl)-butyl]-3-(pyridin-3-yl)-acrylamide dihydrochloride, or
N-[4-(1-diphenylmethylpiperidin-4-yl)-butyl]-3-(pyridin-3-yl)-acrylamide methanesulfonate.

15. Use according to any one of the claims 1 to 14, wherein the compounds according to the general formula (I) are combined with one or more other active ingredients selected from cytostatic agents and immunosuppressive agents.

16. Medicament for cytostatic or immunomodulatory and/or immunosuppressive treatment in human or veterinary medicine, which comprises a compound according to formula (I) corresponding to the substituent definitions given in claims 1 to 14 in combination with one or more other active ingredients selected from cytostatic agents and immunosuppressive agents, optionally together with suitable carriers, adjuvants and additives.

17. Medicament according to claim 16 for cytostatic treatment, which comprises a compound according to formula (I), pharmaceutically acceptable carriers and adjuvants, and in combination one or more cytostatic agents from the series of anti-metabolites selected from cytarabine, 5-fluoruracil, 6-mercaptopurine or methotrexate, alkylating agents selected from busulfan, carmustine, cisplatin, carboplatin, cyclophosphamide, dacarbazine, melphalan and thiotepa, DNA-intercalating substances and topoisomerase inhibitors selected from actinomycin D, daunorubicin, doxorubicin, mitomycin C, mitoxantrone, etoposide, topotecan and irinotecan, spindle poisons selected from vincristine, navelbin, taxol, and taxoter, hormonally active agents selected from tamoxifen, flutiamide, formestan and goserelin or from the series of other cytostatic agents with complex modes of action selected from L-asparaginase, bleomycin and hydroxyurea or from the group of the modulators of P-glycoprotein, MRP, glutathione-S-transferase, metallothionein, optionally together with further medicines effective in these indications.

18. Medicament according to claim 16 for immunosuppressive treatment, which comprises a compound according to formula (I), pharmaceutically acceptable carriers and adjuvants, and in combination one or more immunosuppressive agents from the series of the cyclosporins, selected from cyclosporin A, tacrolimus, and rapamycin, the group of antimetabolites selected from methotrexate and azathioprine and the group of glucocorticoids, optionally together with further medicines effective in these indications.

19. Use according to claims 1 to 15, for the treatment of tumors in connection with the indications gynacological tumors, ovarian carcinomas, testicle tumors, prostate carcinomas, skin cancer, kidney cancer, bladder tumors, esophagus carcinomas, stomach cancer, rectal carcinomas, pancreas carcinomas, thyroid cancer, adrenal tumors, various types of leukemia and lymphomas, Hodgkin's disease, tumor illnesses of the CNS, soft-tissue sarcomas, bone sarcomas, benign and malignant mesotheliomas, especially intestine cancer, liver cancer, breast cancer, bronchial and lung carcinomas, melanomas, acute and chronic leukemias and benign papillomatosis tumors.

20. Use according to one of the claims 1 to 5, wherein the following compound and/or pharmacologically acceptable acid addition salts thereof are used for the production of a medicament:
N-(4-diphenylmethyl-morpholin-2-ylmethyl)-3-(pyridin-3-yl)-acrylamide.

## Patentansprüche

1. Verwendung einer oder mehrerer Verbindungen der Formel (I) 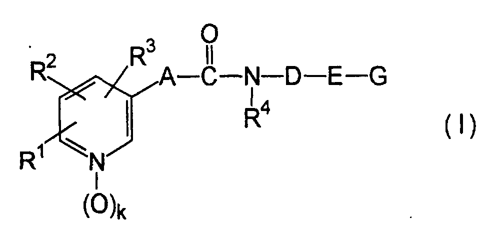 worin:
R¹ ausgewählt ist aus der Gruppe, bestehend aus Wasserstoff, Halogen, Cyano, C₁₋₆-Alkyl, Trifluormethyl, C₃₋₈-Cycloalkyl, C₁₋₄-Hydroxyalkyl, Hydroxy, C₁₋₄-Alkoxy, Benzyloxy, C₁₋₄-Alkanoyloxy, C₁₋₄-Alkylthio, C₂₋₅-Alkoxycarbonyl, Aminocarbonyl, C₃₋₉-Dialkylaminocarbonyl, Carboxy, Phenyl, Phenoxy, Pyridyloxy und NR⁵R⁶, worin
R⁵ und
R⁶ unabhängig voneinander ausgewählt sind aus Wasserstoff und C₁₋₆-Alkyl,
R² ausgewählt ist aus der Gruppe, bestehend aus Wasserstoff, Halogen, C₁₋₆-Alkyl, Trifluormethyl und Hydroxy, worin
R¹ und R², für den Fall, dass sie benachbart sind, gegebenenfalls eine Brücke bilden, die ausgewählt ist aus der Gruppe an Brückenmitgliedern: -(CH₂)₄- und -(CH=CH)₂- und -CH₂O-CR⁷R⁸-O-, worin
R⁷ und
R⁸ unabhängig voneinander Wasserstoff oder C₁₋₆-Alkyl sind,
R³ ausgewählt ist aus Wasserstoff, Halogen und C₁₋₆-Alkyl,
R⁴ ausgewählt ist aus Wasserstoff, C₁₋₆-Alkyl, C₃₋₆-Alkenyl, Hydroxy, C₁₋₆-Alkoxy und Benzyloxy,
k 0 oder 1 ist,
A ausgewählt ist aus C₁₋₆-Alkylen, das gegebenenfalls ein- bis dreimal substituiert ist durch C₁₋₃-Alkyl, Hydroxy, Fluor oder Phenyl, 1,2-Cyclopropylen,
C₂₋₆-Alkenylen, das gegebenenfalls ein- bis dreimal substituiert ist durch C₁₋₃-Alkyl, Hydroxy, Fluor, Cyano oder Phenyl,
C₄₋₆-Alkadienylen, das gegebenenfalls ein- oder zweimal substituiert ist durch C₁₋₃-Alkyl, Fluor, Cyano oder Phenyl,
1,3,5-Hexatrienylen, das gegebenenfalls substituiert ist durch C₁₋₃-Alkyl, Fluor oder Cyano,
Ethinylen und
C₂₋₆-Alkylen, worin eine Methyleneinheit isosterisch ersetzt ist durch 0, S, NR⁹, CO, SO oder SO₂, und worin der isostere Ersatz, mit Ausnahme von =CO, nicht benachbart zu der Amidgruppe ist, und worin
R⁹ ausgewählt ist aus Wasserstoff, C₁₋₃-Alkyl, C₁₋₆-Acyl und Methansulfonyl,
D ausgewählt ist aus C₁₋₁₀-Alkylen, das gegebenenfalls ein- oder zweimal durch C₁₋₃-Alkyl oder Hydroxy substituiert ist,
C₂₋₁₀-Alkenylen, das gegebenenfalls ein- oder zweimal durch C₁₋₃-Alkyl oder Hydroxy substituiert ist, worin die Doppelbindung gegebenenfalls zum Ring E ist,
C₃₋₁₀-Alkinylen, das gegebenenfalls ein- oder zweimal durch C₁₋₃-Alkyl oder Hydroxy substituiert ist, und
der Gruppe, bestehend aus C₁₋₁₀-Alkylen, C₂₋₁₀-Alkenylen oder C₃₋₁₀-Alkinylen, worin ein bis drei Methyleneinheiten isosterisch ersetzt sind durch O, S, MR¹⁰, CO, SO oder SO₂, worin
R¹⁰ die gleiche Bedeutung wie R⁹ besitzt, aber unabhängig davon ausgewählt ist,
E ausgewählt ist aus 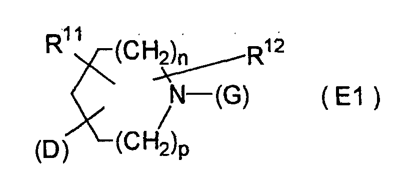 und 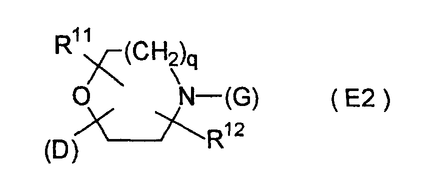
worin der heterocyclische Ring gegebenenfalls eine Doppelbindung aufweist und
n und p unabhängig voneinander 0, 1, 2 oder 3 bedeuten, mit der Massgabe, dass n + p ≤ 4,
q 1 oder 2 ist,
R¹¹ ausgewählt ist aus Wasserstoff, C₁₋₃-Alkyl, Hydroxy, Hydroxymethyl, Carboxy und C₂₋₇-Alkoxycarbonyl,
R¹² ausgewählt ist aus Wasserstoff und einer zum Stickstoffatom benachbarten Oxogruppe,
G ausgewählt ist aus Wasserstoff, G1, G2, G3, G4 und G5, worin
G1 den Rest
**―(CH**_{**2**}**)**_{**r**}**―(CR**^{**14**}**R**^{**15**}**)**_{**s**}**-R**^{**13**} (G1)
bedeutet, worin
r 0, 1 oder 2 ist und
s 0 oder 1 ist,
R¹³ ausgewählt ist aus Wasserstoff, C₁₋₆-Alkyl, C₃₋₆-Alkenyl, C₃₋₆-Alkinyl, C₃₋₈-Cycloalkyl,
Benzyl, Phenyl,
der Gruppe, bestehend aus monocyclischen aromatischen 5- bis 6-gliedrigen Heterocyclen, die ein bis drei Heteroatome, ausgewählt aus N, S oder O, enthalten und entweder direkt oder über eine Methylengruppe gebunden sind,
der Gruppe, bestehend aus anellierten bi- und tricyclischen aromatischen oder teilweise hydrierten carbocyclischen Ringsystemen mit 8 bis 16 Ringatomen und mindestens einem aromatischen Ring, worin die Bindung entweder über einen aromatischen oder einen hydrierten Ring und entweder direkt oder über eine Methylengruppe erfolgt, und
der Gruppe, bestehend aus anellierten bi- und tricyclischen aromatischen oder teilweise hydrierten heterocyclischen Ringsystemen mit 8 bis 16 Ringatomen und mindestens einem aromatischen Ring, worin ein bis drei Ringatome ausgewählt sind aus N, S und O und die Bindung entweder über einen aromatischen oder einen hydrierten Ring, und entweder direkt oder über eine Methylengruppe erfolgt,
R¹⁴ die gleiche Bedeutung wie R¹³ besitzt, aber unabhängig davon ausgewählt ist,
R¹⁵ ausgewählt ist aus Wasserstoff, Hydroxy, Methyl, Benzyl, Phenyl,
der Gruppe, bestehend aus monocyclischen aromatischen 5- bis 6-gliedrigen Heterocyclen, die ein bis drei Heteroatome, ausgewählt aus N, S oder O, enthalten und entweder direkt oder über eine Methylengruppe gebunden sind,
der Gruppe, bestehend aus anellierten bi- und tricyclischen aromatischen oder teilweise hydrierten carbocyclischen Ringsystemen mit 8 bis 16 Ringatomen und mindestens einem aromatischen Ring, worin die Bindung entweder über einen aromatischen oder einen hydrierten Ring und entweder direkt oder über eine Methylengruppe erfolgt, und
der Gruppe, bestehend aus anellierten bi- und tricyclischen aromatischen oder teilweise hydrierten heterocyclischen Ringsystemen mit 8 bis 16 Ringatomen und mindestens einem aromatischen Ring, worin ein bis drei Ringatome ausgewählt sind aus N, S oder O und die Bindung entweder über einen aromatischen oder einen hydrierten Ring, und entweder direkt oder über eine Methylengruppe erfolgt,
G2 ausgewählt ist aus den Resten 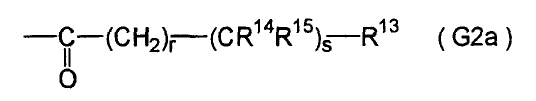 und 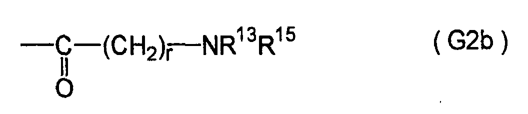 worin die Substituenten R¹³ und R¹⁵ die obigen Bedeutungen besitzen, oder die Gruppe
**―NR**^{**13**}**R**^{**15**}
ein stickstoffhaltiger Heterocyclus ist, der über das Stickstoffatom gebunden ist, worin der stickstoffhaltige Heterocyclus ausgewählt ist aus
der Gruppe, bestehend aus gesättigten und ungesättigten, monocyclischen, 4- bis 8-gliedrigen Heterocyclen, die neben dem essentiellen Stickstoffatom gegebenenfalls ein oder zwei weitere Heteroatome, ausgewählt aus N, S und O, enthalten, und
der Gruppe, bestehend aus gesättigten und ungesättigten, bi- oder tricyclischen, anellierten oder verbrückten Heterocyclen mit 8 bis 16 Ringatomen, die neben dem essentiellen Stickstoffatom gegebenenfalls ein oder zwei weitere Heteroatome, ausgewählt aus N, S und O, enthalten,
G3 der Rest ist:
―SO₂―(CH₂)ᵣR¹³ (G3)
G4 der Rest ist: 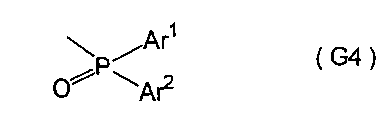
worin
Ar¹ und
Ar² unabhängig voneinander ausgewählt sind aus Phenyl, Pyridyl und Naphthyl,
G5 der Rest ist:
**―COR**^{**16**} (G5)
worin
R¹⁶ ausgewählt ist aus Trifluormethyl, C₁₋₆-Alkoxy, C₃₋₆-Alkenyloxy und Benzyloxy,
worin aromatische Ringsysteme in den Substituenten R¹, R², R⁴, R¹³, R¹⁴, R¹⁵, R¹⁶, Ar¹ und Ar² und in dem Ringsystem -NR¹³R¹⁵ gegebenenfalls unabhängig voneinander ein bis drei der gleichen oder unterschiedlichen Substituenten tragen, ausgewählt aus der Gruppe, bestehend aus Halogen, Cyano, C₁₋₆-Alkyl, Trifluormethyl, C₃₋₈-Cycloalkyl, Phenyl, Benzyl, Hydroxy, C₁₋₆-Alkoxy, das gegebenenfalls vollständig oder teilweise durch Fluor substituiert ist, Benzyloxy, Phenoxy, Mercapto, C₁₋₆-Alkylthio, Carboxy, C₁₋₆-Alkoxycarbonyl, Benzyloxycarbonyl, Nitro, Amino, Mono-C₁₋₆alkylamino und Di-(C₁₋₆-alkyl)-amino, worin zwei benachbarte Gruppen des aromatischen Rings oder Ringsystems gegebenenfalls einen zusätzlichen Ring über eine Methylendioxybrücke bilden,
Tautomere für den Fall der Substitution des Heterocyclus oder in einem anellierten Ringsystem durch freie Hydroxy-, Mercapto- und/oder Aminogruppen,
Stereoisomere und/oder Mischungen davon und pharmakologisch annehmbare Säureadditionssalze,
zur Herstellung von Arzneimitteln zur cytostatischen oder immunomodulatorischen und/oder immunosuppressiven Behandlung.

2. Verwendung von Verbindungen gemäss Anspruch 1, worin:
R¹ ausgewählt ist aus Wasserstoff, Halogen, Cyano, Methyl, Trifluormethyl, Hydroxy, C₁₋₄-Alkoxy, Ethylthio, Methoxycarbonyl, tert-Butoxycarbonyl, Aminocarbonyl, Carboxy und Phenoxy,
R² ausgewählt ist aus Wasserstoff, Halogen, Trifluormethyl und Hydroxy,
R³ ist Wasserstoff oder Halogen,
R⁴ ist ausgewählt aus Wasserstoff, C₁₋₃-Alkyl, Hydroxy und C₁₋₃-Alkoxy,
k ist 0 oder 1,
A ausgewählt ist aus der Gruppe, bestehend aus C₂₋₆-Alkylen, das gegebenenfalls ein- oder zweimal substituiert ist durch C₁₋₃-Alkyl, Hydroxy oder Fluor,
C₂₋₆-Alkenylen, das gegebenenfalls ein- oder zweimal substituiert ist durch C₁₋₃-Alkyl, Hydroxy oder Fluor,
C₄₋₆-Alkadienylen, das gegebenenfalls substituiert ist durch C₁₋₃-Alkyl oder durch 1 oder 2 Fluoratome,
1,3,5-Hexatrienylen, das gegebenenfalls substituiert ist durch Fluor, und
C₂₋₆-Alkylen, worin eine Methyleneinheit isosterisch ersetzt ist durch O, S, CO oder SO₂, und der isosterische Substituent, mit Ausnahme von =CO, nicht zur Amidgruppe benachbart ist,
D ausgewählt ist aus C₁₋₈-Alkylen, das gegebenenfalls ein- oder zweimal substituiert ist durch Methyl oder Hydroxy,
C₂₋₈-Alkenylen, das gegebenenfalls ein- oder zweimal substituiert ist durch Methyl oder Hydroxy, worin die Doppelbindung gegebenenfalls zum Ring E ist,
C₃₋₈-Alkinylen, das gegebenenfalls ein- oder zweimal substituiert ist durch Methyl oder Hydroxy, und
der Gruppe, bestehend aus C₁₋₈-Alkylen, C₂₋₈-Alkenylen und C₃₋₈-Alkinylen, worin ein bis drei Methyleneinheiten isosterisch ersetzt sind durch O, S, NH, N(CH₃), N(COCH₃), N(SO₂CH₃), CO, SO oder SO₂,
E ausgewählt ist aus 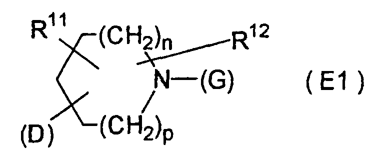 und 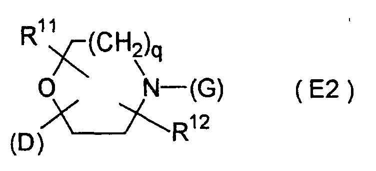
worin der heterocyclische Ring gegebenenfalls eine Doppelbindung aufweist und
n und p unabhängig voneinander 0, 1, 2 oder 3 bedeuten, mit der Massgabe, dass n + p ≤ 3,
q 1 oder 2 ist,
R¹¹ ausgewählt ist aus Wasserstoff, C₁₋₃-Alkyl, Hydroxy und Hydroxymethyl,
R¹² Wasserstoff oder eine Oxogruppe ist, die benachbart zum Stickstoffatom ist,
G ausgewählt ist aus Wasserstoff, G1, G2, G3, G4 und G5, worin
G1 den Rest
**―(CH**_{**2**}**)**_{**r**}**―(CR**^{**14**}**R**^{**15**}**)**_{**s**}**―R**^{**13**} (G1)
bedeutet, worin
r 0, 1 oder 2 ist und
s 0 oder 1 ist,
R¹³ ausgewählt ist Wasserstoff, C₁₋₆-Alkyl, C₃₋₈-Cycloalkyl, Benzyl, Phenyl,
der Gruppe, bestehend aus Benzocyclobutyl, Indanyl, Indenyl, Oxoindanyl, Naphthyl, Dihydronaphthyl, Tetrahydronaphthyl, Oxotetrahydronaphthyl, Biphenylenyl, Fluorenyl, Oxofluorenyl, Anthryl, Dihydroanthryl, Oxodihydroanthryl, Dioxodihydroanthryl, Phenanthryl, Dihydrophenanthryl, Oxodihydrophenanthryl, Dibenzocycloheptenyl, Oxodibenzocycloheptenyl, Dihydrodibenzocycloheptenyl, Oxodihydrodibenzocycloheptenyl, Dihydrodibenzocyclooctenyl, Tetrahydrodibenzocyclooctenyl und Oxotetrahydrodibenzocyclooctenyl, direkt gebunden oder über eine Methylengruppe,
und
der Gruppe, bestehend aus Furyl, Thienyl, Pyrrolyl, Oxazolyl, Isoxazolyl, Thiazolyl, Isothiazolyl, Pyrazolyl, Imidazolyl, Oxadiazolyl, Thiadiazolyl, Triazolyl, Pyridyl, Pyrazinyl, Pyridazinyl, Pyrimidinyl, Triazinyl, Imidazothiazolyl, Benzofuryl, Dihydrobenzofuryl, Benzothienyl, Dihydrobenzothienyl, Indolyl, Indolinyl, Oxoindolinyl, Dioxoindolinyl, Benzoxazolyl, Oxobenzoxazolinyl, Benzisoxazolyl, Oxobenzisoxazolinyl, Benzothiazolyl, Oxobenzothiazolinyl, Benzoisothiazolyl, Oxobenzoisothiazolinyl, Benzimidazolyl, Oxobenzimidazolinyl, Indazolyl, Oxoindazolinyl, Benzofurazanyl, Benzothiadiazolyl, Benzotriazolyl, Oxazolopyridyl, Oxodihydrooxazolopyridyl, Thiazolopyridyl, Oxodihydrothiazolopyridyl, Isothiazolopyridyl, Imidazopyridyl, Oxodihydroimidazopyridyl, Pyrazolopyridyl, Oxodihydropyrazolopyridyl, Thienopyrimidinyl, Chromanyl, Chromanonyl, Benzopyranyl, Chromonyl, Chinolyl, Isochinolyl, Dihydrochinolyl, Oxodihydrochinolinyl, Tetrahydrochinolyl, Oxotetrahydrochinolinyl, Benzodioxanyl, Chinoxalinyl, Chinazolinyl, Naphthyridinyl, Carbazolyl, Tetrahydrocarbazolyl, Oxotetrahydrocarbazolyl, Pyridoindolyl, Acridinyl, Oxodihydroacridinyl, Phenothiazinyl, Dihydrodibenzoxepinyl, Oxodihydrodibenzoxepinyl, Benzocycloheptathienyl, Oxobenzocycloheptathienyl, Dihydrothienobenzothiepinyl, Oxodihydrothienobenzothiepinyl, Dihydrodibenzothiepinyl, Oxodihydrodibenzothiepinyl, Octahydrodibenzothiepinyl, Dihydrodibenzazepinyl, Oxodihydrodibenzazepinyl, Octahydrodibenzazepinyl, Benzocycloheptapyridyl, Oxobenzocycloheptapyridyl, Dihydropyridobenzodiazepinyl, Dihydrodibenzoxazepinyl, Dihydropyridobenzoxepinyl, Dihydropyridobenzoxazepinyl, Oxodihydropyridobenzoxazepinyl, Dihydrodibenzothiazepinyl, Oxodihydrodibenzothiazepinyl, Dihydropyridobenzothiazepinyl und Oxodihydropyridobenzothiazepinyl, direkt gebunden oder über eine Methylengruppe,
R¹⁴ die gleiche Bedeutung wie R¹³ hat, aber unabhängig davon ausgewählt ist,
R¹⁵ ausgewählt ist aus Wasserstoff, Hydroxy, Methyl, Benzyl, Phenyl und
der Gruppe, bestehend aus Indanyl, Indenyl, Naphthyl, Dihydronaphthyl, Tetrahydronaphthyl, Furyl, Thienyl, Pyrrolyl, Oxazolyl, Isoxazolyl, Thiazolyl, Isothiazolyl, Pyrazolyl, Imidazolyl, Oxadiazolyl, Thiadiazolyl, Triazolyl, Pyridyl, Pyrazinyl, Pyridazinyl, Pyrimidinyl, Triazinyl, Benzofuryl, Benzothienyl, Indolyl, Indolinyl, Benzoxazolyl, Benzothiazolyl, Benzimidazolyl, Chromanyl, Chinolyl und Tetrahydrochinolyl, direkt gebunden oder über eine Methylengruppe,
G2 ausgewählt ist aus den Resten: 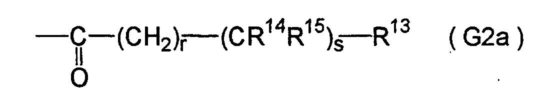 und 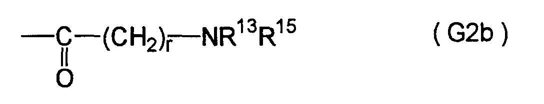 worin die Substituenten R¹³ und R¹⁵ die obigen Bedeutungen haben, oder die Gruppe
**-NR**^{**13**}**R**^{**15**}
bedeutet einen über das Stickstoffatom gebundenen stickstoffhaltigen Heterocyclus, worin der stickstoffhaltige Heterocyclus ausgewählt ist aus der Gruppe, bestehend aus Azetidin, Pyrrolidin, Piperidin, (1H)-Tetrahydropyridin, Hexahydroazepin, (1H)-Tetrahydroazepin, Octahydroazocin, Pyrazolidin, Piperazin, Hexahydrodiazepin, Morpholin, Hexahydrooxazepin, Thiomorpholin, Thiomorpholin-1,1-dioxid, 5-Azabicyclo[2.1.1]hexan, 2-Azabicyclo-[2.2.1]heptan, 7-Azabicyclo[2.2.1]heptan, 2,5-Diazabicyclo[2.2.1]heptan, 2-Azabicyclo[2.2.2]octan, 8-Azabicyclo[3.2.1]octan, 2,5-Diazabicyclo-[2.2.2]octan, 9-Azabicyclo[3.3.1]nonan, Indolin, Isoindolin, (1H)-Dihydrochinolin, (1H)-Tetrahydrochinolin, (2H)-Tetrahydroisochinolin, (1H)-Tetrahydrochinoxalin, (4H)-Dihydrobenzoxazin, (4H)-Dihydrobenzothiazin, (1H)-Tetrahydrobenzo[b]azepin, (1H)-Tetrahydrobenzo[c]azepin, (1H)-Tetrahydrobenzo[d]azepin, (5H)-Tetrahydrobenzo[b]oxazepin, (5H)-Tetrahydrobenzo[b]-thiazepin, 1,2,3,4-Tetrahydro-9H-pyrido[3,4-b]indol, (10H)-Dihydroacridin, 1,2,3,4-Tetrahydroacridanon, (10H)-Phenoxazin, (10H)-Phenothiazin, (5H)-Dibenzazepin, (5H)-Dihydrodibenzazepin, (5H)-Octahydrodibenzazepin, (5H)-Dihydrodibenzodiazepin, (11H)-Dihydrodibenzo[b,e]oxazepin, (11H)-Dihydrodibenzo[b,e]thiazepin, (10H)-Dihydrodibenzo[b,f]oxazepin, (10H)-Dihydrodibenzo[b,f]thiazepin und (5H)-Tetrahydrodibenzazocin,
G3 der Rest ist:
**―SO**_{**2**}**―(CH**_{**2**}**)**_{**r**}**R**^{**13**} (G3)
G4 der Rest ist: 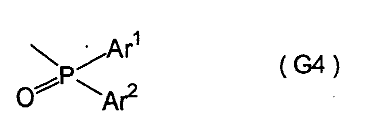
worin
Ar¹ und
Ar² unabhängig voneinander ausgewählt sind aus Phenyl, Pyridyl und Naphthyl,
G5 der Rest ist:
―COR¹⁶ (G5)
worin
R¹⁶ ausgewählt ist aus Trifluormethyl, C₁₋₆-Alkoxy, C₃₋₆-Alkenyloxy und Benzyloxy,
worin aromatische Ringsysteme gegebenenfalls unabhängig voneinander durch ein bis drei der gleichen oder unterschiedlichen Substituenten substituiert sind, ausgewählt aus der Gruppe, bestehend aus Halogen, Cyano, C₁₋₆-Alkyl, Trifluormethyl, C₃₋₈-Cycloalkyl, Phenyl, Benzyl, Hydroxy, C₁₋₆-Alkoxy, C₁₋₆-Alkoxy, das vollständig oder teilweise durch Fluor substituiert ist; Benzyloxy, Phenoxy, Mercapto, C₁₋₆-Alkylthio, Carboxy, C₁₋₆-Alkoxycarbonyl, Benzyloxycarbonyl, Nitro, Amino, Mono-C₁₋₆-alkylamino und Di-(C₁₋₆-alkyl)-amino, worin zwei benachbarte Gruppen des aromatischen Rings oder Ringsystems gegebenenfalls einen zusätzlichen Ring über eine Methylendioxybrücke bilden.

3. Verwendung von Verbindungen gemäss Anspruch 1 oder 2, worin
R¹ ausgewählt ist aus Wasserstoff, Halogen, Cyano, Methyl, Trifluormethyl, Hydroxy, Methoxy und Methoxycarbonyl,
R² Wasserstoff oder Halogen ist,
R³ Wasserstoff ist,
R⁴ ausgewählt ist aus Wasserstoff, C₁₋₃-Alkyl und Hydroxy,
k 0 oder 1 ist,
A ausgewählt ist aus C₂₋₆-Alkylen, das gegebenenfalls ein- oder zweimal substituiert ist durch Hydroxy oder Fluor,
C₂₋₆-Alkenylen, das gegebenenfalls ein- oder zweimal substituiert ist durch Hydroxy oder Fluor,
C₄₋₆-Alkadienylen, das gegebenenfalls substituiert ist durch ein oder zwei Fluoratome,
1,3,5-Hexatrienylen und
C₂₋₆-Alkylen, worin eine Methyleneinheit isosterisch ersetzt ist durch O, S oder CO, und der isosterische Substituent, mit Ausnahme von =CO, nicht zur Amidgruppe benachbart ist,
D. ausgewählt ist aus C₂₋₈-Alkylen, das gegebenenfalls substituiert ist durch Methyl oder Hydroxy,
C₂₋₈-Alkenylen, das gegebenenfalls substituiert ist durch Methyl oder Hydroxy, worin die Doppelbindung gegebenenfalls zum Ring E ist, und
der Gruppe, bestehend aus C₂₋₈-Alkylen und C₂₋₈-Alkenylen, worin ein bis drei Methyleneinheiten isosterisch ersetzt sind durch 0, NH, N(CH₃), N(COCH₃), N(SO₂CH₃) oder CO,
E ausgewählt ist aus: 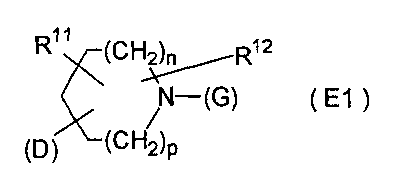 und 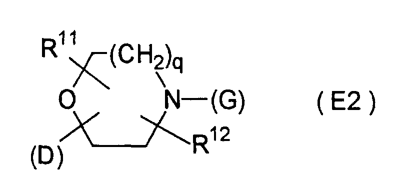 worin der heterocyclische Ring gegebenenfalls eine Doppelbindung aufweist, und
n und p unabhängig voneinander 0, 1, 2 oder 3 sind, mit der Massgabe, dass n + p ≤ 3,
q 1 oder 2 ist,
R¹¹ ausgewählt ist aus der Gruppe, bestehend aus Wasserstoff, Methyl und Hydroxyl,
R¹² Wasserstoff oder eine dem Stickstoffatom benachbarte Oxogruppe ist,
G ausgewählt ist aus Wasserstoff, C₃₋₈-Cycloalkyl, Methoxycarbonyl, tert-Butoxycarbonyl, Benzyloxycarbonyl, Trifluoracetyl, Diphenylphosphinoyl und den Resten
―(CH₂)ᵣ―(CR¹⁴R¹⁵)ₛ―R¹³ (G1) 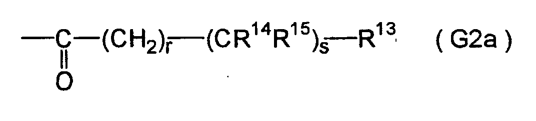 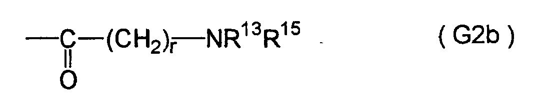
und
―SO₂―(CH₂)ᵣR¹³ (G3)
worin
r 0, 1 oder 2 ist,
s 0 oder 1 ist,
R¹³ ausgewählt ist aus Wasserstoff, Methyl, Benzyl, Phenyl,
der Gruppe, bestehend aus Indanyl, Indenyl, Oxoindanyl, Naphthyl, Dihydronaphthyl, Tetrahydronaphthyl, Oxotetrahydronaphthyl, Fluorenyl, Oxofluorenyl, Anthryl, Dihydroanthryl, Oxodihydroanthryl, Dioxodihydroanthryl, Dibenzocycloheptenyl, Oxodibenzocycloheptenyl, Dihydrodibenzocycloheptenyl und Oxodihydrodibenzocycloheptenyl, direkt gebunden oder über eine Methylengruppe, und
der Gruppe, bestehend aus Furyl, Thienyl, Pyrrolyl, Oxazolyl, Isoxazolyl, Thiazolyl, Isothiazolyl, Pyrazolyl, Imidazolyl, Oxadiazolyl, Thiadiazolyl, Triazolyl, Pyridyl, Pyrazinyl, Pyridazinyl, Pyrimidinyl, Imidazothiazolyl, Benzofuryl, Dihydrobenzofuryl, Benzothienyl, Dihydrobenzothienyl, Indolyl, Indolinyl, Oxoindolinyl, Dioxoindolinyl, Benzoxazolyl, Oxobenzoxazolinyl, Benzisoxazolyl, Oxobenzisoxazolidinyl, Benzothiazolyl, Oxobenzthiazolinyl, Benzoisothiazolyl, Oxobenzoisothiazolinyl, Benzimidazolyl, Oxobenzimidazolinyl, Benzofurazanyl, Benzothiadiazolyl, Benzotriazolyl, Oxazolopyridyl, Oxodihydrooxazolopyridyl, Thiazolopyridyl, Oxodihydrothiazolopyridyl, Isothiazolopyridyl, Imidazopyridyl, Oxodihydroimidazopyridyl, Pyrazolopyridyl, Thienopyrimidinyl, Chromanyl, Chromanonyl, Benzopyranyl, Chromonyl, Chinolyl, Isochinolyl, Dihydrochinolyl, Oxodihydrochinolinyl, Tetrahydrochinolyl, Oxotetrahydrochinolinyl, Benzodioxanyl, Chinoxalinyl, Chinazolinyl, Naphthyridinyl, Carbazolyl, Tetrahydrocarbazolyl, Oxotetrahydrocarbazolyl, Pyridoindolyl, Acridinyl, Oxodihydroacridinyl, Phenothiazinyl, Dihydrodibenzoxepinyl, Benzocycloheptathienyl, Oxobenzocycloheptathienyl, Dihydrothienobenzothiepinyl, Oxodihydrothienobenzothiepinyl, Dihydrodibenzothiepinyl, Oxodihydrobenzothiepinyl, Dihydrodibenzazepinyl, Oxodihydrodibenzazepinyl, Octahydrodibenzazepinyl, Benzocycloheptapyridyl, Oxobenzocycloheptapyridyl, Dihydropyridobenzoxepinyl, Dihydrodibenzothiazepinyl und Oxodihydrodibenzothiazepinyl, direkt gebunden oder über eine Methylengruppe,
R¹⁴ ausgewählt ist aus der Gruppe, bestehend aus Wasserstoff, Methyl, Benzyl und Phenyl,
R¹⁵ ausgewählt ist aus Wasserstoff, Hydroxy, Methyl, Benzyl, Phenyl und
der Gruppe, bestehend aus Naphthyl, Furyl, Thienyl, Oxazolyl, Thiazolyl, Pyrazolyl, Imidazolyl, Oxadiazolyl, Thiadiazolyl, Pyridyl, Benzofuryl, Benzothienyl, Indolyl, Indolinyl, Benzoxazolyl, Benzothiazolyl, Benzimidazolyl, Chromanyl, Chinolyl und Tetrahydrochinolyl, direkt gebunden oder über eine Methylengruppe,
worin in Formel 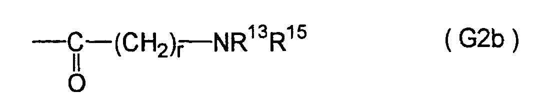
die Gruppe -NR¹³R¹⁵ gegebenenfalls ausgewählt ist aus Pyrrolidin, Piperidin, (1H)-Tetrahydropyridin, Hexahydroazepin, Octahydroazocin, Piperazin, Hexahydrodiazepin, Morpholin, Hexahydrooxazepin, 2-Azabicyclo[2.2.1]heptan, 7-Azabicyclo[2.2.1]heptan, 2,5-Diazabicyclo[2.2.1]-heptan, 8-Azabicyclo[3.2.1]octan, 2,5-Diazabicyclo[2.2.2]octan, Indolin, Isoindolin, (1H)-Dihydrochinolin, (1H)-Tetrahydrochinolin, (2H)-Tetrahydroisochinolin, (1H)-Tetrahydrochinoxalin, (4H)-Dihydrobenzoxazin, (4H)-Dihydrobenzothiazin, (1H)-Tetrahydrobenzo[b]azepin, (1H)-Tetrahydrobenzo-[d]azepin, (5H)-Tetrahydrobenzo[b]oxazepin, (5H)-Tetrahydrobenzo[b]thiazepin, 1,2,3,4-Tetrahydro-9H-pyrido[3,4-b]indol, (10H)-Dihydroacridin, 1,2,3,4-Tetrahydroacridanon, (5H)-Dihydrodibenzazepin, (5H)-Dihydrodibenzodiazepin, (11H)-Dihydrodibenzo[b,e]oxazepin, (11H)-Dihydrodibenzo-[b,e]thiazepin, (10H)-Dihydrodibenzo[b,f]oxazepin und (5H)-Tetrahydrodibenzazocin.

4. Verwendung von Verbindungen gemäss einem der Ansprüche 1 bis 3, worin
R¹ ausgewählt ist aus Wasserstoff, Fluor, Chlor, Brom, Methyl, Trifluormethyl und Hydroxy,
R² und
R³ Wasserstoff sind,
R⁴ Wasserstoff oder Hydroxy ist,
k 0 oder 1 ist,
A ausgewählt ist aus C₂₋₆-Alkylen, das gegebenenfalls ein- oder zweimal substituiert ist durch Hydroxy oder Fluor,
C₂₋₄-Alkenylen, das gegebenenfalls durch Fluor substituiert ist, und
1,3-Butadienylen, das gegebenenfalls durch Fluor substituiert ist,
D ausgewählt ist aus C₂₋₆-Alkylen, C₂₋₆-Alkenylen, worin die Doppelbindung gegebenenfalls zum Ring E ist, und der Gruppe, bestehend aus C₂₋₆-Alkylen und C₂₋₆-Alkenylen, worin eine Methyleneinheit isosterisch ersetzt ist durch O, NH, N(CH₃) oder CO, oder eine Ethylengruppe isosterisch ersetzt ist durch NH-CO oder CO-NH, oder eine Propylengruppe isosterisch ersetzt ist durch NH-CO-O oder O-CO-NH,
E ausgewählt ist aus Pyrrolidin, Piperidin, 1,2,5,6-Tetrahydropyridin, Hexahydroazepin, Morpholin und Hexahydro-1,4-oxazepin, worin der heterocyclische Ring gegebenenfalls durch eine zum Stickstoffatom benachbarte Oxogruppe substituiert ist,
G ausgewählt ist aus Wasserstoff, tert-Butoxycarbonyl, Diphenylphosphinoyl und den Resten
―(CH₂)ᵣ―(CR¹⁴R¹⁵)ₛ―R¹³ (G1) 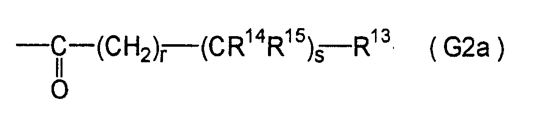 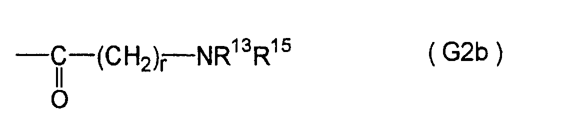
und
―SO₂―(CH₂)ᵣR¹³ (G3)
worin
r 0 oder 1 ist,
s 0 oder 1 ist,
R¹³ ausgewählt ist aus Wasserstoff, Methyl, Benzyl, Phenyl,
der Gruppe, bestehend aus Indenyl, Oxoindanyl, Naphthyl, Tetrahydronaphthyl, Fluorenyl, Oxofluorenyl, Anthryl, Dihydroanthryl, Oxodihydroanthryl, Dioxodihydroanthryl, Dibenzocycloheptenyl und Dihydrodibenzocycloheptenyl, direkt gebunden oder über eine Methylengruppe, und
der Gruppe, bestehend aus Furyl, Thienyl, Oxazolyl, Thiazolyl, Imidazolyl, Oxadiazolyl, Thiadiazolyl, Pyridyl, Pyrazinyl, Pyrimidinyl, Imidazothiazolyl, Benzofuryl, Benzothienyl, Indolyl, Oxoindolinyl, Dioxoindolinyl, Benzoxazolyl, Oxobenzoxazolinyl, Benzothiazolyl, Oxobehzthiazolinyl, Benzimidazolyl, Oxobenzimidazolinyl, Benzofurazanyl, Benzotriazolyl, Oxazolopyridyl, Oxodihydrooxazolopyridyl, Thiazolopyridyl, Oxodihydrothiazolopyridyl, Chromanyl, Chromanonyl, Benzopyranyl, Chromonyl, Chinolyl, Isochinolyl, Oxodihydrochinolinyl, Tetrahydrochinolyl, Oxotetrahydrochinolinyl, Benzodioxanyl, Chinazolinyl, Acridinyl, Oxodihydroacridinyl, Phenothiazinyl, Dihydrodibenzoxepinyl, Benzocycloheptathienyl, Dihydrothienobenzothiepinyl, Dihydrodibenzothiepinyl, Oxodihydrodibenzothiepinyl, Dihydrodibenzazepinyl, Oxodihydrodibenzazepinyl, Octahydrodibenzazepinyl, Benzocycloheptapyridyl, Oxobenzocycloheptapyridyl und Dihydrodibenzothiazepinyl, direkt gebunden oder über eine Methylengruppe,
R¹⁴ ist Wasserstoff, Methyl, Benzyl oder Phenyl,
R¹⁵ ausgewählt ist aus der Gruppe, bestehend aus Wasserstoff, Hydroxy, Methyl, Benzyl, Phenyl und
der Gruppe, bestehend aus Naphthyl, Furyl, Thienyl, Pyridyl, Benzofuryl, Benzothienyl, Indolyl, Benzoxazolyl, Benzothiazolyl, Benzimidazolyl, Chromanyl, Chinolyl und Tetrahydrochinolyl, direkt gebunden oder über eine Methylengruppe,
worin in Formel 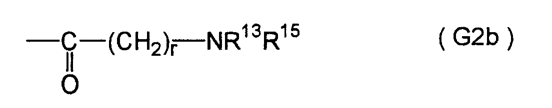
die Gruppe -NR¹³R¹⁵ gegebenenfalls ausgewählt ist aus Pyrrolidin, Piperidin, Hexahydroazepin, Morpholin, 2,5-Diazabicyclo[2.2.1]heptan, Indolin, Isoindolin, (1H)-Dihydrochinolin, (1H)-Tetrahydrochinolin, (2H)-Tetrahydroisochinolin, (1H)-Tetrahydrobenzo[b]azepin, (1H)-Tetrahydrobenzo-[d]azepin, (5H)-Tetrahydrobenzo[b]oxazepin, (5H)-Tetrahydrobenzo[b]thiazepin, 1,2,3,4-Tetrahydroacridanon, (5H)-Dihydrodibenzazepin, (11H)-Dihydrodibenzo[b,e]oxazepin und (11H)-Dihydrodibenzo[b,e]thiazepin,
worin aromatische Ringsysteme gegebenenfalls unabhängig voneinander durch ein bis drei der gleichen oder unterschiedlichen Substituenten substituiert sind, ausgewählt aus der Gruppe, bestehend aus Halogen, Cyano, C₁₋₆-Alkyl, Trifluormethyl, C₃₋₈-Cycloalkyl, Phenyl, Benzyl, Hydroxy, C₁₋₆-Alkoxy, C₁₋₆-Alkoxy, das vollständig oder teilweise durch Fluor substituiert ist; Benzyloxy, Phenoxy, Mercapto, C₁₋₆-Alkylthio, Carboxy, C₁₋₆-Alkoxycarbonyl, Benzyloxycarbonyl, Nitro, Amino, Mono-C₁₋₆-alkylamino und Di-(C₁₋₆-alkyl)-amino, worin zwei benachbarte Gruppen des aromatischen Rings oder Ringsystems gegebenenfalls einen zusätzlichen Ring über eine Methylendioxybrücke bilden.

5. Verwendung von Verbindungen gemäss Ansprüchen 1 bis 4, worin
R¹ ausgewählt ist aus Wasserstoff, Fluor, Methyl, Trifluormethyl und Hydroxy,
R² und
R³ Wasserstoff sind,
R⁴ Wasserstoff oder Hydroxy ist,
k 0 ist
A ausgewählt ist aus der Gruppe, bestehend aus Ethylen, Propylen und Butylen, die gegebenenfalls substituiert sind durch Hydroxy oder ein- oder zweimal durch Fluor,
Ethenylen und
1,3-Butadienylen,
D ausgewählt ist aus C₂₋₆-Alkylen und C₂₋₆-Alkenylen, worin die Doppelbindung gegebenenfalls zum Ring E ist,
E ausgewählt ist aus Pyrrolidin, Piperidin, Hexahydroazepin und Morpholin,
G ausgewählt ist aus Benzyl, Phenethyl, Fluorenylmethyl, Anthrylmethyl, Diphenylmethyl, Fluorenyl oder Dihydrodibenzocycloheptenyl, Furylmethyl, Thienylmethyl, Thiazolylmethyl, Pyridylmethyl, Benzothienylmethyl, Chinolylmethyl, Phenyl-thienylmethyl, Phenyl-pyridylmethyl, Dihydrodibenzoxepinyl, Dihydrodibenzothiepinyl,
Acetyl, Pivaloyl, Phenylacetyl, Diphenylacetyl, Diphenylpropionyl, Naphthylacetyl, Benzoyl, Naphthoyl, Anthrylcarbonyl, Oxofluorenylcarbonyl, Oxodihydroanthrylcarbonyl, Dioxodihydroanthrylcarbonyl,
Furoyl, Pyridylcarbonyl, Chromonylcarbonyl, Chinolylcarbonyl,
Naphthylaminocarbonyl, Dibenzylaminocarbonyl, Benzylphenylaminocarbonyl, Diphenylaminocarbonyl, Indolinyl-1-carbonyl, Dihydrodibenzazepin-N-carbonyl, Tetrahydrochinolinyl-N-carbonyl, Tetrahydrobenzo[b]azepinyl-N-carbonyl,
Methansulfonyl, Phenylsulfonyl, p-Toluolsulfonyl, Naphthylsulfonyl, Chinolinsulfonyl und
Diphenylphosphinoyl,
worin aromatische Ringsysteme gegebenenfalls unabhängig voneinander durch ein bis drei der gleichen oder unterschiedlichen,Substituenten substituiert sind, ausgewählt aus der Gruppe, bestehend aus Halogen, Cyano, C₁₋₆-Alkyl, Trifluormethyl, C₃₋₈-Cycloalkyl, Phenyl, Benzyl, Hydroxy, C₁₋₆-Alkoxy, vollständig oder teilweise durch Fluor substituiertes C₁₋₆-Alkoxy; Benzyloxy, Phenoxy, Mercapto, C₁₋₆-Alkylthio, Carboxy, C₁₋₆-Alkoxycarbonyl, Benzyloxycarbonyl, Nitro, Amino, Mono-C₁₋₆-alkylamino und Di-(C₁₋₆-alkyl)-amino, worin zwei benachbarte Gruppen im Ring oder Ringsystem gegebenenfalls einen zusätzlichen Ring über eine Methylendioxybrücke bilden.

6. Verwendung gemäss einem der Ansprüche 1 bis 5, worin ein oder mehrere der folgenden Verbindungen und/oder pharmakologisch annehmbare Säureadditionssalze davon verwendet werden für die Herstellung des Medikaments:
N-[2-(1-Benzylpiperidin-4-yl)-ethyl]-3-(pyridin-3-yl)-propionamid,
N-{2-[1-(2-Phenylethyl)-piperidin-4-yl]-ethyl}-3-(pyridin-3-yl)-propionamid,
N-[2-(1-Diphenylmethylpiperidin-4-yl)-ethyl]-3-(pyridin-3-yl)-propionamid,
N-[3-(1-Diphenylmethylpiperidin-4-yl)-propyl]-3-(pyridin-3-yl)-propionamid oder
N-[4-(1-Diphenylmethylpiperidin-4-yl)-butyl]-3-(pyridin-3-yl)-propionamid.

7. Verwendung gemäss einem der Ansprüche 1 bis 4, worin die folgende Verbindung und/oder pharmakologisch annehmbare Säureadditionssalze davon verwendet werden für die Herstellung eines Medikaments:
N-{2-[1-(4-Phenylbutyl)-piperidin-4-yl]-ethyl}-3-(pyridin-3-yl)-propionamid.

8. Verwendung von N-{2-[1-(4-Hydroxy-4-phenylbutyl)-piperidin-4-yl]-ethyl}-3-(pyridin-3-yl)-propionamid, Stereoisomeren, Mischungen und/oder pharmakologisch annehmbaren Säureadditionssalzen davon für die Herstellung eines Medikaments zur cytostatischen oder immunomodulatorischen und/oder immunosuppressiven Behandlung.

9. Verwendung gemäss einem der Ansprüche 1 bis 5, worin ein oder mehrere der folgenden Verbindungen und/oder pharmakologisch annehmbare Säureadditionssalze davon verwendet werden für die Herstellung eines Medikaments:
N-[4-(1-Benzylpiperidin-4-yl)-butyl)-3-(pyridin-3-yl)-acrylamid,
N-{4-[1-(2-Phenylethyl)-piperidin-4-yl]-butyl}-3-(pyridin-3-yl)-acrylamid,
N-{4-[1-(4-Biphenylmethyl)-piperidin-4-yl]-butyl}-3-(pyridin-3-yl)-acrylamid,
N-{4-[1-(9-Anthrylmethyl)-piperidin-4-yl]-butyl}-3-(pyridin-3-yl)-acrylamid oder
N-{4-[1-(10,11-Dihydro-5H-dibenzo[a,d]cyclohepten-5-yl)-piperidin-4-yl]-butyl}-3-(pyridin-3-yl)-acrylamid.

10. Verwendung gemäss einem der Ansprüche 1 bis 4, worin die folgende Verbindung und/oder pharmakologisch annehmbare Säureadditionssalze davon verwendet werden für die Herstellung eines Medikaments:
N-{4-[1-(1-Naphthylmethyl)-piperidin-4-yl]-butyl}-3-(pyridin-3-yl)-acrylamid.

11. Verwendung gemäss Anspruch 1 oder 2, worin die folgende Verbindung und/oder pharmakologisch annehmbare Säureadditionssalze davon verwendet werden für die Herstellung eines Medikaments:
N-{4-[1-(Cyclohexylphenylmethyl)-piperidin-4-yl]-butyl}-3-(pyridin-3-yl)-acrylamid.

12. Verwendung gemäss einem der Ansprüche 1 bis 5, worin ein oder mehrere der folgenden Verbindungen und/oder pharmakologisch annehmbare Säureadditionssalze davon verwendet werden für die Herstellung eines Medikaments
N-[2-(1-Diphenylmethylpiperidin-4-yl)-ethyl]-3-(pyridin-3-yl)-acrylamid,
M-[3-(1-Diphenylmethylpiperidin-4-yl)-propyl]-3-(pyridin-3-yl)-acrylamid,
N-[5-(1-Diphenylmethylpiperidin-4-yl)-pentyl]-3-(pyridin-3-yl)-acrylamid,
N-[6-(1-Diphenylmethylpiperidin-4-yl)-hexyl]-3-(pyridin-3-yl)-acrylamid oder
N-[4-(1-Diphenylmethylpiperidin-4-yl)-butyl]-5-(pyridin-3-yl)-2,4-pentadiensäureamid.

13. Verwendung gemäss einem der Ansprüche 1 bis 5, worin ein oder mehrere der folgenden Verbindungen und/oder pharmakologisch annehmbare Säureadditionssalze davon verwendet werden für die Herstellung eines Medikaments:
N-(4-{1-[Bis-(4-fluorphenyl)-methyl]-piperidin-4-yl}-butyl)-3-(pyridin-3-yl)-acrylamid,
N-(4-{1-[Bis-(2-chlorphenyl)-methyl]-piperidin-4-yl}-butyl)-3-(pyridin-3-yl)-acrylamid,
N-[4-(1-Diphenylmethylpiperidin-4-yl)-butyl]-3-(2-fluorpyridin-3-yl)-acrylamid oder
N-[4-(1-Diphenylmethylpiperidin-4-yl)-butyl]-3-(6-fluorpyridin-3-yl)-acrylamid.

14. Verwendung gemäss einem der Ansprüche 1 bis 5, worin ein oder mehrere der folgenden Verbindungen verwendet werden für die Herstellung eines Medikaments:
N-[4-(1-Diphenylmethylpiperidin-4-yl)-butyl]-3-(pyridin-3-yl)-acrylamid,
N-[4-(1-Diphenylmethylpiperidin-4-yl)-butyl]-3-(pyridin-3-yl)-acrylamid-dihydrochlorid oder
N-[4-(1-Diphenylmethylpiperidin-4-yl)-butyl]-3-(pyridin-3-yl)-acrylamid-methansulfonat.

15. Verwendung gemäss einem der Ansprüche 1 bis 14, worin die Verbindungen gemäss allgemeiner Formel (I) mit einem oder mehreren Wirkstoff(en), ausgewählt aus Cytostatika und Immunosuppressiva, kombiniert werden.

16. Medikament zur cytostatischen oder immunomodulatorischen und/oder immunosuppresiven Behandlung in der Human- oder Tiermedizin, umfassend eine Verbindung gemäss Formel (I), entsprechend den in den Ansprüchen 1 bis 14 gegebenen Substituentendefinitionen in Kombination mit einem oder mehreren Wirkstoff(en), ausgewählt aus Cytostatika und Immunosuppressiva, gegebenenfalls zusammen mit weiteren geeigneten Trägern, Hilfsstoffen und Additiven.

17. Medikament gemäss Anspruch 16 zur cytostatischen Behandlung, das umfasst: eine Verbindung gemäss Formel (I), pharmazeutisch annehmbare Träger und Hilfsstoffe, und in Kombination ein oder mehrere Cytostatika aus der Reihe der Antimetabolite, ausgewählt aus Cytarabin, 5-Fluoruracil, 6-Mercaptopurin oder Methotrexat, Alkylierungsmittel, ausgewählt aus Busulfan, Carmustin, Cisplatin, Carboplatin, Cyclophosphamid, Dacarbazin, Melphalan und Thiotepa, DNA-interkalierende Substanzen und Topoisomerasehemmer, ausgewählt aus Actinomycin D, Daunorubicin, Doxorubicin, Mitomycin C, Mitoxantron, Etoposid, Topotecan und Irinotecan, Spindelgifte, ausgewählt aus Vincristin, Navelbin, Taxol und Taxoter, hormonell aktive Wirkstoffe, ausgewählt aus Tamoxifen, Flutiamid, Formestan und Goserelin, oder aus der Reihe anderer Cytostatika mit komplexen Wirkmechanismen, ausgewählt aus L-Asparaginase, Bleomycin und Hydroxyharnstoff, oder aus der Gruppe der Modulatoren von P-Glycoprotein, MRP, Glutathion-S-Transferase, Metallothionin, gegebenenfalls zusammen mit weiteren bei diesen Indikationen wirksamen Pharmaka.

18. Medikament gemäss Anspruch 16 zur immunosuppressiven Behandlung, das umfasst: eine Verbindung gemäss Formel (I), pharmazeutisch annehmbare Träger und Hilfsstoffe, und in Kombination ein oder mehrere Immunosuppressiva aus der Reihe der Cyclosporine, ausgewählt aus Cyclosporin A, Tacrolimus und Rapamycin, der Gruppe der Antimetabolite, ausgewählt aus Methotrexat und Azathioprin, und der Gruppe der Glucocorticoide, gegebenenfalls zusammen mit weiteren, bei diesen Indikationen wirksamen Pharmaka.

19. Verwendung gemäss Ansprüchen 1 bis 15, zur Behandlung von Tumoren in Zusammenhang mit den Indikationen: gynäkologische Tumore, Eierstockkarzinome, Hodentumore, Prostatakarzinome, Hautkrebs, Nierenkrebs, Blasentumore, Ösophaguskarzinom, Magenkrebs, Rektalkarzinome, Pankreaskarzinome, Schilddrüsenkrebs, Nebennierentumore, verschiedene Arten von Leukämie und Lymphome, Morbus Hodgkin, Tumorerkrankungen des ZNS, Weichteilsarkoma, Knochensarkoma, benigne und maligne Mesotheliome, insbesondere Darmkrebs, Leberkrebs, Brustkrebs, Bronchial- und Lungenkarzinome, Melanome, akute und chronische Leukämie und benigne papillomatöse Tumore.

20. Verwendung gemäss einem der Ansprüche 1 bis 5, worin die folgende Verbindung und/oder pharmakologisch annehmbare Säureadditionssalze davon für die Herstellung eines Medikaments verwendet werden:
N-(4-Diphenylmethyl-morpholin-2-ylmethyl)-3-(pyridin-3-yl)-acrylamid.

## Revendications

1. Utilisation d'un ou plusieurs des composés de formule (I) 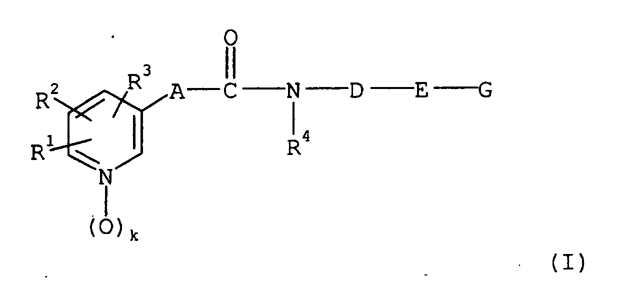 où
R¹ est choisi dans le groupe consistant en l'hydrogène, halogène, cyano, alkyle en C₁-C₆, trifluorométhyle, cycloalkyle en C₃-C₈, hydroxyalkyle en C₁-C₄, hydroxyle, alcoxy en C₁-C₄, benzyloxy, alcanoyloxy en C₁-C₄, alkylthio en C₁-C₄, alcoxy en C₂-C₅-carbonyle, aminocarbonyle, dialkyle en C₃-C₉-aminocarbonyle, carboxyle, phényle, phénoxy, pyridyloxy et NR⁵R⁶, où
R⁵ et
R⁶ sont choisis, indépendamment l'un de l'autre, parmi l'hydrogène et alkyle en C₁-C₆,
R² est choisi dans le groupe consistant en l'hydrogène, halogène, alkyle en C₁-C₆, trifluorométhyle et hydroxyle, où
R¹ et R², dans le cas où ils sont adjacents, forment éventuellement un pont qui est choisi dans le groupe de membres de pontage -(CH₂)₄- et -(CH=CH)₂- et -CH₂O-CR⁷R⁸-O-, où
R⁷ et
R⁸ sont indépendamment l'un de l'autre l'hydrogène ou alkyle en C₁-C₆,
R³ est choisi parmi l'hydrogène, halogène et alkyle en C₁-C₆,
R⁴ est choisi parmi l'hydrogène, alkyle en C₁-C₆, alcényle en C₃-C₆, hydroxyle, alcoxy en C₁-C₆ et benzyloxy,
k est 0 ou 1,
A est choisi parmi alkylène en C₁-C₆, qui est éventuellement substitué une à trois fois par alkyle en C₁-C₃, hydroxyle, le fluor ou phényle,
1,2-cyclopropylène,
alcénylène en C₂-C₆, qui est éventuellement substitué une à trois fois par alkyle en C₁-C₃, hydroxyle, le fluor, cyano ou phényle,
alcadiénylène en C₄-C₆, qui est éventuellement substitué une ou deux fois par alkyle en C₁-C₃, le fluor, cyano ou phényle,
1,3,5-hexatriénylène, qui est éventuellement substitué par alkyle en C₁-C₃, le fluor ou cyano,
éthynylène et
alkylène en C₂-C₆, où une unité méthylène est remplacée isostériquement par O, S, NR⁹, CO, SO ou SO₂, et où le substituant isostérique, à l'exception de =CO, n'est pas adjacent au groupe amide, et où
R⁹ est choisi parmi l'hydrogène, alkyle en C₁-C₃, acyle en C₁-C₆ et méthanesulfonyle,
D est choisi parmi alkylène en C₁-C₁₀, qui est éventuellement substitué une ou deux fois par alkyle en C₁-C₃ ou hydroxyle,
alcénylène en C₂-C₁₀, éventuellement substitué une ou deux fois par alkyle en C₁-C₃ ou hydroxyle, où la double liaison est éventuellement avec le cycle E,
alcynylène en C₃-C₁₀, qui est éventuellement substitué une ou deux fois par alkyle en C₁-C₃ ou hydroxyle, et
le groupe consistant en alkylène en C₁-C₁₀, alcénylène en C₂-C₁₀ et alcynylène en C₃-C₁₀, où une à trois unités méthylène sont remplacées isostériquement par O, S, NR¹⁰, CO, SO ou SO₂, où
R¹⁰ a la même signification que R⁹ mais est choisi indépendamment de lui,
E est choisi parmi 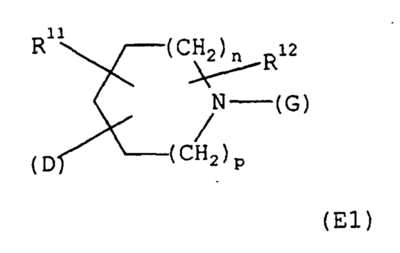 et 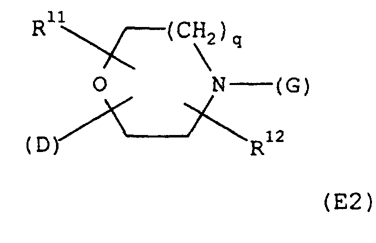 où le cycle hétérocyclique a éventuellement une double liaison et
n et p sont, indépendamment l'un de l'autre, 0, 1, 2 ou 3, avec la condition que n + p ≤ 4,
q est 1 ou 2,
R¹¹ est choisi parmi l'hydrogène, alkyle en C₁-C₃, hydroxyle, hydroxyméthyle, carboxyle et alcoxy en C₂-C₇-carbonyle,
R¹² est choisi parmi l'hydrogène et un groupe oxo adjacent à l'atome d'azote,
G est choisi parmi l'hydrogène,
G1, G2, G3, G4 et G5, où
G1 représente le résidu
- (CH₂)ᵣ- (CR¹⁴R¹⁵)ₛ-R¹³ (G1)
où
r est 0, 1 ou 2,
s est 0 ou 1,
R¹³ est choisi parmi l'hydrogène, alkyle en C₁-C₆, alcényle en C₃-C₆, alcynyle en C₃-C₆, cycloalkyle en C₃-C₈,
benzyle, phényle,
le groupe consistant en hétérocycles aromatiques monocycliques à cinq à six chaînons, qui contiennent un à trois hétéroatomes parmi N, S et O et qui sont liés directement ou par le biais d'un groupe méthylène,
le groupe consistant en les systèmes cycliques carbocycliques condensés bi- et tricycliques aromatiques ou partiellement hydrogénés avec 8 à 16 atomes cycliques et au moins un cycle aromatique, où la liaison a lieu par le biais d'un cycle aromatique ou hydrogéné et directement ou par le biais d'un groupe méthylène, et
le groupe consistant en les systèmes cycliques hétérocycliques condensés bi- et tricycliques aromatiques ou partiellement hydrogénés avec 8 à 16 atomes cycliques et au moins un cycle aromatique, où un à trois atomes cycliques sont choisis parmi N, S et O et la liaison a lieu par le biais d'un cycle aromatique ou hydrogéné et directement ou par le biais d'un groupe méthylène,
R¹⁴ a la même signification que R¹³, mais est choisi indépendamment de lui,
R¹⁵ est choisi parmi l'hydrogène, hydroxyle, méthyle, benzyle, phényle,
le groupe consistant en les hétérocycles monocycliques aromatiques à cinq à six chaînons, qui contiennent un à trois hétéroatomes choisis parmi N, S et O et qui sont liés directement ou par le biais d'un groupe méthylène,
le groupe consistant en systèmes cycliques carbocycliques condensés bi- et tricycliques aromatiques ou partiellement hydrogénés avec 8 à 16 atomes cycliques et au moins un cycle aromatique, où la liaison a lieu par le biais d'un cycle aromatique ou hydrogéné et directement ou par le biais d'un groupe méthylène, et
le groupe consistant en systèmes cycliques hétérocycliques condensés bi- et tricycliques aromatiques ou partiellement hydrogénés avec 8 à 16 atomes cycliques et au moins un cycle aromatique, où un à trois atomes cycliques sont choisis parmi N, S et O et la liaison a lieu par le biais d'un cycle aromatique ou hydrogéné et directement ou par le biais d'un groupe méthylène,
G2 est choisi parmi les résidus 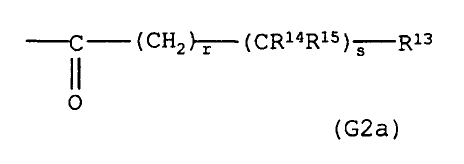 et 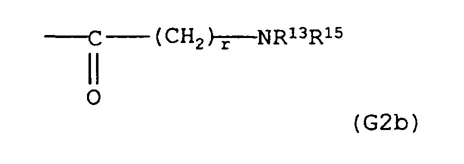 où les substituants R¹³ et R¹⁵ ont la signification ci-dessus, ou le groupe
-NR¹³R¹⁵
est un hétérocycle azoté lié par l'atome d'azote, l'hétérocycle azoté étant choisi dans le groupe consistant en hétérocycles monocycliques saturés et insaturés, à quatre à huit chaînons, qui, mis à part l'atome d'azote essentiel, contiennent éventuellement un ou deux autres hétéroatomes choisis parmi N, S et O, et
le groupe consistant en hétérocycles saturés et insaturés bi- ou tricycliques, condensés ou pontés avec 8 à 16 atomes cycliques, qui, mis à part l'atome d'azote essentiel, contiennent éventuellement un ou deux autres hétéroatomes choisis parmi N, S et O,
G3 est le résidu
-SO₂-(CH₂)ᵣR¹³ (G3)
G4 est le résidu 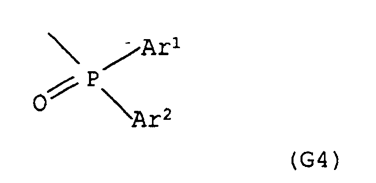
où
Ar¹ et
Ar² sont choisis indépendamment l'un de l'autre parmi phényle, pyridyle et naphtyle,
G5 est le résidu
-COR¹⁶ (G5)
où
R¹⁶ est choisi parmi trifluorométhyle, alcoxy en C₁-C₆, alcényloxy en C₃-C₆ et benzyloxy,
où les systèmes cycliques aromatiques dans les substituants R¹, R², R⁴, R¹³, R¹⁴, R¹⁵, R¹⁶, Ar¹ et Ar² et le système cyclique -NR¹³R¹⁵ portent éventuellement indépendamment les uns des autres un à trois substituants identiques ou différents choisis dans le groupe consistant en halogène, cyano, alkyle en C₁-C₆, trifluorométhyle, cycloalkyle en C₃-C₈, phényle, benzyle, hydroxyle, alcoxy en C₁-C₆, qui est éventuellement substitué totalement ou partiellement par le fluor, benzyloxy, phénoxy, mercapto, alkylthio en C₁-C₆, carboxyle, alcoxy en C₁-C₆-carbonyle, benzyloxycarbonyle, nitro, amino, mono-alkyle en C₁-C₆-amino et di-(alkyle en C₁-C₆)amino, où deux groupes adjacents du cycle aromatique ou du système cyclique aromatique forment éventuellement un cycle supplémentaire par le biais d'un pont méthylènedioxy, de leurs tautomères en cas de substitution de l'hétérocycle ou dans un système cyclique condensé par des groupes hydroxyle, mercapto et/ou amino libres, de leurs stéréoisomères et/ou de leurs mélanges et de leurs sels d'addition - d'acide pharmacologiquement acceptables
pour la production de médicaments pour un traitement cytostatique ou immunomodulateur et/ou immunosuppresseur.

2. Utilisation de composés selon la revendication 1 où
R¹ est choisi parmi l'hydrogène, halogène, cyano, méthyle, trifluorométhyle, hydroxyle, alcoxy en C₁-C₄, éthylthio, méthoxycarbonyle, tert-butoxycarbonyle, aminocarbonyle, carboxyle et phénoxy,
R² est choisi parmi l'hydrogène, halogène, trifluorométhyle et hydroxyle,
R³ est l'hydrogène ou halogène,
R⁴ est choisi parmi l'hydrogène, alkyle en C₁-C₃, hydroxyle et alcoxy en C₁-C₃,
k est 0 ou 1,
A est choisi dans le groupe consistant en alkylène en C₂-C₆, qui est éventuellement substitué une ou deux fois par alkyle en C₁-C₃, hydroxyle ou le fluor, et
alcénylène en C₂-C₆, qui est éventuellement substitué une ou deux fois par alkyle en C₁-C₃, hydroxyle ou le fluor,
alcadiénylène en C₄-C₆, qui est éventuellement substitué par alkyle en C₁-C₃ ou par un ou deux atomes de fluor,
1,3,5-hexatriénylène, qui est éventuellement substitué par le fluor, et
alkylène en C₂-C₆, où une unité méthylène est remplacée isostériquement par O, S, CO ou SO₂, et le substituant isostérique, à l'exception de =CO, n'est pas adjacent au groupe amide, et
D est choisi parmi alkylène en C₁-C₈, qui est éventuellement substitué une ou deux fois par méthyle ou hydroxyle,
alcénylène en C₂-C₈, qui est éventuellement substitué une ou deux fois par méthyle ou hydroxyle, où la double liaison est éventuellement avec le cycle E,
alcynylène en C₃-C₈, qui est éventuellement substitué une ou deux fois par méthyle ou hydroxyle, et
le groupe consistant en alkylène en C₁-C₈, alcénylène en C₂-C₈ et alcynylène en C₃-C₈, où une à trois unités méthylène sont remplacées isostériquement par O, S, NH, N(CH₃), N(COCH₃), N(SO₂CH₃), CO, SO ou SO₂,
E est choisi parmi 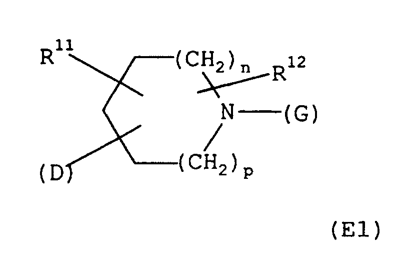 et 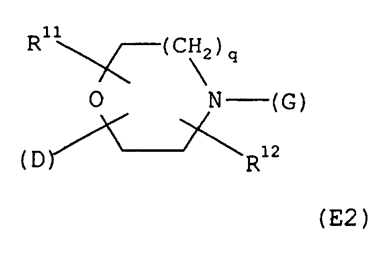 où le cycle hétérocyclique a éventuellement une double liaison et
n et
p sont, indépendamment l'un de l'autre, 0, 1, 2 ou 3, avec la condition que n + p ≤ 3,
q est 1 ou 2,
R¹¹ est choisi parmi l'hydrogène, alkyle en C₁-C₃, hydroxyle et hydroxyméthyle,
R¹² est l'hydrogène ou un groupe oxo qui est adjacent à l'atome d'azote,
G est choisi parmi l'hydrogène,
G1, G2, G3, G4 et G5, où
G1 représente le résidu
- (CH₂)ᵣ-(CR¹⁴R¹⁵)ₛ-R¹³ (G1)
où
r est 0, 1 ou 2,
s est 0 ou 1,
R¹³ est choisi parmi l'hydrogène, alkyle en C₁-C₆, cycloalkyle en C₃-C₈, benzyle, phényle,
le groupe consistant en benzocyclobutyle, indanyle, indényle, oxoindanyle, naphtyle, dihydronaphtyle, tétrahydronaphtyle, oxotétrahydronaphtyle, biphénylényle, fluorényle, oxofluorényle, anthryle, dihydroanthryle, oxodihydroanthryle, dioxodihydroanthryle, phénanthryle, dihydrophénanthryle, oxodihydrophénanthryle, dibenzocycloheptényle, oxodibenzocycloheptényle, dihydrodibenzocycloheptényle, oxodihydrodibenzocycloheptényle, dihydrodibenzocyclooctényle, tétrahydrodibenzocyclooctényle et oxotétrahydrodibenzocyclooctényle, lié directement ou par le biais d'un groupe méthylène, et
le groupe consistant en furyle, thiényle, pyrrolyle, oxazolyle, isoxazolyle, thiazolyle, isothiazolyle, pyrazolyle, imidazolyle, oxadiazolyle, thiadiazolyle, triazolyle, pyridyle, pyrazinyle, pyridazinyle, pyrimidinyle, triazinyle, imidazothiazolyle, benzofuryle, dihydrobenzofuryle, bénzothiényle, dihydrobenzothiényle, indolyle, indolinyle, oxoindolinyle, dioxoindolinyle, benzoxazolyle, oxobenzoxazolinyle, benzisoxazolyle, oxobenzisoxazolinyle, benzothiazolyle, oxobenzothiazolinyle, benzoisothiazolyle, oxobenzoisothiazolinyle, benzimidazolyle, oxobenzimidazolinyle, indazolyle, oxoindazolinyle, benzofurazanyle, benzothiadiazolyle, benzotriazolyle, oxazolopyridyle, oxodihydrooxazolopyridyle, thiazolopyridyle, oxodihydrothiazolopyridyle, isothiazolopyridyle, imidazopyridyle, oxodihydroimidazopyridyle, pyrazolopyridyle, oxodihydropyrazolopyridyle, thiénopyrimidinyle, chromanyle, chromanonyle, benzopyranyle, chromonyle, quinolyle, isoquinolyle, dihydroquinolyle, oxodihydroquinolinyle, tétrahydroquinolyle, oxotétrahydroquinolinyle, benzodioxanyle, quinoxalinyle, quinazolinyle, naphtyridinyle, carbazolyle, tétrahydrocarbazolyle, oxotétrahydrocarbazolyle, pyridoindolyle, acridinyle, oxodihydroacridinyle, phénothiazinyle, dihydrodibenzoxépinyle, oxodihydrodibenzoxépinyle, benzocycloheptathiényle, oxobenzocycloheptathiényle, dihydrothiénobenzothiépinyle, oxodihydrothiénobenzothiépinyle, dihydrodibenzothiépinyle, oxodihydrodibenzothiépinyle, octahydrodibenzothiépinyle, dihydrodibenzazépinyle, oxodihydrodibenzazépinyle, octahydrodibenzazépinyle, benzocycloheptapyridyle, oxobenzocycloheptapyridyle, dihydropyridobenzodiazépinyle, dihydrodibenzoxazépinyle, dihydropyridobenzoxépinyle, dihydropyridobenzoxazépinyle, oxodihydropyridobenzoxazépinyle, dihydrodibenzothiazépinyle, oxodihydrodibenzothiazépinyle, dihydropyridobenzothiazépinyle et oxodihydropyridobenzothiazépinyle, lié directement ou par le biais d'un groupe méthylène,
R¹⁴ a la même signification que R¹³, mais est choisi indépendamment de lui,
R¹⁵ est choisi parmi l'hydrogène, hydroxyle, méthyle, benzyle, phényle, et
le groupe consistant en indanyle, indényle, naphtyle, dihydronaphtyle, tétrahydronaphtyle, furyle, thiényle, pyrrolyle, oxazolyle, isoxazolyle, thiazolyle, isothiazolyle, pyrazolyle, imidazolyle, oxadiazolyle, thiadiazolyle, triazolyle, pyridyle, pyrazinyle, pyridazinyle, pyrimidinyle, triazinyle, benzofuryle, benzothiényle, indolyle, indolinyle, benzoxazolyle, benzothiazolyle, benzimidazolyle, chromanyle, quinolyle et tétrahydroquinolyle, lié directement ou par le biais. d'un groupe méthylène,
G2 est choisi parmi les résidus 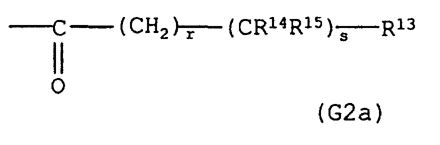 et 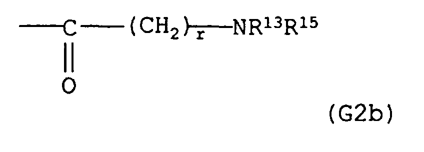 où les substituants R¹³ et R¹⁵ ont les significations ci-dessus, ou le groupe
-NR¹³R¹⁵
représente un hétérocycle azoté lié par l'atome d'azote, l'hétérocycle azoté étant choisi dans le groupe consistant en azétidine, pyrrolidine, pipéridine, (1H)-tétrahydropyridine, hexahydroazépine, (1H)-tétrahydroazépine, octahydroazocine, pyrazolidine, pipérazine, hexahydrodiazépine, morpholine, hexahydrooxazépine, thiomorpholine, thiomorpholine-1, 1-dioxyde, 5-aza-bicyclo[2.1.1]hexane, 2-azabicyclo-[2.2.1]heptane, 7-aza-bicyclo[2.2.1]heptane, 2,5-diazabicyclo[2.2.1]heptane, 2-aza-bicyclo[2.2.2]octane, 8-aza-bicyclo[3.2.1]octane, 2,5-diazabicyclo[2.2.2]-octane, 9-azabicyclo[3.3.1]nonane, indoline, isoindoline, (1H)-dihydroquinoléine, (1H)-tétrahydroquinoléine, (2H)-tétrahydroisoquinoléine, (1H)-tétrahydroquinoxaline, (4H)-dihydrobenzoxazine, (4H)-dihydrobenzothiazine, (1H)-tétrahydrobenzo[b]-azépine, (1H)-tétrahydrobenzo-[c]azépine, (1H)-tétrahydrobenzo[d]azépine, (5H)-tétra-hydrobenzo[b]-oxazépine, (5H)-tétrahydrobenzo[b]-thiazépine, 1,2,3,4-tétrahydro-9H-pyrido[3,4-b]indole, (10H)-dihydroacridine, 1,2,3,4-tétrahydroacridanone, (10H)-phénoxazine, (10H)-phénothiazine, (5H)-dibenzazépine, (5H)-dihydrodibenzazépine, (5H)-octahydrodibenzazépine, (5H)-dihydrodibenzodiazépine, (11H)-dihydrodibenzo[b,e]oxazépine, (11H)-dihydrodibenzo[b,e]thiazépine, (10H)-dihydrodibenzo[b,f]-oxazépine, (10H)-dihydrodibenzo[b,f]thiazépine et (5H)-tétrahydrodibenzazocine,
G3 est le résidu
-SO₂-(CH₂)ᵣR¹³ (G3)
G4 est le résidu 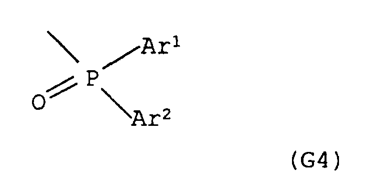
où
Ar¹ et
Ar² sont choisis indépendamment l'un de l'autre parmi phényle, pyridyle et naphtyle,
G5 est le résidu
-COR¹⁶ (G5)
où
R¹⁶ est choisi parmi trifluorométhyle, alcoxy en C₁-C₆, alcényloxy en C₃-C₆ et benzyloxy,
où les systèmes cycliques aromatiques sont éventuellement substitués indépendamment les uns des autres par un à trois substituants identiques ou différents choisis dans le groupe consistant en halogène, cyano, alkyle en C₁-C₆, trifluorométhyle, cycloalkyle en C₃-C₈, phényle, benzyle, hydroxyle, alcoxy en C₁-C₆, alcoxy en C₁-C₆ substitué totalement ou partiellement par le fluor ; benzyloxy, phénoxy, mercapto, alkylthio en C₁-C₆, carboxyle, alcoxy en C₁-C₆-carbonyle, benzyloxycarbonyle, nitro, amino, mono-alkyle en C₁-C₆-amino et di-(alkyle en C₁-C₆)amino, où deux groupes adjacents du cycle aromatique ou du système cyclique aromatique forment éventuellement un cycle supplémentaire par le biais d'un pont méthylènedioxy.

3. Utilisation de composés selon la revendication 1 ou 2 où
R¹ est choisi parmi l'hydrogène, halogène, cyano, méthyle, trifluorométhyle, hydroxyle, méthoxy et méthoxycarbonyle,
R² est l'hydrogène ou halogène,
R³ est l'hydrogène,
R⁴ est l'hydrogène, alkyle en C₁-C₃ ou hydroxyle,
k est 0 ou 1,
A est choisi parmi alkylène en C₂-C₆, qui est éventuellement substitué une ou deux fois par hydroxyle ou le fluor,
alcénylène en C₂-C₆, qui est éventuellement substitué une ou deux fois par hydroxyle ou le fluor,
alcadiénylène en C₄-C₆, qui est éventuellement substitué par un ou deux atomes de fluor,
1,3,5-hexatriénylène et
alkylène en C₂-C₆, où une unité méthylène est remplacée isostériquement par O, S ou CO, et le substituant isostérique, à l'exception de =CO, n'est pas adjacent au groupe amide, et
D est choisi parmi alkylène en C₂-C₈, qui est éventuellement substitué par méthyle ou hydroxyle,
alcénylène en C₂-C₈, qui est éventuellement substitué par méthyle ou hydroxyle, où la double liaison est éventuellement avec le cycle E, et
le groupe consistant en alkylène en C₂-C₈ et alcénylène en C₂-C₈, où une à trois unités méthylène sont remplacées isostériquement par O, NH, N(CH₃), N(COCH₃), N(SO₂CH₃) ou CO,
E est choisi parmi 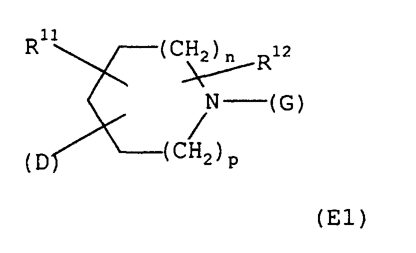 et 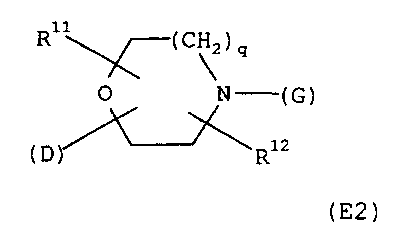 où le cycle hétérocyclique a éventuellement une double liaison et
n et p sont, indépendamment l'un de l'autre, 0, 1, 2 ou 3, avec la condition que n + p ≤ 3,
q est 1 ou 2,
R¹¹ est choisi dans le groupe consistant en l'hydrogène, méthyle et hydroxyle,
R¹² est l'hydrogène ou un groupe oxo adjacent à l'atome d'azote,
G est choisi parmi l'hydrogène, cycloalkyle en C₃-C₈, méthoxycarbonyle, tert-butoxycarbonyle, benzyloxycarbonyle, trifluoroacétyle, diphénylphosphinoyle et les résidus
-(CH₂)ᵣ-(CR¹⁴R¹⁵)ₛ-R¹³ (G1) 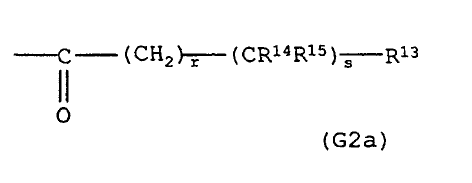 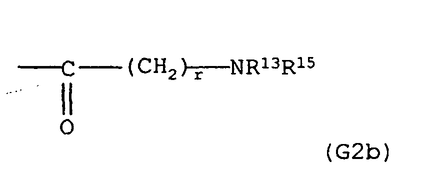
et
-SO₂-(CH₂)ᵣR¹³ (G3)
où
r est 0, 1 ou 2,
s est 0 ou 1,
R¹³ est choisi parmi l'hydrogène, méthyle, benzyle, phényle,
le groupe consistant en indanyle, indényle, oxoindanyle, naphtyle, dihydronaphtyle, tétrahydronaphtyle, oxotétrahydronaphtyle, fluorényle, oxofluorényle, anthryle, dihydroanthryle, oxodihydroanthryle, dioxodihydroanthryle, dibenzocycloheptényle, oxodibenzocycloheptényle, dihydrodibenzocycloheptényle et oxodihydrodibenzocycloheptényle lié directement ou par le biais d'un groupe méthylène, et le groupe consistant en furyle, thiényle, pyrrolyle, oxazolyle, isoxazolyle, thiazolyle, isothiazolyle, pyrazolyle, imidazolyle, oxadiazolyle, thiadiazolyle, triazolyle, pyridyle, pyrazinyle, pyridazinyle, pyrimidinyle, imidazothiazolyle, benzofuryle, dihydrobenzofuryle, benzothiényle, dihydrobenzothiényle, indolyle, indolinyle, oxoindolinyle, dioxoindolinyle, benzoxazolyle, oxobenzoxazolinyle, benzisoxazolyle, oxobenzisoxazolinyle, benzothiazolyle, oxobenzothiazolinyle, benzoisothiazolyle, oxobenzoisothiazolinyle, benzimidazolyle, oxobenzimidazolinyle, benzofurazanyle, benzothiadiazolyle, benzotriazolyle, oxazolopyridyle, oxodihydrooxazolopyridyle, thiazolopyridyle, oxodihydrothiazolopyridyle, isothiazolopyridyle, imidazopyridyle, oxodihydroimidazopyridyle, pyrazolopyridyle, thiénopyrimidinyle, chromanyle, chromanonyle, benzopyranyle, chromonyle, quinolyle, isoquinolyle, dihydroquinolyle, oxodihydroquinolinyle, tétrahydroquinolyle, oxotétrahydroquinolinyle, benzodioxanyle, quinoxalinyle, quinazolinyle, naphtyridinyle, carbazolyle, tétrahydrocarbazolyle, oxotétrahydrocarbazolyle, pyridoindolyle, acridinyle, oxodihydroacridinyle, phénothiazinyle, dihydro-dibenzoxépinyle, benzocycloheptathiényle, oxobenzocycloheptathiényle, dihydrothiénobenzothiépinyle, oxodihydrothiénobenzothiépinyle, dihydrodibenzothiépinyle, oxodihydrodibenzothiépinyle, dihydrodibenzazépinyle, oxodihydrodibenzazépinyle, octahydrodibenzazépinyle, benzocycloheptapyridyle, oxobenzocycloheptapyridyle, dihydropyridobenzoxépinyle, dihydrodibenzothiazépinyle et oxodihydrodibenzothiazépinyle, lié directement ou par le biais d'un groupe méthylène,
R¹⁴ est choisi dans le groupe consistant en l'hydrogène, méthyle, benzyle et phényle,
R¹⁵ est choisi parmi l'hydrogène, hydroxyle, méthyle, benzyle, phényle, et
le groupe consistant en naphtyle, furyle, thiényle, oxazolyle, thiazolyle, pyrazolyle, imidazolyle, oxadiazolyle, thiadiazolyle, pyridyle, benzofuryle, benzothiényle, indolyle, indolinyle, benzoxazolyle, benzothiazolyle, benzimidazolyle, chromanyle, quinolyle et tétrahydroquinolyle, lié directement ou par le biais d'un groupe méthylène,
où, dans la formule 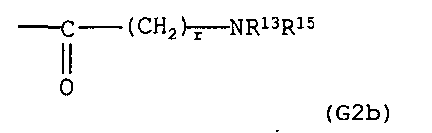
le groupe NR¹³R¹⁵ est éventuellement choisi parmi pyrrolidine, pipéridine, (1H)-tétrahydropyridine, hexahydroazépine, octahydroazocine, pipérazine, hexahydrodiazépine, morpholine, hexahydrooxazépine, 2-azabicyclo[2.2.1]heptane, 7-azabicyclo[2.2.l]heptane, 2,5-diazabicyclo[2.2.1]heptane, 8-azabicyclo[3.2.1]-octane, 2,5-diazabicyclo[2.2.2]octane, indoline, isoindoline, (1H)-dihydroquinoléine, (1H)-tétrahydroquinoléine, (2H)-tétrahydroisoquinoléine, (1H)-tétrahydroquinoxaline, (4H)-dihydrobenzoxazine, (4H)-dihydrobenzothiazine, (1H)-tétrahydrobenzo[b]-azépine, (1H)-tétrahydrobenzo[d]azépine, (5H)-tétrahydrobenzo[b]oxazépine, (5H)-tétrahydrobenzo[b]-thiazépine, 1,2,3,4-tétrahydro-9H-pyrido[3,4-b]indole, (10H)-dihydroacridine, 1,2,3,4-tétrahydroacridanone, (5H)-dihydrodibenzazépine, (5H)-dihydrodibenzodiazépine, (11H)-dihydrodibenzo[b,e]oxazépine, (11H)-dihydrodibenzo[b,e]thiazépine, (10H)-dihydrodibenzo-[b,f]oxazépine et (5H)-tétrahydrodibenzazocine.

4. Utilisation de composés selon les revendications 1 à 3 où
R¹ est choisi parmi l'hydrogène, le fluor, le chlore, le brome, méthyle, trifluorométhyle et hydroxyle,
R² et
R³ sont l'hydrogène,
R⁴ est l'hydrogène ou hydroxyle,
k est 0 ou 1,
A est choisi parmi alkylène en C₂-C₆, qui est éventuellement substitué une ou deux fois par hydroxyle ou le fluor,
alcénylène en C₂-C₄, qui est éventuellement substitué par le fluor, et
1,3-butadiénylène, qui est éventuellement substitué par le fluor,
D est choisi parmi alkylène en C₂-C₆, alcénylène en C₂-C₆, où la double liaison est éventuellement avec le cycle E, et le groupe consistant en alkylène en C₂-C₆ et alcénylène en C₂-C₆, où une unité méthylène est remplacée isostériquement par O, NH, N(CH₃) ou CO, ou un groupe éthylène est remplacé isostériquement par NH-CO ou CO-NH ou un groupe propylène est remplacé isostériquement par NH-CO-O ou O-CO-NH,
E est choisi parmi pyrrolidine, pipéridine, 1,2,5,6-tétrahydropyridine, hexahydroazépine, morpholine et hexahydro-1,4-oxazépine, où le cycle hétérocyclique est éventuellement substitué par un groupe oxo adjacent à l'atome d'azote
G est choisi parmi l'hydrogène, tert-butoxycarbonyle, diphénylphosphinoyle et les résidus
-(CH₂)ᵣ-(CR¹⁴R¹⁵)ₛ-R¹³ (G1) 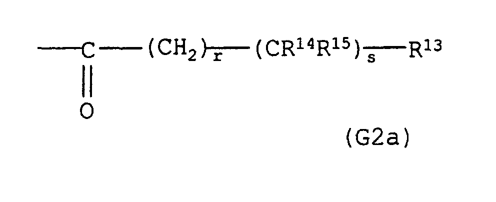 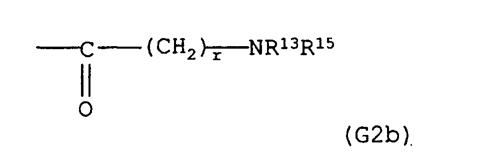
et
-SO₂-(CH₂)ᵣR¹³ (G3)
où
r est 0 ou 1,
s est 0 ou 1,
R¹³ est choisi parmi l'hydrogène, méthyle, benzyle, phényle,
le groupe consistant en indényle, oxoindanyle, naphtyle, tétrahydronaphtyle, fluorényle, oxofluorényle, anthryle, dihydroanthryle, oxodihydroanthryle, dioxodihydroanthryle, dibenzocycloheptényle et dihydrodibenzocycloheptényle lié directement ou par le biais d'un groupe méthylène, et
le groupe consistant en furyle, thiényle, oxazolyle, thiazolyle, imidazolyle, oxadiazolyle, thiadiazolyle, pyridyle, pyrazinyle, pyrimidinyle, imidazothiazolyle, benzofuryle, benzothiényle, indolyle, oxoindolinyle, dioxoindolinyle, benzoxazolyle, oxobenzoxazolinyle, benzothiazolyle, oxobenzothiazolinyle, benzimidazolyle, oxobenzimidazolinyle, benzofurazanyle, benzotriazolyle, oxazolopyridyle, oxodihydrooxazolopyridyle, thiazolopyridyle, oxodihydrothiazolopyridyle, chromanyle, chromanonyle, benzopyranyle, chromonyle, quinolyle, isoquinolyle, oxodihydroquinolinyle, tétrahydroquinolyle, oxotétrahydroquinolinyle, benzodioxanyle, quinazolinyle, acridinyle, oxodihydroacridinyle, phénothiazinyle, dihydrodibenzoxépinyle, benzocycloheptathiényle, dihydrothiénobenzothiépinyle, dihydrodibenzothiépinyle, oxodihydrodibenzothiépinyle, dihydrodibenzazépinyle, oxodihydrodibenzazépinyle, octahydrodibenzazépinyle, benzocycloheptapyridyle, oxobenzocycloheptapyridyle et dihydrodibenzo-thiazépinyle, lié directement ou par le biais d'un groupe méthylène,
R¹⁴ est l'hydrogène, méthyle, benzyle ou phényle,
R¹⁵ est choisi dans le groupe consistant en l'hydrogène, hydroxyle, méthyle, benzyle, phényle, et
le groupe consistant en naphtyle, furyle, thiényle, pyridyle, benzofuryle, benzothiényle, indolyle, benzoxazolyle, benzothiazolyle, benzimidazolyle, chromanyle, quinolyle et tétrahydroquinolyle, lié directement ou par le biais d'un groupe méthylène,
où, dans la formule 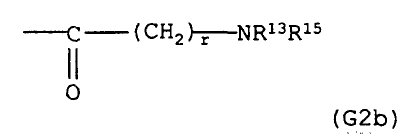
le groupe NR¹³R¹⁵ est éventuellement choisi parmi pyrrolidine, pipéridine, hexahydroazépine, morpholine, 2,5-diazabicyclo[2.2.1]heptane, indoline, isoindoline, (1H)-dihydroquinoléine, (1H)-tétrahydroquinoléine, (2H) -tétrahydroisoquinoléine, (1H)-tétrahydrobenzo[b]-azépine, (1H)-tétrahydrobenzo[d]azépine, (5H)-tétrahydrobenzo[b]oxazépine, (5H)-tétrahydrobenzo[b]-thiazépine, 1,2,3,4-tétrahydroacridanone, (5H)-dihydrodibenzazépine, (11H)-dihydrodibenzo[b,e]oxazépine et (11H)-dihydrodibenzo[b,e]thiazépine,
où les systèmes cycliques aromatiques sont éventuellement substitués indépendamment les uns des autres par un à trois substituants identiques ou différents choisis dans le groupe consistant en cycloalkyle en C₃-C₈, phényle, benzyle, hydroxyle, alcoxy en C₁-C₆, alcoxy en C₁-C₆ qui est substitué totalement ou partiellement par le fluor ; benzyloxy, phénoxy, mercapto, alkylthio en C₁-C₆, carboxyle, alcoxy en C₁-C₆-carbonyle, benzyloxycarbonyle, nitro, amino, mono-alkyle en C₁-C₆-amino et di-(alkyle en C₁-C₆)amino, où deux groupes adjacents du cycle aromatique ou du système cyclique aromatique forment éventuellement un cycle supplémentaire par le biais d'un pont méthylènedioxy.

5. Utilisation de composés selon les revendications 1 à 4 où
R¹ est choisi parmi l'hydrogène, le fluor, méthyle, trifluorométhyle et hydroxyle,
R² et
R³ sont l'hydrogène,
R⁴ est l'hydrogène ou hydroxyle,
k est 0,
A est choisi dans le groupe consistant en éthylène, propylène et butylène, qui sont éventuellement substitués par hydroxyle ou une ou deux fois par le fluor,
éthénylène et
1,3-butadiénylène
D est choisi parmi alkylène en C₂-C₆ et alcénylène en C₂-C₆, où la double liaison est éventuellement avec le cycle E,
E est choisi parmi pyrrolidine, pipéridine, hexahydroazépine et morpholine
G est choisi parmi benzyle, phénéthyle, fluorénylméthyle, anthrylméthyle, diphénylméthyle, fluorényl et dihydrodibenzocycloheptényle, furylméthyle, thiénylméthyle, thiazolylméthyle, pyridylméthyle, benzothiénylméthyle, quinolylméthyle, phényl-thiénylméthyle, phénylpyridylméthyle, dihydrodibenzoxépinyle, dihydrodibenzothiépinyle,
acétyle, pivaloyle, phénylacétyle, diphénylacétyle, diphénylpropionyle, naphtylacétyle, benzoyle, naphtoyle, anthrylcarbonyle, oxofluorénylcarbonyle, oxodihydroanthrylcarbonyle, dioxodihydroanthrylcarbonyle, furoyle, pyridylcarbonyle, chromonylcarbonyle, quinolylcarbonyle, naphtylaminocarbonyle, dibenzylaminocarbonyle, benzylphénylaminocarbonyle, diphénylaminocarbonyle, indolinyl-1-carbonyle, dihydrodibenzazépine-N-carbonyle, tétrahydroquinolinyl-N-carbonyle, tétrahydrobenzo[b]azépinyl-N-carbonyle, méthanesulfonyle, phénylsulfonyle, p-toluènesulfonyle, naphtylsulfonyle, quinoléinesulfonyle et diphénylphosphinolyle,
où les systèmes cycliques aromatiques sont éventuellement substitués indépendamment les uns des autres par un à trois substituants identiques ou différents choisis dans le groupe consistant en halogène, cyano, alkyle en C₁-C₆, trifluorométhyle, cycloalkyle en C₃-C₈, phényle, benzyle, hydroxyle, alcoxy en C₁-C₆, alcoxy en C₁-C₆ qui est substitué totalement ou partiellement par le fluor, benzyloxy, phénoxy, mercapto, alkylthio en C₁-C₆, carboxyle, alcoxy en C₁-C₆-carbonyle, benzyloxycarbonyle, nitro, amino, mono-alkyle en C₁-C₆-amino et di-(alkyle en C₁-C₆)amino, où deux groupes adjacents dans le cycle aromatique ou dans le système cyclique aromatique forment éventuellement un cycle supplémentaire par le biais d'un pont méthylènedioxy.

6. Utilisation selon l'une des revendications 1 à 5 où un ou plusieurs des composés suivants et/ou leurs sels d'addition d'acide pharmacologiquement acceptables sont utilisés pour la production d'un médicament
N-[2-(1-benzylpipéridin-4-yl)-éthyl]-3-(pyridin-3-yl)-propionamide,
N-{2-[1-(2-phényléthyl)-pipéridin-4-yl]-éthyl}-3-(pyridin-3-yl)-propionamide,
N-[2-(1-diphénylméthylpipéridin-4-yl)-éthyl]-3-(pyridin-3-yl)-propionamide,
N-[3-(1-diphénylméthylpipéridin-4-yl)-propyl]-3-(pyridin-3-yl)-propionamide, ou
N-[4-(1-diphénylméthylpipéridin-4-yl)-butyl]-3-(pyridin-3-yl)-propionamide.

7. Utilisation selon l'une des revendications 1 à 4 où le composé suivant et/ou ses sels d'addition d'acide pharmacologiquement acceptables sont utilisés pour la production d'un médicament
N-{2-[1-(4-phénylbutyl)-pipéridin-4-yl]-éthyl}-3-(pyridin-3-yl)-propionamide.

8. Utilisation du N-{2-[1-(4-hydroxy-4-phénylbutyl)-pipéridin-4-yl]-éthyl}-3-(pyridin-3-yl)-propionamide, de ses stéréoisomères, mélanges et/ou sels d'addition d'acide pharmacologiquement acceptables pour la production d'un médicament pour un traitement cytostatique ou immunomodulateur et/ou immunosuppresseur.

9. Utilisation selon l'une quelconque des revendications 1 à 5 où un ou plusieurs des composés suivants et/ou leurs sels d'addition d'acide pharmacologiquement acceptables sont utilisés pour la production d'un médicament
N-[4-(1-benzylpipéridin-4-yl)-butyl]-3-(pyridin-3-yl)-acrylamide,
N-{4-[1-(2-phényléthyl)-pipéridin-4-yl]-butyl}-3-(pyridin-3-yl)-acrylamide,
N-{4-[1-(4-biphénylylméthyl)-pipéridin-4-yl]-butyl}-3-(pyridin-3-yl)-acrylamide,
N-{4-[1-(9-anthrylméthyl)-pipéridin-4-yl]-butyl}-3-(pyridin-3-yl)-acrylamide,
ou
N-{4-[1-(10,11-dihydro-5H-dibenzo[a,d]cycloheptén-5-yl)-pipéridin-4-yl]-butyl}-3-(pyridin-3-yl)-acrylamide.

10. Utilisation selon l'une des revendications 1 à 4 où le composé suivant et/ou ses sels d'addition d'acide pharmacologiquement acceptables sont utilisés pour la production d'un médicament
N-{4-[1-(1-naphtylméthyl)-pipéridin-4-yl]-butyl}-3-(pyridin-3-yl)-acrylamide.

11. Utilisation selon la revendication 1 ou 2 où le composé suivant et/ou ses sels d'addition d'acide pharmacologiquement acceptables sont utilisés pour la production d'un médicament :
N-{4-[1-(cyclohexylphénylméthyl)-pipéridin-4-yl]-butyl}-3-(pyridin-3-yl)-acrylamide.

12. Utilisation selon l'une quelconque des revendications 1 à 5 où un ou plusieurs des composés suivants et/ou leurs sels d'addition d'acide pharmacologiquement acceptables sont utilisés pour la production d'un médicament
N-[2-(1-diphénylméthylpipéridin-4-yl)-éthyl]-3-(pyridin-3-yl)-acrylamide,
N-[3-(1-diphénylméthylpipéridin-4-yl)-propyl]-3-(pyridin-3-yl)-acrylamide,
N-[5-(1-diphénylméthylpipéridin-4-yl)-pentyl]-3-(pyridin-3-yl)-acrylamide,
N-[6-(1-diphénylméthylpipéridin-4-yl)-hexyl]-3-(pyridin-3-yl)-acrylamide
et
N-[4-(1-diphénylméthylpipéridin-4-yl)-butyl]-5-(pyridin-3-yl)-2,4-pentadiénamide.

13. Utilisation selon l'une quelconque des revendications 1 à 5 où un ou plusieurs des composés suivants et/ou leurs sels d'addition d'acide pharmacologiquement acceptables sont utilisés pour la production d'un médicament
N-(4-{1-[bis-(4-fluorophényl)-méthyl]-pipéridin-4-yl}-butyl)-3-(pyridin-3-yl)-acrylamide,
N-(4-{1-[bis-(2-chlorophényl)-méthyl]-pipéridin-4-yl}-butyl)-3-(pyridin-3-yl)-acrylamide,
N-[4-(1-diphénylméthylpipéridin-4-yl)-butyl]-3-(2-fluoropyridin-3-yl)-acrylamide
et
N-[4-(1-diphénylméthylpipéridin-4-yl)-butyl]-3-(6-fluoropyridin-3-yl)-acrylamide.

14. Utilisation selon l'une quelconque des revendications 1 à 5 où un ou plusieurs des composés suivants sont utilisés pour la production d'un médicament
N-[4-(1-diphénylméthylpipéridin-4-yl)-butyl]-3-(pyridin-3-yl)-acrylamide,
dichlorhydrate de N-[4-(1-diphénylméthylpipéridin-4-yl)-butyl]-3-(pyridin-3-yl)-acrylamide, et
méthanesulfonate de N-[4-(1-diphénylméthylpipéridin-4-yl)-butyl]-3-(pyridin-3-yl)-acrylamide.

15. Utilisation selon l'une quelconque des revendications 1 à 14 où les composés selon la formule générale (I) sont combinés avec un ou plusieurs autres ingrédients actifs choisis parmi les agents cytostatiques et les agents immunosuppresseurs.

16. Médicament pour un traitement cytostatique ou immunomodulateur et/ou immunosuppresseur en médecine humaine ou vétérinaire, qui comprend un composé selon la formule (I) correspondant aux définitions des substituants données dans les revendications 1 à 14 en combinaison avec un ou plusieurs autres ingrédients actifs choisis parmi les agents cytostatiques et les agents immunosuppresseurs, éventuellement avec des supports, adjuvants et additifs appropriés.

17. Médicament selon la revendication 16 pour un traitement cytostatique qui comprend un composé selon la formule (I), des supports et adjuvants pharmaceutiquement acceptables, et en combinaison un ou plusieurs agents cytostatiques de la série des antimétabolites choisis parmi la cytarabine, le 5-fluorouracile, la 6-mercaptopurine et le méthotrexate, des agents alkylants choisis parmi le busulfan, la carmustine, le cisplatine, le carboplatine, le cyclophosphamide, la dacarbazine, le melphalan et le thiotépa, des substances qui s'intercalent dans l'ADN et des inhibiteurs de topoisomérase choisis parmi l'actinomycine D, la daunorubicine, la doxorubicine, la mitomycine C, la mitoxantrone, l'étoposide, le topotecan et l'irinotecan, des poisons fusoriaux choisis parmi la vincristine, la navelbine, le taxol et le taxotère, des agents actifs du point de vue hormonal choisis parmi le tamoxifène, le flutiamide, le formestan et la goséréline ou de la série d'autres agents cytostatiques ayant des modes d'action complexes choisis parmi la L-asparaginase, la bléomycine et l'hydroxyurée ou du groupe des modulateurs de la P-glycoprotéine, de MRP, de la glutathion-S-transférase, de la métallothionéine, éventuellement avec d'autres médicaments efficaces dans ces indications.

18. Médicament selon la revendication 16 pour un traitement immunosuppresseur qui comprend un composé selon la formule (I), des supports et adjuvants pharmaceutiquement acceptables, et en combinaison un ou plusieurs agents immunosuppresseurs de la série des cyclosporines choisies parmi la cyclosporine A, le tacrolimus et la rapamycine, du groupe des antimétabolites choisis parmi le méthotrexate et l'azathioprine et du groupe des glucocorticoïdes, éventuellement avec d'autres médicaments efficaces dans ces indications.

19. Utilisation selon les revendications 1 à 15 pour le traitement des tumeurs en liaison avec les indications tumeurs gynécologiques, cancers ovariens, tumeurs des testicules, cancers de la prostate, cancer de la peau, cancer du rein, tumeurs de la vessie, cancers de l'oesophage, cancer de l'estomac, cancers du rectum, cancers du pancréas, cancer de la thyroïde, tumeurs surrénales, différents types de leucémies et de lymphomes, maladie de Hodgkin, maladies tumorales du SNC, sarcomes des tissus mous, ostéosarcomes, mésothéliomes bénins et malins, en particulier cancer de l'intestin, cancer du foie, cancer du sein, cancers des bronches et du poumon, mélanomes, leucémies aiguës et chroniques et tumeurs papillomateuses bénignes.

20. Utilisation selon l'une des revendications 1 à 5 où le composé suivant et/ou ses sels d'addition d'acide pharmacologiquement acceptables sont utilisés pour la production d'un médicament :
N-(4-diphénylméthyl-morpholin-2-ylméthyl)-3-(pyridin-3-yl)-acrylamide.
